# EUROPÄISCHE PATENTSCHRIFT

(11) **EP 2 558 460 B1**
(45) Veröffentlichungstag und Bekanntmachung des Hinweises auf die Patenterteilung: **04.03.2015**
(21) Anmeldenummer: 11713817.2
(22) Anmeldetag: 12.04.2011
(51) Int. Cl.: C07D 401/14, C07D 403/06, A01N 43/647

(54) **TRIAZOLSUBSTITUIERTE ANTHRANILSÄUREAMIDE ALS PESTIZIDE**
TRIAZOL-SUBSTITUTED ANTHRANILIC ACID AMIDES AS PESTICIDES
AMIDES D'ACIDE ANTHRANILIQUE SUBSTITUÉS AU TRIAZOLE EN TANT QUE PESTICIDES

(30) Priorität: 19.04.2010 US 325500 P; 16.04.2010 EP 10160117
(43) Veröffentlichungstag der Anmeldung: 20.02.2013
(73) Patentinhaber: Bayer Intellectual Property GmbH, 40789 Monheim am Rhein (DE)
(72) Erfinder: FISCHER, Rüdiger, 50259 Pulheim (DE); GRONDAL, Christoph, 50937 Köln (DE); GESING, Ernst, Rudolf, 40699 Erkrath (DE); WROBLOWSKY, Heinz-Jürgen, 40764 Langenfeld (DE); HENSE, Achim, 51379 Leverkusen (DE); FRANKEN, Eva-Maria, 51381 Leverkusen (DE); VOERSTE, Arnd, 50674 Köln (DE); GÖRGENS, Ulrich, 40882 Ratingen (DE)
(74) Vertreter: BIP Patents
(86) Internationale Anmeldenummer: PCT/EP2011/055693
(87) Internationale Veröffentlichungsnummer: WO 2011/128329

(56) Entgegenhaltungen:
- WO-A1-2007/144100

## Beschreibung

Die vorliegende Erfindung betrifft triazolsubstituierte Anthranilsäureamide, mehrere Verfahren zu ihrer Herstellung und ihre Verwendung als Wirkstoffe auch in Kombination mit weiteren Mitteln zur Wirksamkeitssteigerung, insbesondere ihre Verwendung als Schädlingsbekämpfungsmittel.

In der Literatur ist bereits beschrieben, daß bestimmte Anthranilsäureamide (z.B. WO 01/70671, WO 03/015519, WO 03/016284, WO 03/015518, WO 03/024222, WO 03/016282, WO 03/016283, WO 03/062226, WO 03/027099, WO 04/027042, WO 04/033468, WO 2004/046129, WO 2004/067528, WO 2005/118552, WO 2005/077934, WO 2005/085234, WO 2006/023783, WO 2006/000336, WO 2006/040113, WO 2006/111341, WO 2007/006670, WO 2007/024833, WO2007/020877 und WO 07/144100) insektizide Eigenschaften besitzen.

Es wurde nun gefunden, dass die neuen triazolhaltigen Anthranilsäureamide Vorteile gegenüber dem Stand der Technik darstellen, z.B. durch bessere biologische oder ökologische Eigenschaften. Als weitere Vorteile seien breitere Anwendungsmethoden, eine bessere insektizide, akarizide Wirkung, sowie eine gute Verträglichkeit gegenüber Nutzpflanzen beispielhaft genannt. Die triazolhaltigen Anthranilsäureamide können in Kombination mit weiteren Mitteln zur Verbesserung der Wirksamkeit insbesondere gegen schwierig zu bekämpfende Insekten eingesetzt werden.

Gegenstand der vorliegenden Erfindung sind Anthranilsäureamide der allgemeinen Formel (I) 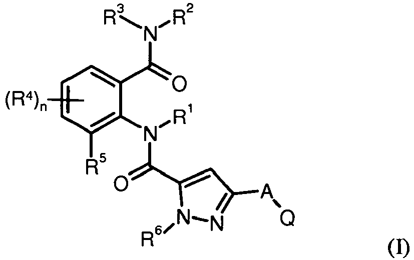 in welcher
- R¹: für Wasserstoff, Amino, Hydroxy oder für jeweils gegebenenfalls einfach oder mehrfach, gleich oder verschieden substituiertes C₁-C₆-Alkyl, C₂-C₆-Alkenyl, C₂-C₆-Alkinyl oder C₃-C₆-Cycloalkyl steht, wobei die Substituenten unabhängig voneinander ausgewählt sein können aus Halogen, Cyano, Nitro, Hydroxy, C₁-C₄-Alkoxy, C₁-C₄-Alkylthio, C₁-C₄-Alkylsulfinyl, C₁-C₄-Al-kylsulfonyl, (C₁-C₄-Alkoxy)carbonyl, C₁-C₄-Alkylamino, Di-(C₁-C₄-alkyl)amino, C₃-C₆-Cycloalkylamino oder (C₁-C₄-Alkyl)C₃-C₆-cycloalkylamino,
- R²: für Wasserstoff, C₁-C₆-Alkyl, C₂-C₆-Alkenyl, C₂-C₆-Alkinyl, C₃-C₆-Cycloalkyl, C₁-C₄-Alkoxy, C₁-C₄-Alkylamino, Di-(C₁-C₄-alkyl)amino, C₃-C₆-Cycloalkylamino, C₂-C₆-Alkoxycarbonyl oder C₂-C₆-Alkylcarbonyl steht,
- R³: für Wasserstoff oder für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl steht, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
- R³: weiterhin für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl steht, wobei die Substituenten unabhängig voneinander ausgewählt sein können aus Amino, C₃-C₆-Cycloalkylamino oder einem 5- oder 6-gliedrigen heteroaromatischen Ring,
- R³: ebenfalls weiterhin für C₃-C₁₂-Cycloalkyl, C₃-C₁₂-Cycloalkyl-C₁-C₆-Alkyl und C₄-C₁₂-Bicycloalkyl steht, wobei die Substituenten unabhängig voneinander ausgewählt sein können aus Halogen, Cyano, Nitro, Hydroxy, Amino, C₁-C₆-Alkyl, C₃-C₆-Cycloalkyl, C₃-C₆-Cycloalkylamino, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl, C₃-C₆-Trialkylsilyl oder einem 5- oder 6-gliedrigen heteroaromatischen Ring,
- R² und: R³ miteinander über zwei bis sechs Kohlenstoffatome verbunden sein können und einen Ring ausbilden, der gegebenenfalls zusätzlich ein weiteres Stickstoff-, Schwefel- oder Sauerstoffatom enthält und gegebenenfalls einfach bis vierfach mit C₁-C₂-Alkyl, Halogen, Cyano, Amino oder C₁-C₂-Alkoxy substituiert sein kann,
- R², R³: weiterhin gemeinsam für =S(C₁-C₄-Alkyl)₂, =S(O)(C₁-C₄-Alkyl)₂, stehen,
- R⁴: für Wasserstoff, Halogen, Cyano, Nitro C₁-C₄-Alkyl, C₁-C₄-Haloalkyl, C₂-C₆-Alkenyl, C₂-C₆-Haloalkenyl, C₂-C₆-Alkinyl, C₁-C₄-Alkoxy, C₁-C₄-Haloalkoxy, SF₅, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₄-Alkoxy)imino, (C₁-C₄-Alkyl)(C₁-C₄-Alkoxy)imino, (C₁-C₄-Haloalkyl)(C₁-C₄-Alkoxy)imino oder C₃-C₆-Trialkylsilyl steht, oder

zwei R⁴ über benachbarte Kohlenstoffatome einen Ring ausbilden, der für -(CH₂)₃-, -(CH₂)₄-, - (CH₂)₅-, -(CH=CH-)₂-, -OCH₂O- -O(CH₂)₂O-, -OCF₂O-, -(CF₂)₂O-, -O(CF₂)₂O-, -(CH=CH-CH=N)- oder -(CH=CH-N=CH)- steht,
zwei R⁴ weiterhin über benachbarte Kohlenstoffatome die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, C₁-C₆-Alkyl, C₃-C₆-Cycloalkyl, C₁-C₆-Haloalkyl, C₃-C₆-Halocycloalkyl, Halogen, C₁-C₆-Alkoxy, C₁-C₄-Alkylthio(C₁-C₆-alkyl), C₁-C₄-Alkylsulfinyl(C₁-C₆-alkyl), C₁-C₄-Alkylsulfonyl(C₁-C₆-alkyl), C₁-C₄-Alkylamino, Di-(C₁-C₄-alkyl)amino oder C₃-C₆-Cycloalkylamino, 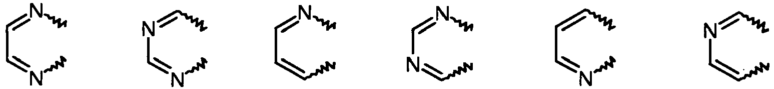 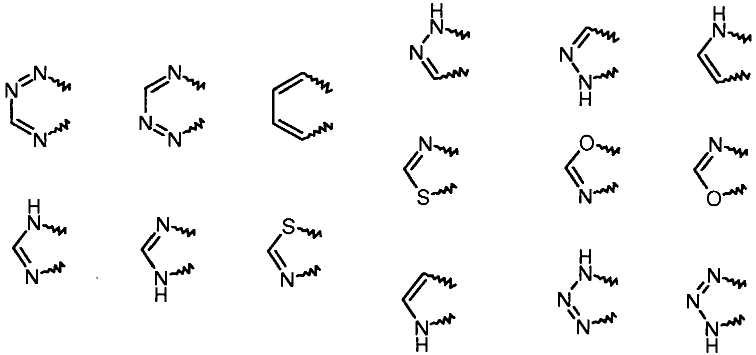
- n: für 0 bis 3 steht,
- R⁵: für C₁-C₆-Alkyl, C₃-C₆-Cycloalkyl, C₁-C₆-Haloalkyl, C₁-C₆-Halocycloalkyl, C₂-C₆-Alkenyl, C₂-C₆-Haloalkenyl, C₂-C₆-Alkinyl, C₂-C₆-Haloalkinyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, Halogen, Cyano, Nitro oder C₃-C₆-Trialkylsilyl steht,
- R⁶: für Wasserstoff, C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl oder 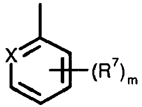 steht,
- R⁶: weiterhin für C₃-C₆-Cycloalkoxy steht,
- R⁷: steht unabhängig voneinander für Wasserstoff, C₁-C₆-Alkyl, C₃-C₆-Cycloalkyl, C₁-C₆-Haloalkyl, Halogen, Cyano, Nitro, C₁-C₄-Alkoxy, C₁-C₄-Haloalkoxy, C₁-C₄-Alkylthio oder C₁-C₄-Haloalkylthio,
- m: für 0 bis 4 steht,
- X: für N, CH, CF, CCl, CBr oder Cl steht,
- A: für -CH₂-, -CH₂O-, -CH₂OCH₂-, -CH₂S-, -CH₂SCH₂-, -CH₂N(C₁-C₆-Alkyl)-, -CH₂N(C₁-C₆-Alkyl)CH₂-, -CH[CO₂(C₁-C₆-Alkyl)]-, -CH(CN)-, -CH(C₁-C₆-Alkyl)-, -C(Di-C₁-C₆-Alkyl)-, -CH₂CH₂-, -C=NO(C₁-C₆-Alkyl)- steht,
- Q: für einen 5-gliedrigen heteroaromatischen Ring oder ein aromatisches 9-gliedriges annelliertes heterobicyclisches Ringsystem steht, wobei der Ring oder das Ringsystem drei Stickstoffe enthalten und wobei die Stickstoffe im Ring oder Ringsystem benachbarte Positionen einnehmen und die Bindung des Rings oder des Ringsystems an den Rest A in Formel (I) über Stickstoff erfolgt. Der Ring oder das Ringsystem sind gegebenenfalls einfach oder mehrfach, gleich oder verschieden substituiert, wobei die Substituenten unabhängig voneinander ausgewählt sein können aus Wasserstoff, C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, CO₂H, CO₂NH₂, NO₂, OH, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino,
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, OH, C₁-C₄-Alkoxy, C₁-C₄-Haloalkoxy substituiert sein können,
die Verbindungen der allgemeinen Formel (I) außerdem N-Oxide und Salze umfassen.

Schließlich wurde gefunden, dass die erfindungsgemäßen Verbindungen der Formel (I) sehr gute insektizide Eigenschaften besitzen und sich sowohl im Pflanzenschutz als auch im Materialschutz zur Bekämpfung unerwünschter Schädlinge, wie Insekten, verwenden lassen.

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäßen Anthranilamide sind durch die Formel (I) allgemein definiert. Bevorzugte Restedefinitionen der vorstehenden und nachfolgend genannten Formeln sind im Folgenden angegeben. Die allgemeinen oder in Vorzugsbereichen aufgeführten Restedefinitionen bzw. Erläuterungen können auch untereinander, also zwischen den jeweiligen Bereichen und Vorzugsbereichen beliebig kombiniert werden. Die Definitionen gelten für die Endprodukte der Formel (I) wie für alle Zwischenprodukte gleichermaßen.
- R¹: steht bevorzugt für Wasserstoff, C₁-C₆-Alkyl, C₂-C₆-Alkenyl, C₂-C₆-Alkinyl, C₃-C₆-Cyclo-alkyl, Cyano(C₁-C₆-alkyl), C₁-C₆-Haloalkyl, C₂-C₆-Haloalkenyl, C₂-C₆-Haloalkinyl, C₁-C₄-Alkoxy-C₁-C₄-alkyl, C₁-C₄-Alkylthio-C₁-C₄-alkyl, C₁-C₄-Alkylsulfinyl-C₁-C₄-alkyl oder C₁-C₄-Alkylsulfonyl-C₁-C₄-alkyl,
- R¹: steht besonders bevorzugt für Wasserstoff, Methyl, Cyclopropyl, Cyanomethyl, Methoxymethyl, Methylthiomethyl, Methylsulfinylmethyl oder Methylsulfonylmethyl,
- R¹: steht ganz besonders bevorzugt für Wasserstoff.
- R²: steht bevorzugt für Wasserstoff oder C₁-C₆-Alkyl,
- R²: steht besonders bevorzugt für Wasserstoff oder Methyl,
- R²: steht ganz besonders bevorzugt für Wasserstoff.
- R³: steht bevorzugt für Wasserstoff oder für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
- R³: steht weiterhin bevorzugt für C₃-C₁₂-Cycloalkyl und C₄-C₁₀-Bicycloalkyl, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
- R³: steht besonders bevorzugt für Wasserstoff oder für jeweils gegebenenfalls einfach oder mehrfach, gleich oder verschieden substituiertes C₁-C₆-Alkyl oder C₁-C₆-Alkoxy, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
- R³: steht weiterhin besonders bevorzugt für gegebenenfalls einfach oder mehrfach, gleich oder verschieden substituiertes C₃-C₆-Cycloalkyl wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
- R³: steht ganz besonders bevorzugt für Methyl, Ethyl, n-Propyl, iso-Propyl, n-Butyl, iso-Butyl, sec-Butyl ,tert-Butyl cyclo-Propyl, cyclo-Butyl, cyclo-Pentyl, cyclo-Hexyl, cycloPropylmethyl, cyclo-Propylethyl, cyclo-Butylmethyl, cyclo-Butylethyl ,cyclo-Propylcyclo-Propyl, Ethyl-cyclo-Propyl, Cyanomethyl, 1-Cyanoethyl, 2-Cyanoethyl, 1-Cyano-n-Propyl, 2-Cyano-n-Propyl, 3-Cyano-n-Propyl, 1-Cyano-iso-Propyl, 2-Cyano-iso-Propyl, C₁-C₄-Alkylthio-(C₁-C₄-Alkyl), C₁-C₄-Alkylsulfinyl-(C₁-C₄-Alkyl) oder C₁-C₄-Alkylsulfonyl-(C₁-C₄-Alkyl),
- R⁴: steht bevorzugt für Wasserstoff, C₁-C₄-Alkyl, C₁-C₄-Haloalkyl, Halogen, Cyano, C₁-C₄-Alkoxy, C₁-C₄-Haloalkoxy, C₁-C₄-Alkylthio oder C₁-C₄-Haloalkylthio.

Bevorzugt stehen ausserdem zwei benachbarte Reste R⁴ für -(CH₂)₃-, -(CH₂)₄-, -(CH₂)₅-, - (CH=CH-)₂-, -OCH₂O-, -O(CH₂)₂O-, -OCF₂O-, -(CF₂)₂O-, -O(CF₂)₂O-, -(CH=CH-CH=N)- oder - (CH=CH-N=CH)-.
- R⁴: steht besonders bevorzugt für Wasserstoff, C₁-C₄-Alkyl, C₁-C₂-Haloalkyl, Halogen, Cyano oder C₁-C₂-Haloalkoxy.

Besonders bevorzugt stehen ausserdem zwei benachbarte Reste R⁴ für -(CH₂)₄-, -(CH=CH-)₂-, -O(CH₂)₂O-, -O(CF₂)₂O-, -(CH=CH-CH=N)- oder -(CH=CH-N=CH)-.
- R⁴: steht ganz besonders bevorzugt für Wasserstoff, Methyl, Trifluormethyl, Cyano, Fluor, Chlor, Brom, Iod oder Trifluormethoxy. Ganz besonders bevorzugt stehen ausserdem zwei benachbarte Reste R⁴ für -(CH₂)₄-, oder -(CH=CH-)₂-.
- R⁴: steht insbesondere bevorzugt für Chlor, Brom oder Fluor,
- R⁴: steht weiterhin insbesondere bevorzugt für Iod oder Cyano. Insbesondere bevorzugt stehen ausserdem zwei benachbarte Reste R⁴ für -(CH=CH-)₂-,
- n: steht bevorzugt für 1,2 oder 3,
- n: steht besonders bevorzugt für 1 oder 2,
- n: steht ganz besonders bevorzugt für 2,
- R⁵: steht bevorzugt für C₁-C₄-Alkyl, C₃-C₆-Cycloalkyl, C₁-C₄-Haloalkyl, C₁-C₆-Halocycloalkyl, C₂-C₆-Alkenyl, C₂-C₄-Haloalkenyl, C₂-C₄-Alkinyl, C₂-C₄-Haloalkinyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, Halogen, Cyano, Nitro oder C₃-C₆-Trialkylsilyl.
- R⁵: steht besonders bevorzugt für C₁-C₄-Alkyl, C₃-C₆-Cycloalkyl, C₁-C₄-Haloalkyl, C₁-C₆-Halocycloalkyl, C₂-C₆-Alkenyl, C₂-C₄-Haloalkenyl, C₂-C₄-Alkinyl, C₂-C₄-Haloalkinyl, C₁-C₄-Alkoxy, C₁-C₄-Haloalkoxy, Fluor, Chlor, Brom, Iod, Cyano, Nitro oder C₃-C₆-Trialkylsilyl,
- R⁵: steht ganz besonders bevorzugt für Methyl, Fluor, Chlor, Brom oder Iod.
- R⁵: steht insbesondere bevorzugt für Methyl oder Chlor.
- R⁶: steht bevorzugt für C₁-C₆-Alkyl oder 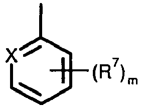
- R⁶: steht weiterhin bevorzugt für C₃-C₆-Cycloalkoxy,
- R⁶: steht besonders bevorzugt für Methyl oder 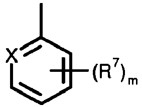
- R⁷: steht unabhängig voneinander bevorzugt für Wasserstoff, Halogen, Cyano, C₁-C₄-Alkyl, C₁-C₄-Alkoxy, C₁-C₄-Haloalkyloder C₁-C₄-Haloalkoxy
- R⁷: steht unabhängig voneinander besonders bevorzugt für Wasserstoff, Halogen oder C₁-C₄-Haloalkyl,
- R⁷: steht ganz besonders bevorzugt für Fluor, Chlor oder Brom,
- R⁷: steht insbesondere bevorzugt für Chlor und Brom.
- m: steht bevorzugt für 1, 2 oder 3,
- m: steht besonders bevorzugt für 1 oder 2,
- m: steht ganz besonders bevorzugt für 1,
- X: steht bevorzugt für N, CH, CF, CCl, CBr oder CI,
- X: steht besonders bevorzugt für N, CH, CF, CCl oder CBr,
- X: steht ganz besonders bevorzugt für N, CCl oder CH.
- A: steht bevorzugt für -CH₂-, -CH₂O-, -CH₂OCH₂-, -CH₂S-, -CH₂SCH₂-, -CH₂N(C₁-C₆-Alkyl)-, -CH₂N(C₁-C₆-Alkyl)CH₂-, -CH(CN)-, -CH(C₁-C₆-Alkyl)-, -C(Di-C₁-C₆-Alkyl)-, -CH₂CH₂-, -C=NO(C₁-C₆-Alkyl)-,
- A: steht besonders bevorzugt für -CH₂-, -CH(CH₃), C(CH₃)₂ oder CH₂CH₂,
- A: steht weiterhin besonders bevorzugt für -CH(CN)-,
- A: steht ganz besonders bevorzugt für CH₂ oder CH(CH₃),
- A: steht insbesondere bevorzugt für CH₂,
- Q: steht bevorzugt und besonders bevorzugt für einen 5-gliedrigen heteroaromatischen Ring oder ein aromatisches 9-gliedriges annelliertes heterobicyclisches Ringsystem, wobei der Ring oder das Ringsystem drei Stickstoffe enthalten und wobei die Stickstoffe im Ring oder Ringsystem benachbarte Positionen einnehmen und die Bindung des Rings oder des Ringsystems an den Rest A in Formel (I) über Stickstoff erfolgt. Der Ring oder das Ringsystem sind gegebenenfalls einfach oder mehrfach, gleich oder verschieden substituiert, wobei die Substituenten unabhängig voneinander ausgewählt sein können aus Wasserstoff, C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, CO₂H, CO₂NH₂, NO₂, OH, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino,
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, OH, C₁-C₄-Alkoxy, C₁-C₄-Haloalkoxy substituiert sein können,
- Q: steht ganz besonders bevorzugt für einen 5-gliedrigen heteroaromatischen Ring oder ein aromatisches 9-gliedriges annelliertes heterobicyclisches Ringsystem, wobei der Ring oder das Ringsystem drei Stickstoffe enthalten und wobei die Stickstoffe im Ring oder Ringsystem benachbarte Positionen einnehmen und die Bindung des Rings oder des Ringsystems an den Rest A in Formel (I) über Stickstoff erfolgt. Der Ring oder das Ringsystem sind gegebenenfalls einfach oder mehrfach, gleich oder verschieden substituiert, wobei die Substituenten unabhängig voneinander ausgewählt sein können aus Wasserstoff, C₁-C₄-Alkyl, C₂-C₄-Alkenyl, C₂-C₄-Alkinyl, C₃-C₆-Cycloalkyl, C₁-C₄-Haloalkyl, C₂-C₄-Haloalkenyl, C₂-C₄-Haloalkinyl, C₃-C₆-Halocycloalkyl, Halogen, CN, NO₂, C₁-C₄-Alkoxy, C₁-C₄-Haloalkoxy,
oder wobei die Substituenten unabhängig voneinander ausgewählt sein können aus Phenyl, welches gegebenenfalls einfach oder mehrfach, gleich oder verschieden mit C₁-C₄-Alkyl, C₂-C₆-Alkenyl, C₂-C₆-Alkinyl, C₃-C₆-Cycloalkyl, C₁-C₄-Haloalkyl, Halogen, CN, NO₂, C₁-C₄-Alkoxy, C₁-C₄-Haloalkoxy substituiert sein kann.,

Durch Halogen substituierte Reste, z.B. Haloalkyl, sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Bevorzugt, besonders bevorzugt, ganz besonders bevorzugt und insbesondere bevorzugt sind Verbindungen, welche jeweils die unter bevorzugt, besonders bevorzugt, ganz besonders bevorzugt und insbesondere bevorzugt genannten Substituenten tragen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die Verbindungen der Formeln (I) können in Form verschiedener Isomere vorliegen. Gegenstand der vorliegenden Erfindung sind daher auch die Isomere von Verbindungen der Formeln (I) sowie Mischungen verschiedener isomerer Formen.

Insbesondere können die Verbindungen der Formeln (I) in Form verschiedener Regioisomere vorliegen. Beispielsweise in Form von Mischungen aus Verbindungen, wobei die Bindung des Rings oder des Ringsystems Q an den Rest (A) jeweils über unterschiedliche Kohlenstoff- oder Stickstoffatome erfolgt.

### Herstellung der erfindungsgemäßen Verbindungen der allegemeinen Formel (I)

Anthranilamide der Formel (I) werden nach nach einem der folgenden Verfahren erhalten. Anthranilamide der Formel (I) 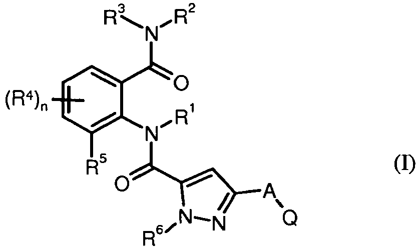 in welcher A, R¹, R², R³, R⁴, R⁵, R⁶, Q und n die oben angegebenen Bedeutungen haben, werden erhalten, indem man
(A) Aniline der Formel (II) 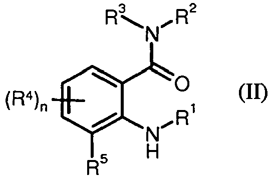 in welcher A, R¹, R², R³, R⁴, R⁵ und n die oben angegebenen Bedeutungen haben,
   mit Carbonsäurechloriden der Formel (III) 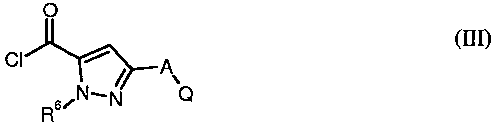 in welcher R⁶, A und Q die oben angegebenen Bedeutungen haben, in Gegenwart eines Säurebindemittels umsetzt,
(B) Aniline der Formel (II) 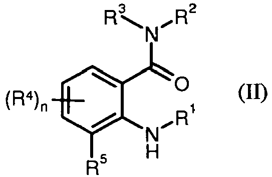 in welcher A, R¹, R², R³, R⁴, R⁵ und n die oben angegebenen Bedeutungen haben,
   mit einer Carbonsäure der Formel (IV) 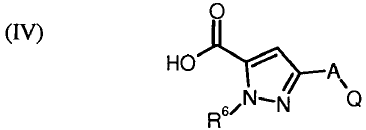 in welcher R⁶, A und Q die oben angegebenen Bedeutungen hat,
   in Gegenwart eines Kondensationsmittels umsetzt oder indem man
(C) zur Synthese von Anthranilamiden der Formel (I), in welcher R¹ für Wasserstoff steht, Benzoxazinone der Formel (V) 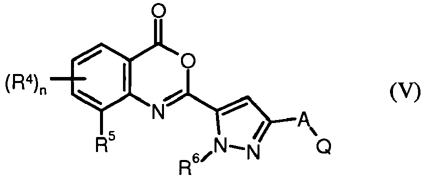 in welcher R⁴, R⁵, R⁶, A, Q und n die oben angegebenen Bedeutungen haben,
   mit einem Amin der Formel (VI) 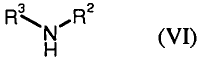 in welcher R² und R³ die oben angegebenen Bedeutungen haben,
   in Gegenwart eines Verdünnungsmittels umsetzt.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..
Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.
Aus der Klasse der Bivalva z.B. Dreissena spp..
Aus der Ordnung der Chilopoda z.B. Geophilus spp., Scutigera spp..
Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..
Aus der Ordnung der Collembola z.B. Onychiurus armatus.
Aus der Ordnung der Dermaptera z.B. Forficula auricularia.
Aus der Ordnung der Diplopoda z.B. Blaniulus guttulatus.
Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.
Aus der Klasse der Gastropoda z.B. Arion spp., Biomphalaria spp., Bulinus spp., Deroceras spp., Galba spp., Lymnaea spp., Oncomelania spp., Succinea spp..
Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.
Weiterhin lassen sich Protozoen, wie Eimeria, bekämpfen.
Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.
Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.
Aus der Ordnung der Hymenoptera z.B. Diprion spp., Hoplocampa spp., Lasius spp., Monomorium pharaonis, Vespa spp..
Aus der Ordnung der Isopoda z.B. Armadillidium vulgare, Oniscus asellus, Porcellio scaber.
Aus der Ordnung der Isoptera z.B. Reticulitermes spp., Odontotermes spp..
Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..
Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.
Aus der Ordnung der Siphonaptera z.B. Ceratophyllus spp., Xenopsylla cheopis.
Aus der Ordnung der Symphyla z.B. Scutigerella immaculata.
Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..
Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die Wirksamkeit der Verbindungen der Formel (I) lässt sich durch die Zugabe von Ammonium- und Phosphoniumsalzen steigern. Die Ammonium- und Phosphoniumsalze werden durch Formel (XI) definiert 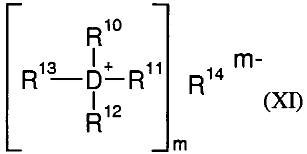 in welcher
- D: für Stickstoff oder Phosphor steht,
- D: bevorzugt für Stickstoff steht,
- R¹⁰, R¹¹, R¹², and R¹³: unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
- R¹⁰, R¹¹, R¹², and R¹³: bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
- R¹⁰, R¹¹, R¹², and R¹³: besonders bevorzugt unabhängig voneinander für Wasserstoff, Methyl, Ethyl, n-Propyl, i-Propyl, n-Butyl, i-Butyl, s-Butyl oder t-Butyl stehen,
- R¹⁰, R¹¹, R¹², and R¹³: ganz besonders bevorzugt für Wasserstoff stehen,
- m: für 1, 2, 3 oder 4 steht,
- m: bevorzugt für 1 oder 2 steht,
- R¹⁴: für ein anorganisches oder organisches Anion steht,
- R¹⁴: bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat, Citrat oder Oxalat steht,
- R¹⁴: besonders bevorzugt für Laktat, Sulfat, Monohydrogenphosphat, Dihydrogenphosphat, Nitrat, Thiosulfat, Thiocyanat, Citrat, Oxalat oder Formiat steht.
- R¹⁴: ganz besonders bevorzugt für Sulfat steht.

Die Ammonium- und Phosphoniumsalze der Formel (XI) können in einem breiten Konzentrationsbereich zur Steigerung der Wirkung von Pflanzenschutzmitteln enthaltend Verbindungen der Formel (I) eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern ein Penetrationsförderer zugegeben. Selbst in diesen Fällen ist eine Wirkungssteigerung zu beobachten. Gegenstand der vorliegenden Erfindung ist also ebenfalls die Verwendung eines Penetrationsförders, sowie die Verwendung einer Kombination von Penetrationsförderer und Ammonium- und/oder Phosphoniumsalzen zur Wirkungssteigerung von Pflanzenschutzmitteln, die akarizid/insektizid wirksame Verbindungen der Formel (I) als Wirkstoff enthalten. Gegenstand der Erfindung ist schließlich weiterhin die Verwendung dieser Mittel zur Bekämpfung von Schadinsekten.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel

R-O-(-AO)ᵥ-R' (XII)

in welcher
- R: für geradkettiges oder verzweigtes Alkyl mit 4 bis 20 Kohlenstoffatomen steht,
- R': für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- v: für Zahlen von 2 bis 30 steht.

Eine bevorzugte Gruppe von Penetrationsförderern sind Alkanolalkoxylate der Formel

R-O-(-EO-)ₙ-R' (XII-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (XII-b)

in welcher
- R: die oben angegebene Bedeutung hat,
- R': die oben angegebene Bedeutung hat,
- EO: für -CH₂-CH₂-O- steht,
- PO: für 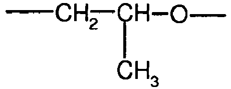 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (XII-c)

in welcher
- R: die oben angegebene Bedeutung hat,
- R': die oben angegebene Bedeutung hat,
- EO: für -CH₂-CH₂-O- steht,
- PO: für 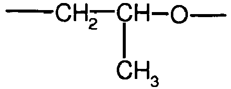 steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (XII-d)

in welcher
- R und R': die oben angegebenen Bedeutungen haben,
- EO: für CH₂-CH₂-O- steht,
- BO: für 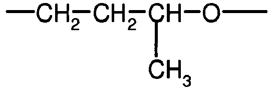 steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (XII-e)

in welcher
- R und R': die oben angegebenen Bedeutungen haben,
- BO: für 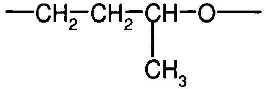 steht,
- EO: für CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (XII-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (XII-c) sei 2-Ethyl-hexyl-alkoxylat der Formel 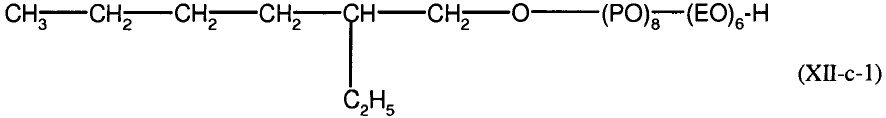 in welcher
- EO: für -CH₂-CH₂-O- steht,
- PO: für 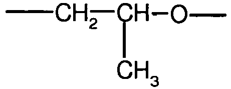 steht und
die Zahlen 8 und 6 Durchschnittswerte darstellen, genannt.

Als Beispiel für ein Alkanol-Alkoxylat der Formel (XII-d) sei die Formel

CH₃-(CH₂)₁₀-O-(-EO-)₆-(-BO-)₂-CH₃ (XII-d-1)

in welcher
- EO: für CH₂-CH₂-O- steht,
- BO: für 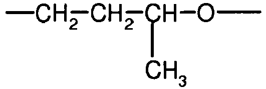 steht und
die Zahlen 10, 6 und 2 Durchschnittswerte darstellen, genannt.

Besonders bevorzugte Alkanol-Alkoxylate der Formel (XII-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (XII-f-1)

CH₃-(CH₂)ₜ-CH₂-O-(-CH₂-CH₂-O-)ᵤ-R' (XII-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Löslichkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäß hervorgehobene Kombinationen von Wirkstoff, Salz und Penetrationsförderer sind in folgender Tabelle aufgeführt."Gemäß Test" bedeutet dabei, dass jede Verbindung geeignet ist, die in dem Test für die Kutikelpenetration (Baur et al., 1997, Pesticide Science 51, 131-152) als Penetrationsförderer wirkt:

| # | Wirkstoff | Salz | Penetrationsförderer |
|---|---|---|---|
| 1 | I | Ammoniumsulfat | Gemäß Test |
| 2 | I | Ammoniumlaktat | Gemäß Test |
| 3 | I | Ammoniumnitrat | Gemäß Test |
| 4 | I | Ammoniumthiosulfat | Gemäß Test |
| 5 | I | Ammoniumthiocyanat | Gemäß Test |
| 6 | I | Ammoniumcitrat | Gemäß Test |
| 7 | I | Ammoniumoxalat | Gemäß Test |
| 8 | I | Ammoniumformiat | Gemäß Test |
| 9 | I | Ammoniumhydrogenphosphat | Gemäß Test |
| 10 | I | Ammoniumdihydrogenphosphat | Gemäß Test |
| 11 | I | Ammoniumcarbonat | Gemäß Test |
| 12 | I | Ammoniumbenzoat | Gemäß Test |
| 13 | I | Ammoniumsulfit | Gemäß Test |
| 14 | I | Ammoniumbenzoat | Gemäß Test |
| 15 | I | Ammoniumhydrogenoxalat | Gemäß Test |
| 16 | I | Ammoniumhydrogencitrat | Gemäß Test |
| 17 | I | Ammoniumacetat | Gemäß Test |
| 18 | I | Tetramethylammoniumsulfat | Gemäß Test |
| 19 | I | Tetramethylammoniumlaktat | Gemäß Test |
| 20 | I | Tetramethylammoniumnitrat | Gemäß Test |
| 21 | I | Tetramethylammoniumthiosulfat | Gemäß Test |
| 22 | I | Tetramethylammoniumthiocyanat | Gemäß Test |
| 23 | I | Tetramethylammoniumcitrat | Gemäß Test |
| 24 | I | Tetramethylammoniumoxalat | Gemäß Test |
| 25 | I | Tetramethylammoniumformiat | Gemäß Test |
| 26 | I | Tetramethylammoniumhydrogenphosphat | Gemäß Test |
| 27 | I | Tetramethylammoniumdihydrogenphosphat | Gemäß Test |
| 28 | I | Tetraethylammoniumsulfat | Gemäß Test |
| 29 | I | Tetraethylammoniumlaktat | Gemäß Test |
| 30 | I | Tetraethylammoniumnitrat | Gemäß Test |
| 31 | I | Tetraethylammoniumthiosulfat | Gemäß Test |
| 32 | I | Tetraethylammoniumthiocyanat | Gemäß Test |
| 33 | I | Tetraethylammoniumcitrat | Gemäß Test |
| 34 | I | Tetraethylammoniumoxalat | Gemäß Test |
| 35 | I | Tetraethylammoniumformiat | Gemäß Test |
| 36 | I | Tetraethylammoniumhydrogenphosphat | Gemäß Test |
| 37 | I | Tetraethylammoniumdihydrogenphosphat | Gemäß Test |

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen. Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

### Verfahren (A)

Verwendet man beispielsweise 2-Amino-N-ethyl-5-iod-3-methylbenzamid und 1-(3-Chlorpyridin-2-yl)-3-{[4-(trifluormethyl)-1H-1,2,3-triazol-1-yl]methyl}-1H-pyrazol-5-carbonsäurechlorid als Ausgangsstoffe, so kann der Verlauf des Verfahrens (A) durch das folgende Formelschema veranschaulicht werden.

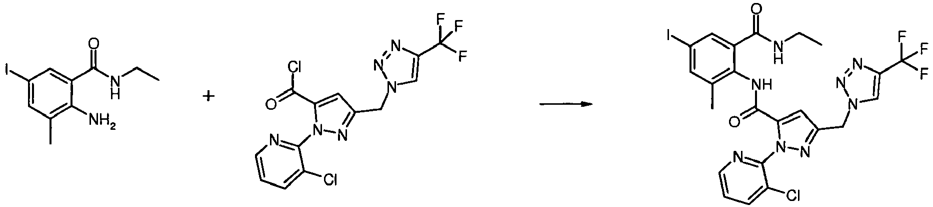

Die bei der Durchführung des Verfahrens (A) als Ausgangsstoffe benötigten Aminobenzamide sind durch die Formel (II) allgemein definiert.

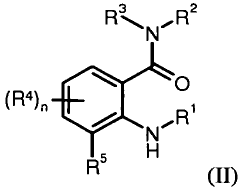

In dieser Formel (II) haben R¹, R², R³, R⁴, R⁵ und n die oben angegebene Bedeutung.

Das Verfahren (A) wird in Gegenwart eines Säurebindemittels durchgeführt. Hierzu eignen sich alle für solche Kupplungsreaktionen üblichen anorganischen oder organischen Basen. Vorzugsweise verwendbar sind Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natrium-methylat, Natriumethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, Diisopropylethylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU). Ebenso lassen sich gegebenenfalls polymer-gestützte Säurebindemittel, wie z.B. polymer-gebundenes Diisopropylamin und polymer-gebundenes Dimethylaminopyridin einsetzen.

Das Verfahren (A) kann gegebenenfalls in Gegenwart eines für solche Reaktionen üblichen inerten organischen Verdünnungsmittel durchgeführt werden. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser.

Aminobenzamide der Formel (II) sind bekannt oder können nach bekannten Methoden hergestellt werden (vgl. z.B. M. J. Kornet, J. Heterocyl. Chem. 1992, 29, 103-105; G. P. Lahm et al., Bioorg. Med. Chem. Letters 2005, 15, 4898-4906; WO 2003/016284, WO 2006/055922, WO 2006/062978, WO 2008/010897, WO 2008/070158).

Die bei der Durchführung des Verfahrens (A) als Ausgangsstoffe benötigten Pyrazolcarbonsäurechloride sind durch die Formel (III) allgemein definiert.

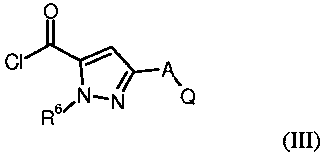

In dieser Formel (III) haben Q, A und R⁶ die oben angegebene Bedeutung.

Pyrazolcarbonsäurechloride der Formel (III) sind neu. Sie lassen sich beispielsweise herstellen, indem man
Pyrazolcarbonsäure-Derivate der Formel (IV) 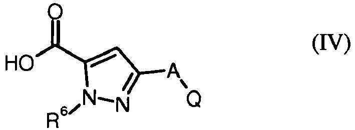 in welcher Q, A und R⁶ und n die oben angegebene Bedeutung haben,
mit einem Chlorierungsmittel (z.B. Thionylchlorid und Oxalylchlorid) in Gegenwart eines inerten Verdünnungsmittels (z.B. Toluol und Dichlormethan) in Gegenwart von einer katalytischen Menge von N,N-Dimethylformamid umsetzt.

Pyrazolcarbonsäure-Derivate der Formel (IV) sind neu. Sie lassen sich beispielsweise herstellen, indem man
Pyrazolcarbonsäureester der Formel (VII) 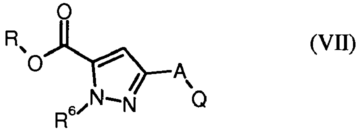 in welcher Q, A und R⁶ die oben angegebene Bedeutungen haben und R für C₁-C₆-Alkyl steht, mit einem Alkalimetallhydroxid (z.B. Natriumhydroxid oder Kaliumhydroxid) in Gegenwart eines inerten Verdünnungsmittels (z.B. Dioxan/Wasser oder Ethanol/Wasser) umsetzt.

Pyrazolcarbonsäureester der Formel (VII) sind bekannt oder können nach bekannten Verfahren erhalten werden (vgl. z.B. Smallheer, Joanne M.; Alexander, Richard S.; Wang, Jianmin; Wang, Shuaige; Nakajima, Suanne; Rossi, Karen A.; Smallwood, Angela; Barbera, Frank; Burdick, Debra; Luettgen, Joseph M.; Knabb, Robert M.; Wexler, Ruth R.; Jadhav, Prabhakar Bioorganic & Medicinal Chemistry Letters 2004, 14(21), 5263-5267).

### Verfahren (B)

Verwendet man beispielsweise 2-Amino-N-ethyl-5-iod-3-methylbenzamid und 1-(3-Chlorpyridin-2-yl)-3-{[4-(trifluormethyl)-1H-1,2,3-triazol-1-yl]methyl}-1H-pyrazol-5-carbonsäure als Ausgangsstoffe, so kann der Verlauf des Verfahrens (B) durch das folgende Formelschema veranschaulicht werden.

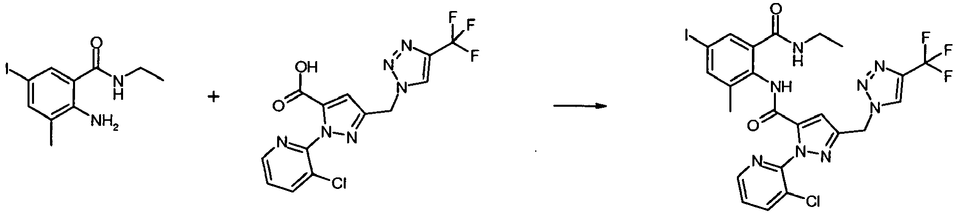

Die bei der Durchführung des Verfahrens (B) als Ausgangsstoffe benötigten Anthranilamide der Formel (II) sind bereits in Zusammenhang mit dem Verfahren (A) beschrieben worden.

Die bei der Durchführung des Verfahrens (B) weiterhin als Ausgangsstoffe benötigten Pyrazolcarbonsäuren sind durch die Formel (IV) allgemein definiert.

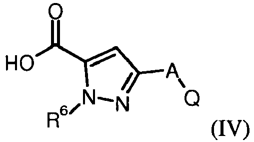

In dieser Formel (IV) haben Q, A und R⁶ die oben angegebene Bedeutung.

Das Verfahren (B) wird in Gegenwart eines Kondensationsmittels durchgeführt. Hierzu eignen sich alle für solche Kupplungsreaktionen üblichen Mittel. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, 1,1'-Carbonyldiimidazol, 2-Ethoxy-N-ethoxycarbonyl-1,2-dihydrochinolin (EEDQ), Triphenylphosphin/Tetrachlorkohlenstoff, Brom-tripyrrolidinophosphonium-hexafluorophosphat, Bis(2-oxo-3-oxazolidinyl)-phosphinchlorid oder Benzotriazol-1-yloxytris(dimethylamino)-phosphonium-hexafluorphosphat. Auf Polymer gestützte Reagenzien, wie z.B. polymer-gebundenes Cyclohexylcarbodiimid, können ebenfalls verwendet werden.

Das Verfahren (B) wird gegebenenfalls in Gegenwart eines Katalysators durchgeführt. Beispielsweise genannt seien 4-Dimethylaminopyridin, 1-Hydroxy-benzotriazol oder Dimethylformamid.

Das Verfahren (B) kann gegebenenfalls in Gegenwart eines für solche Reaktionen üblichen inerten organischen Verdünnungsmittel durchgeführt werden. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser.

### Verfahren (C)

Verwendet man 2-[1-(3-chlorpyridin-2-yl)-3-{[4-(trifluormethyl)-1H-1,2,3-triazol-1-yl]methyl}-1H-pyrazol-5-yl]-6-iod-8-methyl-4H-3,1-benzoxazin-4-on und Ethylamin, so kann der Verlauf des Verfahrens (C) durch das folgende Formelschema veranschaulicht werden.

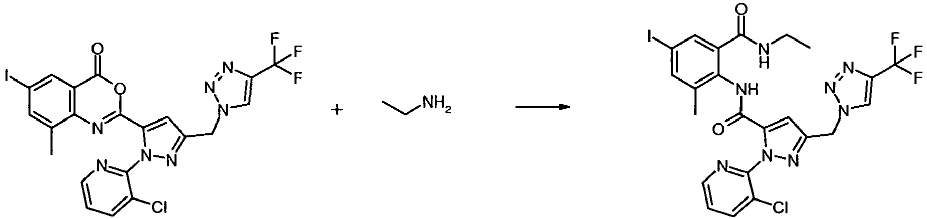

Die bei der Durchführung des Verfahrens (C) als Ausgangsstoffe benötigten Benzoxazinone sind durch die Formel (V) allgemein definiert.

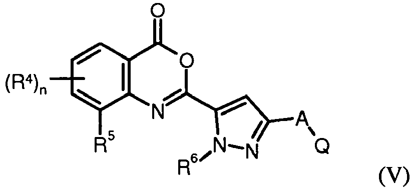

In dieser Formel (V) haben R⁴, R⁵, R⁶, A, Q und n die oben angegebene Bedeutung.

Benzoxazinone der Formel (V) sind neu. Sie werden beispielsweise erhalten, indem man
Pyrazolcarbonsäure-Derivate der Formel (IV) 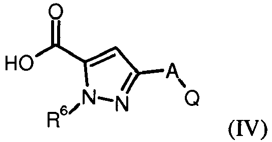 in welcher Q, A und R⁶ die oben angegebene Bedeutung hat,
mit Anthranilsäuren der Formel (VIII) 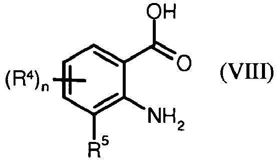 in welcher R⁴, R⁵ und n die oben angegebenen Bedeutungen haben, in Anwesenheit einer Base (z.B. Triethylamin oder Pyridin) und in Gegenwart eines Sulfonsäurechlorids (z.B. Methansulfonsäurechlorid) sowie gegebenenfalls in Gegenwart eines Verdünnungsmittels (z.B. Acetonitril) umsetzt.

Die bei der Durchführung des Verfahrens als Ausgangsstoffe benötigten Pyrazolcarbonsäure-Derivate der Formel (IV) sind bereits oben in Zusammenhang mit dem Verfahren (A) beschrieben worden.

Anthranilsäuren der Formel VIII) sind bekannt oder können nach allgemeinen Synthesemethoden hergestellt werden (vgl. z. B. Baker et al. J. Org. Chem. 1952, 149-153; G. Reissenweber et al., Angew. Chem 1981, 93, 914-915, P.J. Montoya-Pelaez, J. Org. Chem. 2006, 71, 5921-5929; F. E. Sheibley, J. Org. Chem. 1938, 3, 414-423, WO 2006023783).

### Herstellungsbeispiele

### Verbindungen:

### Synthese von 1-(3-Chlorpyridin-2-yl)-N-[2-(ethylcarbamoyl)-4-iod-6-methylphenyl]-3-{[4-(trifluormethyl)-1H-1,2,3-triazol-1-yl]methyl}-1H-pyrazol-5-carboxamid (Beispiel 1)

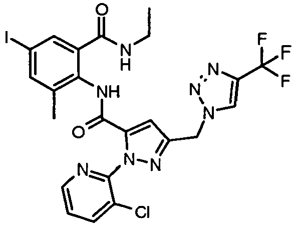

140 mg (0.228 mmol) 2-[1-(3-chlorpyridin-2-yl)-3-{[4-(trifluormethyl)-1H-1,2,3-triazol-1-yl]methyl}-1H-pyrazol-5-yl]-6-iod-8-methyl-4H-3,1-benzoxazin-4-on wurden in 20 ml Tetrahydrofuran vorgelegt. Nach Einleiten von 103 mg (2.281 mmol) Ethylamin wurde die resultierende Lösung 12 h bei Raumtemperatur gerührt, das Lösungsmittel und der Aminüberschuss anschließend im Vakuum bei 40°C abdestilliert und der ölige blassgelbe Rückstand mit Petrolether kristallisiert. Es wurden 150mg (99% d. Th.; Gehalt 100%) des gewünschten Produktes isoliert.
logP: 3.04; MH⁺: 659; ¹H-NMR (400 MHz, DMSO-d₆, δ, ppm): 1.00 (t, 3H), 2.10 (s, 3H), 3.14 (m, 2H), 5.86 (s, 2H), 7.19 (s, 1H), 7.55 (dd, 1H), 7.59 (s, 1H), 7.69 (d, 1H), 7.98 (t, 1H), 8.09 (dd, 1H), 8.44 (dd, 1H), 8.93 (s, 1H), 10,06 (s, 1H).

Die folgenden Beispiele können auf analoge Art und Weise erhalten werden. Die Verbindungen der Formel (I) können teils als verschiedene Regioisomere vorliegen. Dabei sind in der folgenden Tabelle in Bezug auf die NMR-Daten jeweils die chemischen Verschiebungen und die dazugehörigen Signalintensitäten angegeben, beispielsweise steht für Verbindung 1:
Signal 1
   10.081, 2.12; für 10.081 ppm (chemische Verschiebung), 2.12 (Signalintensität);
Signal 2
   8.795, 8.04; für 8.795 ppm (chemische Verschiebung), 8.04 (Signalintensität).

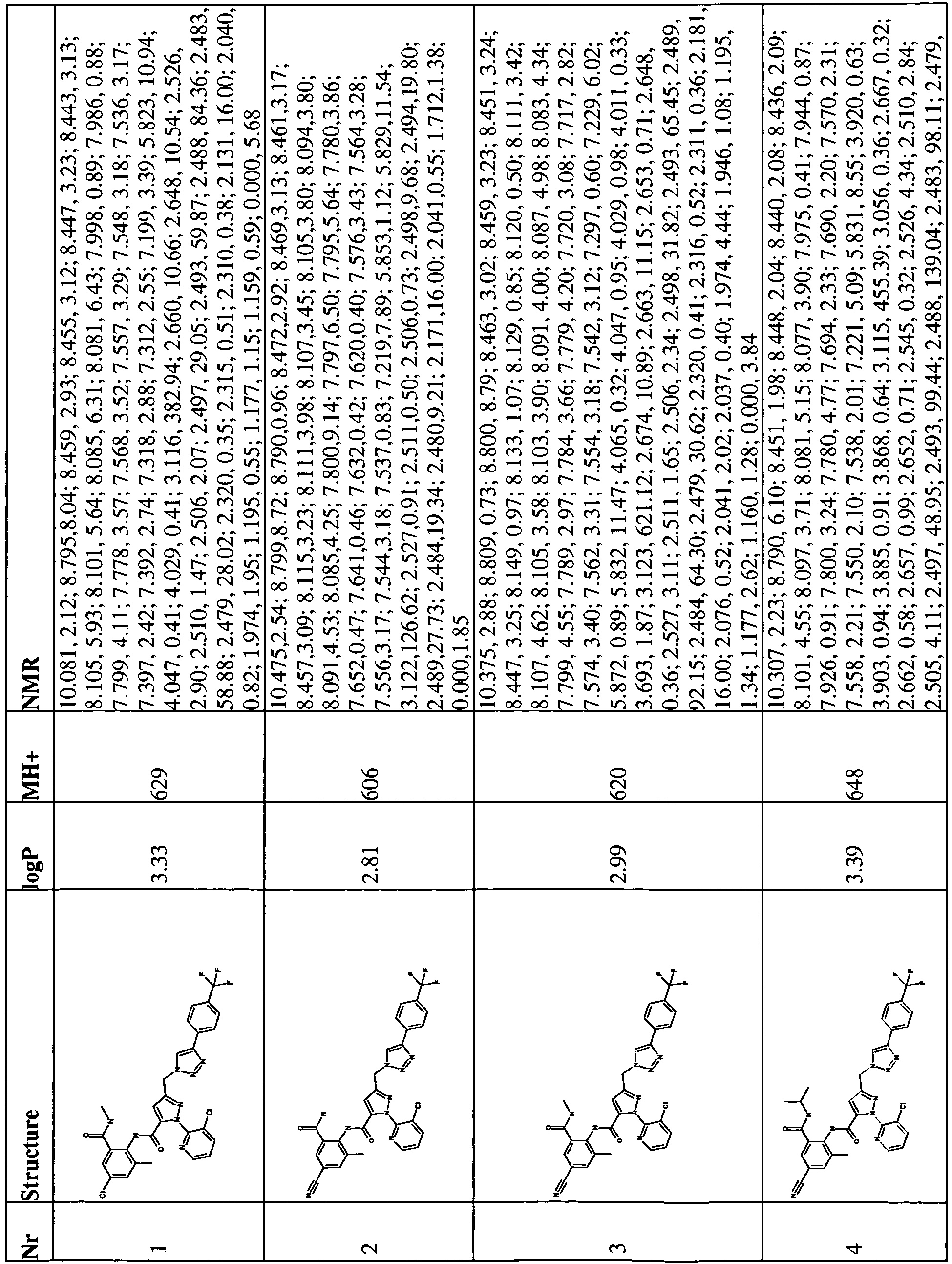

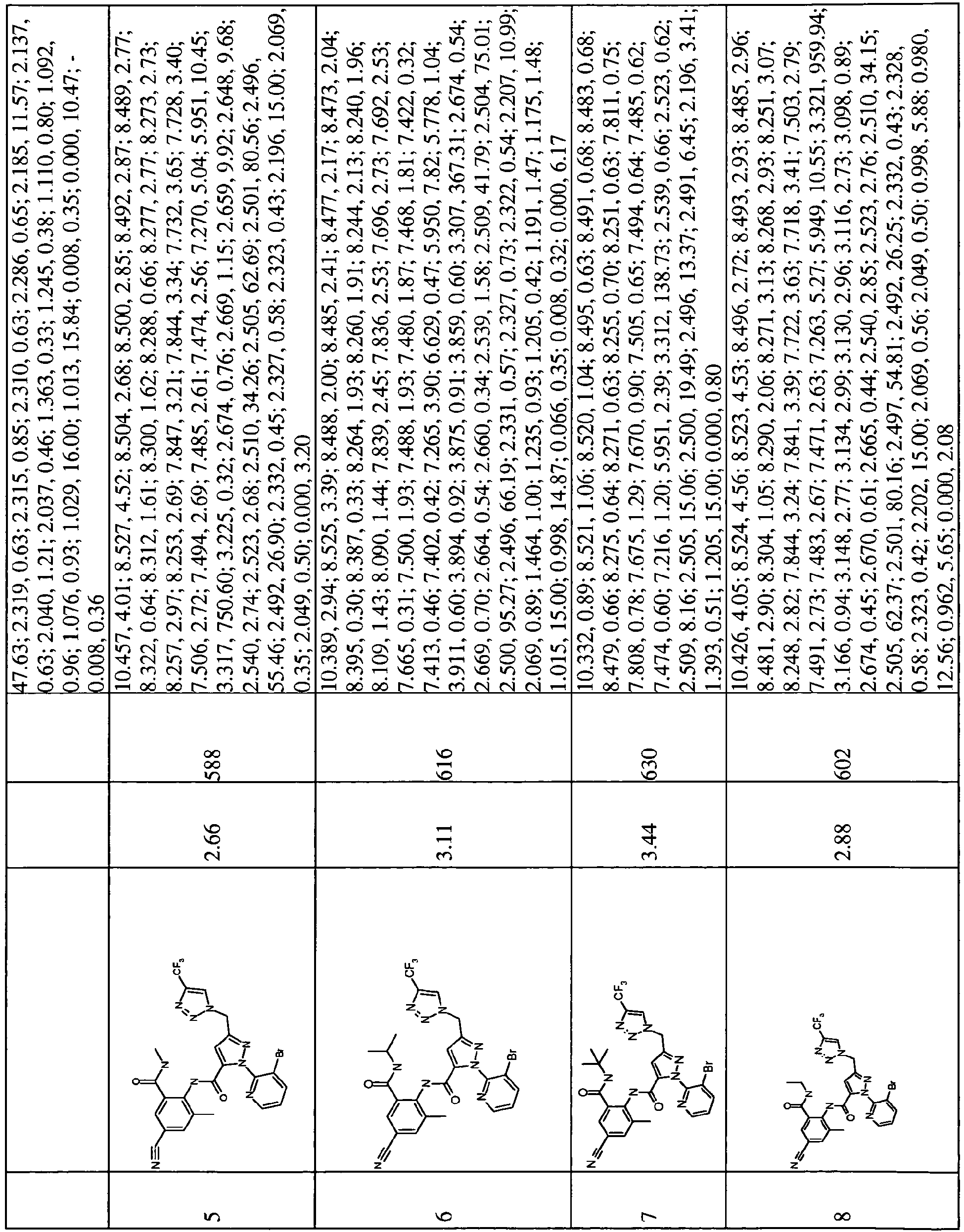

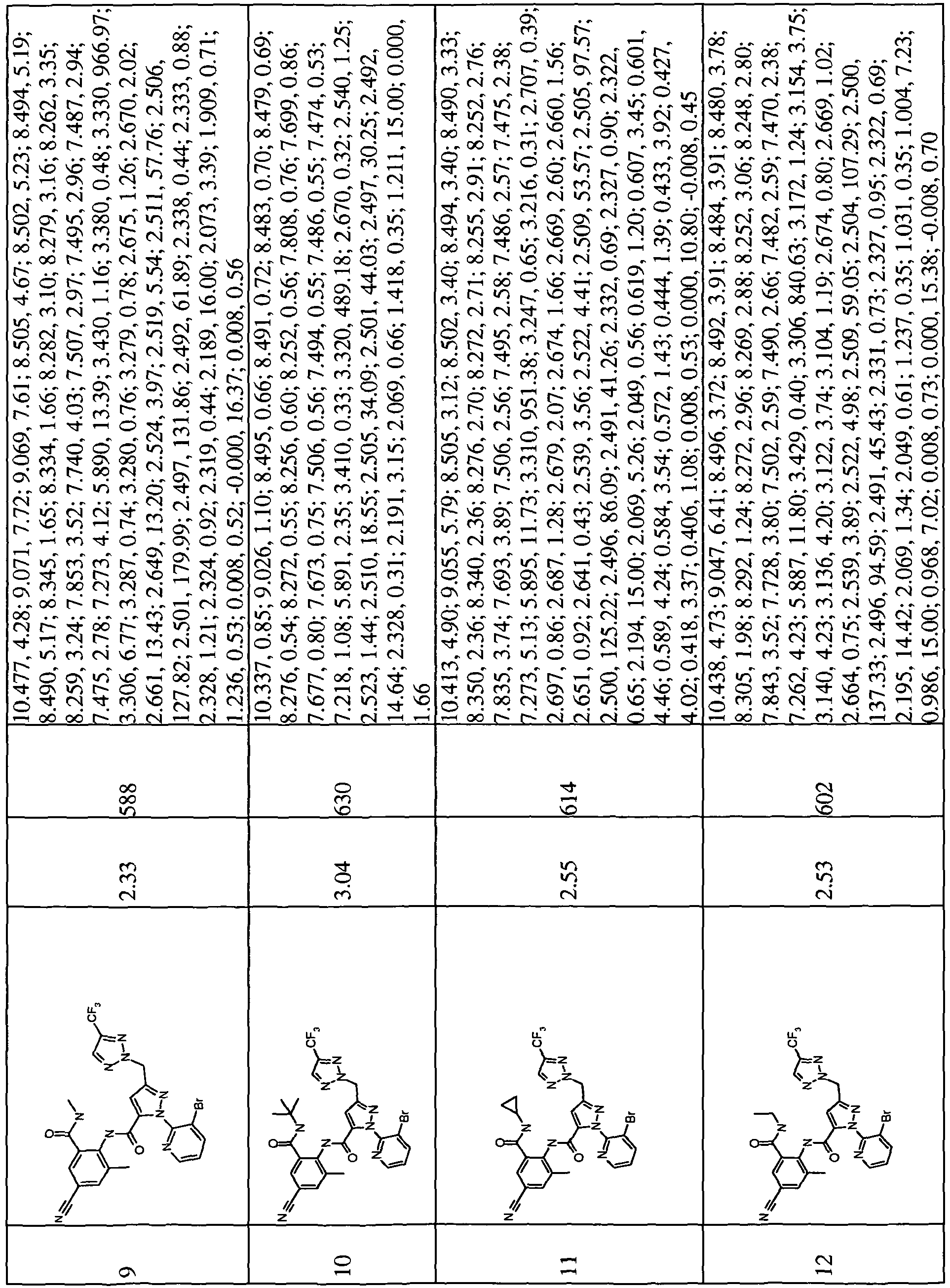

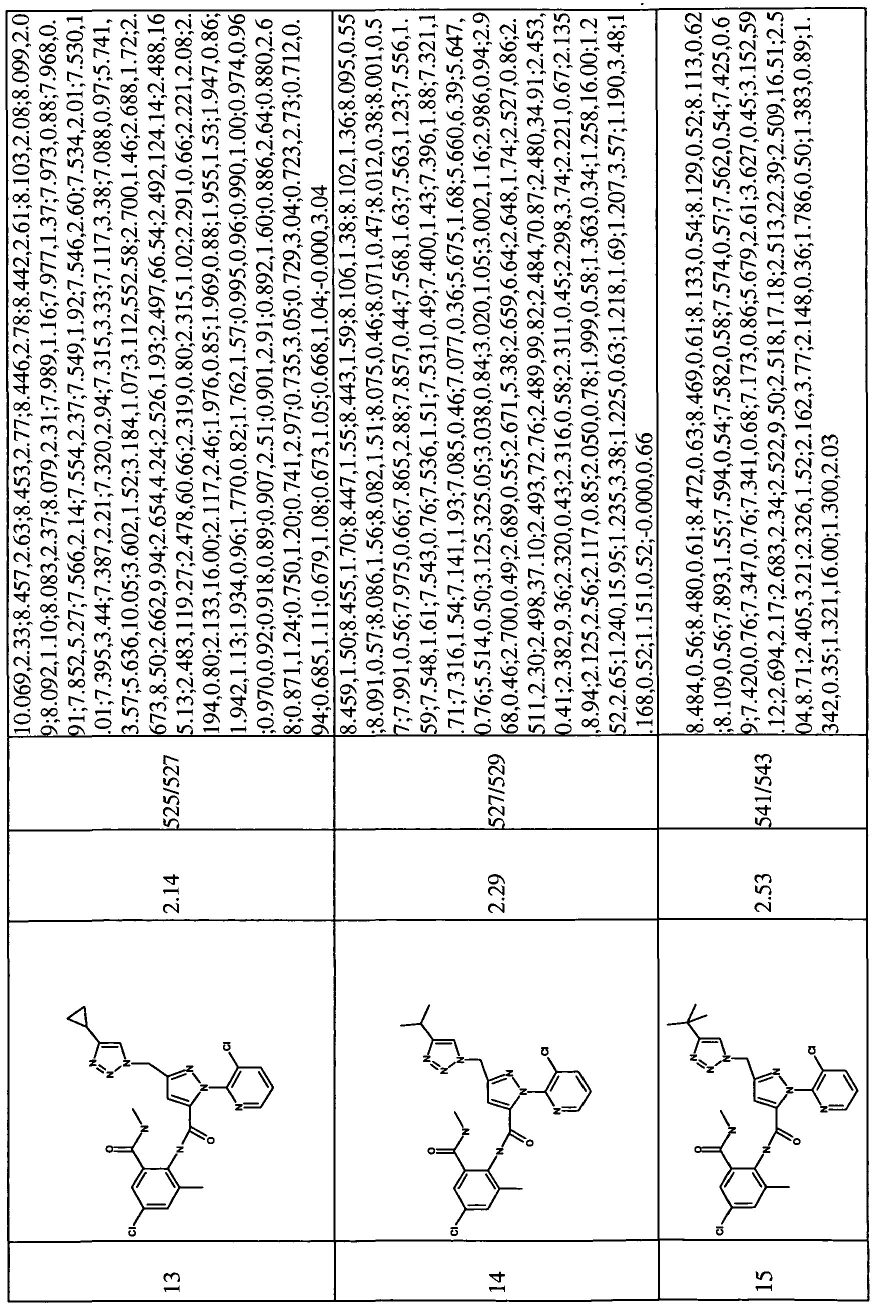

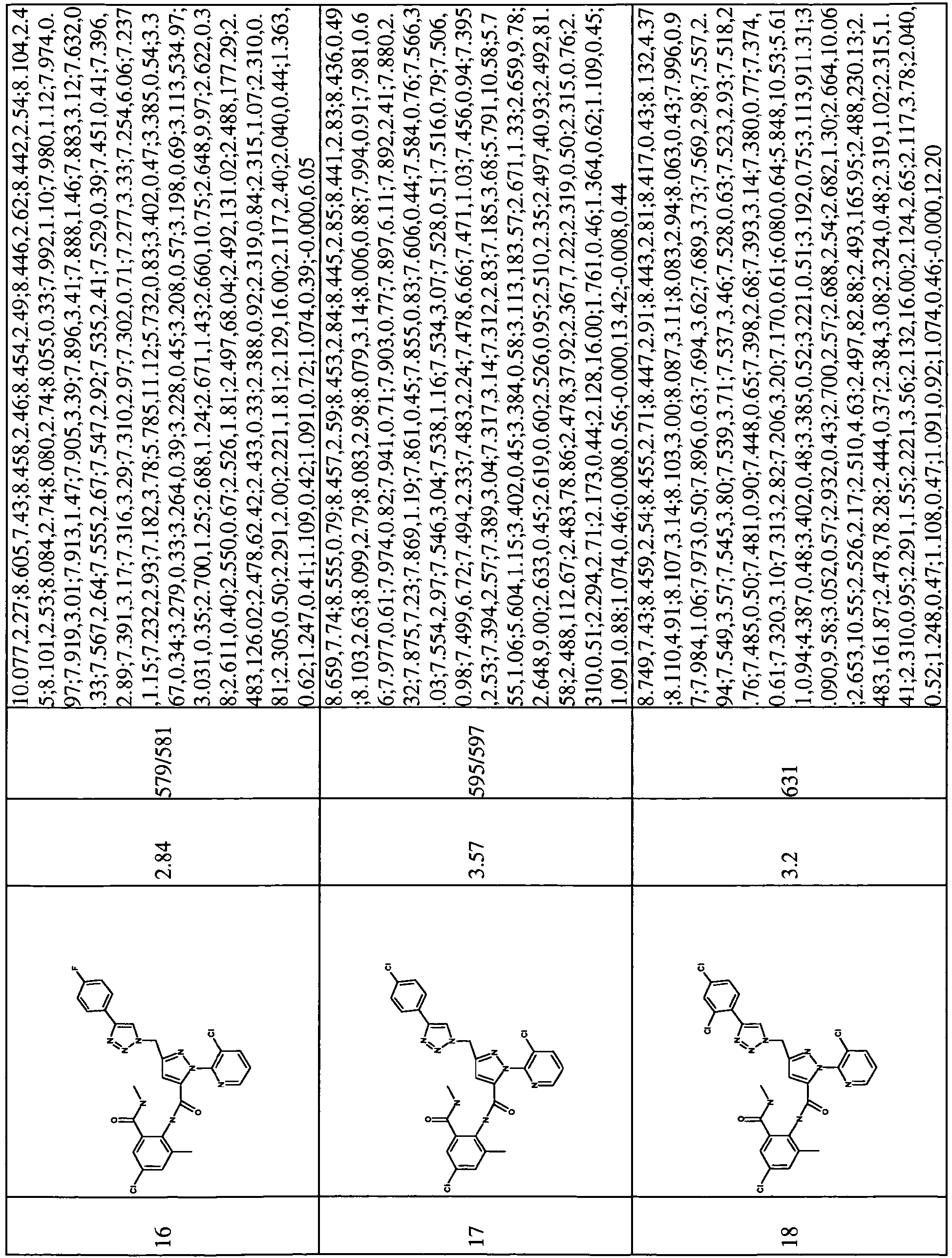

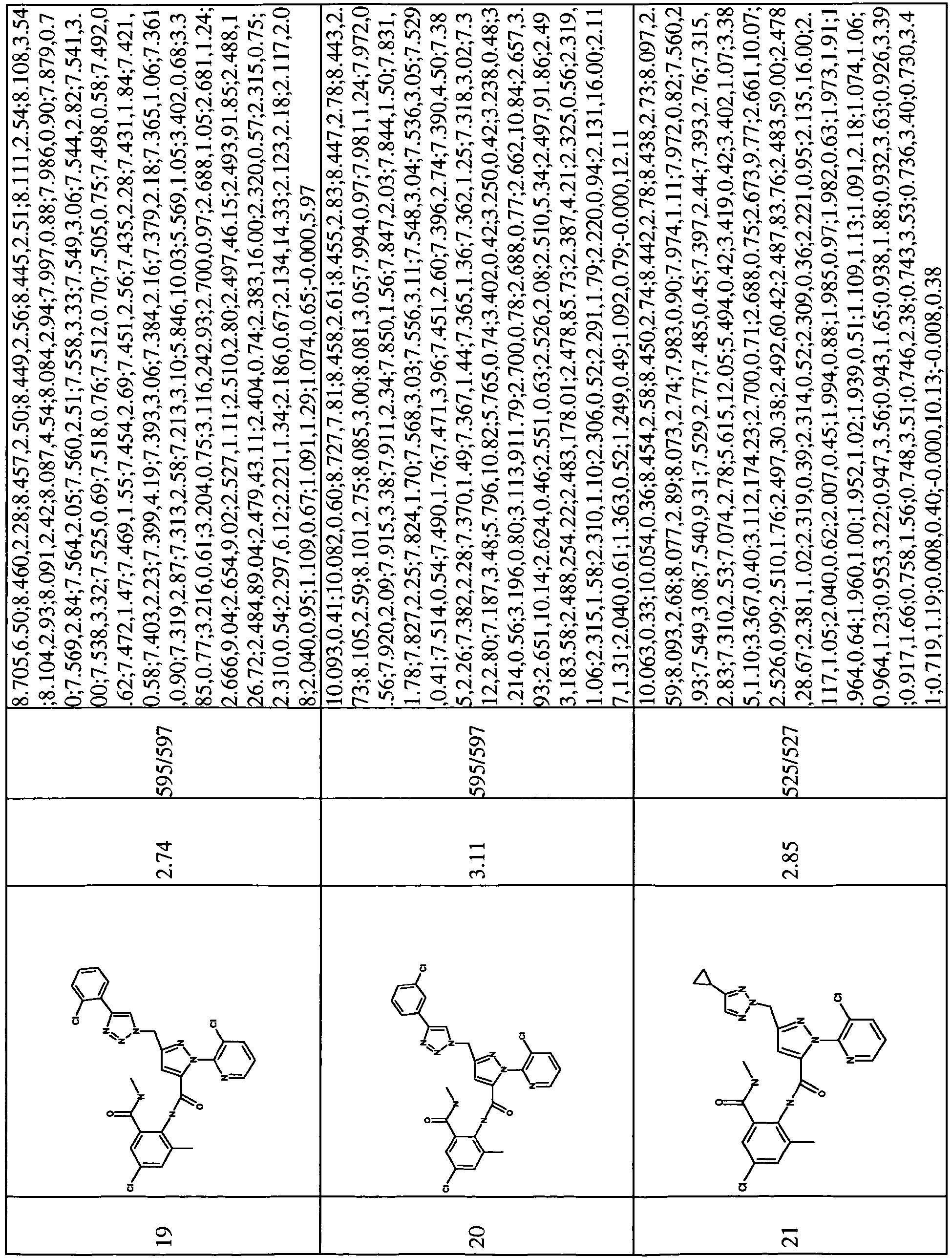

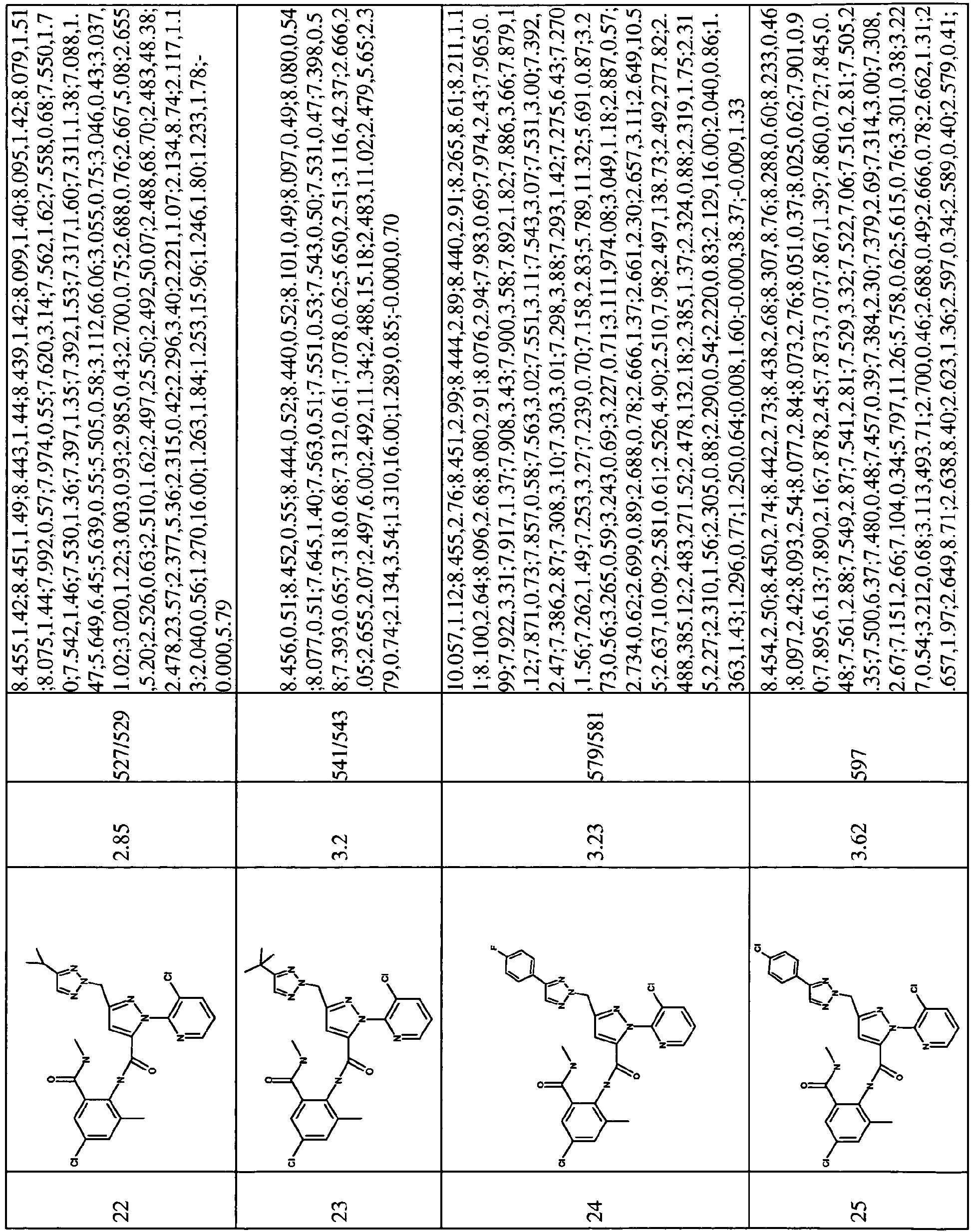

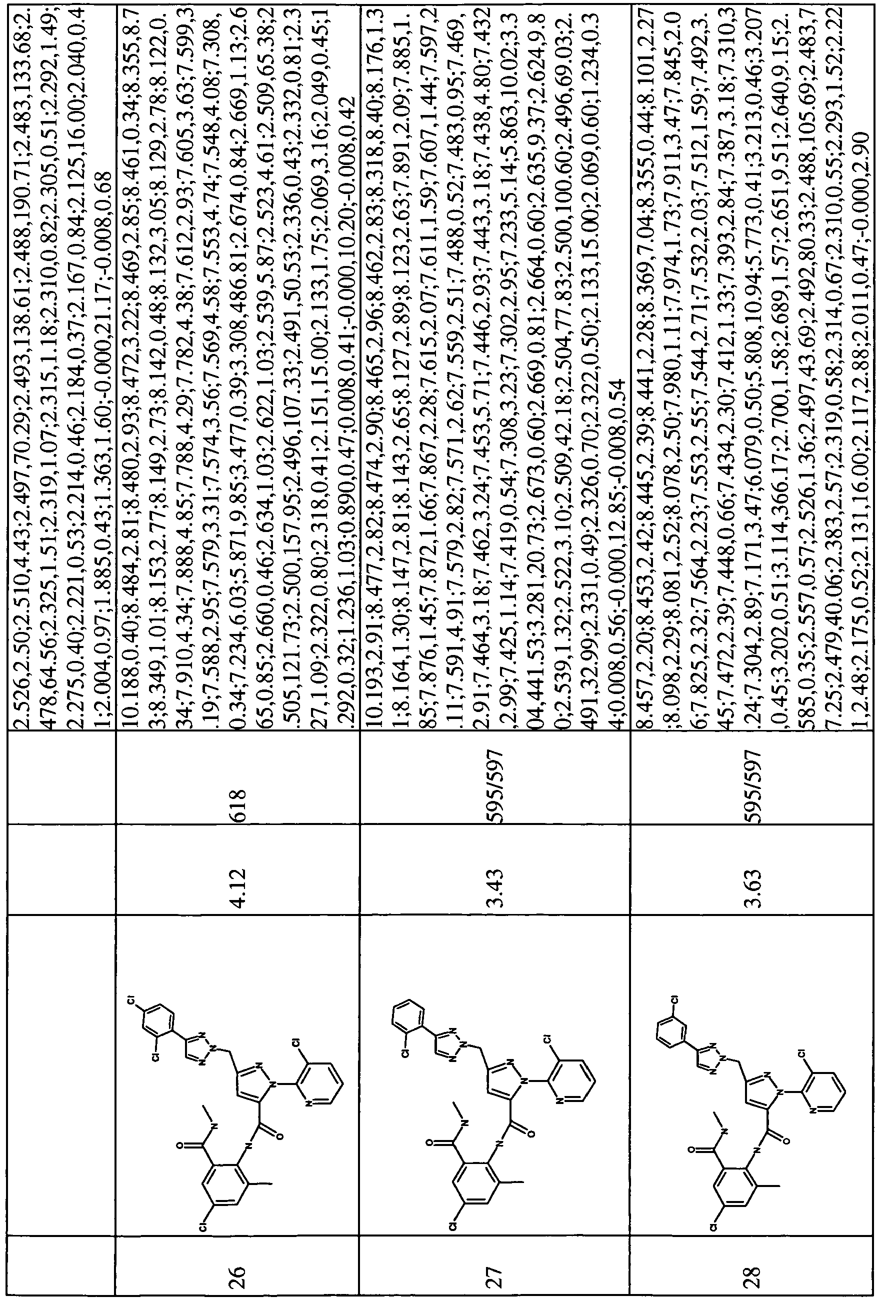

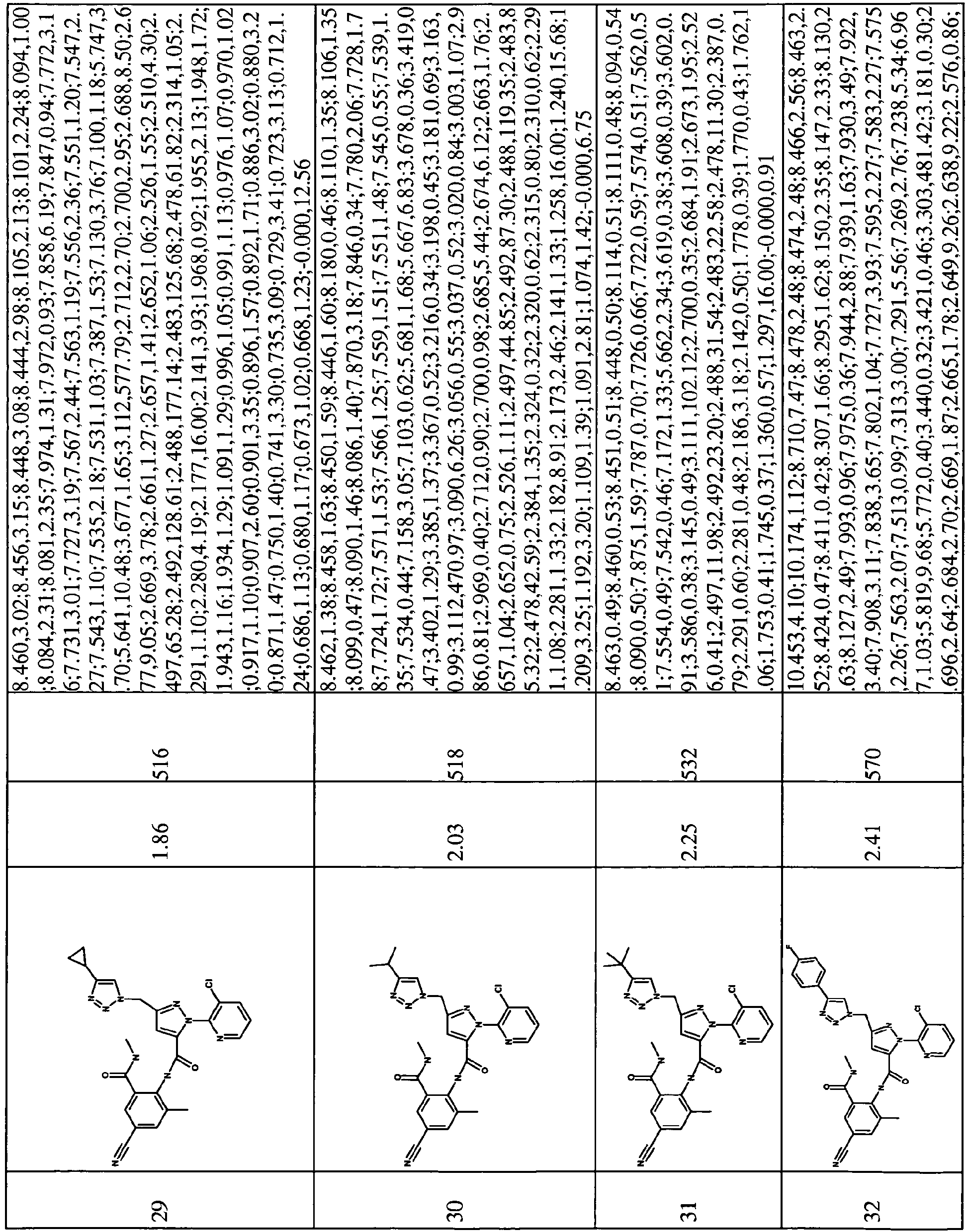

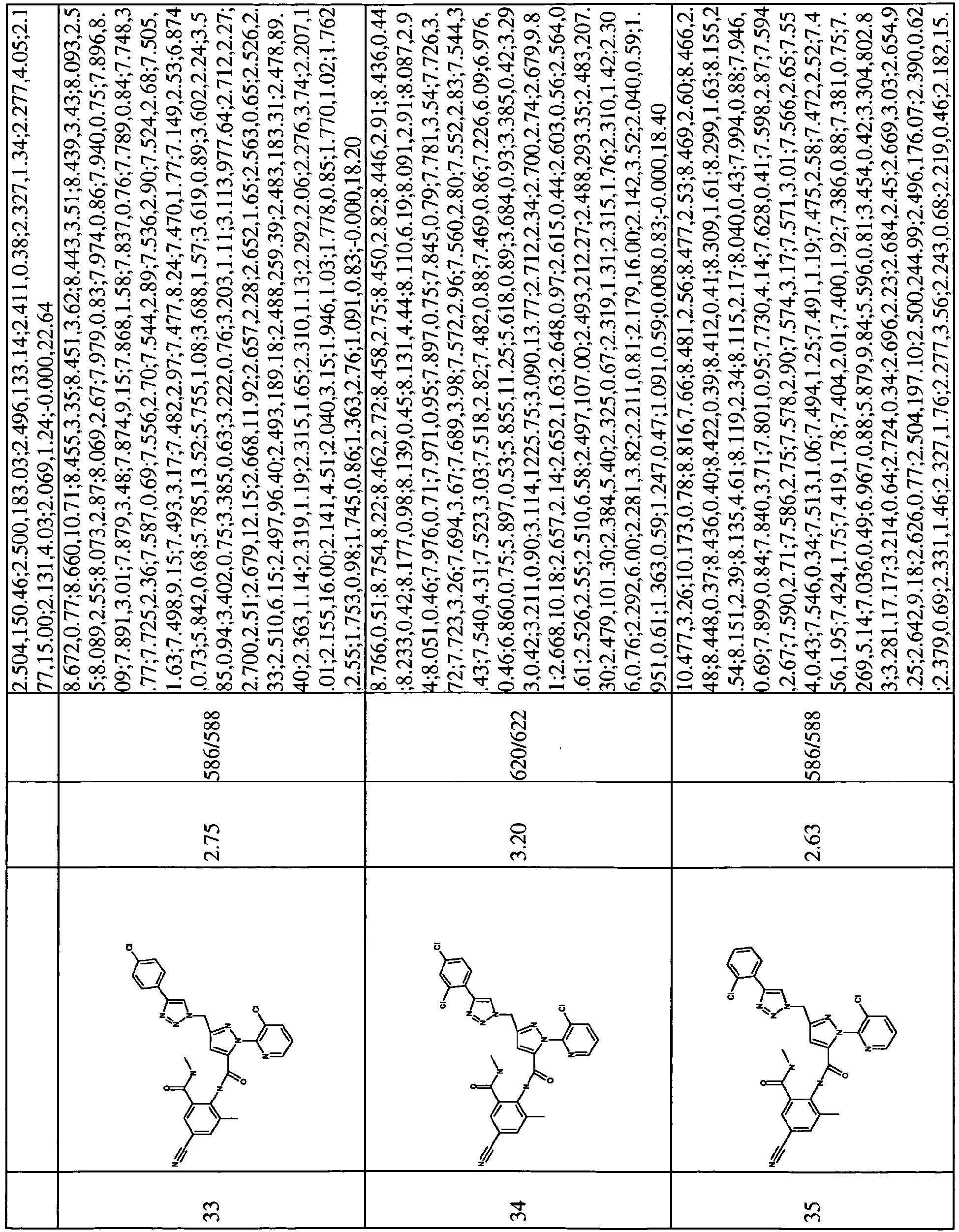

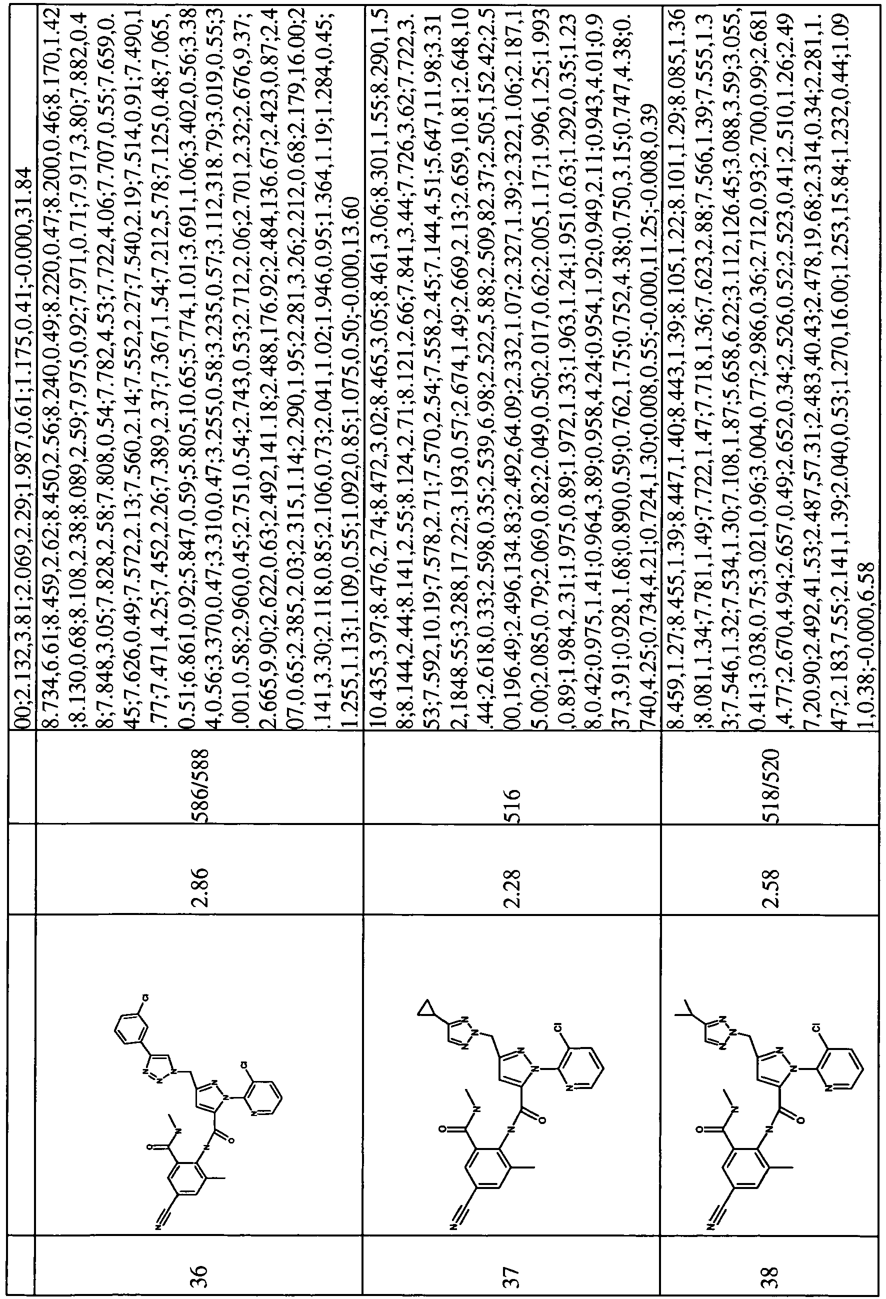

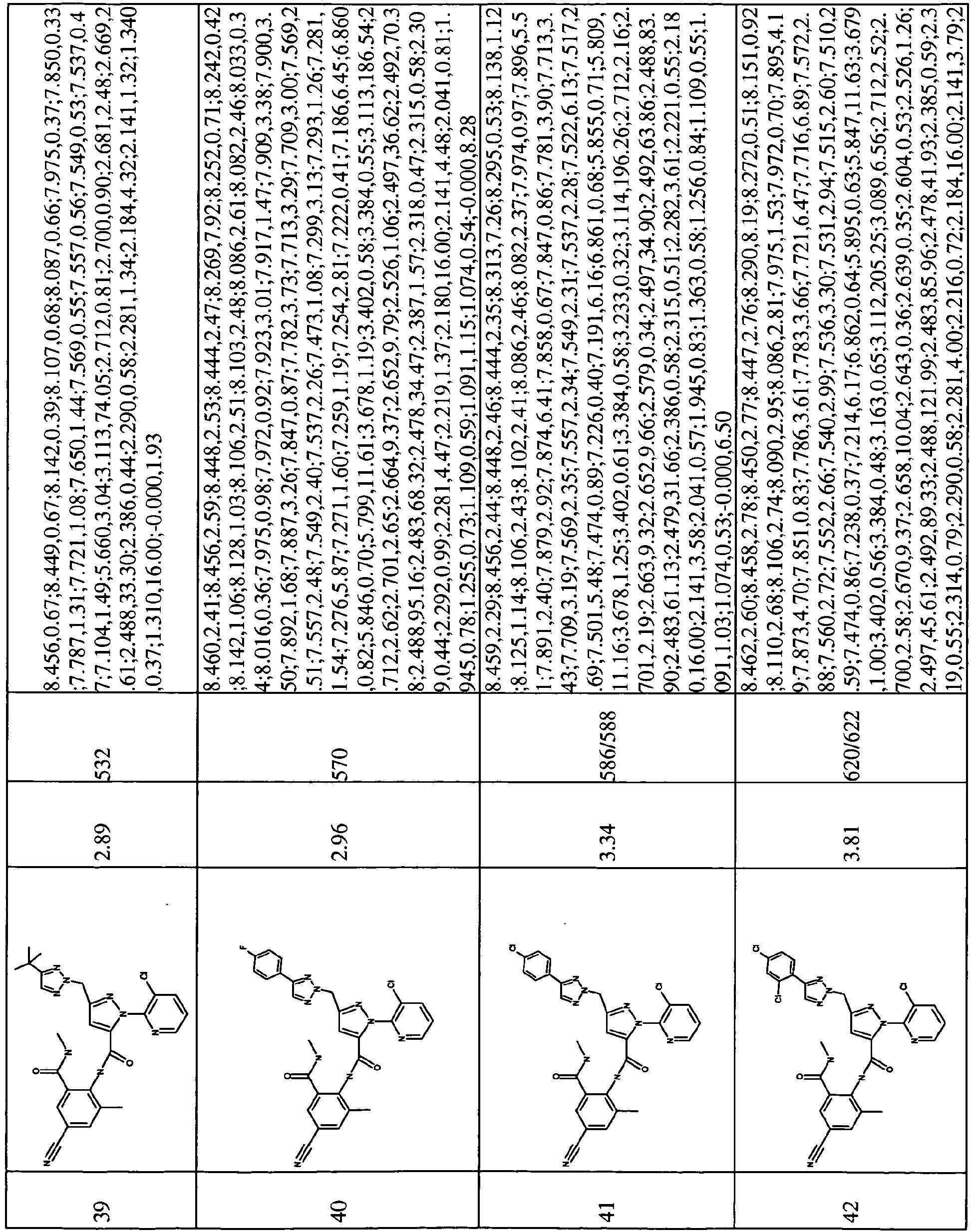

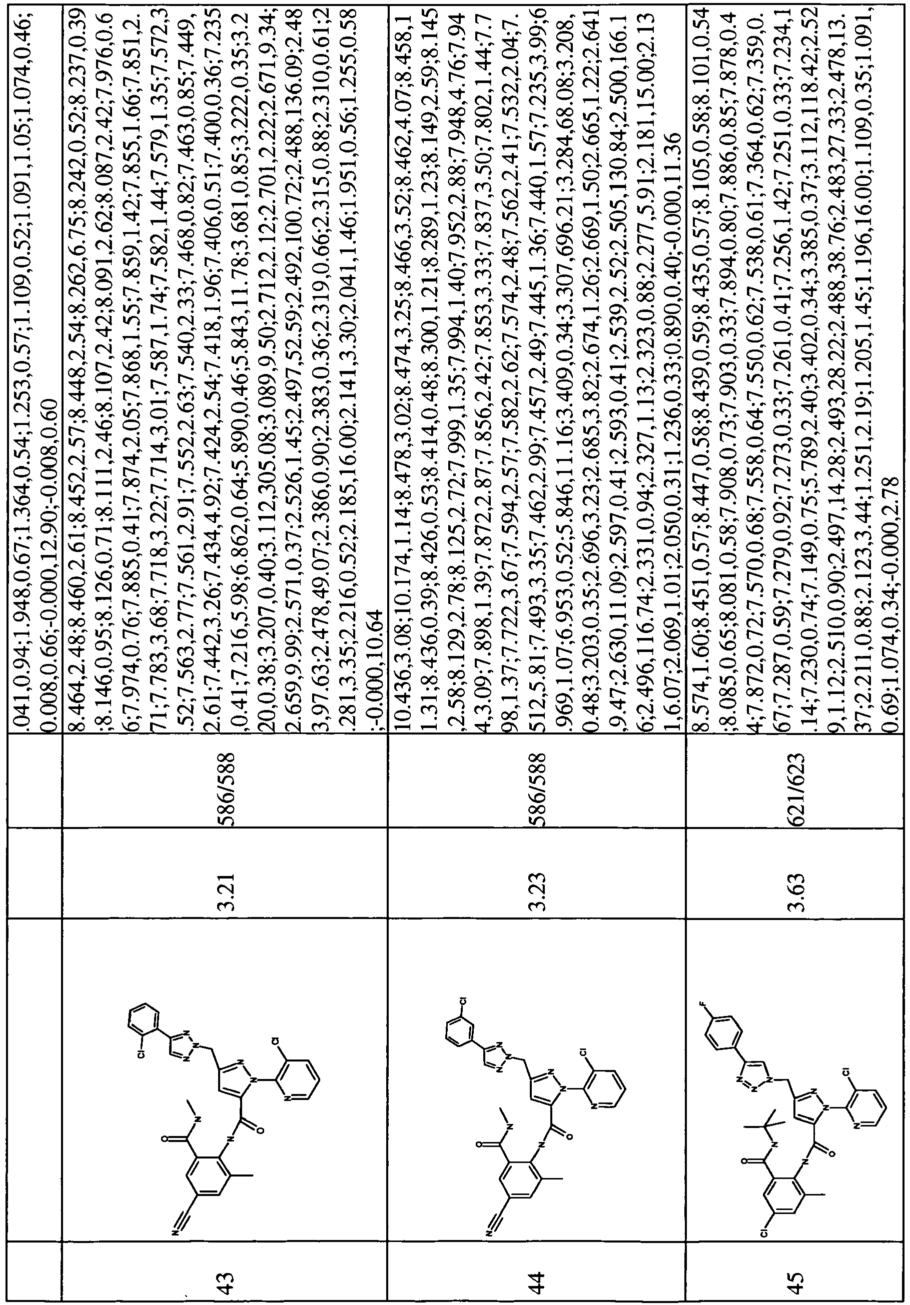

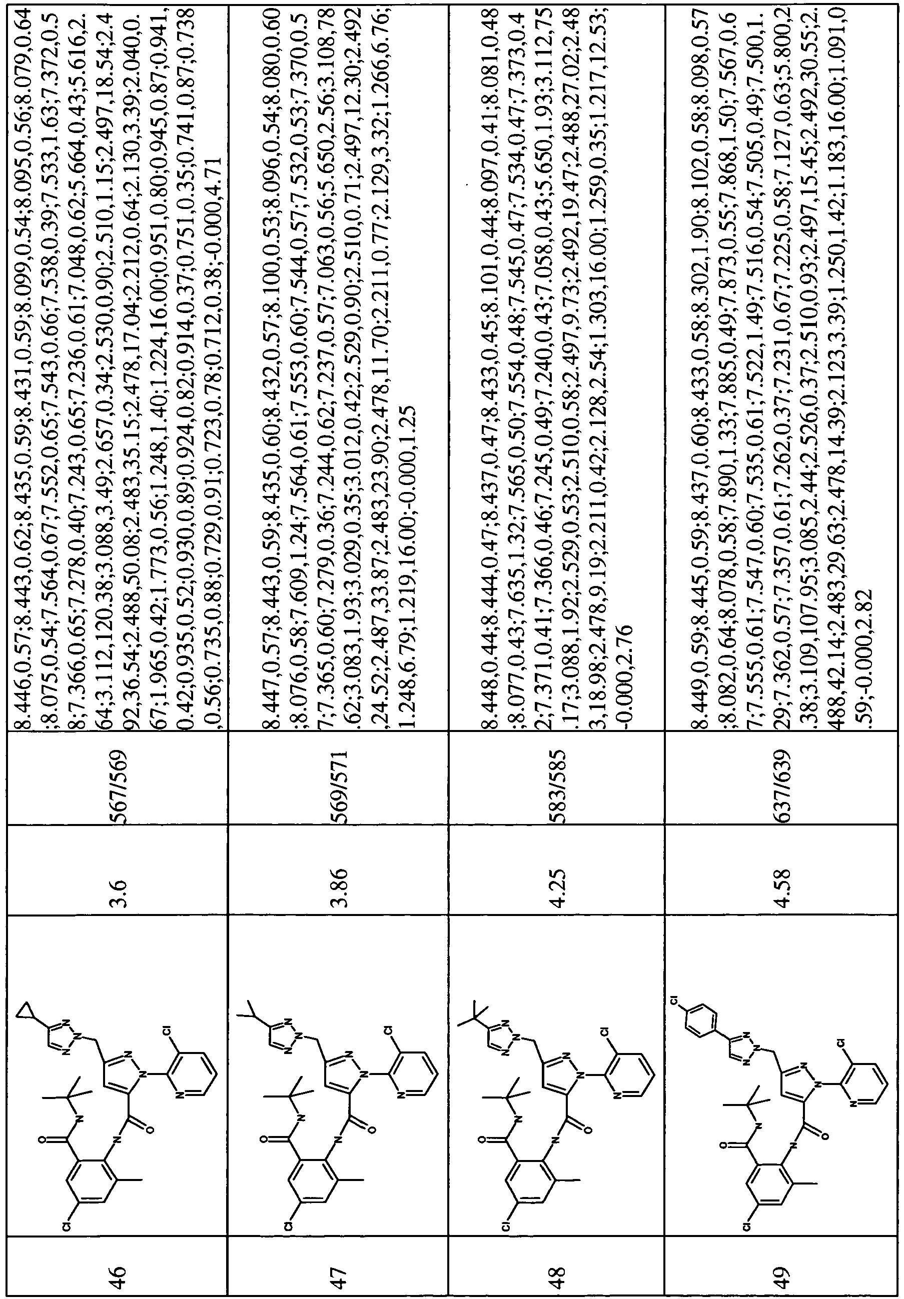

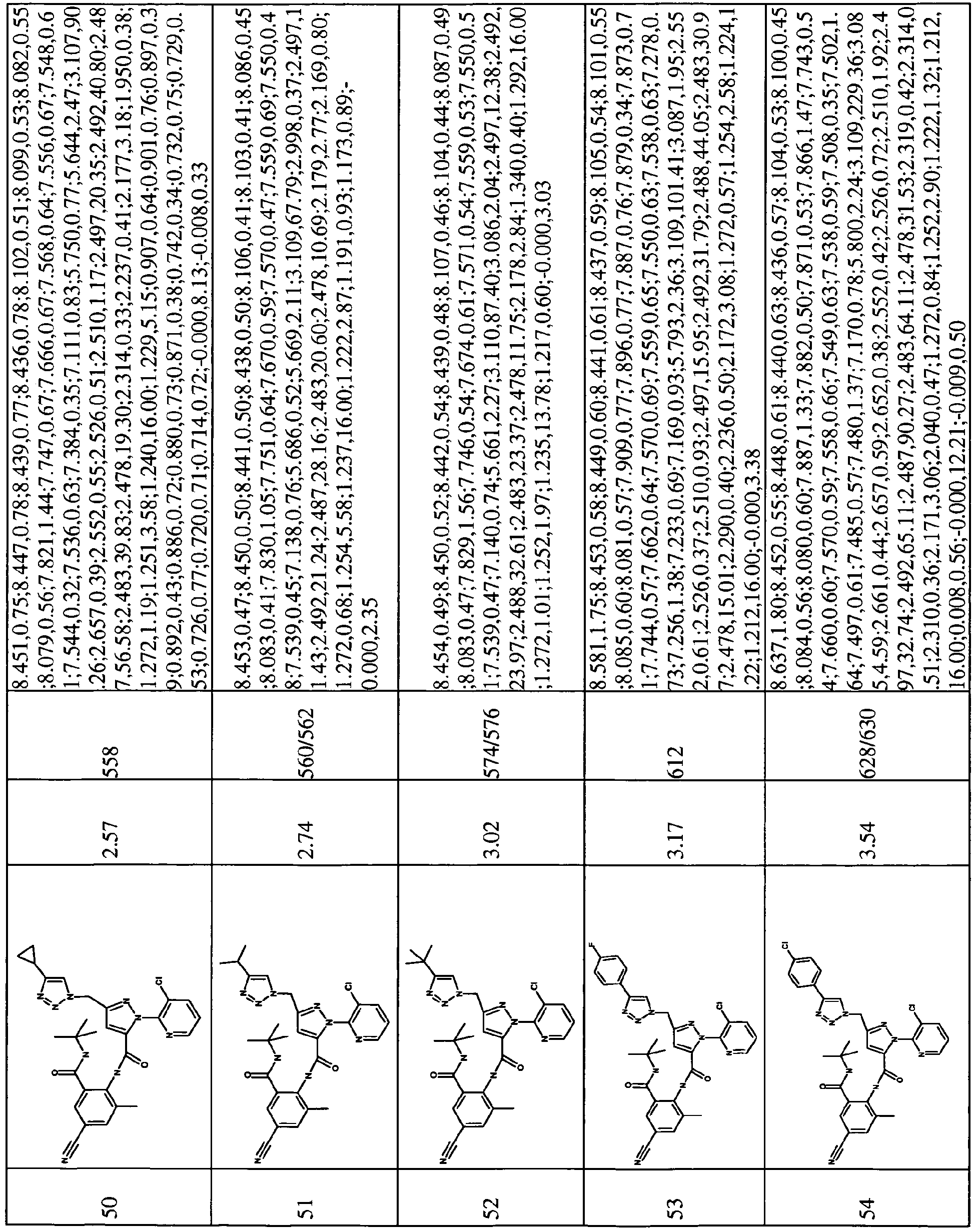

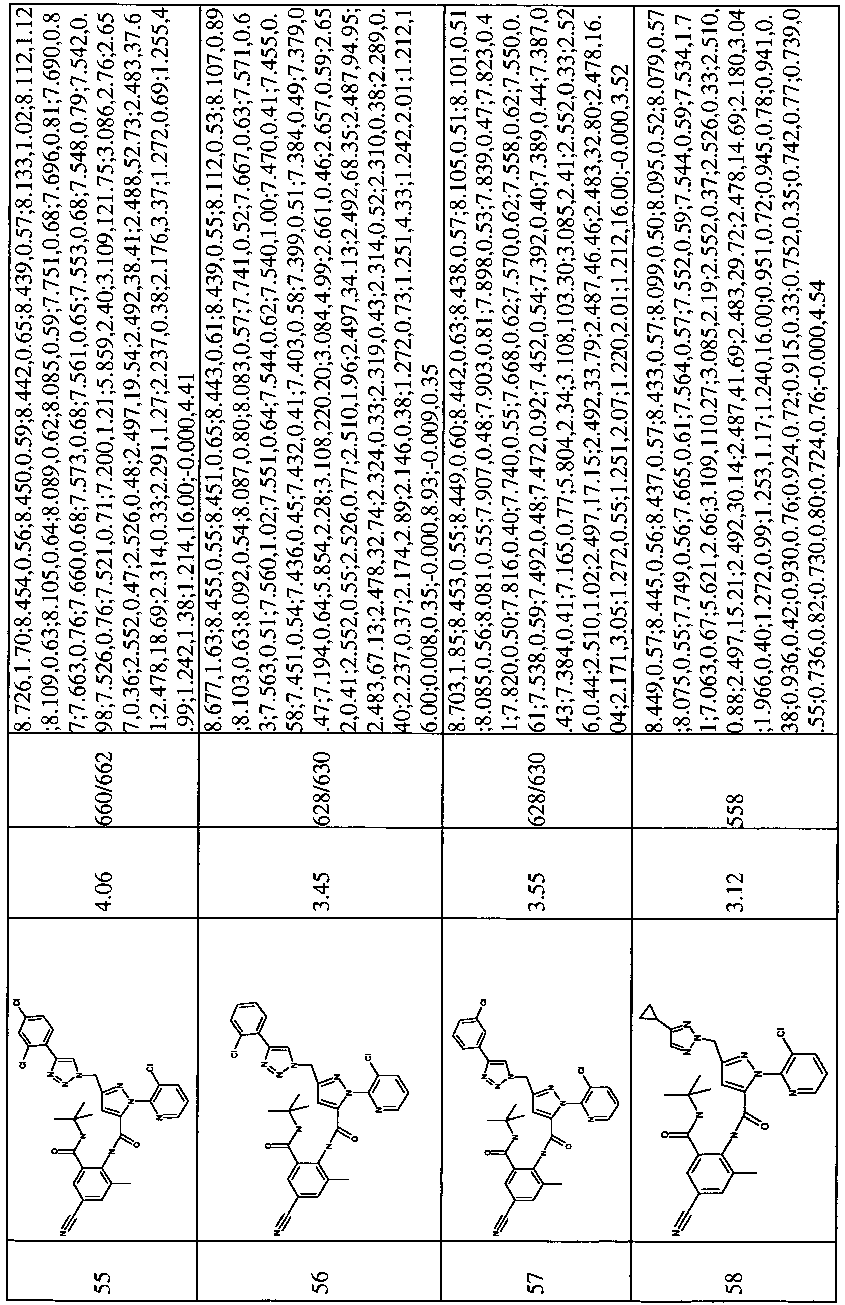

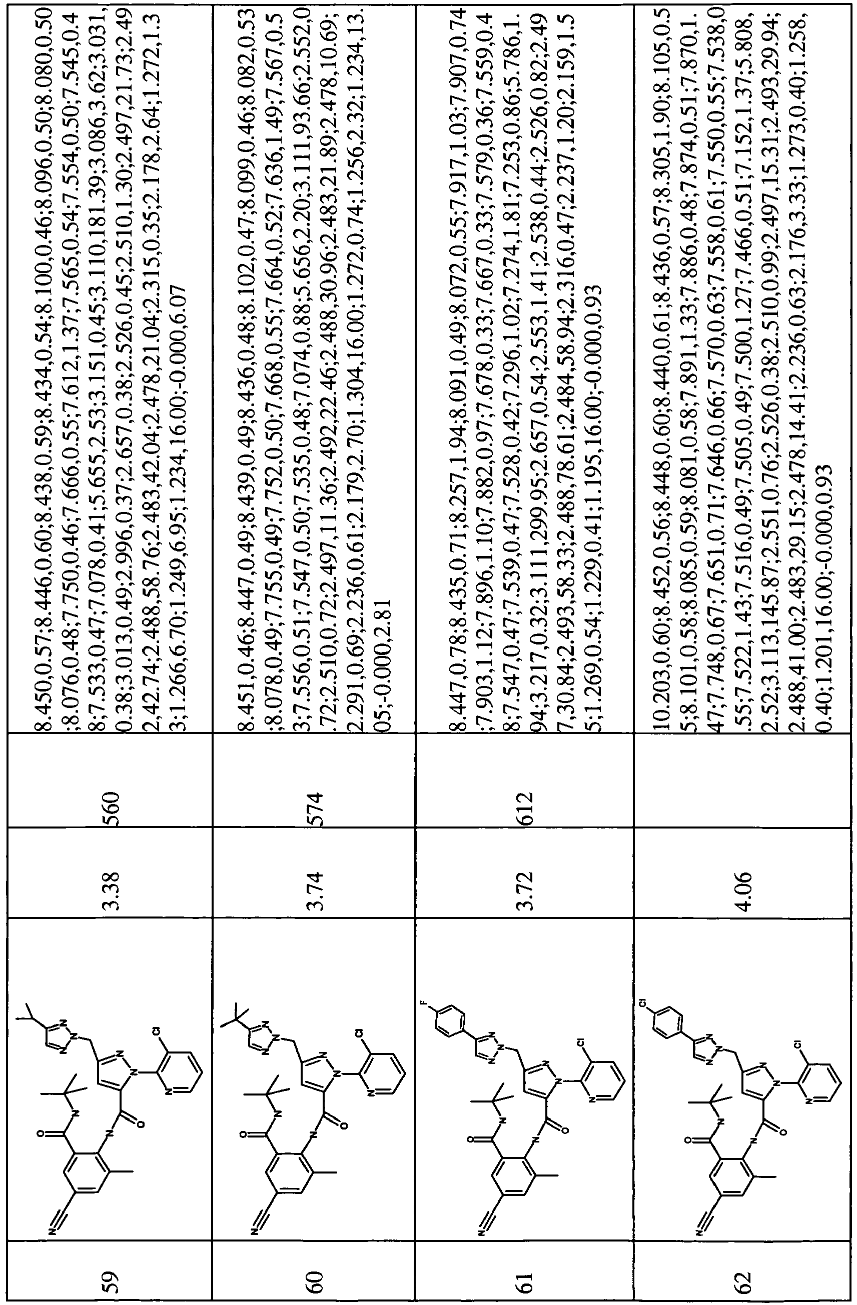

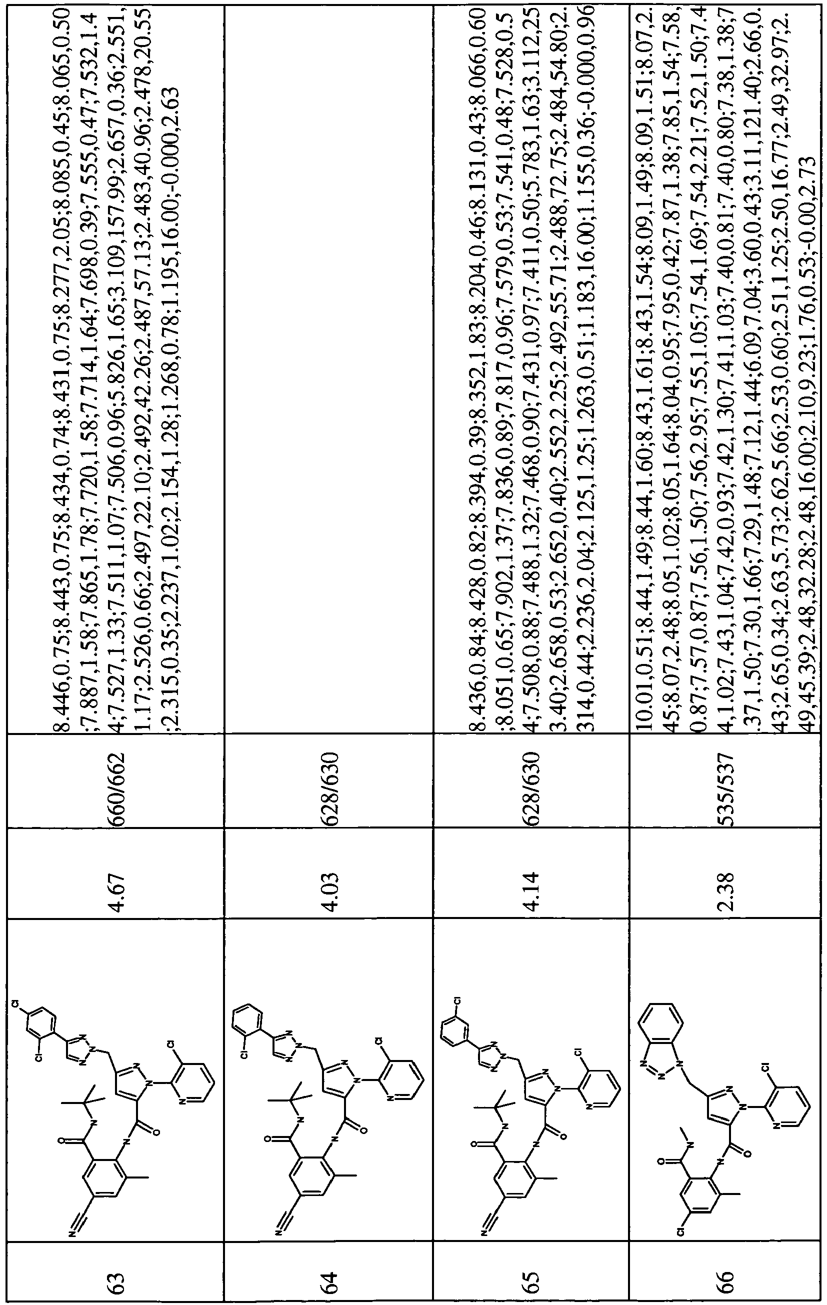

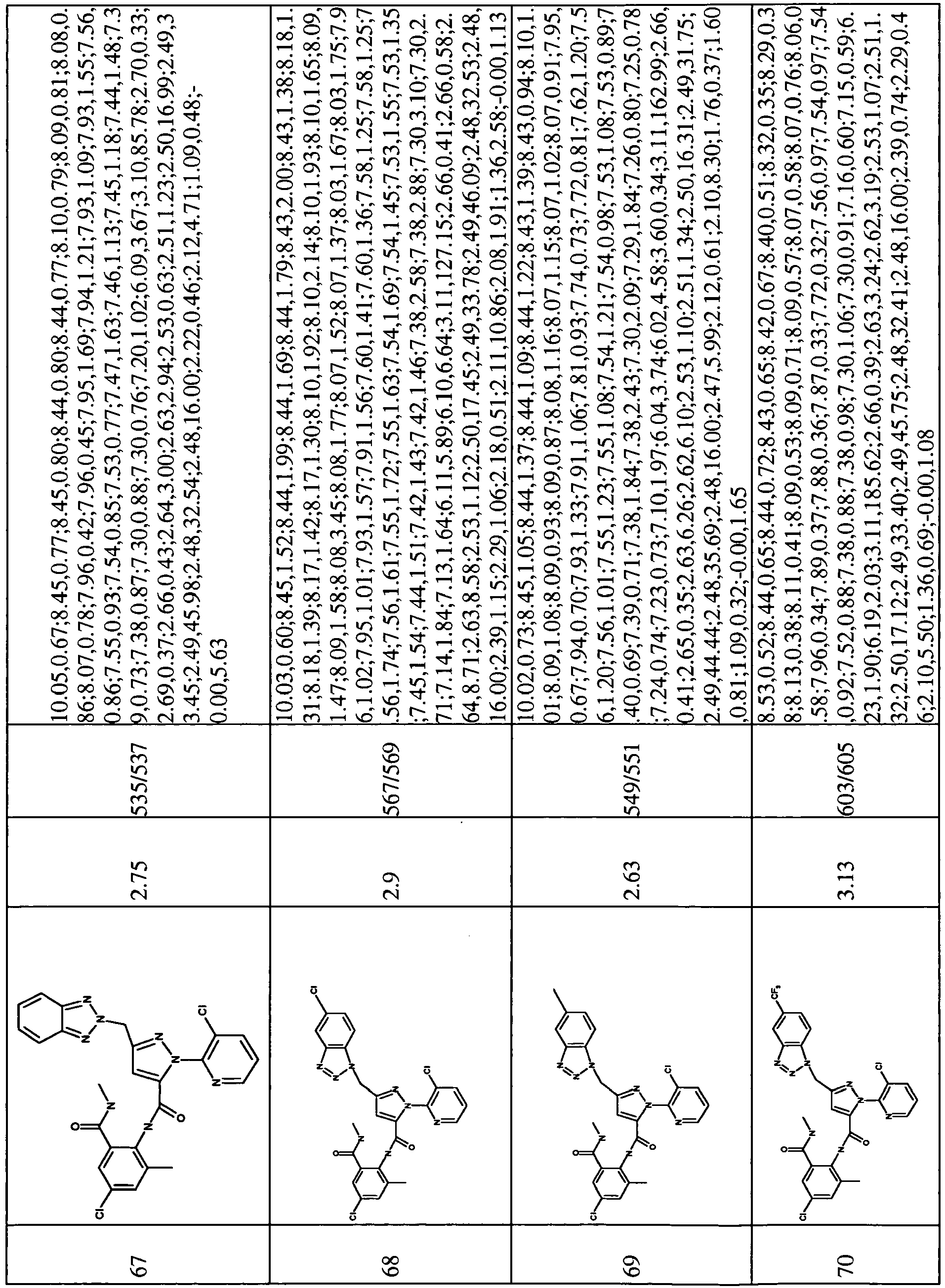

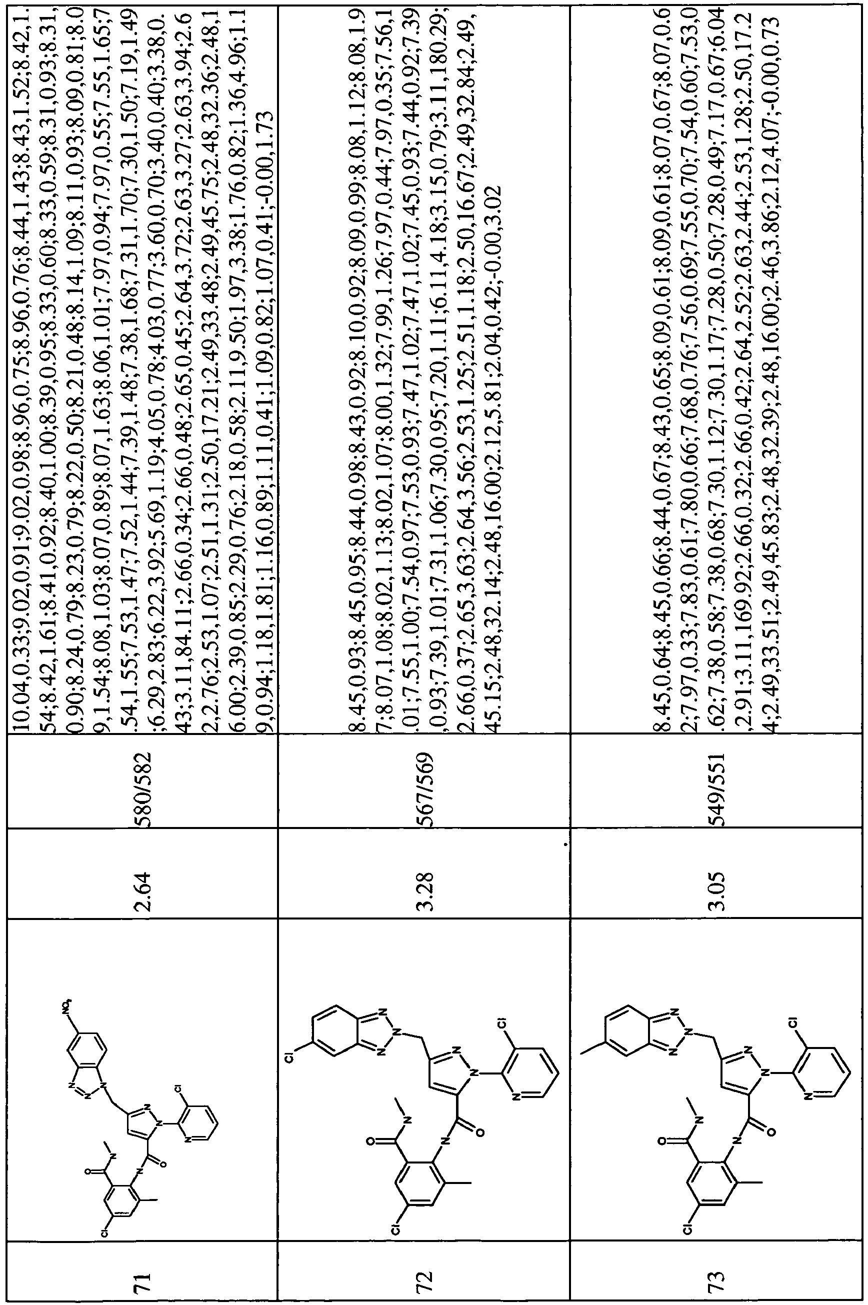

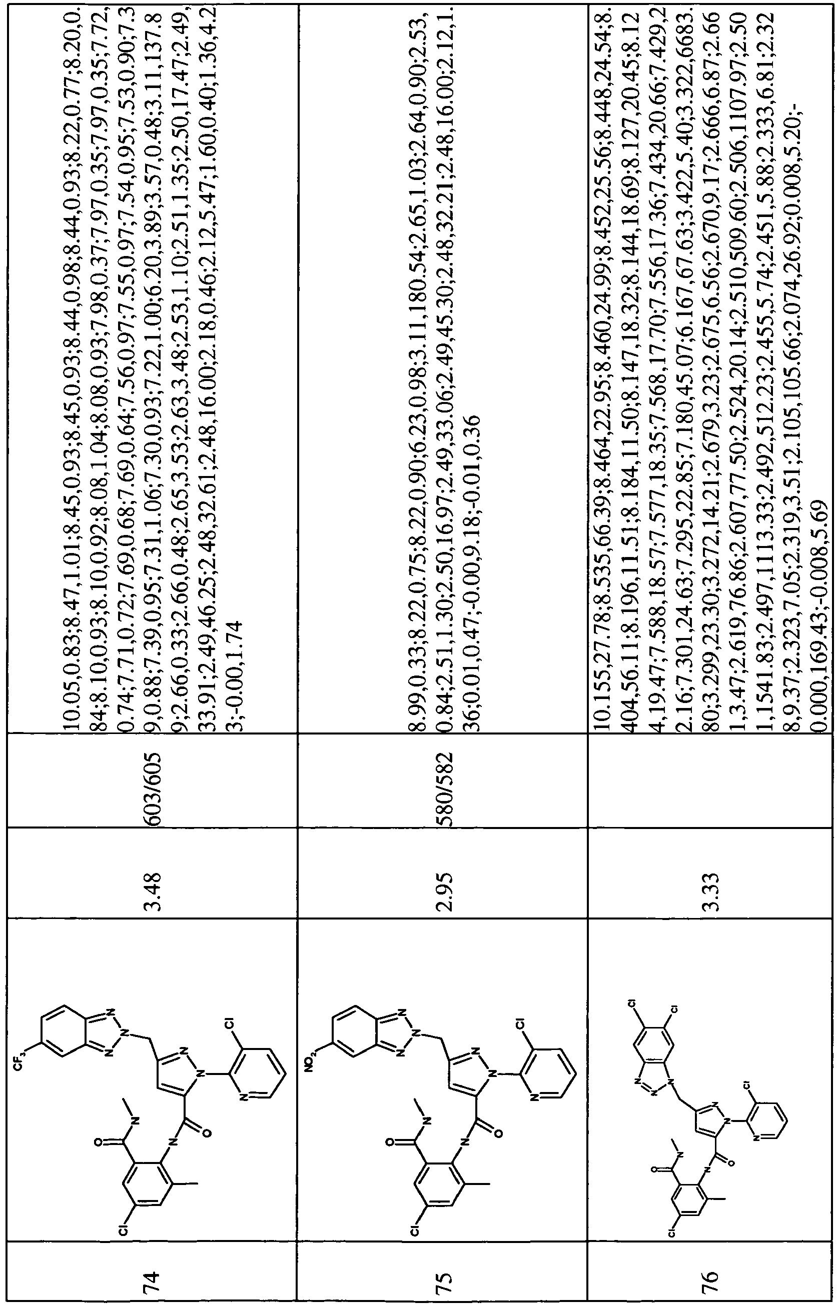

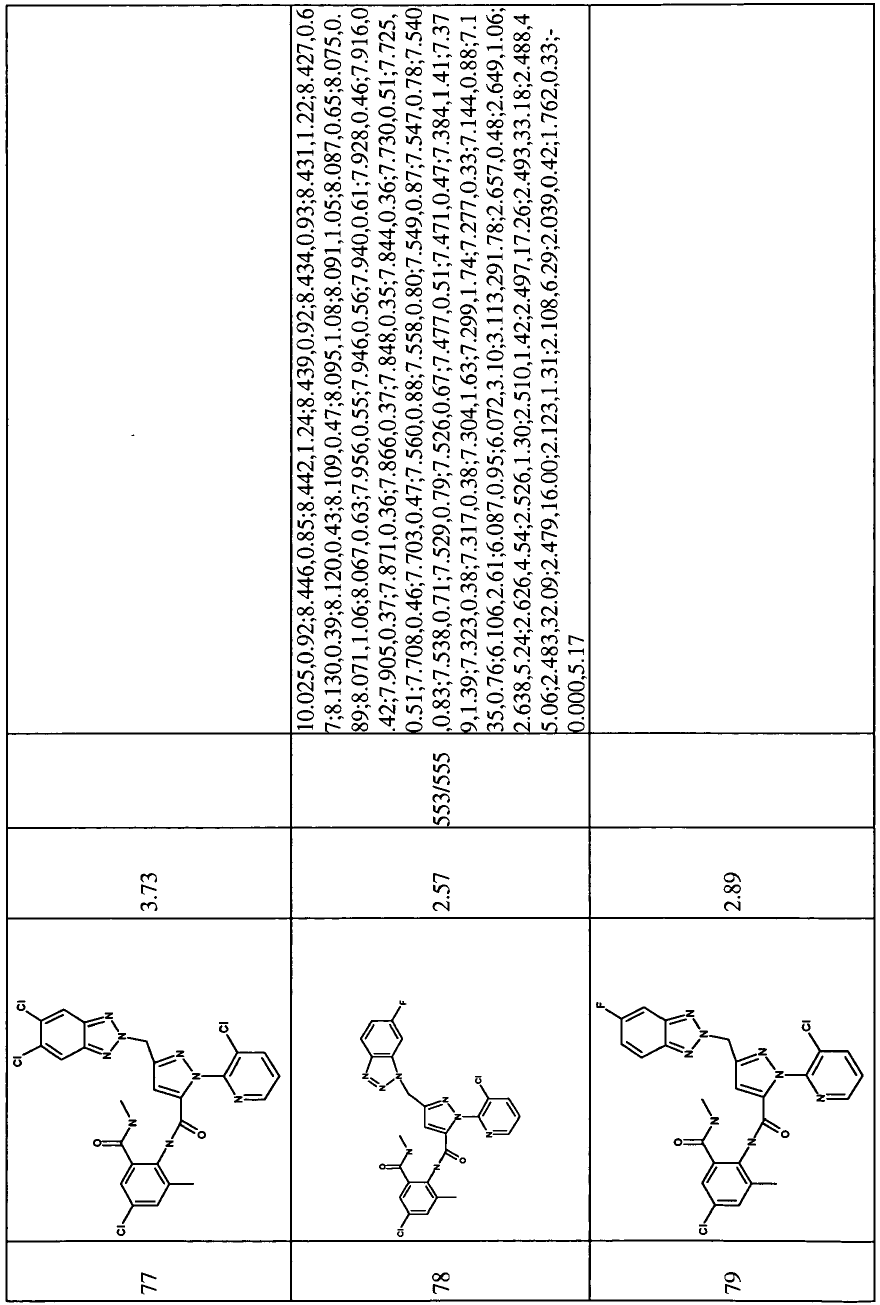

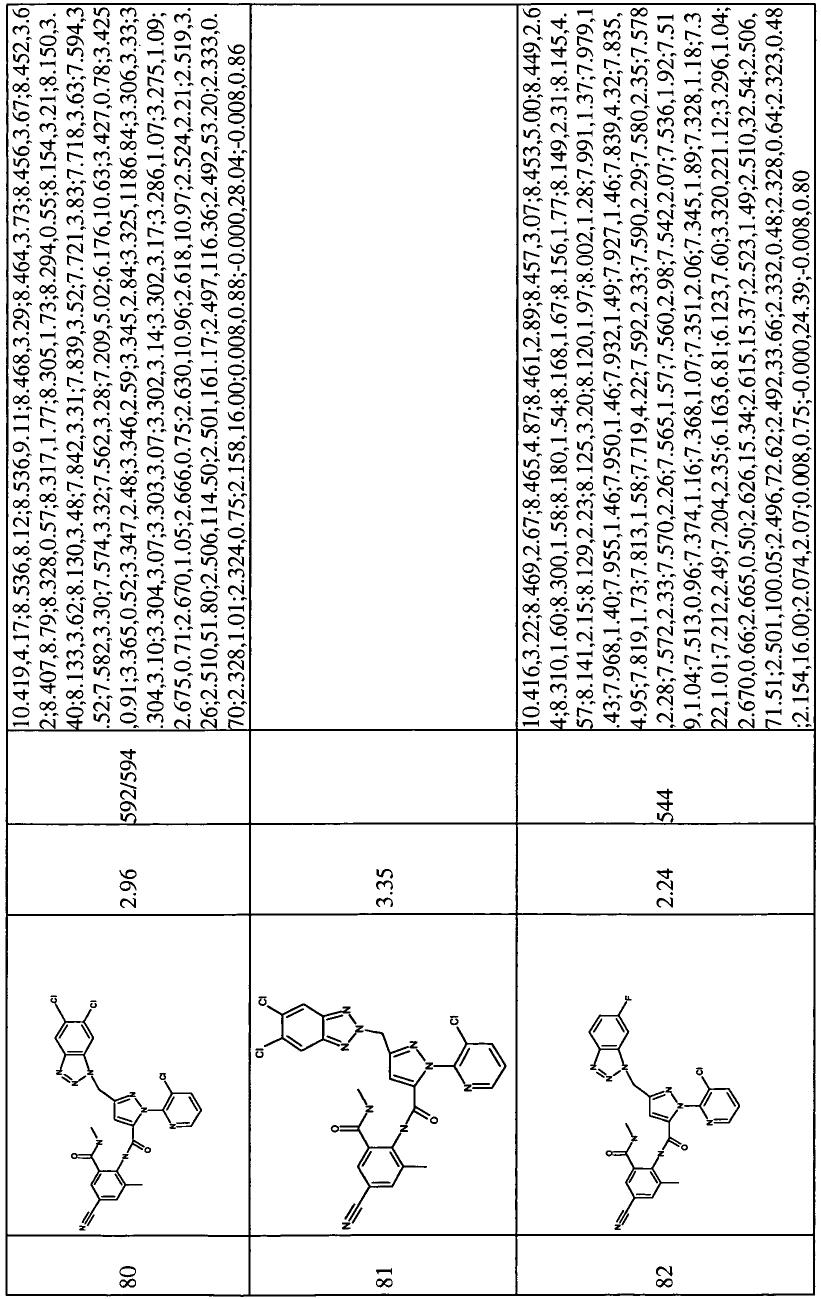

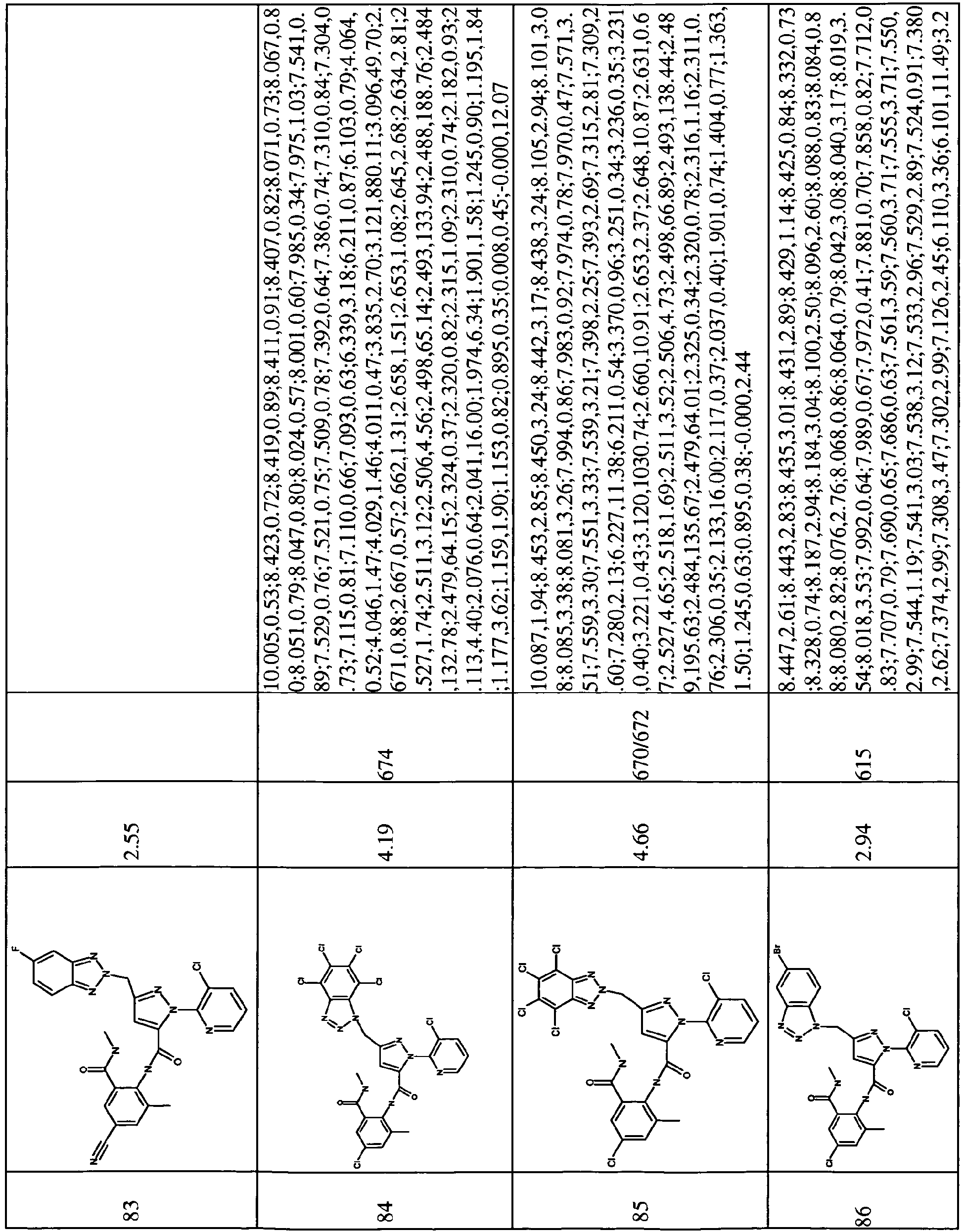

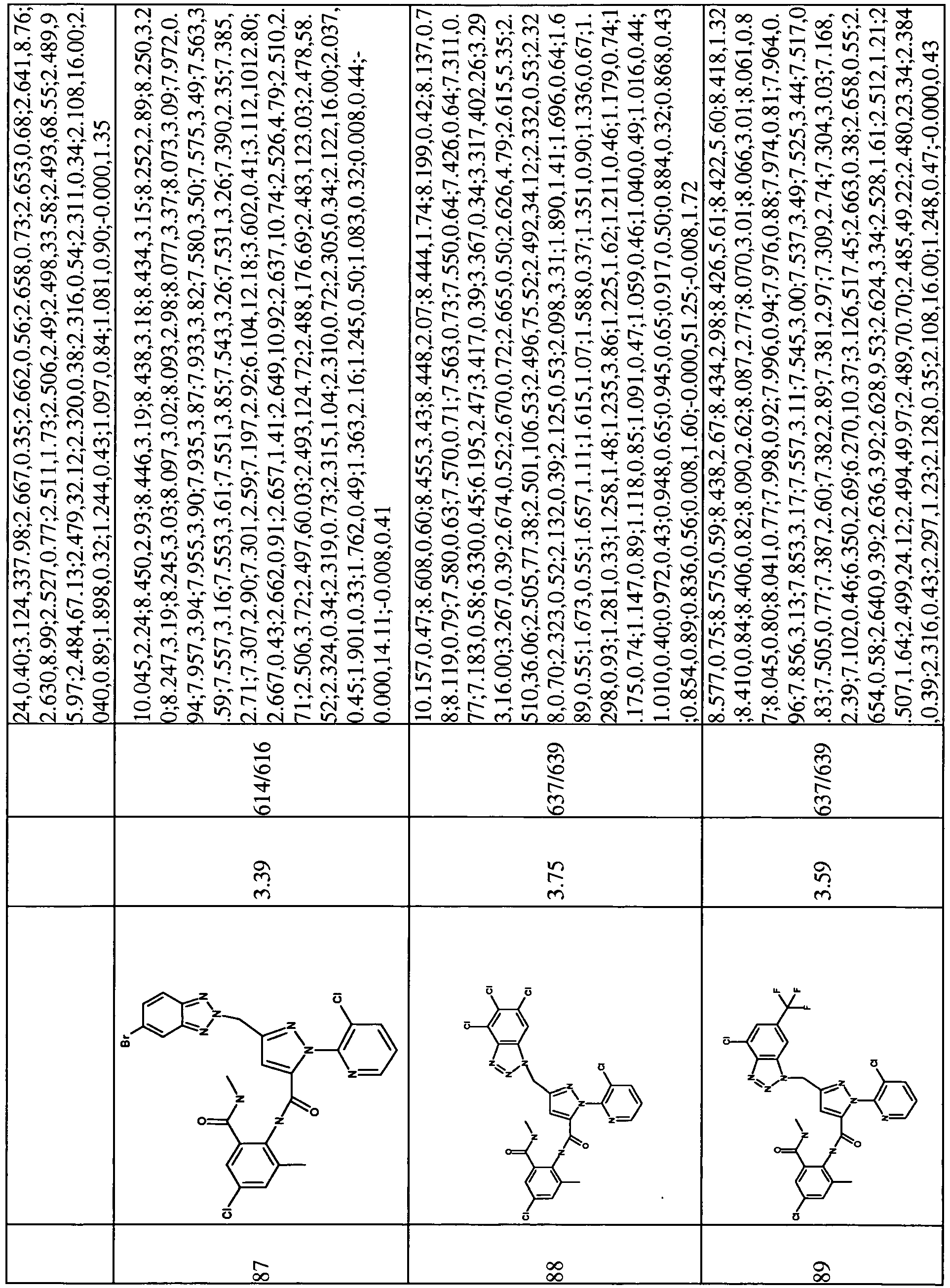

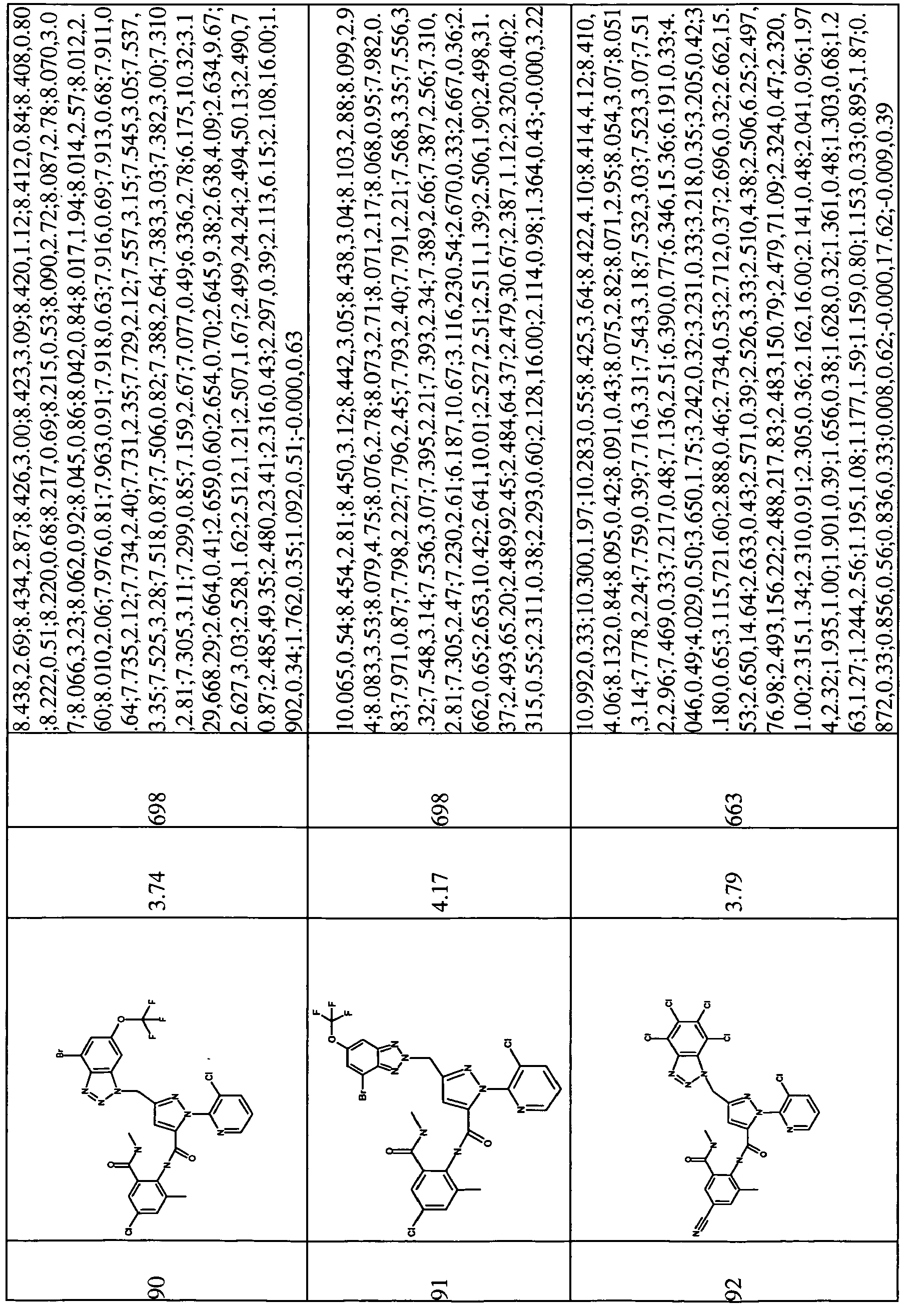

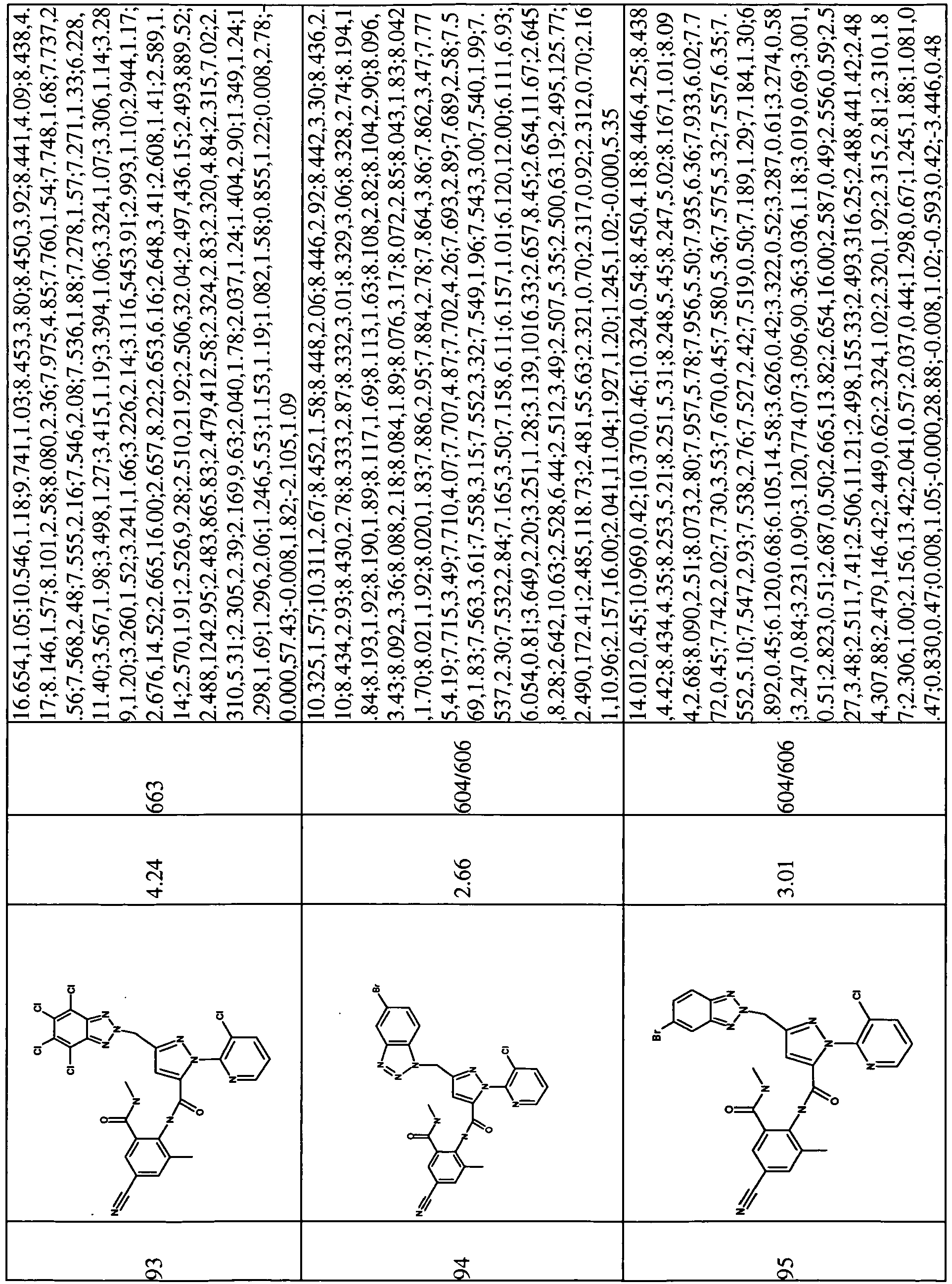

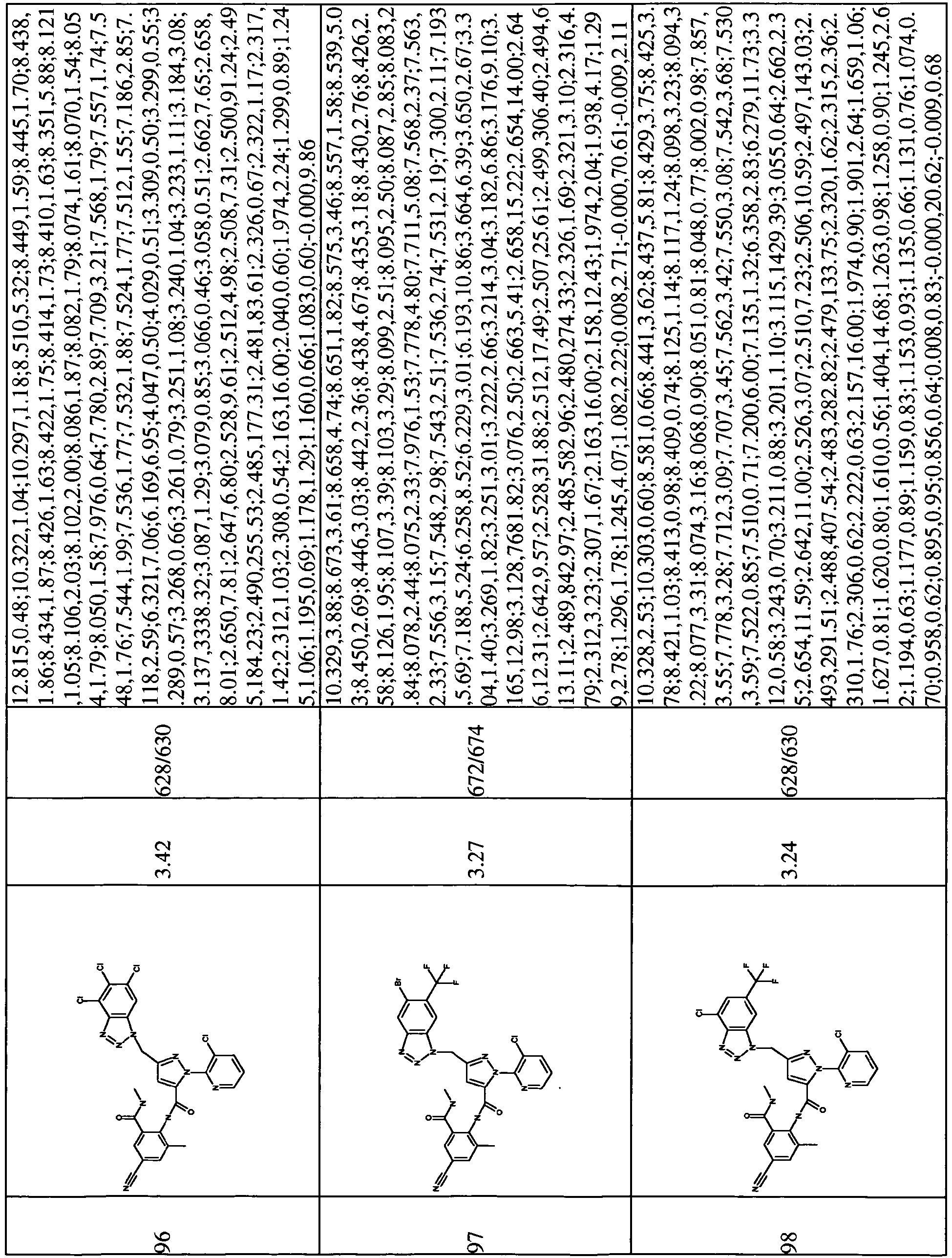

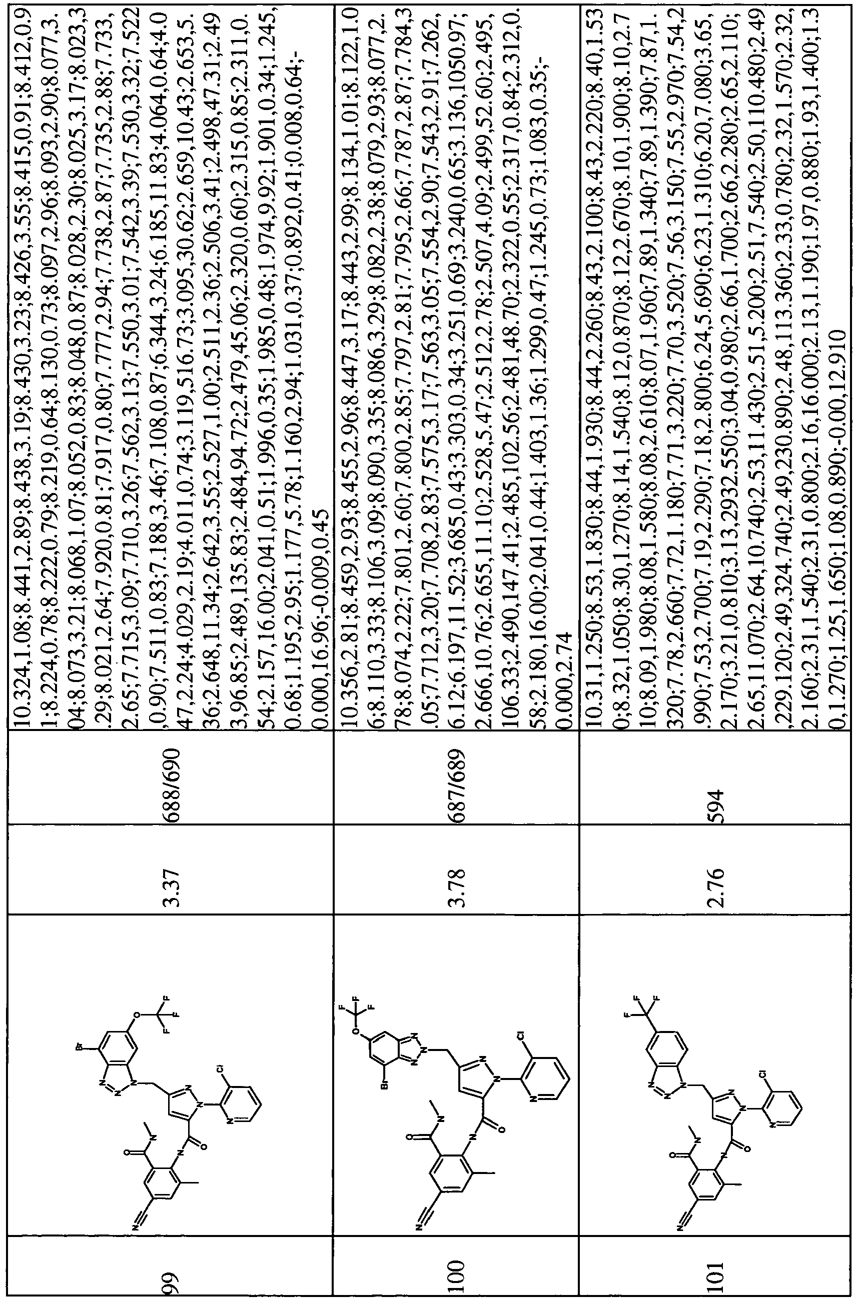

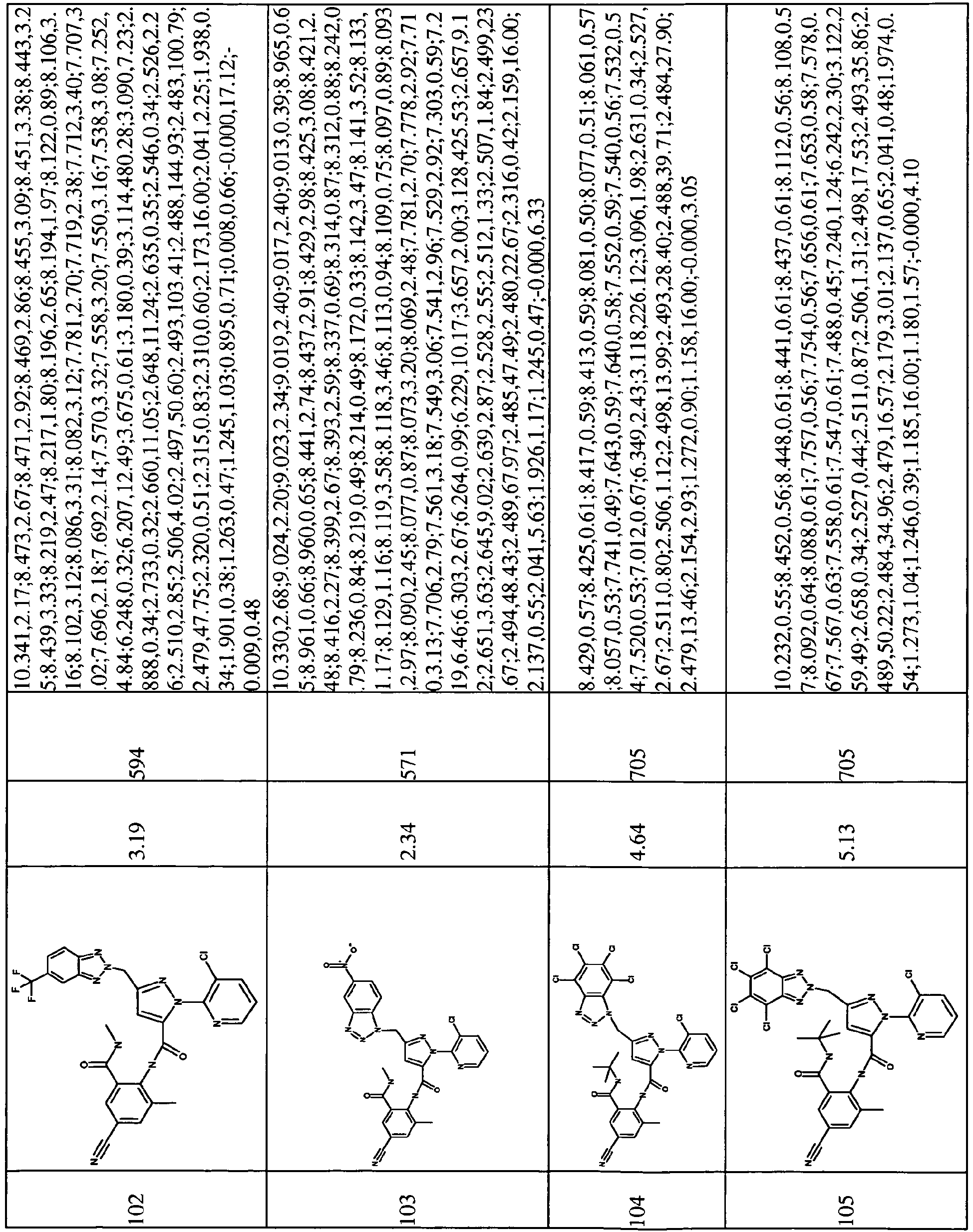

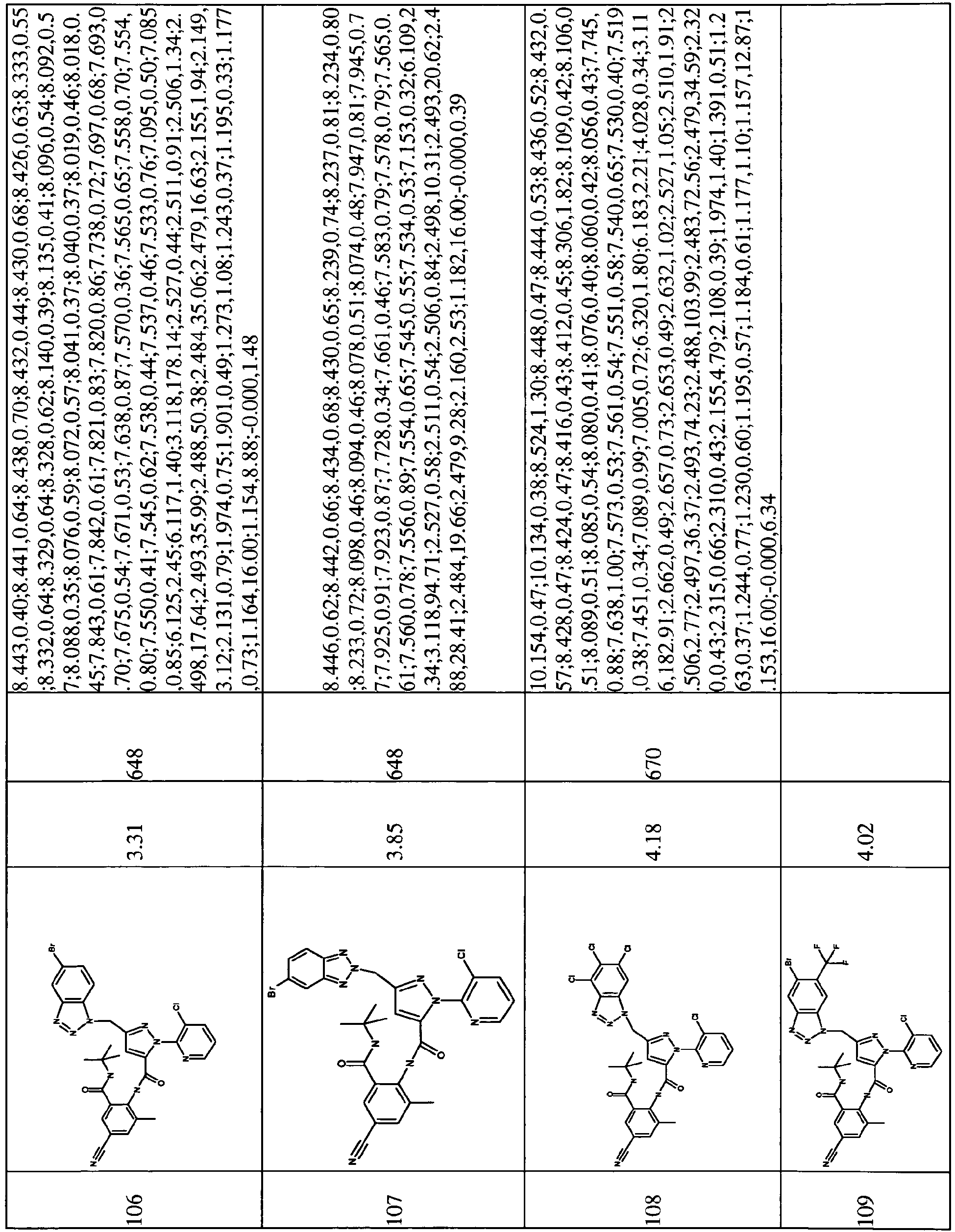

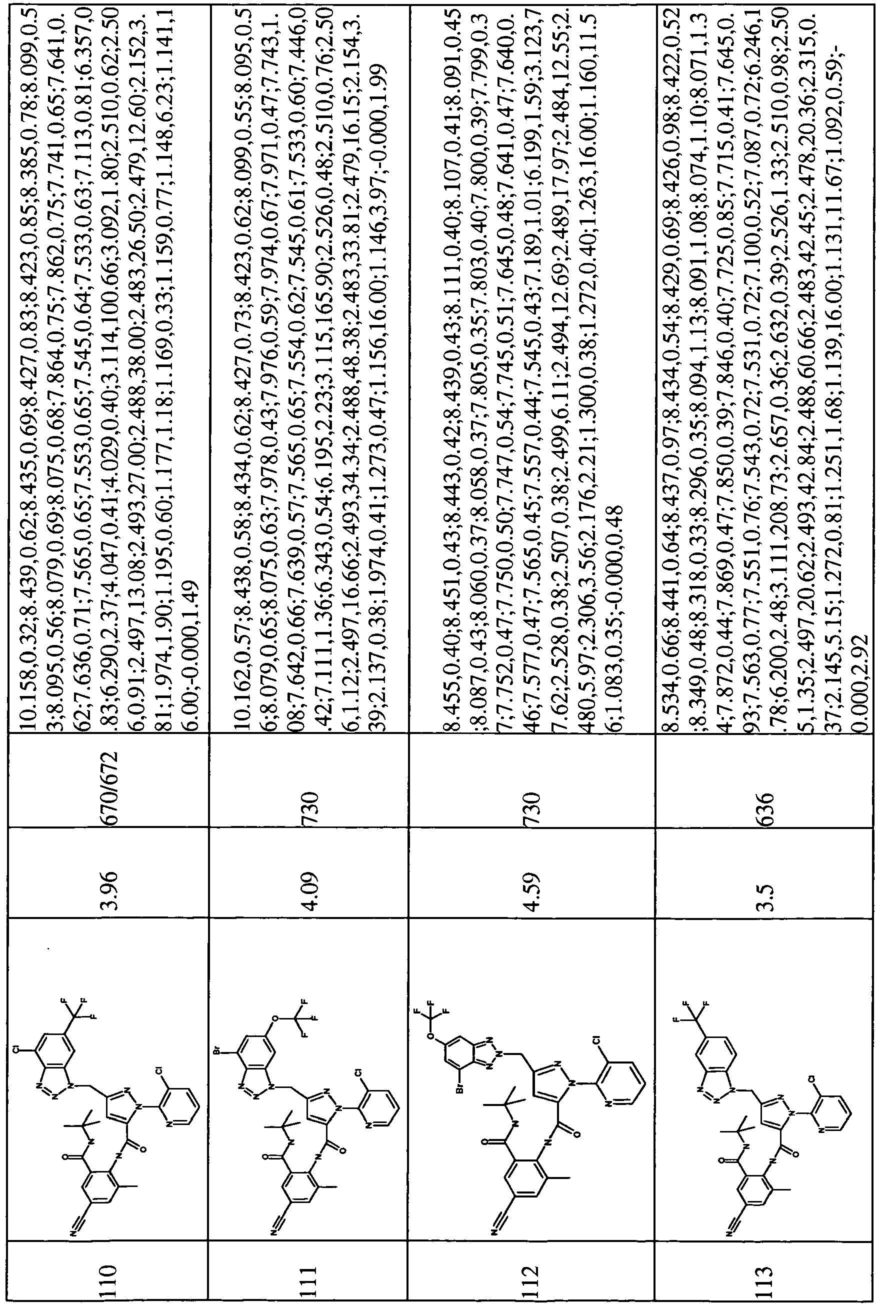

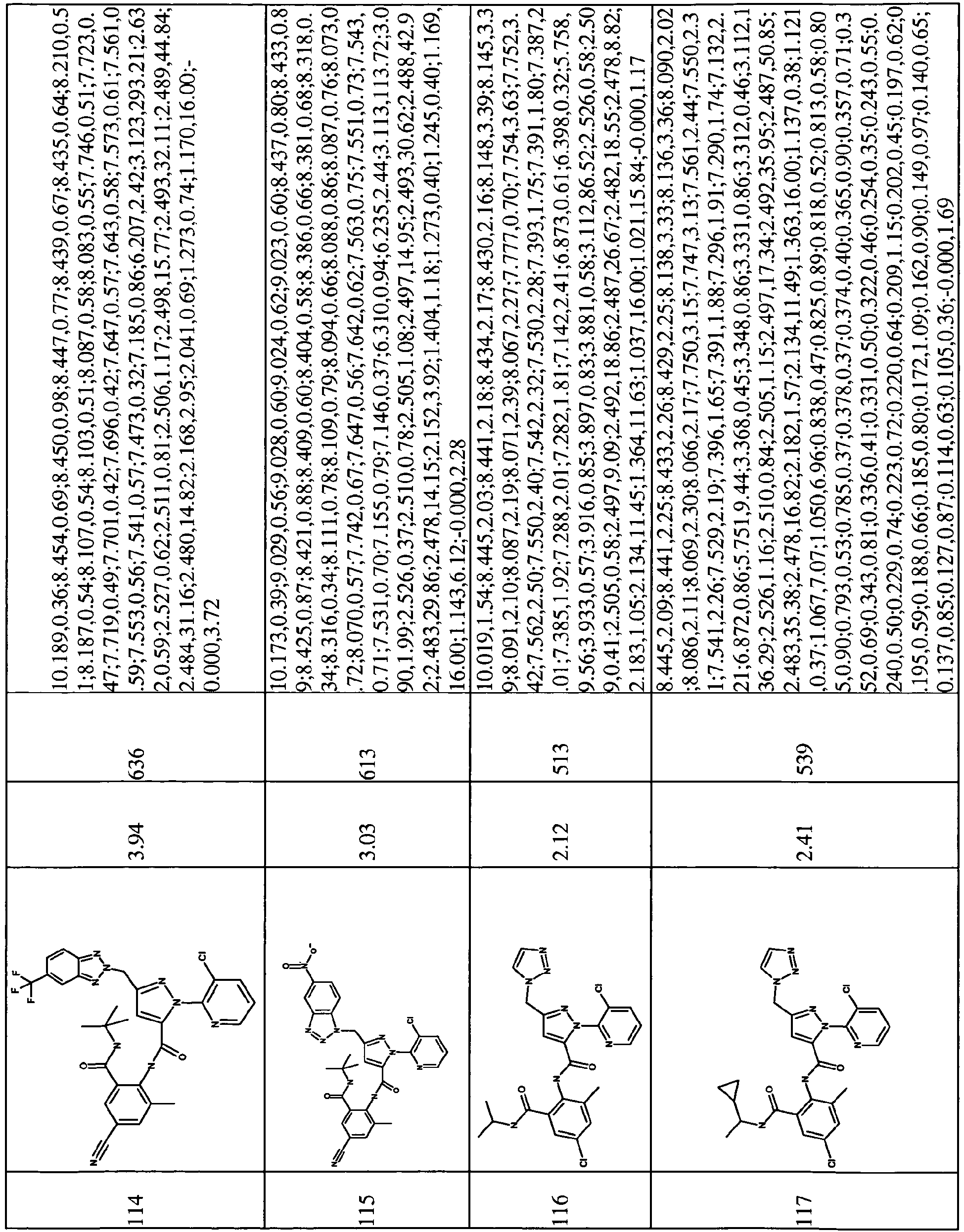

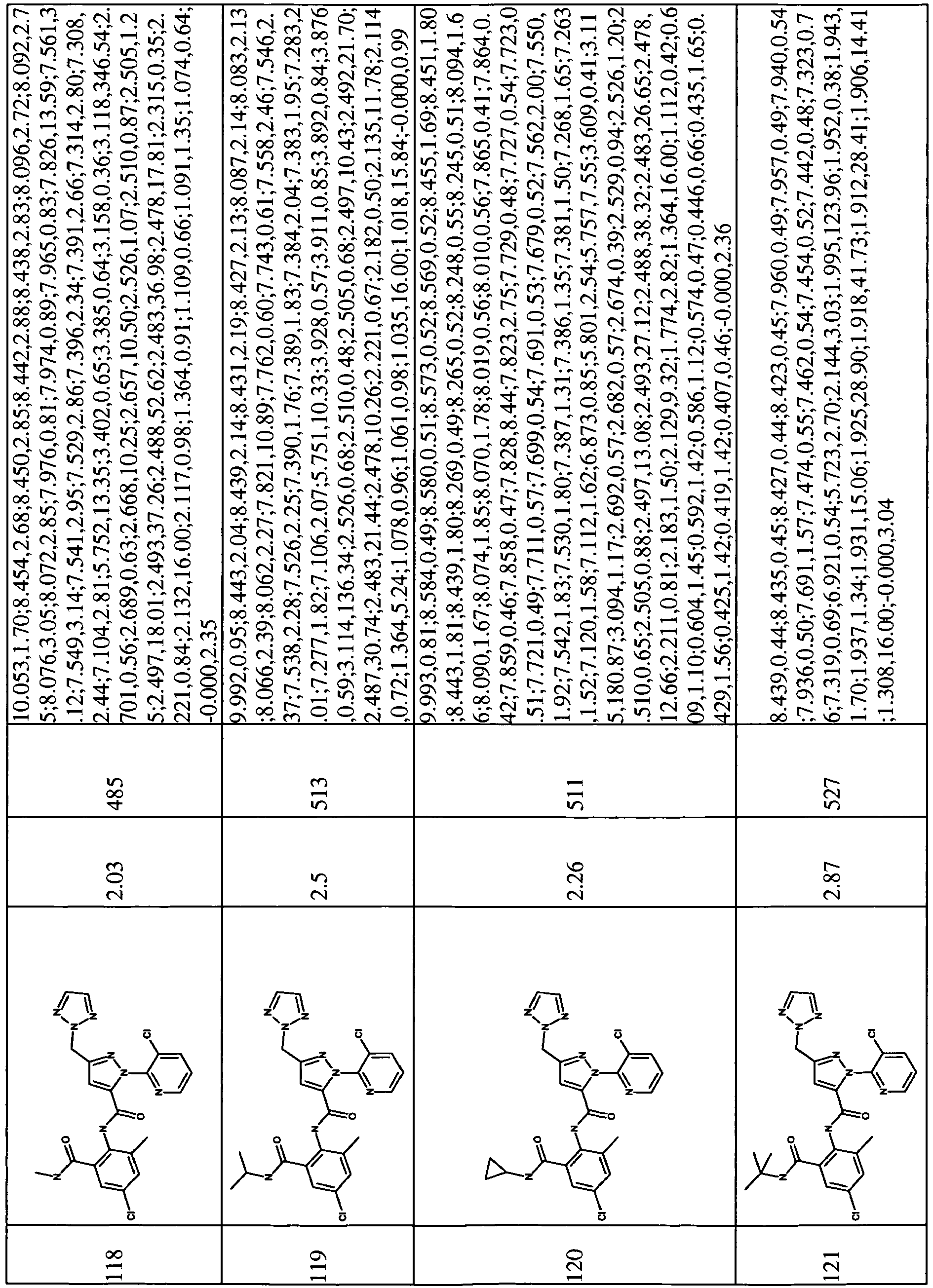

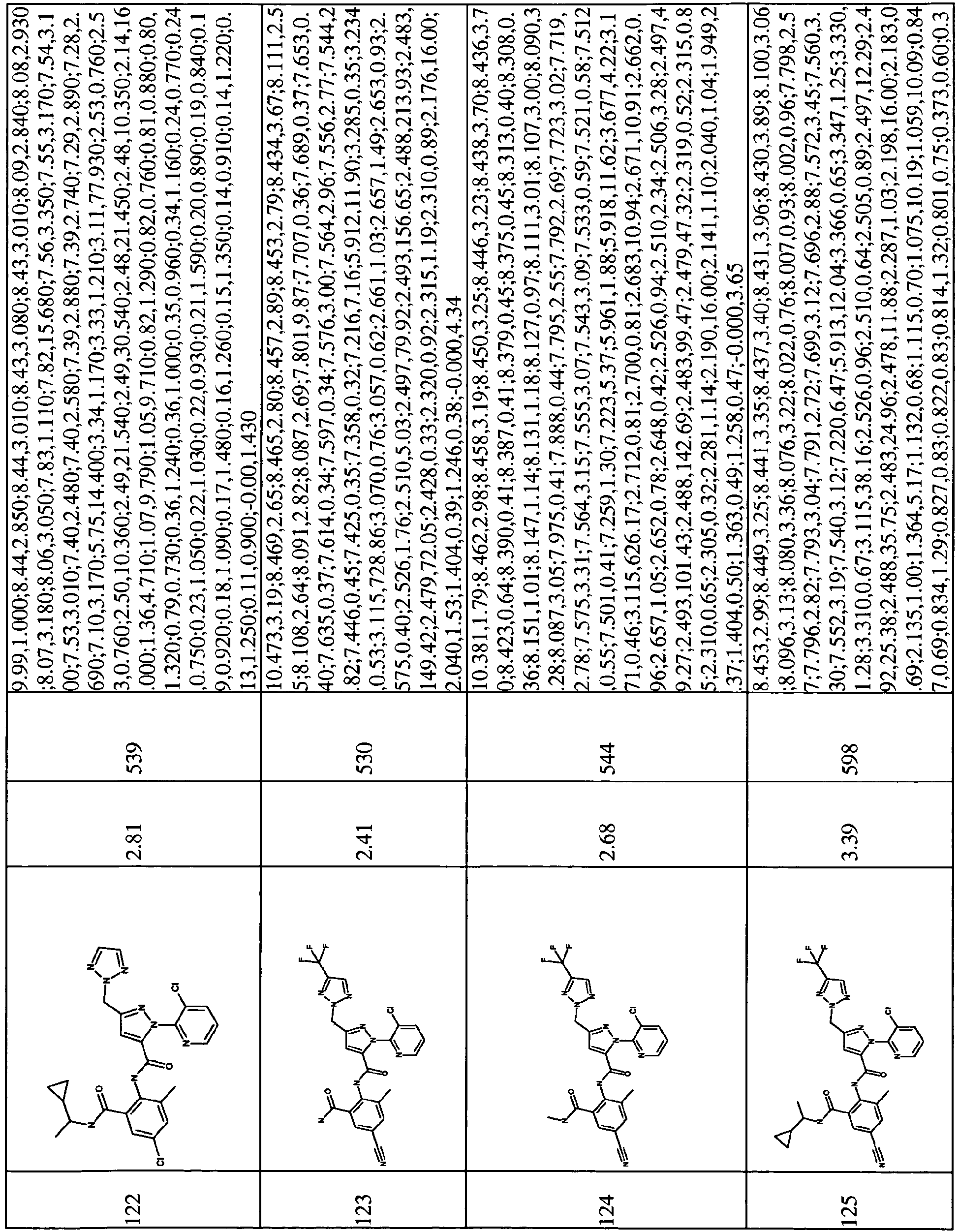

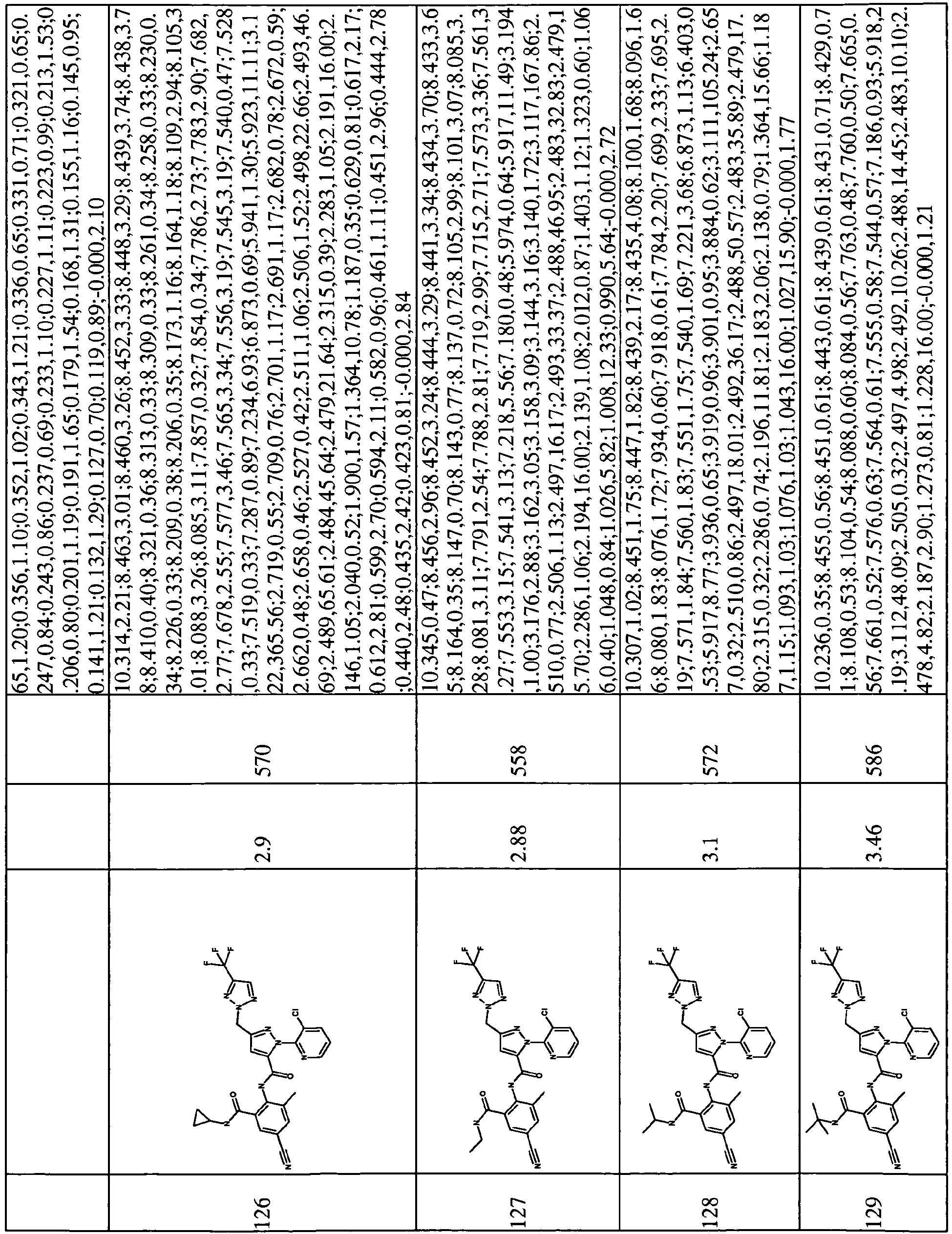

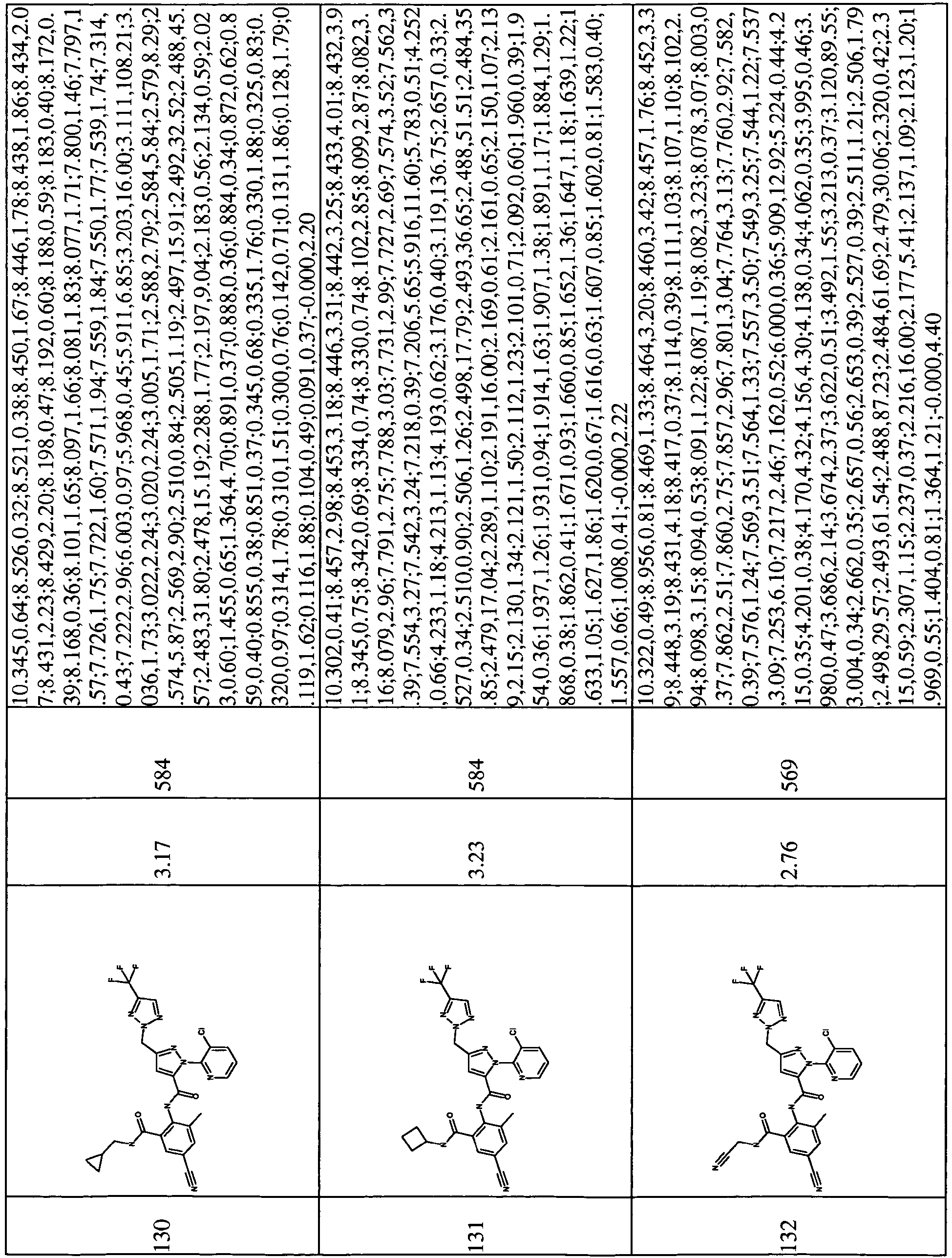

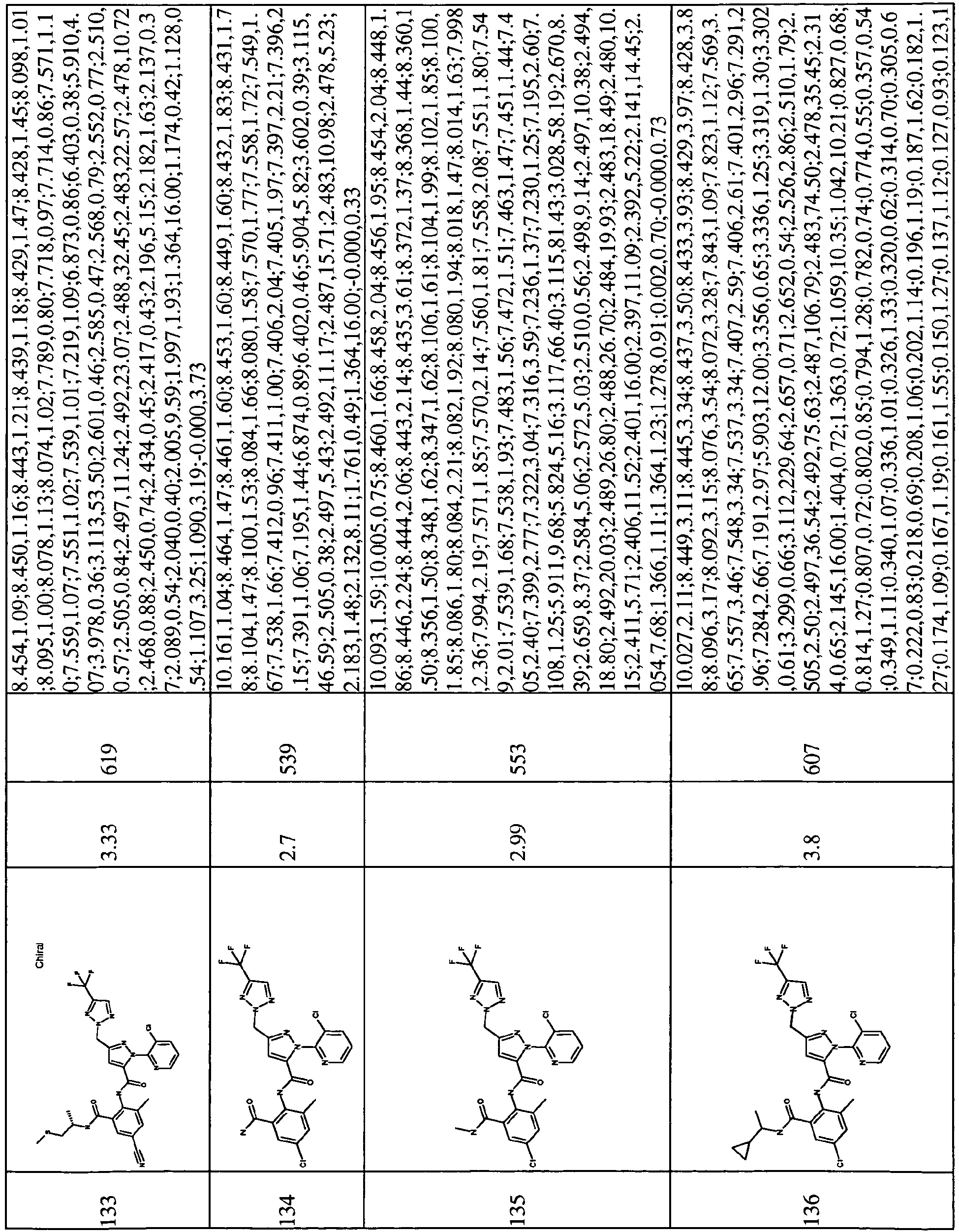

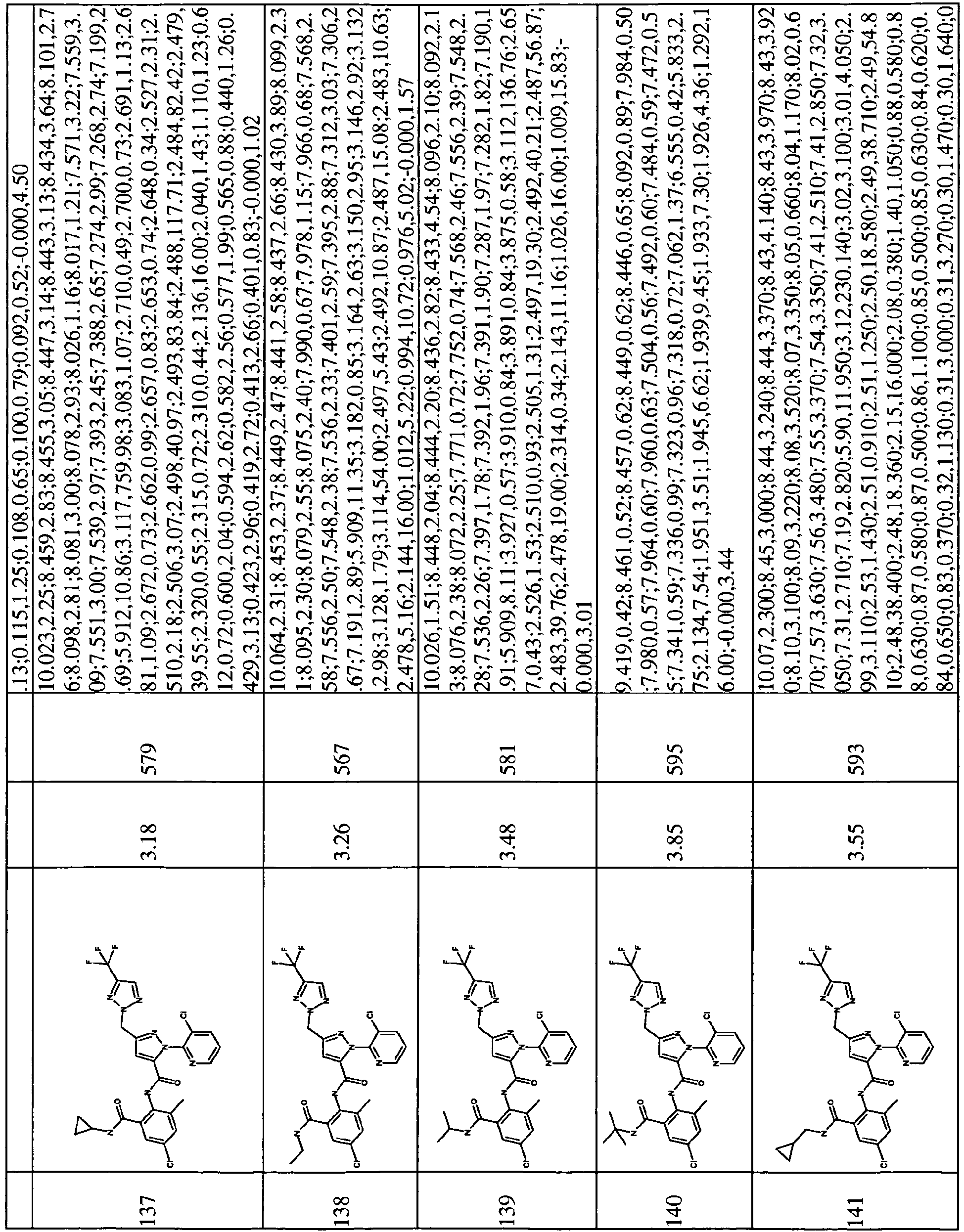

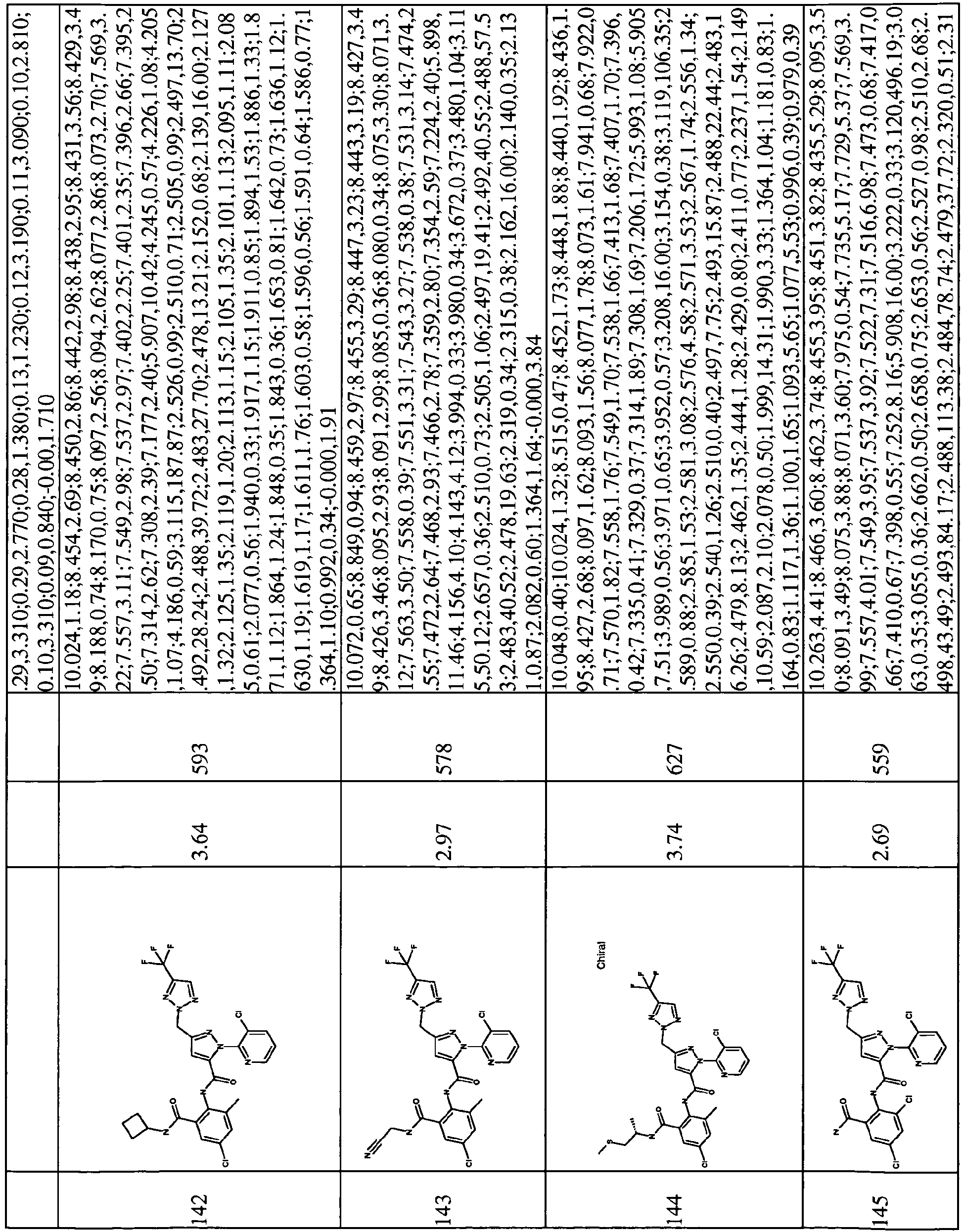

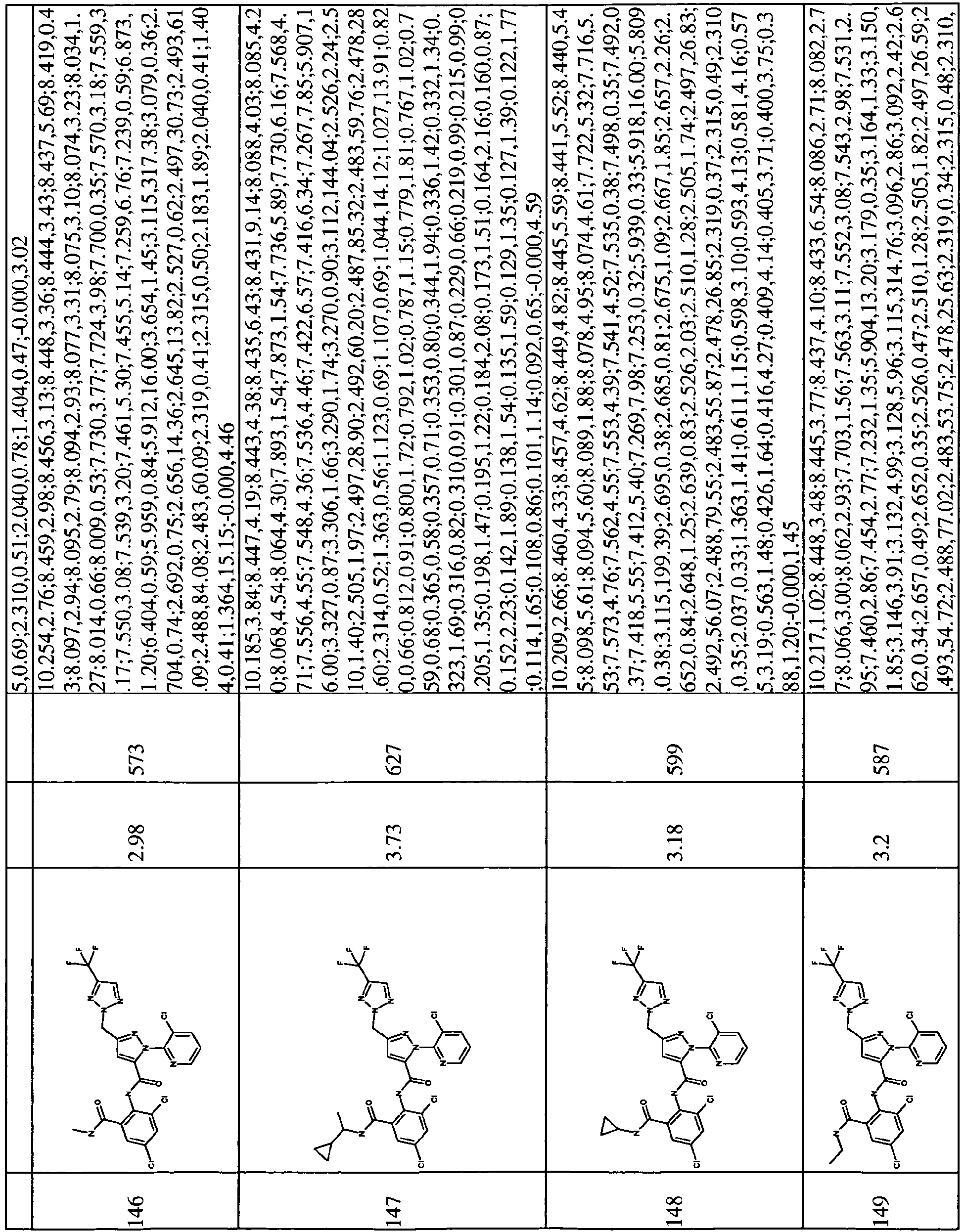

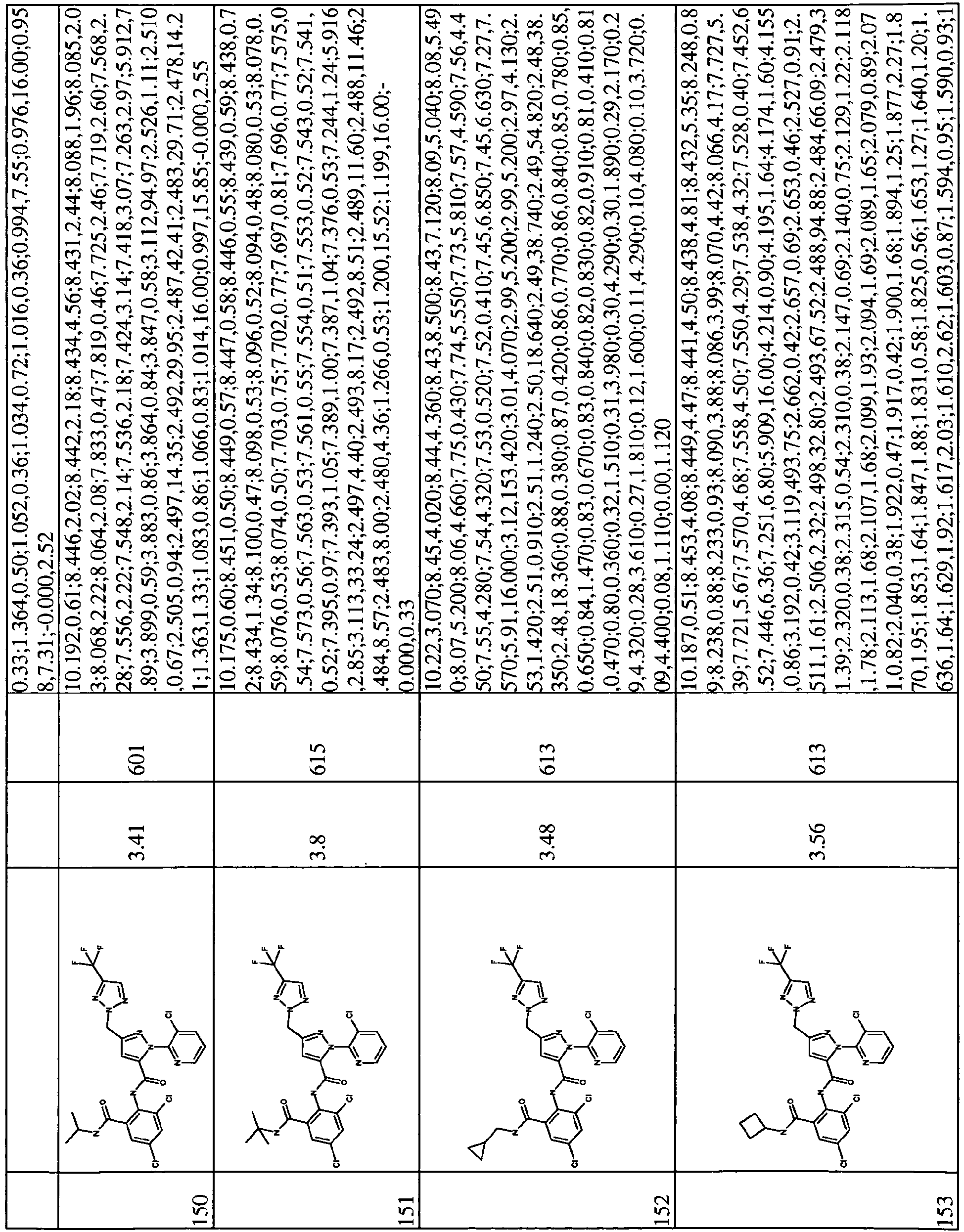

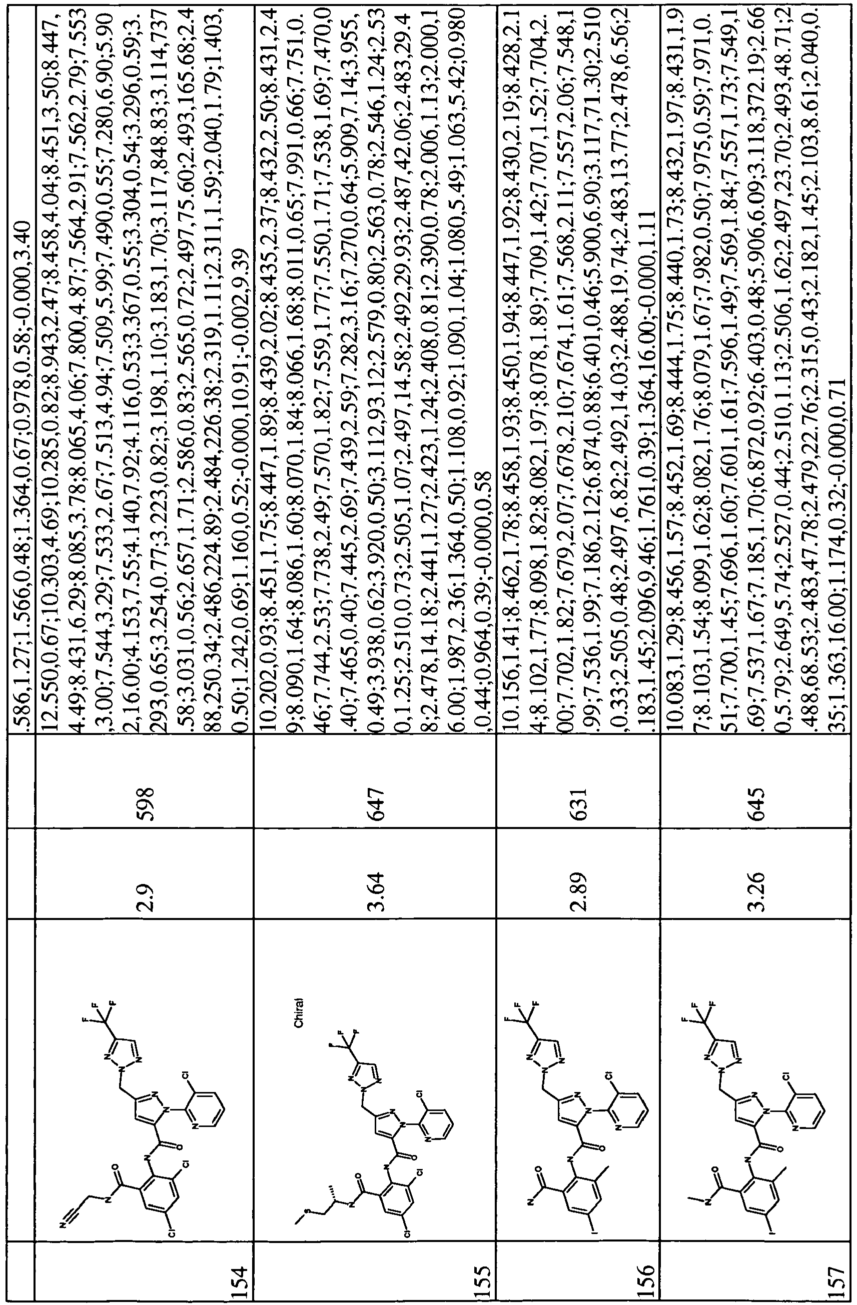

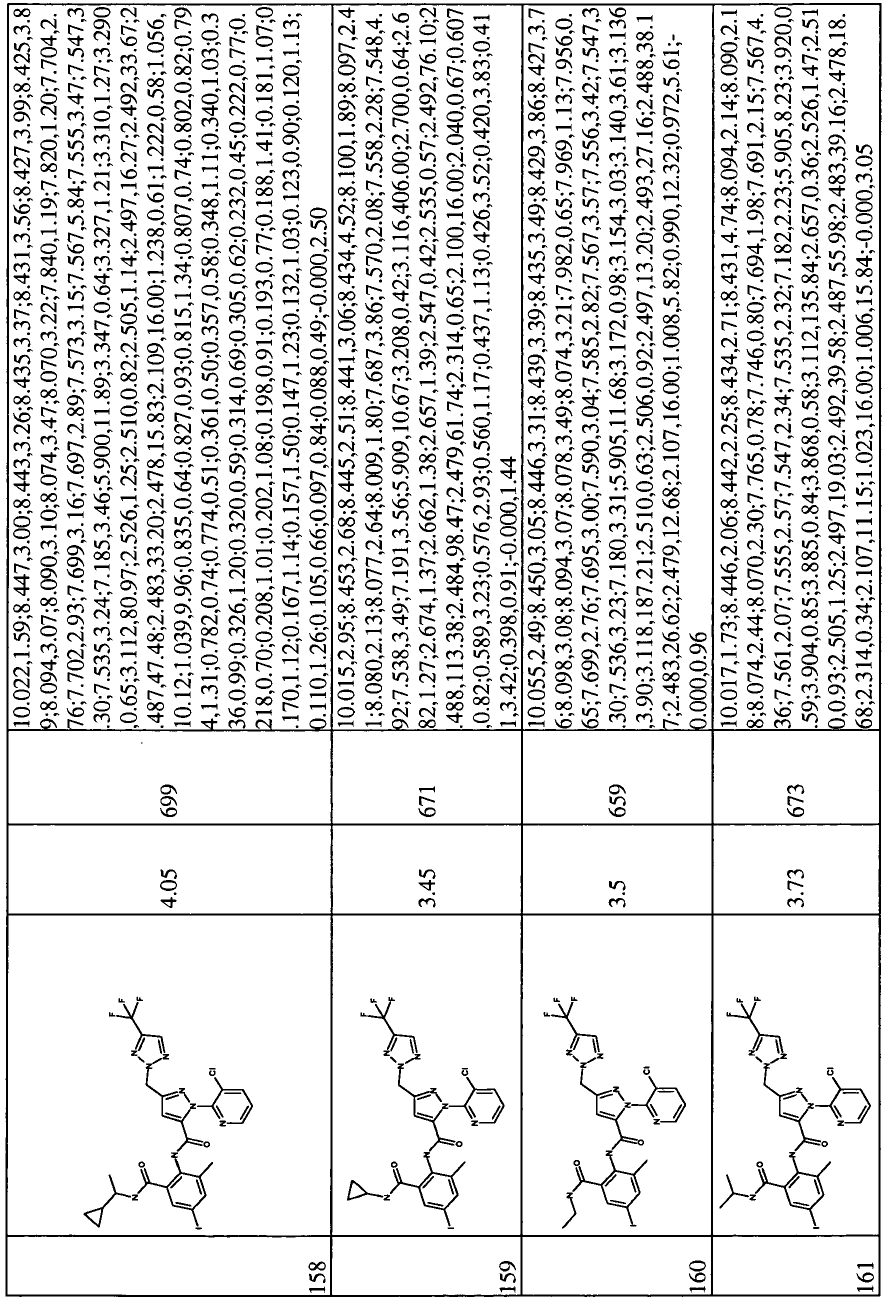

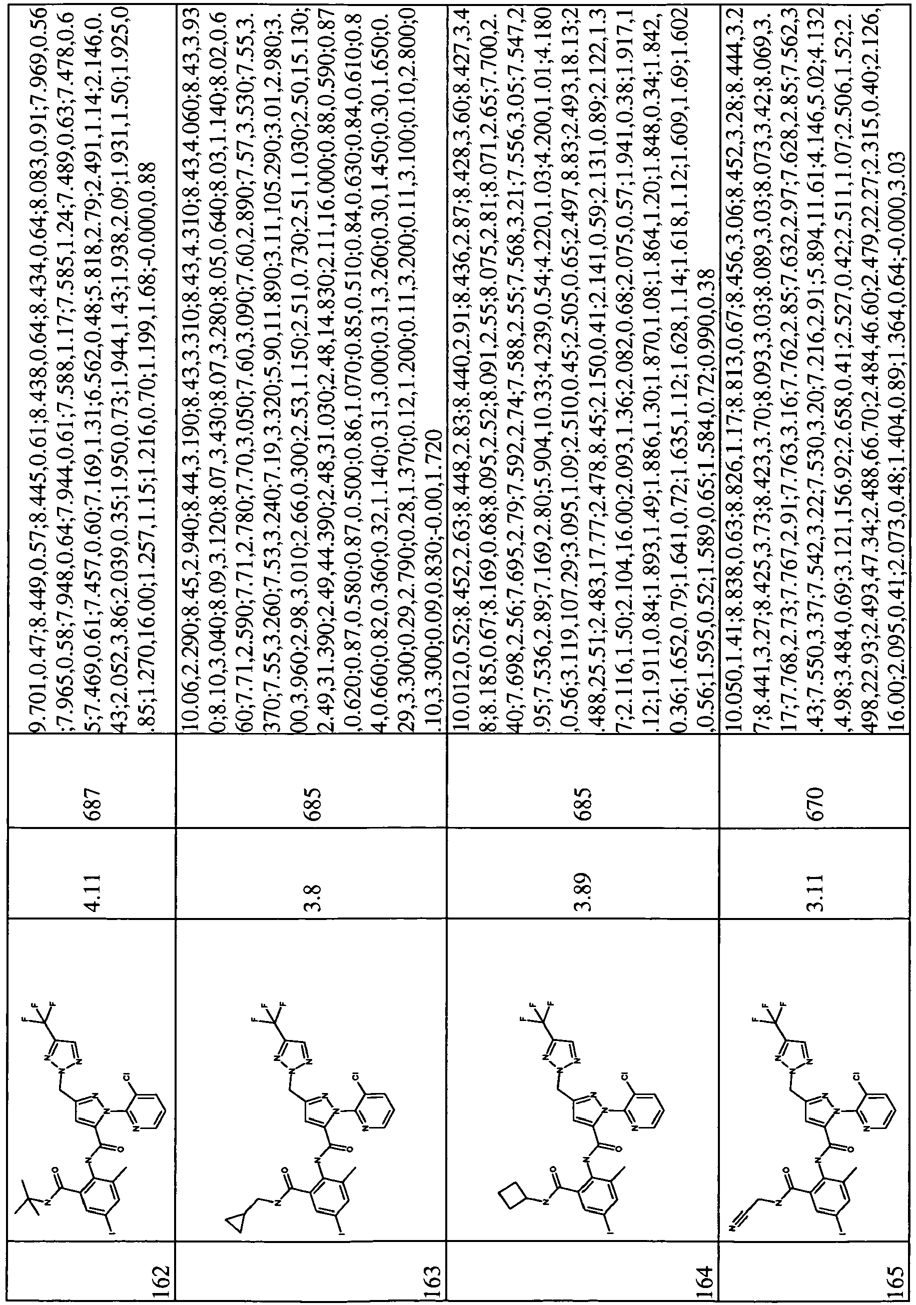

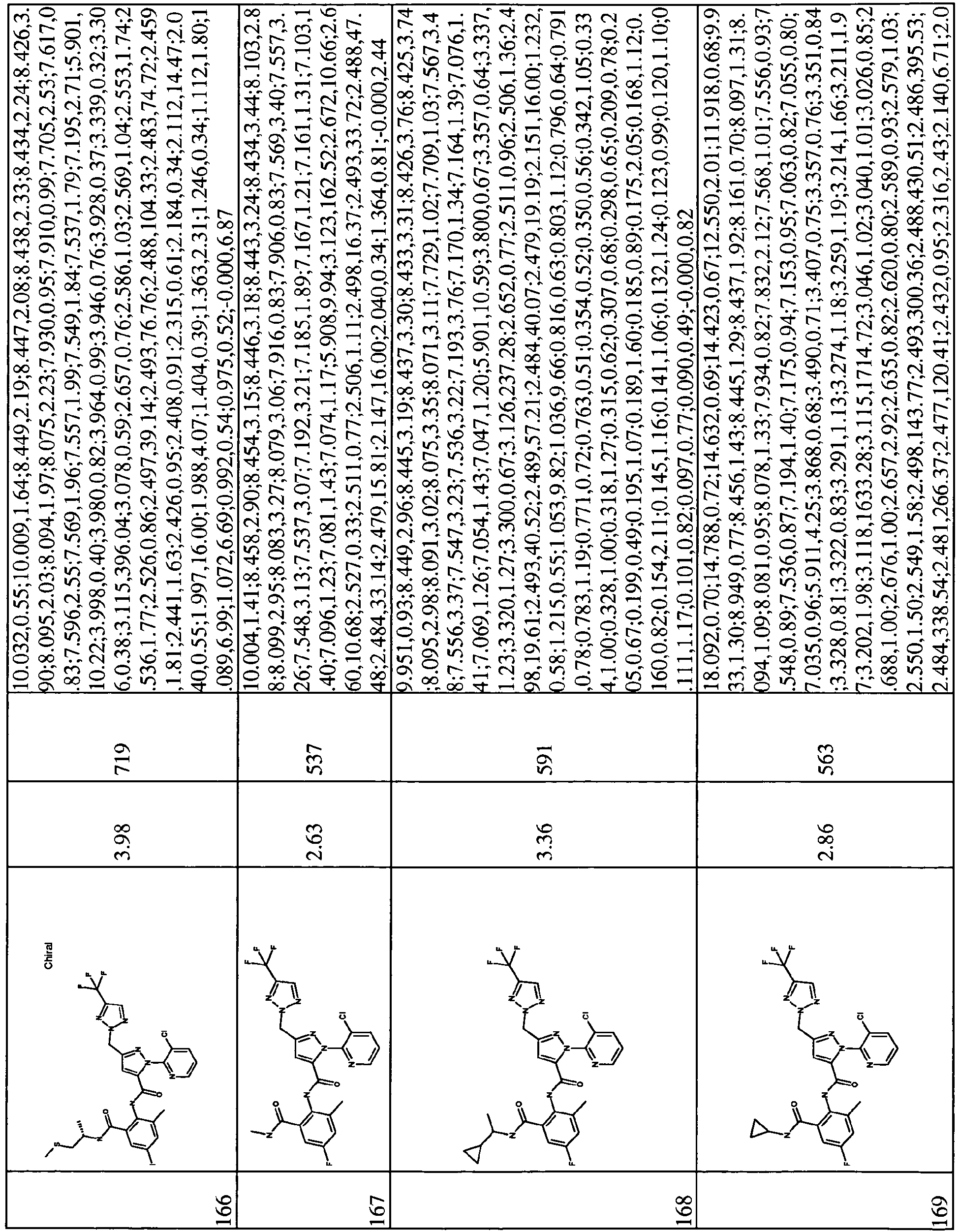

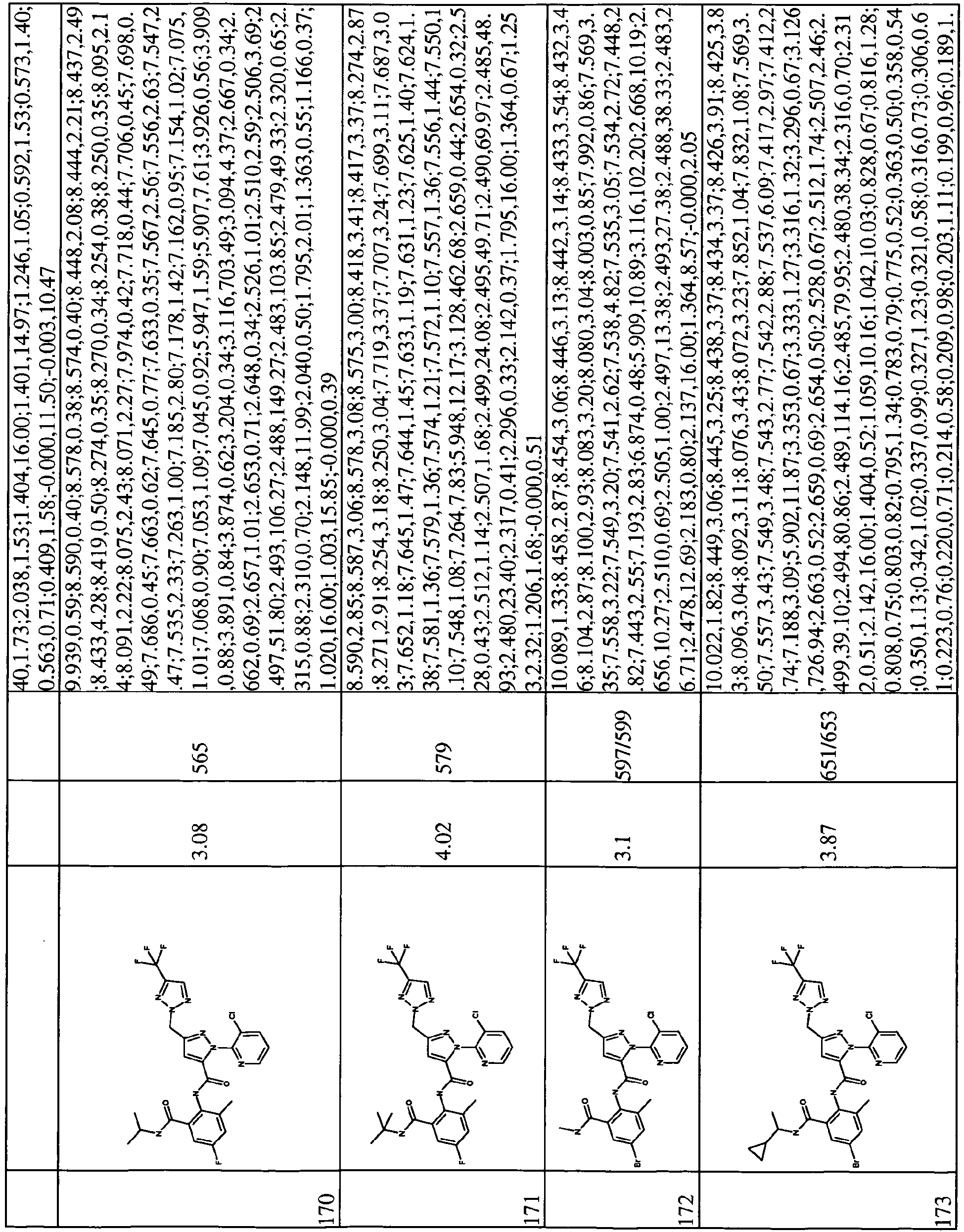

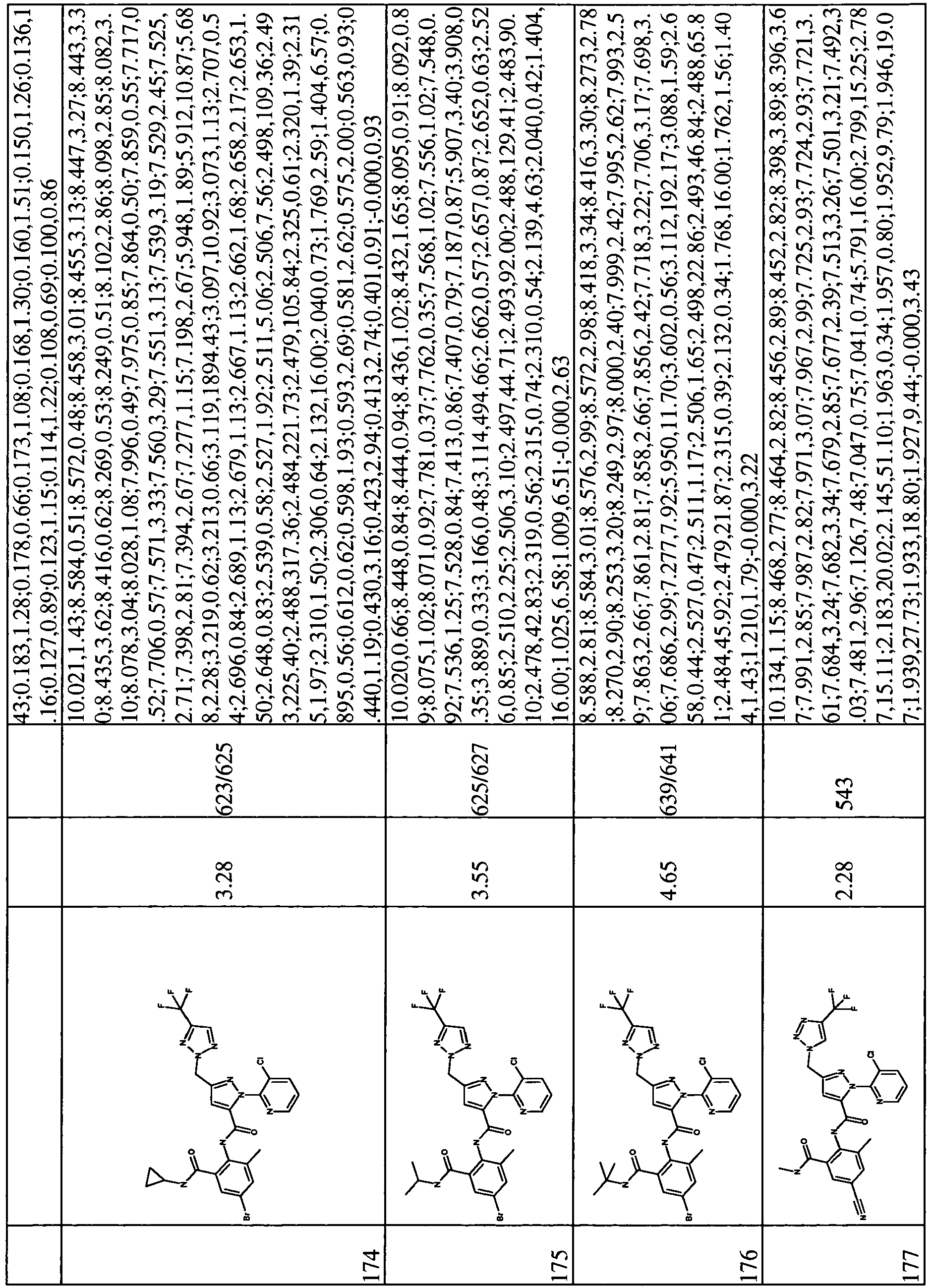

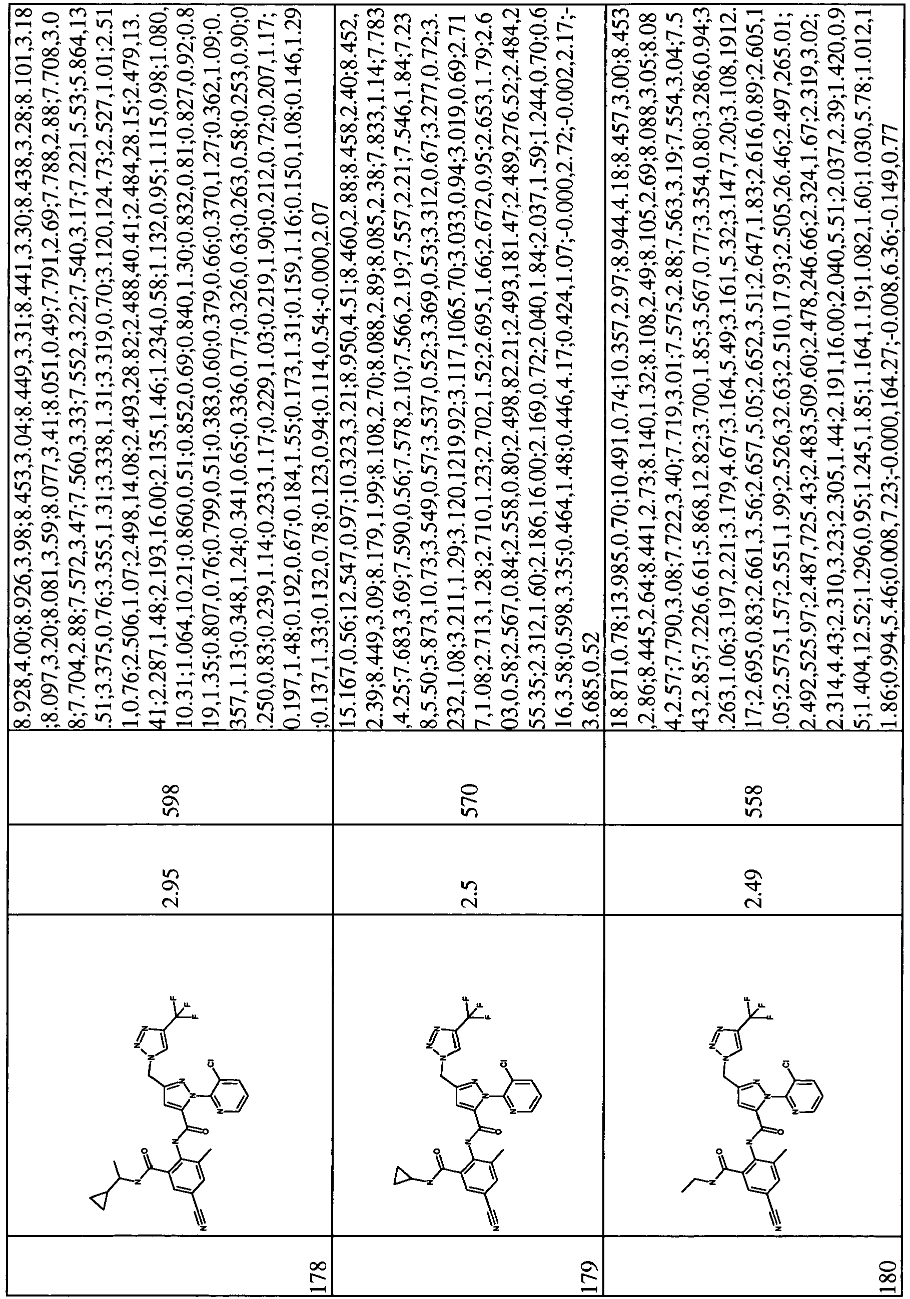

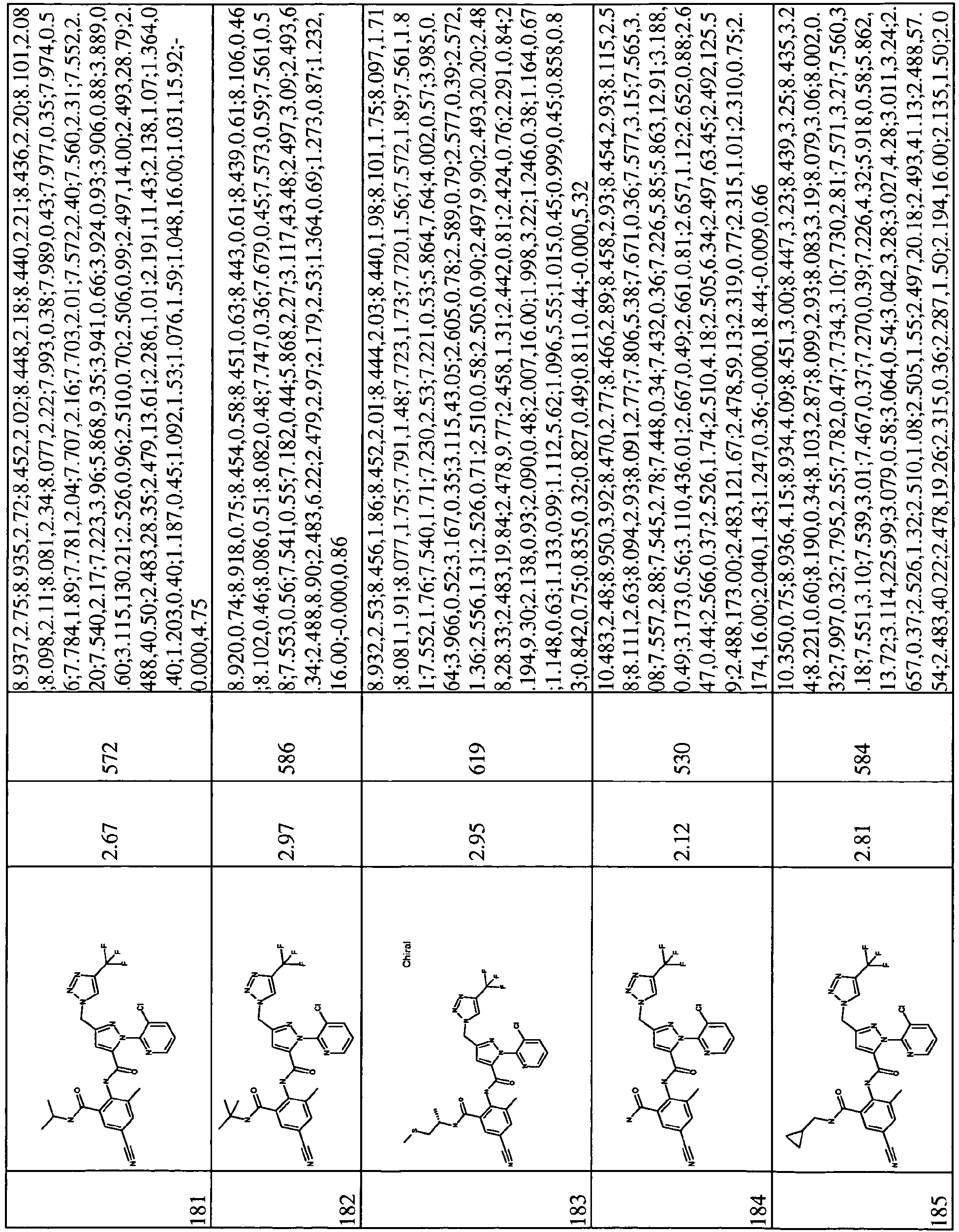

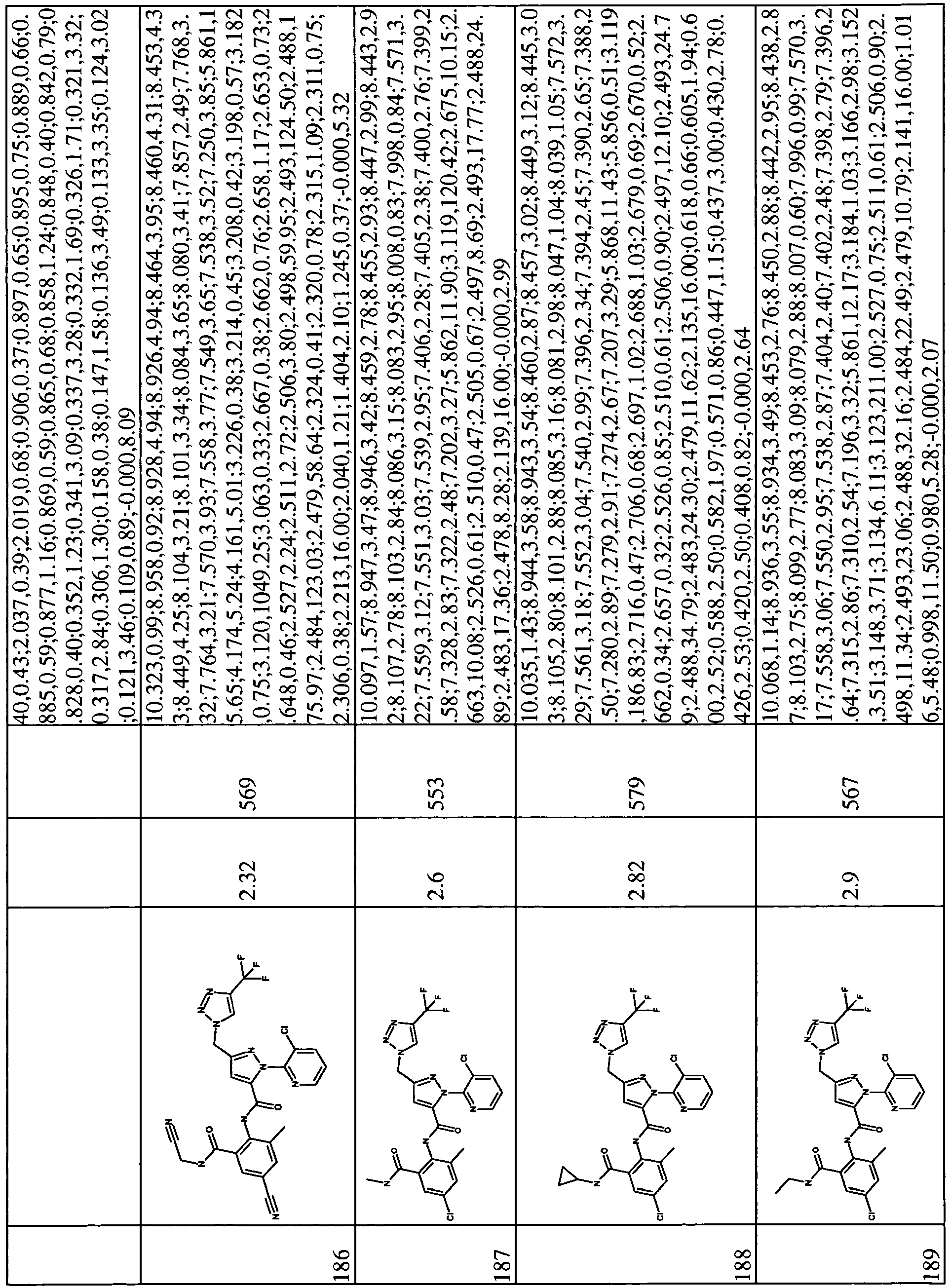

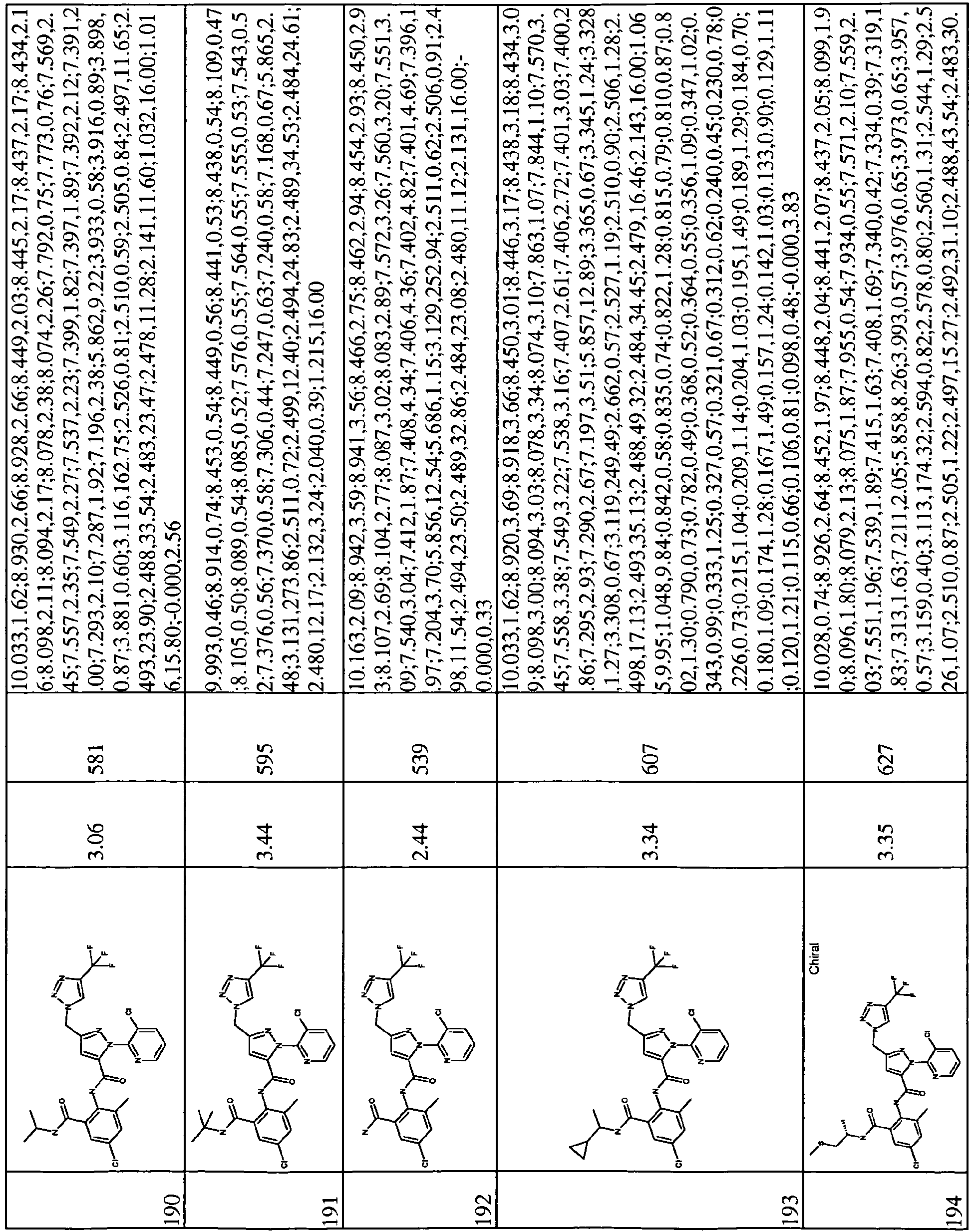

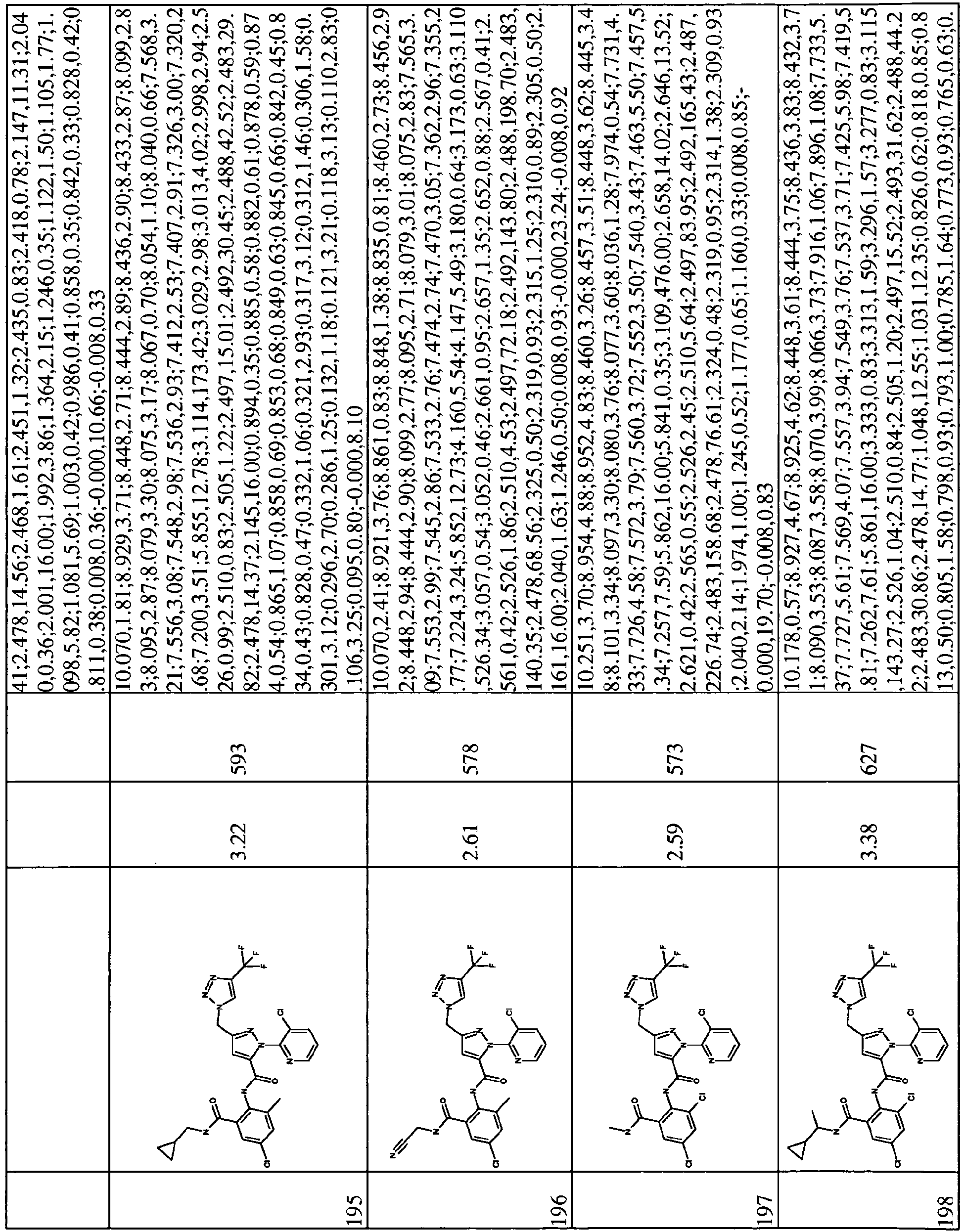

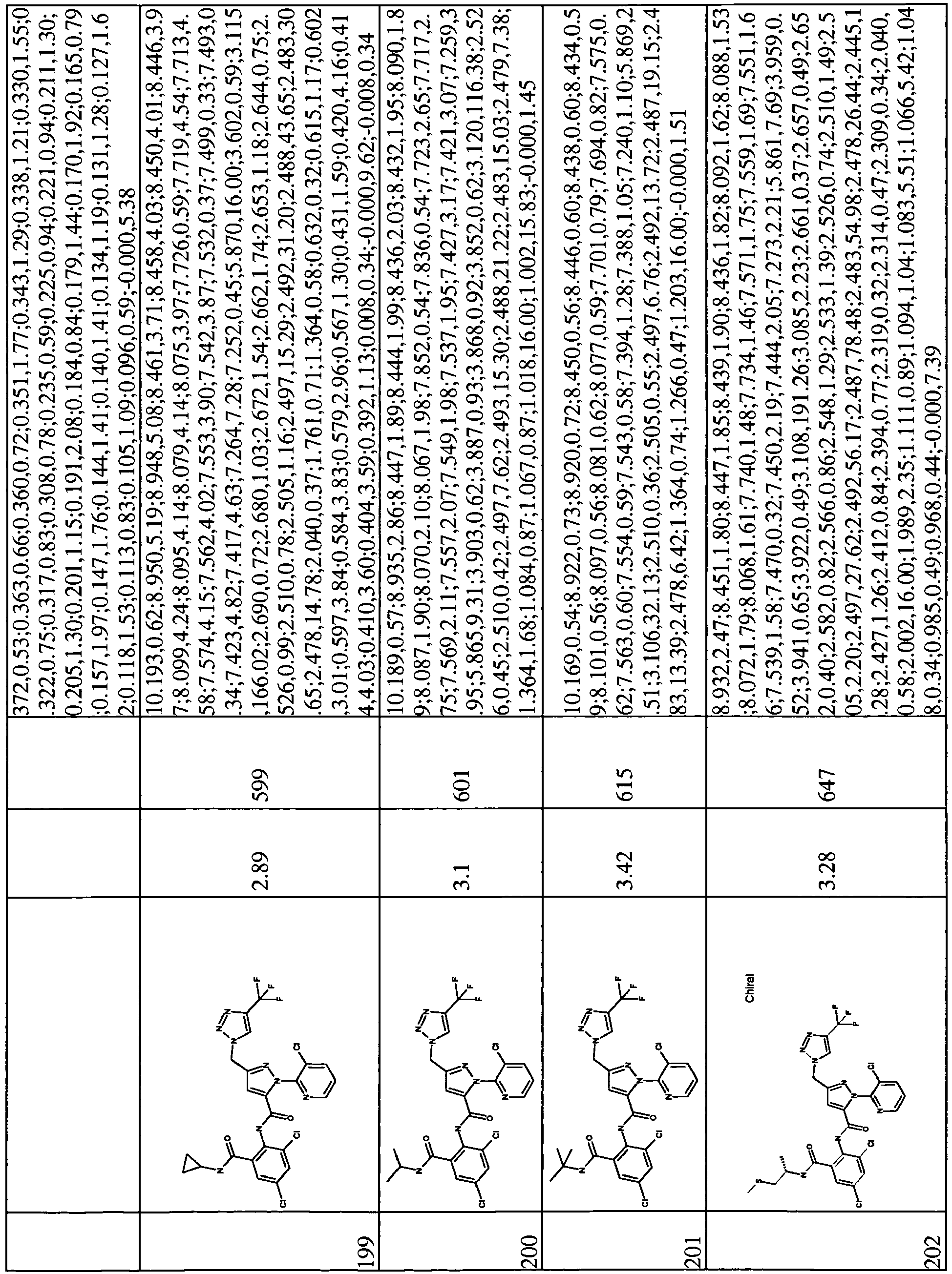

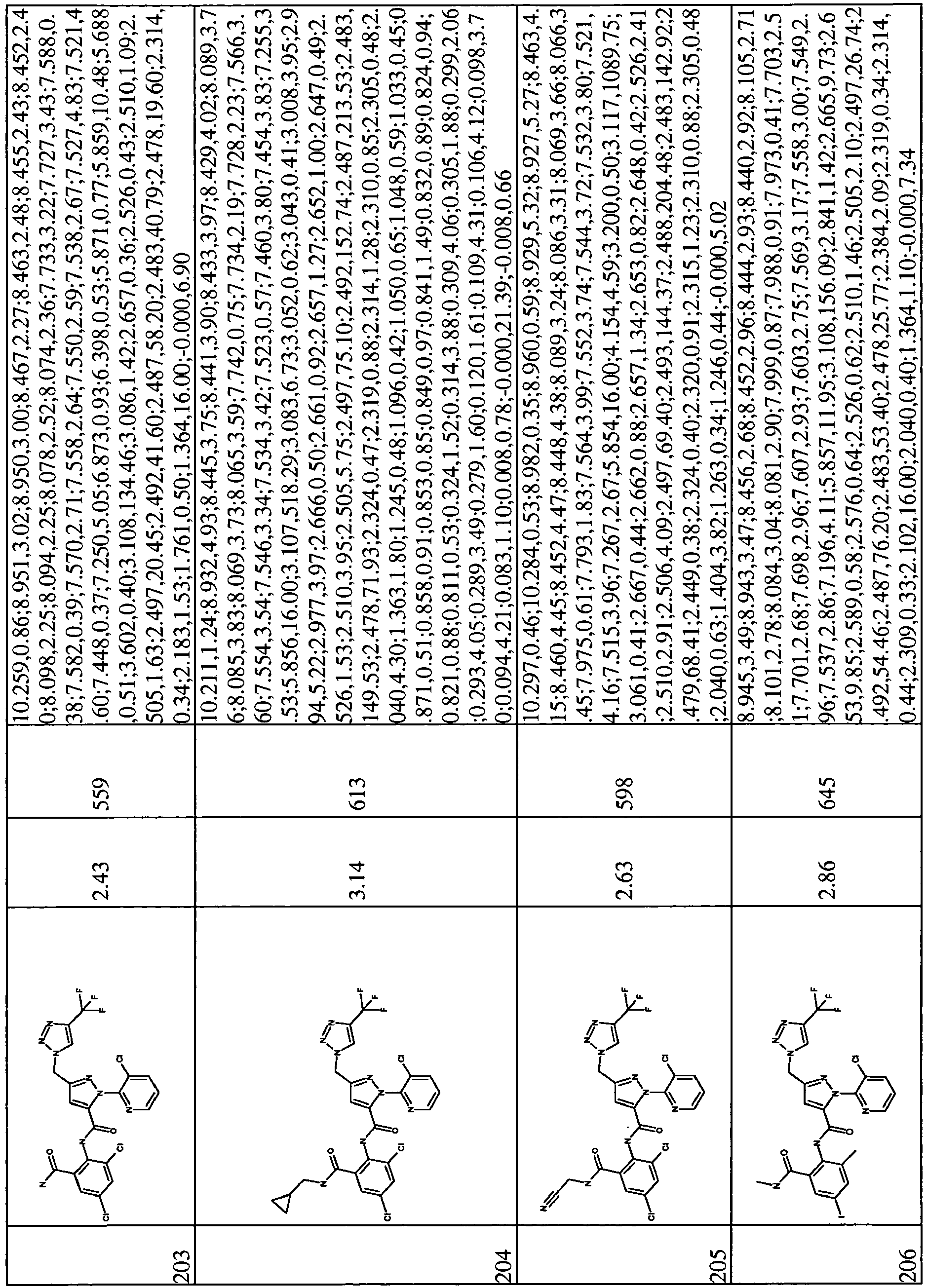

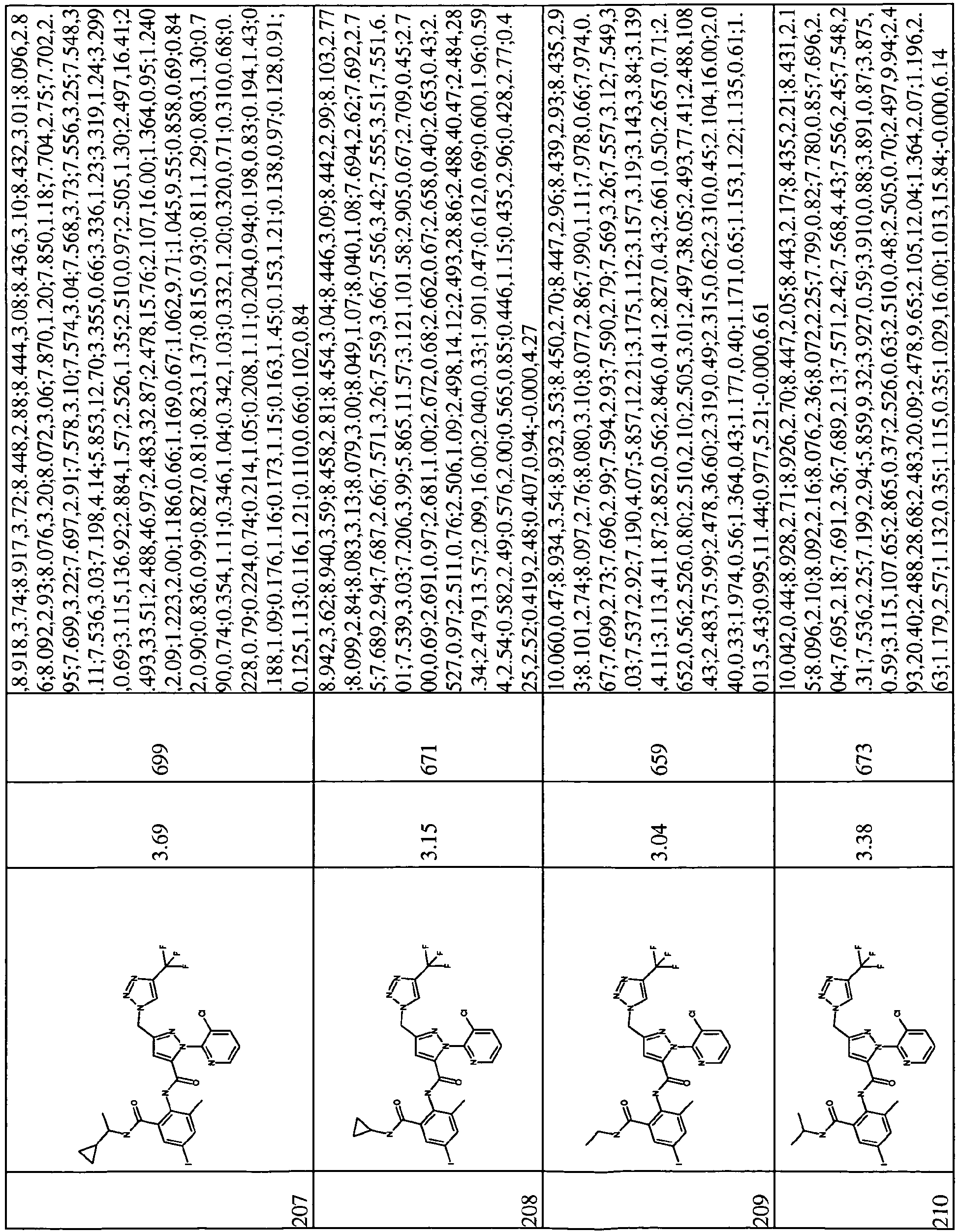

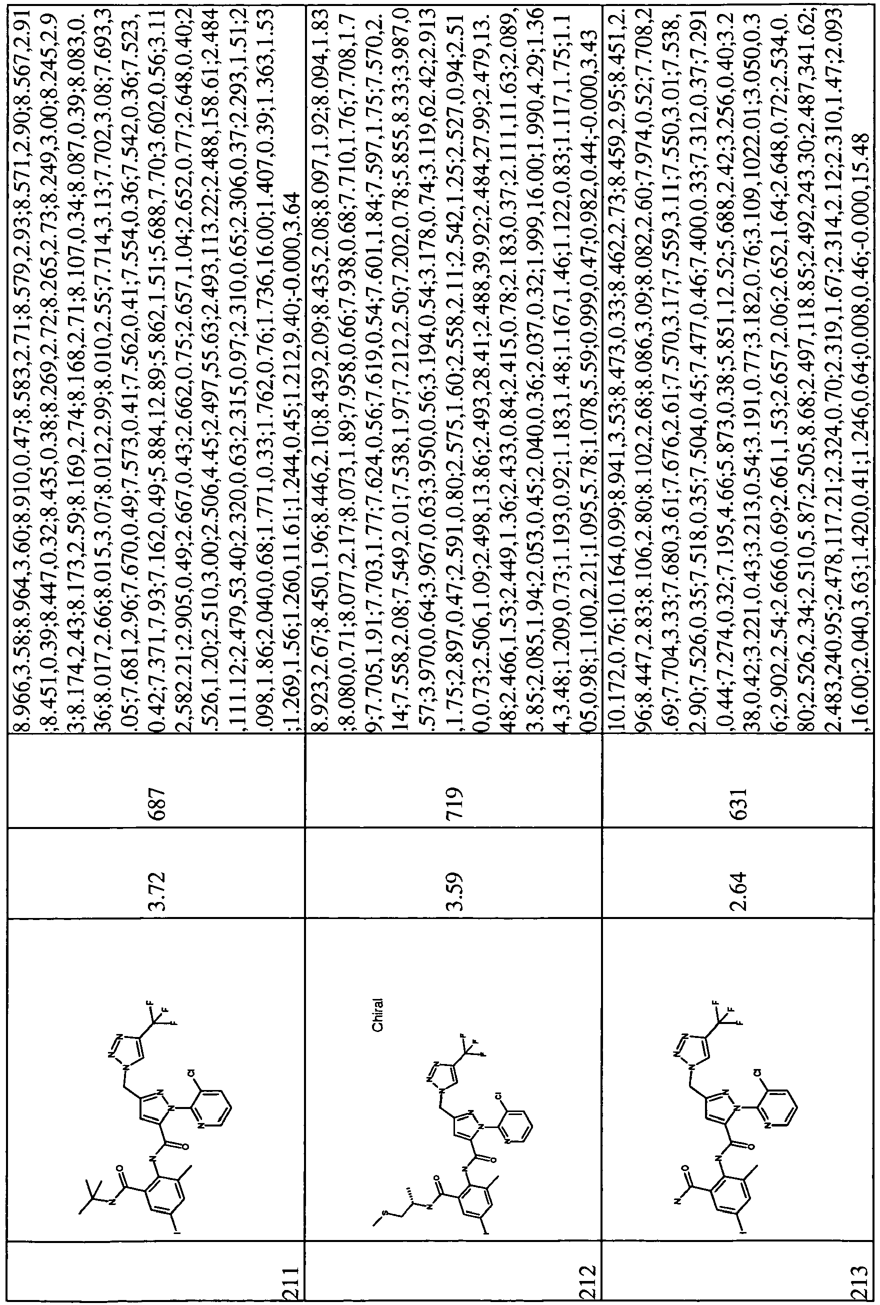

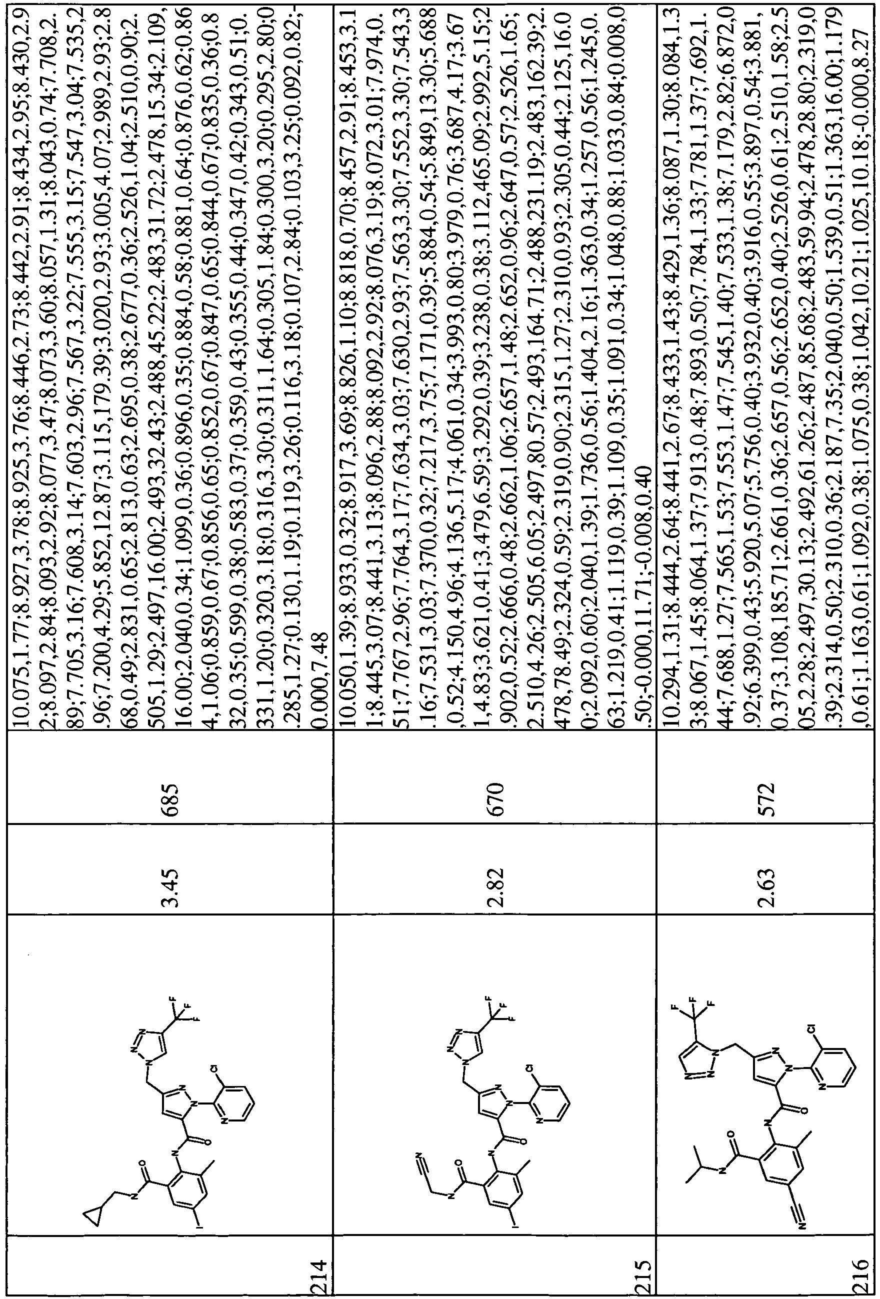

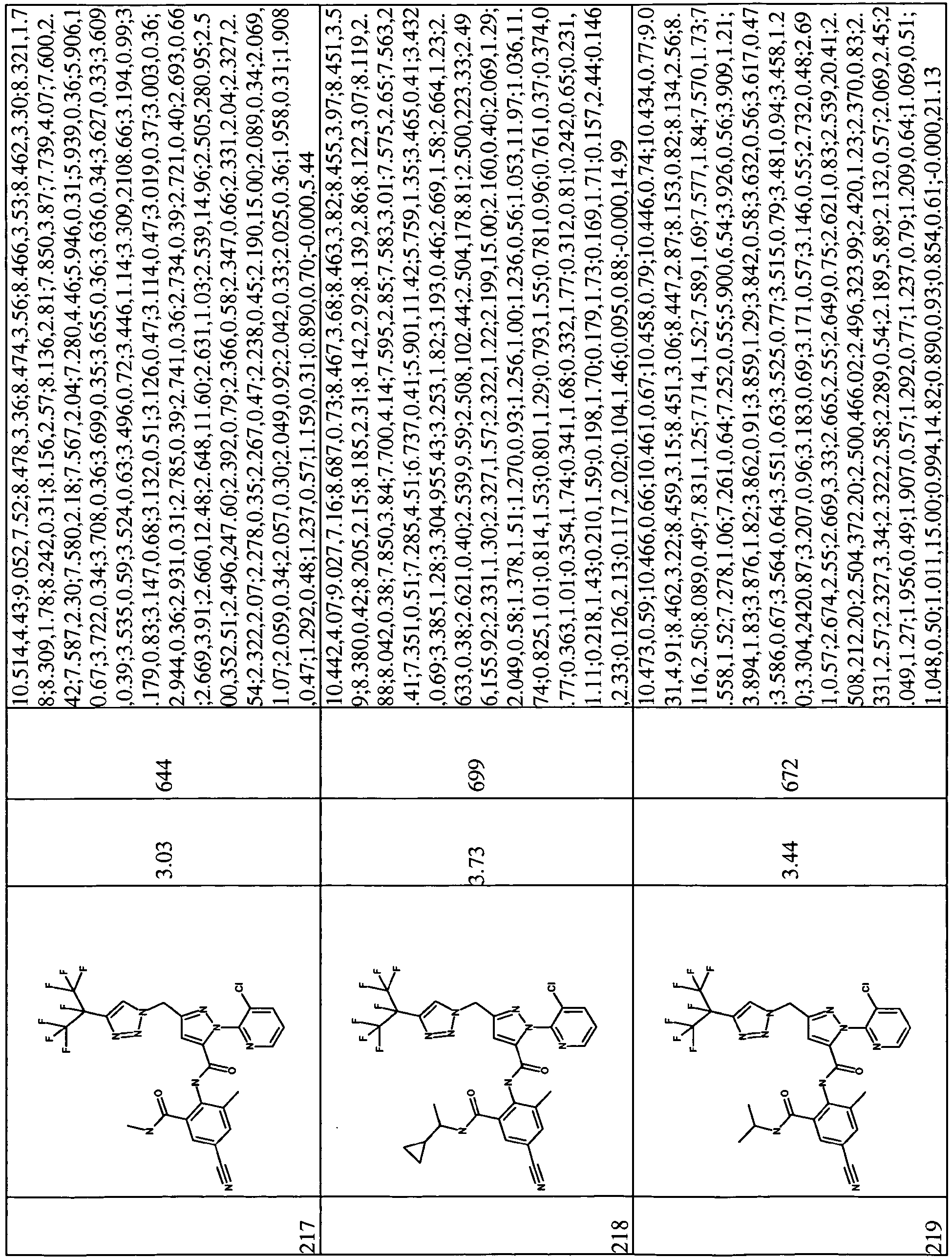

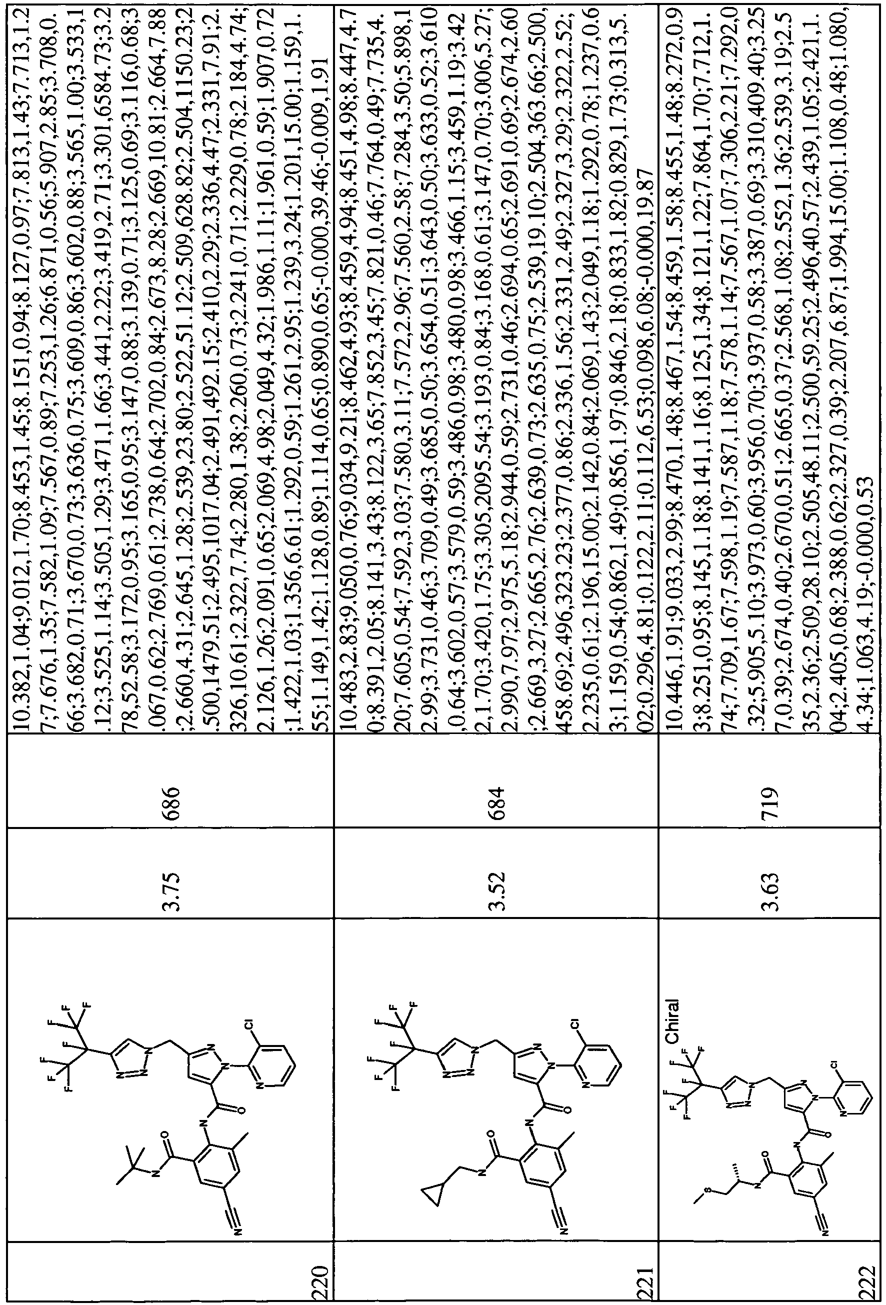

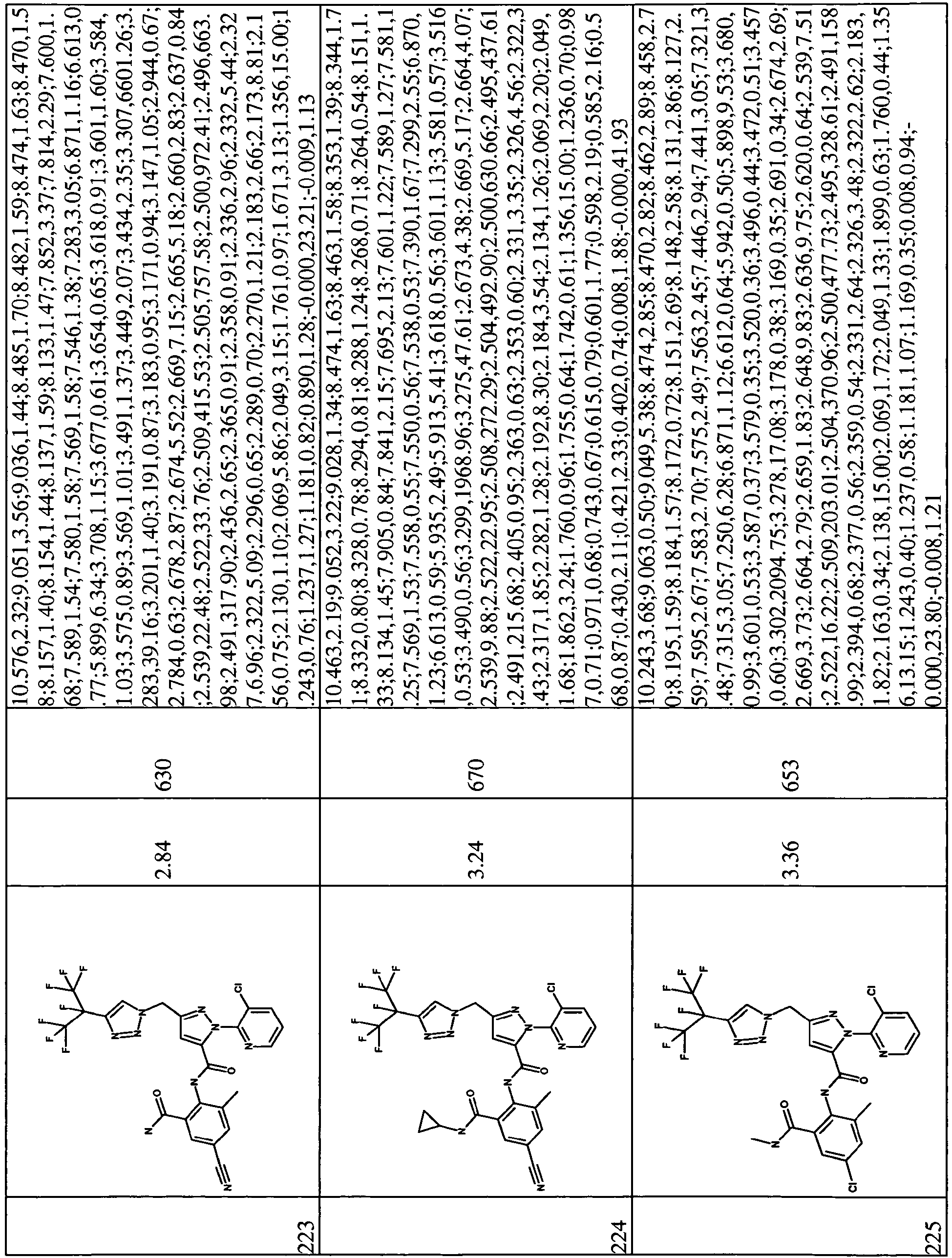

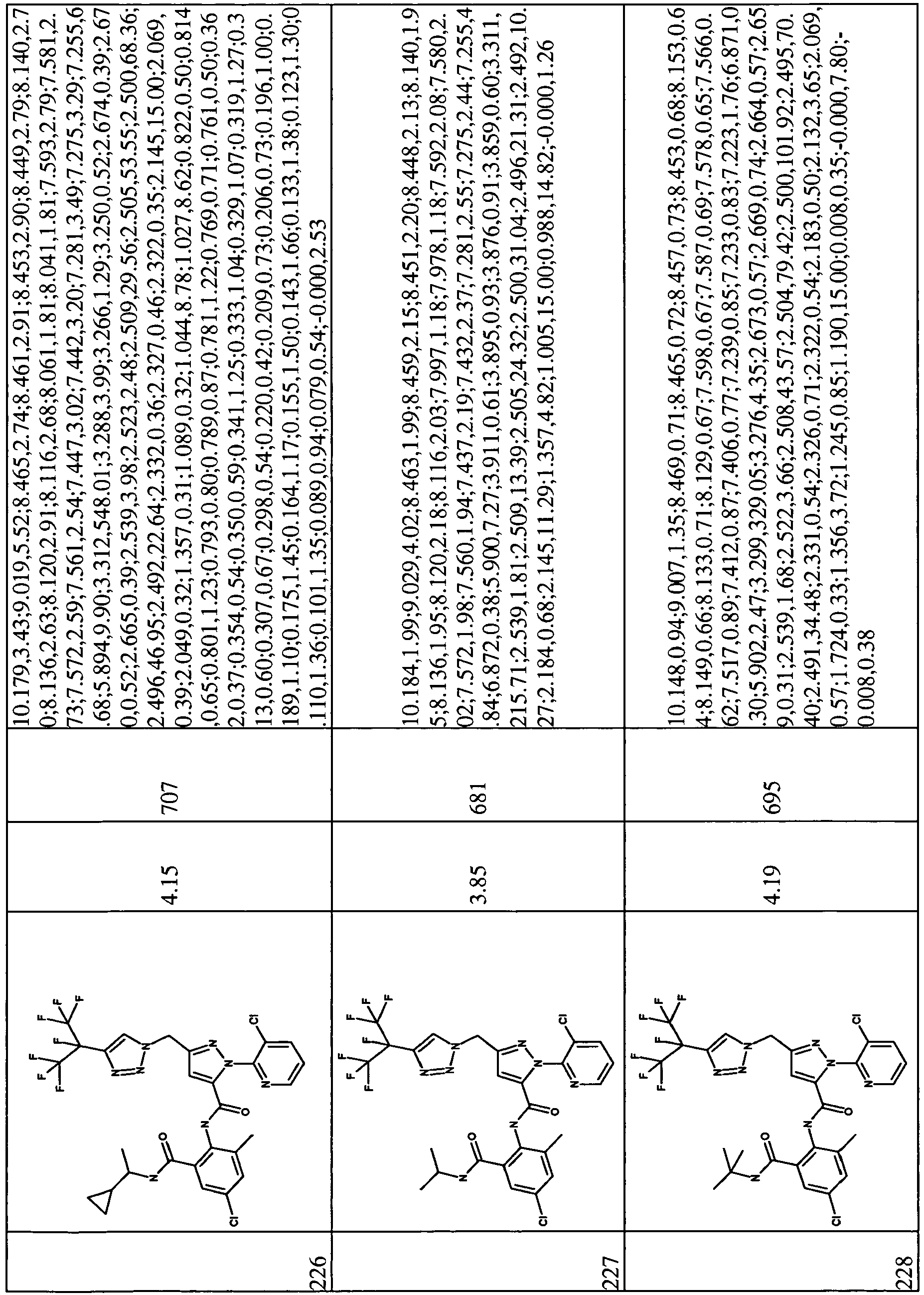

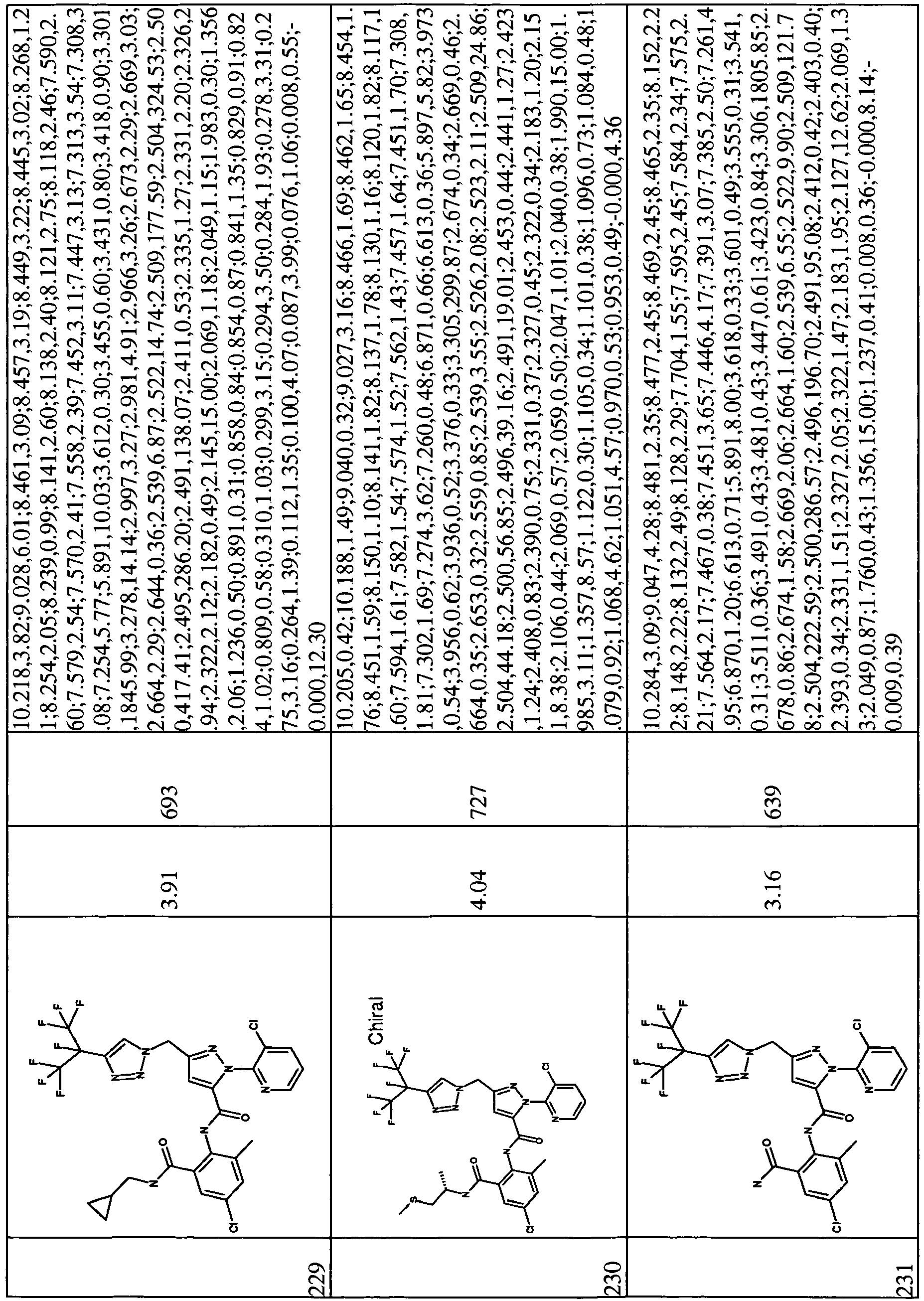

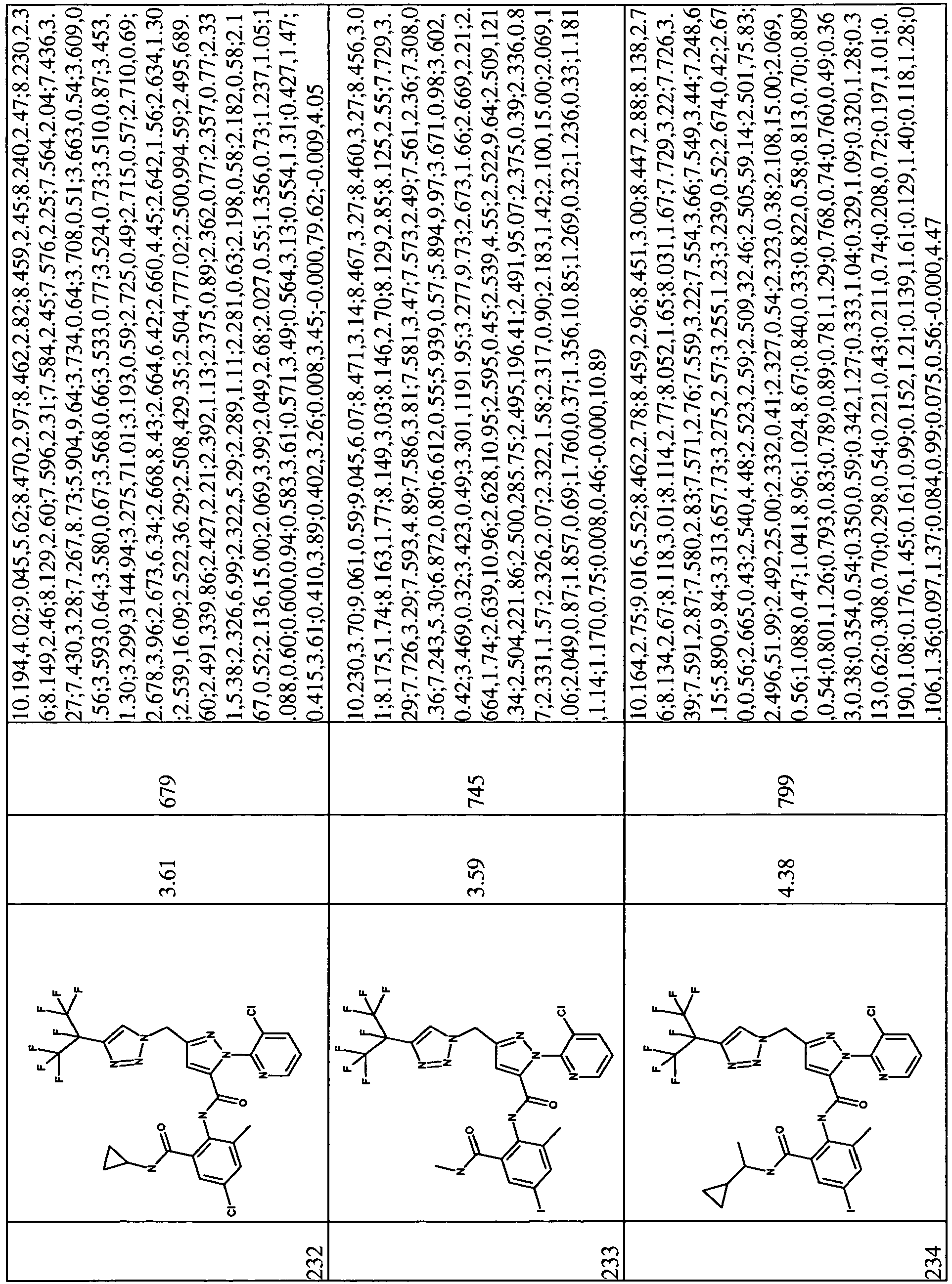

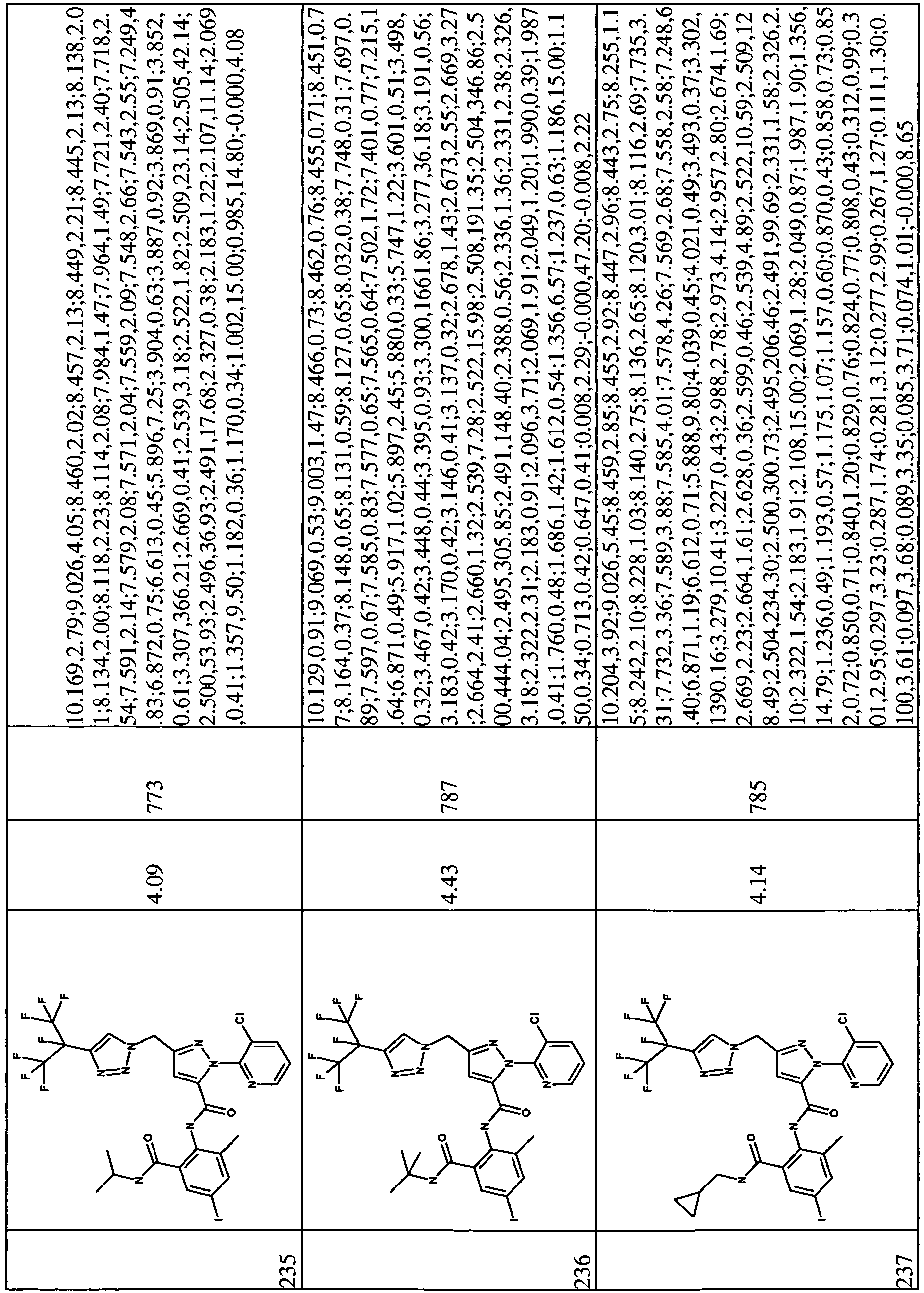

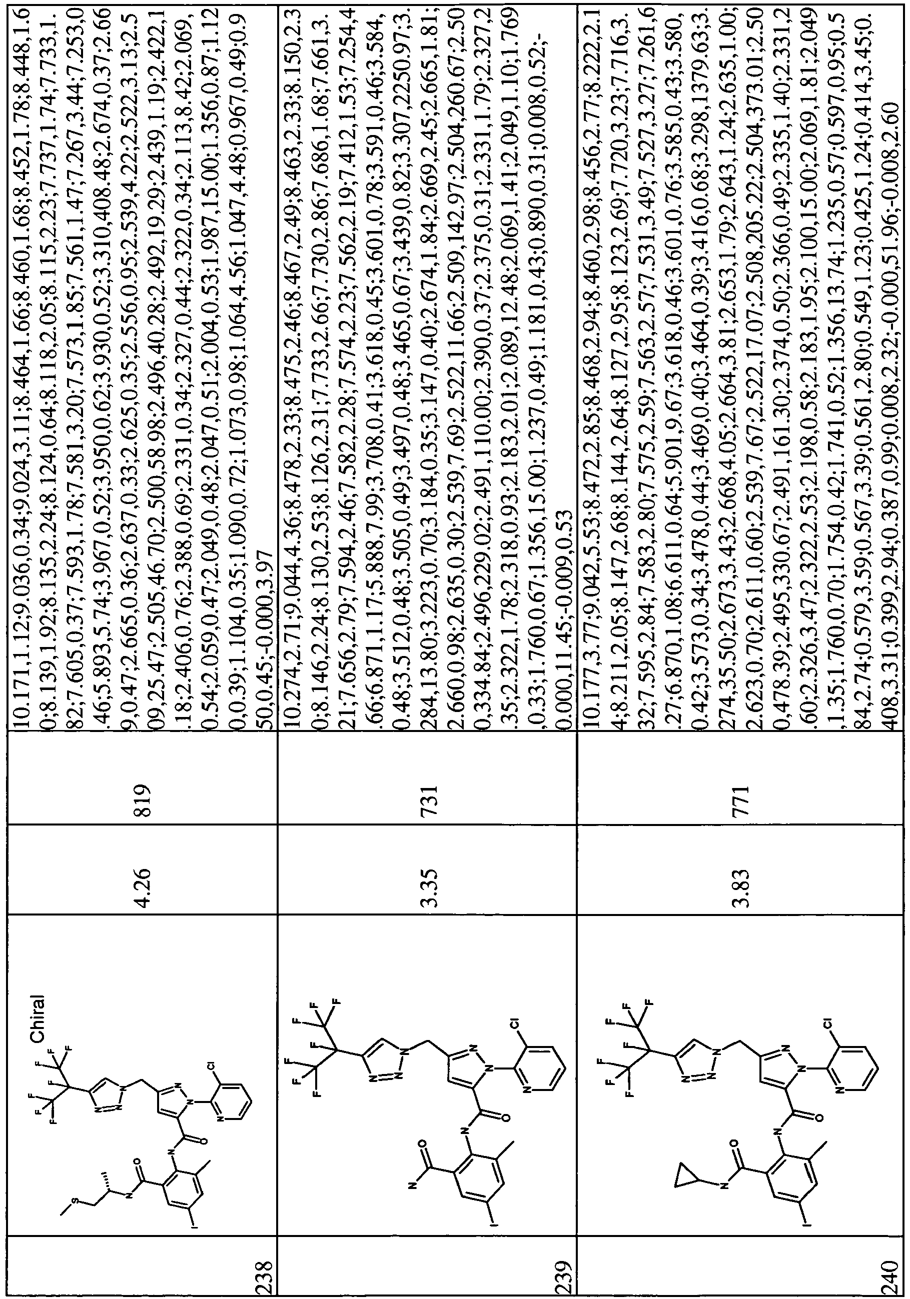

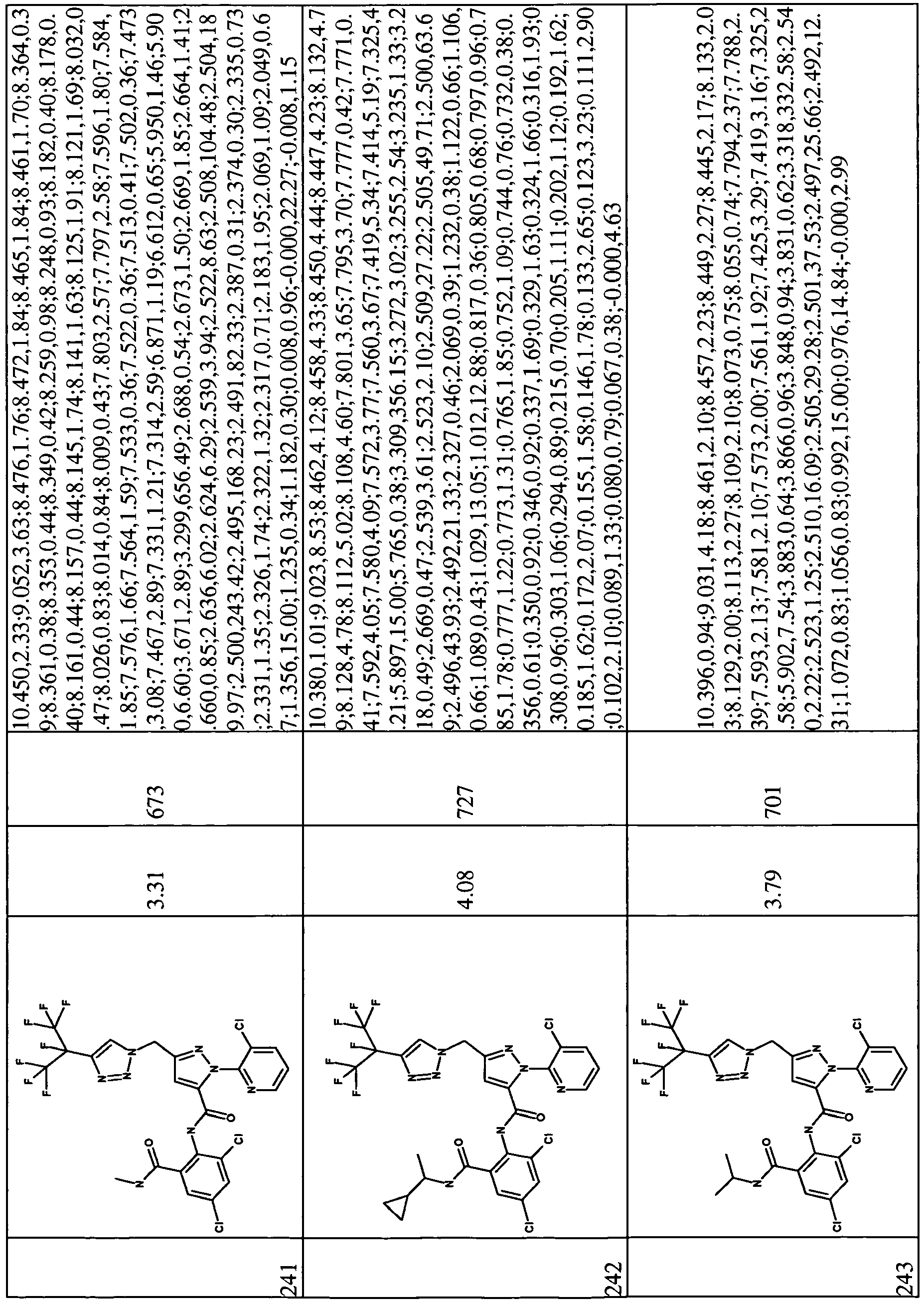

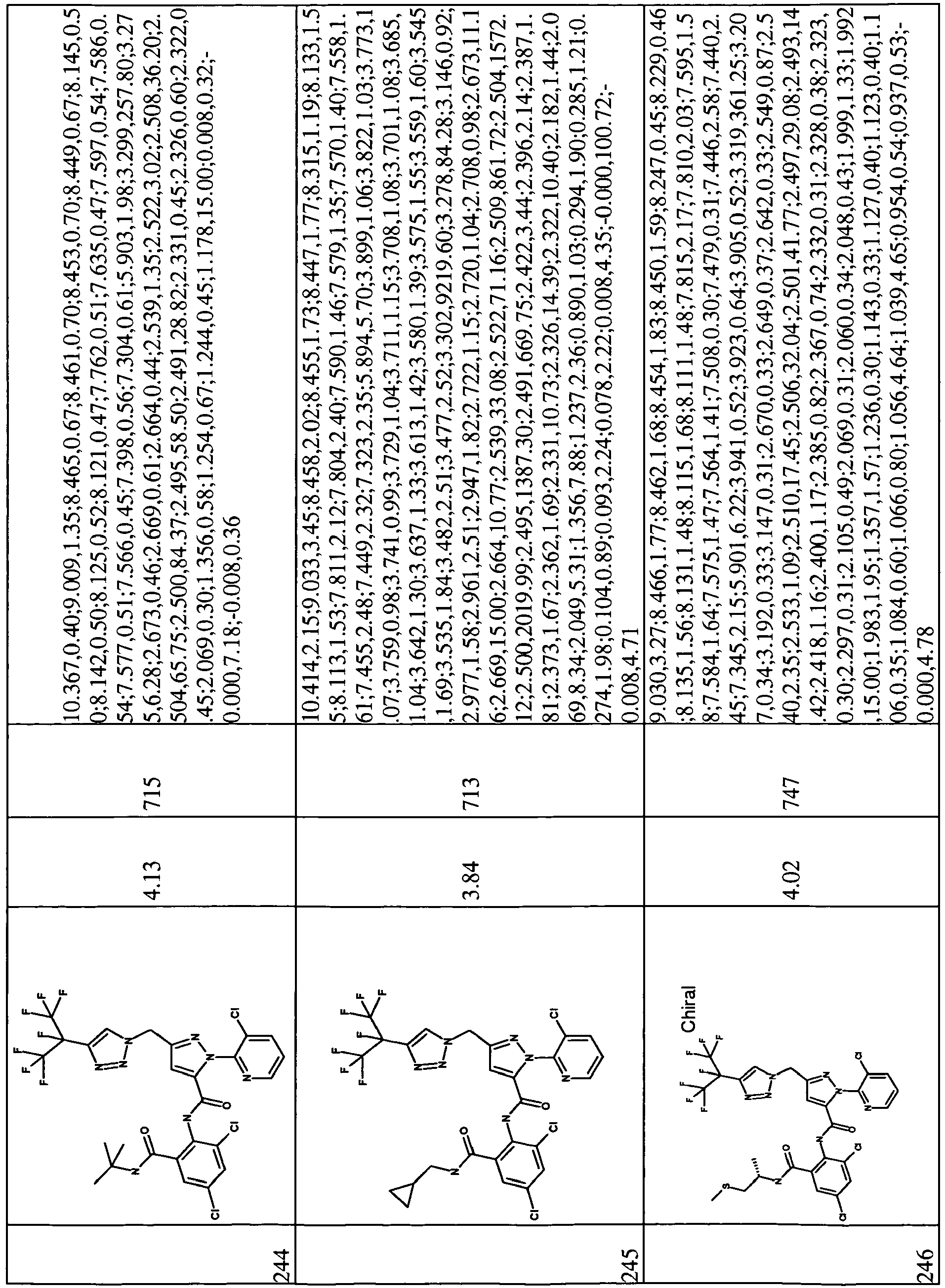

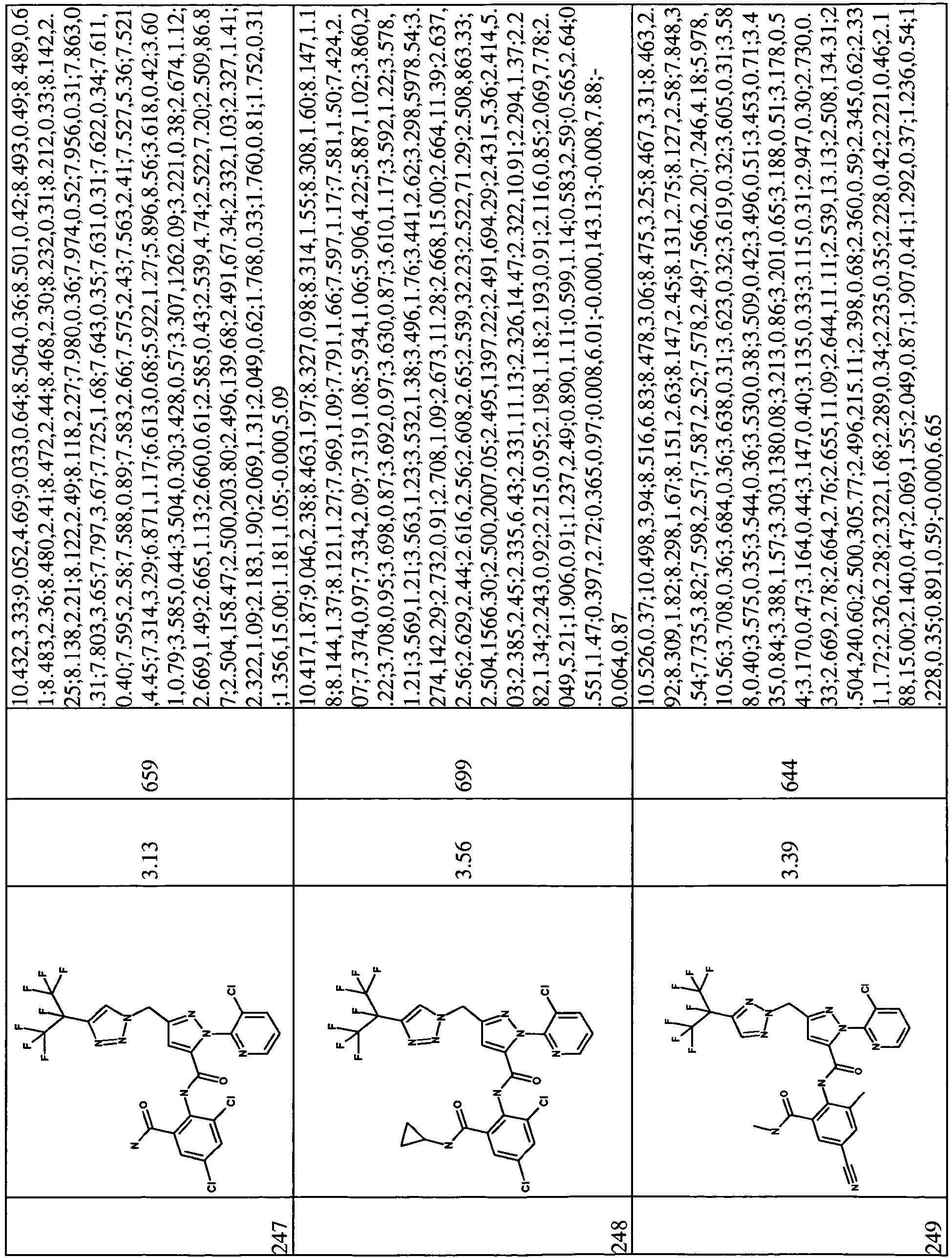

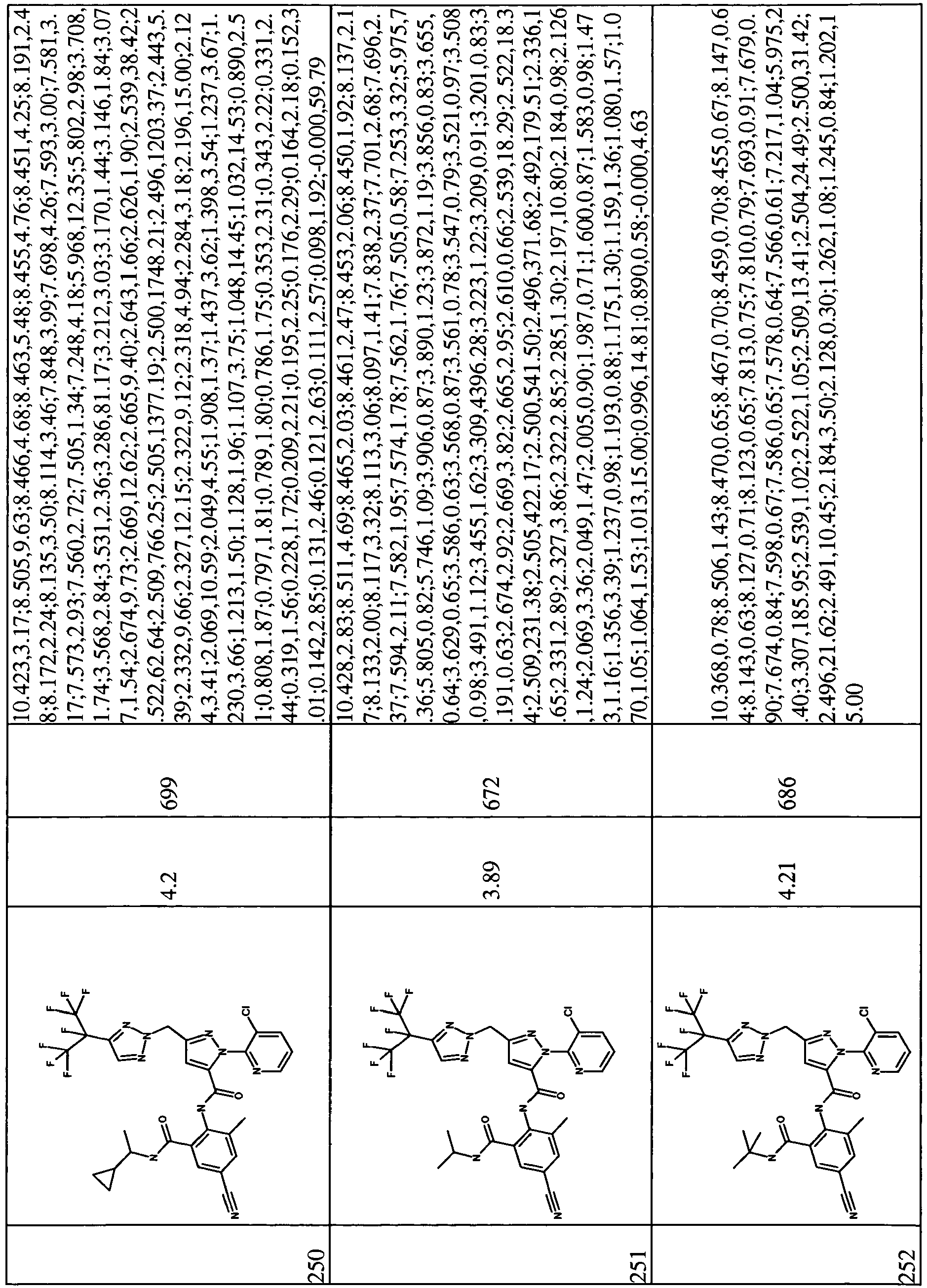

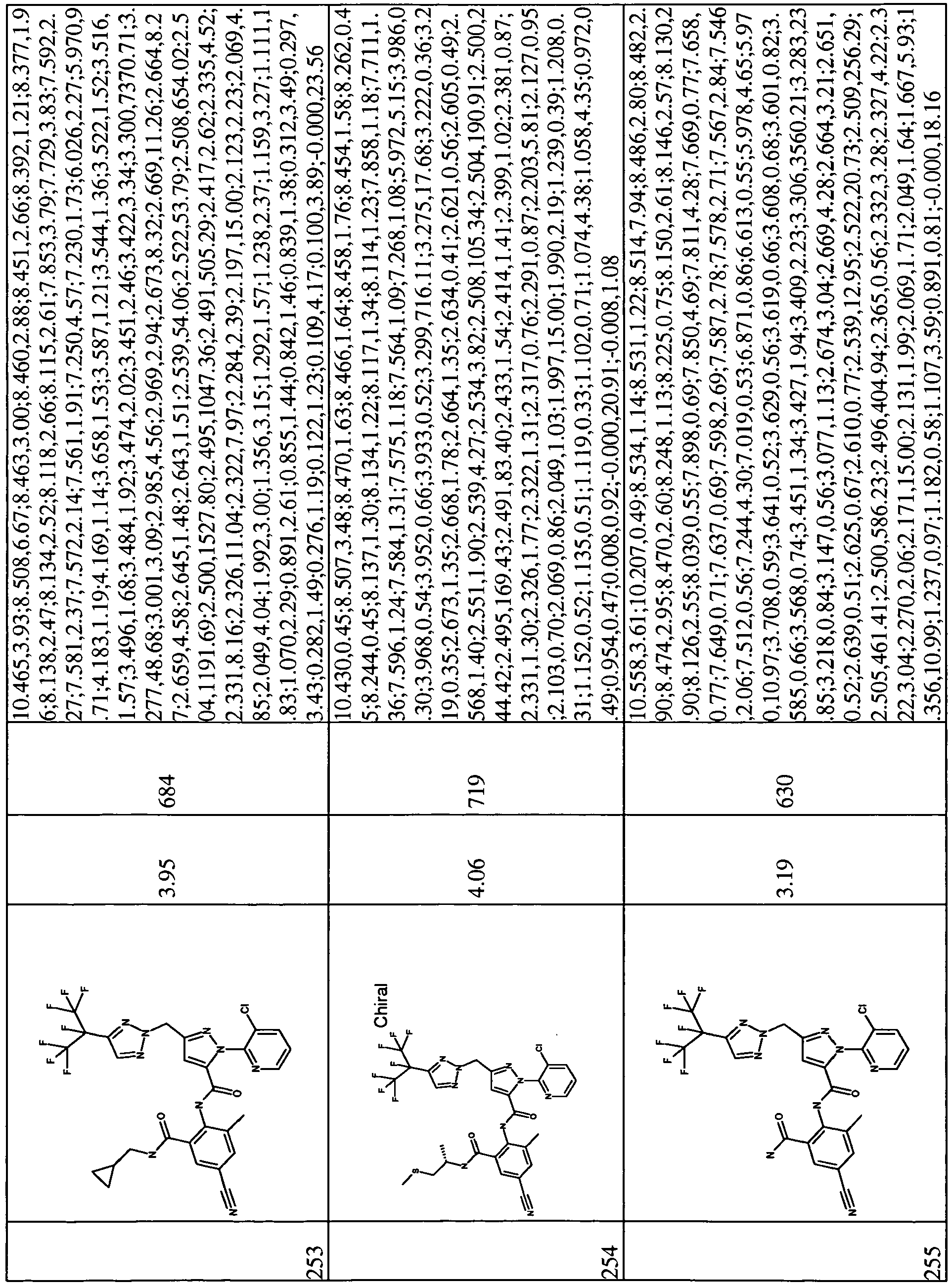

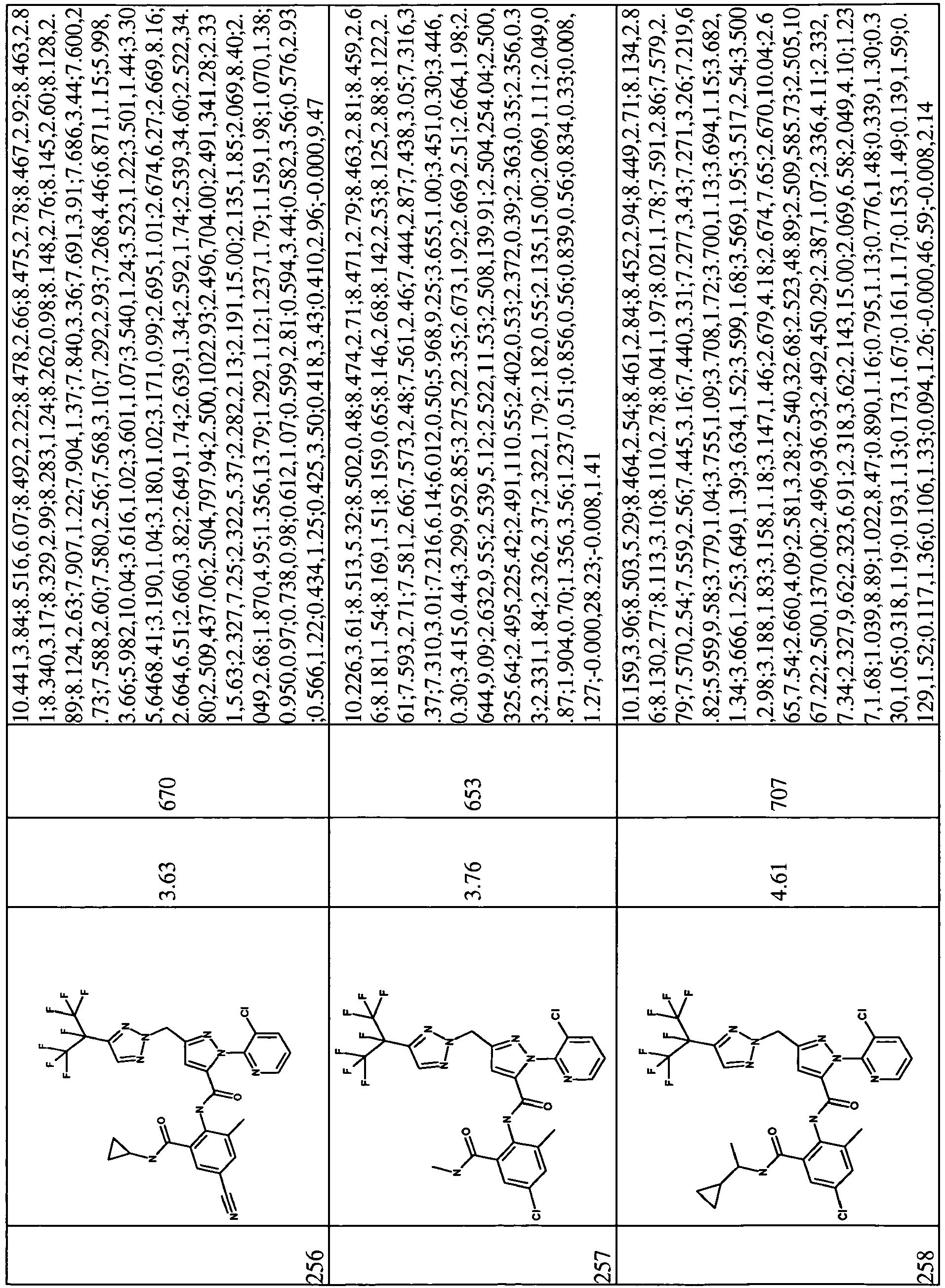

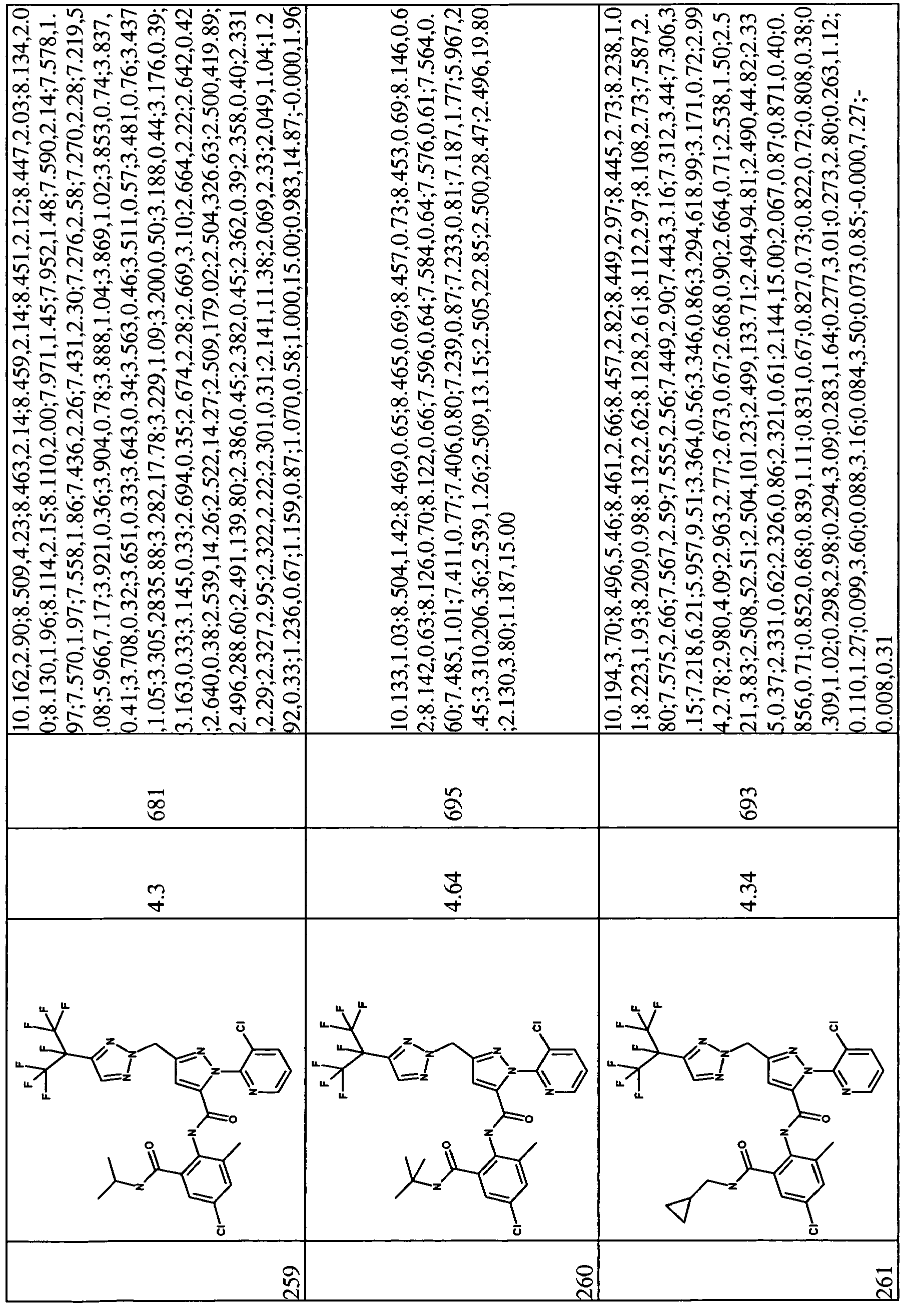

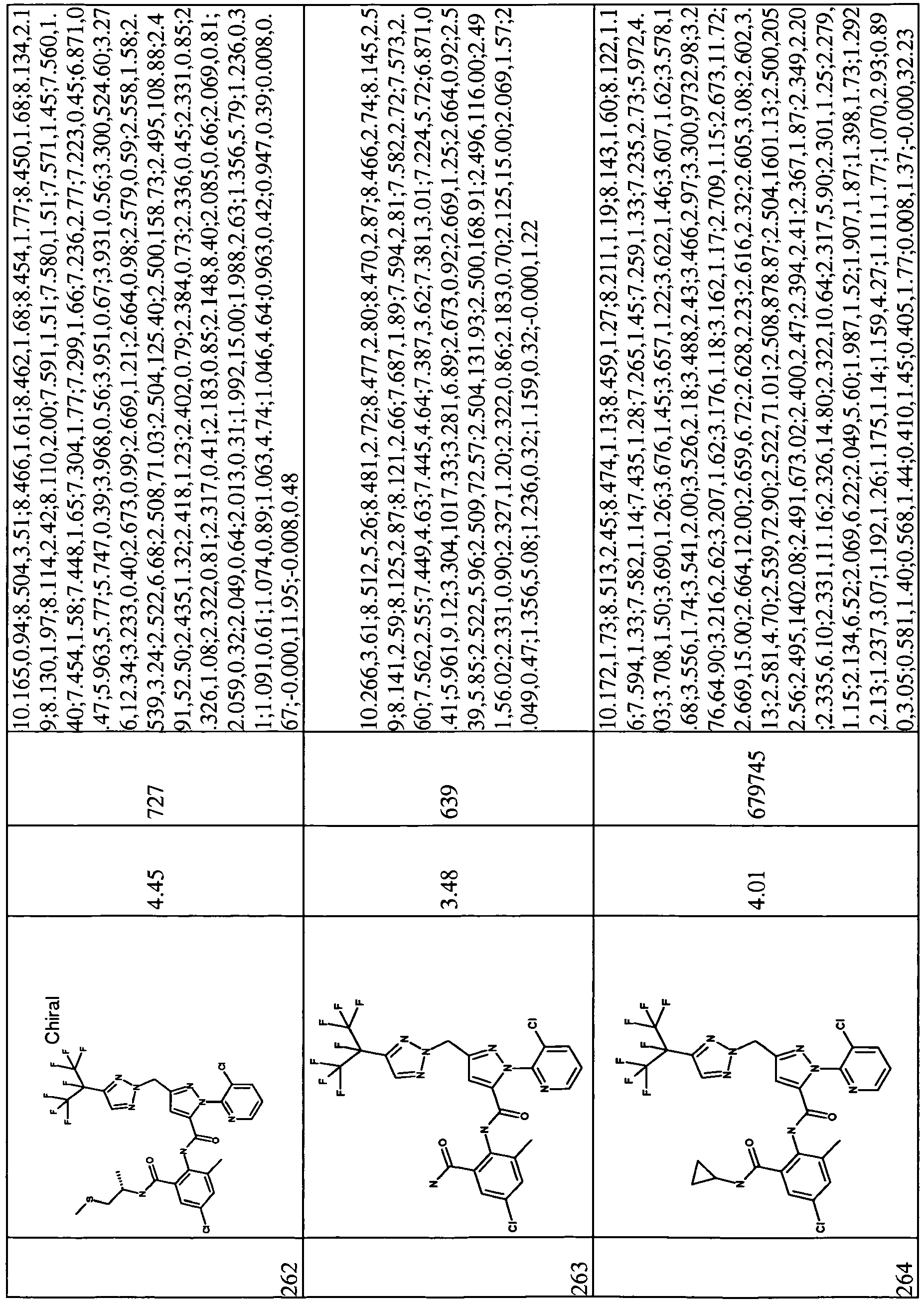

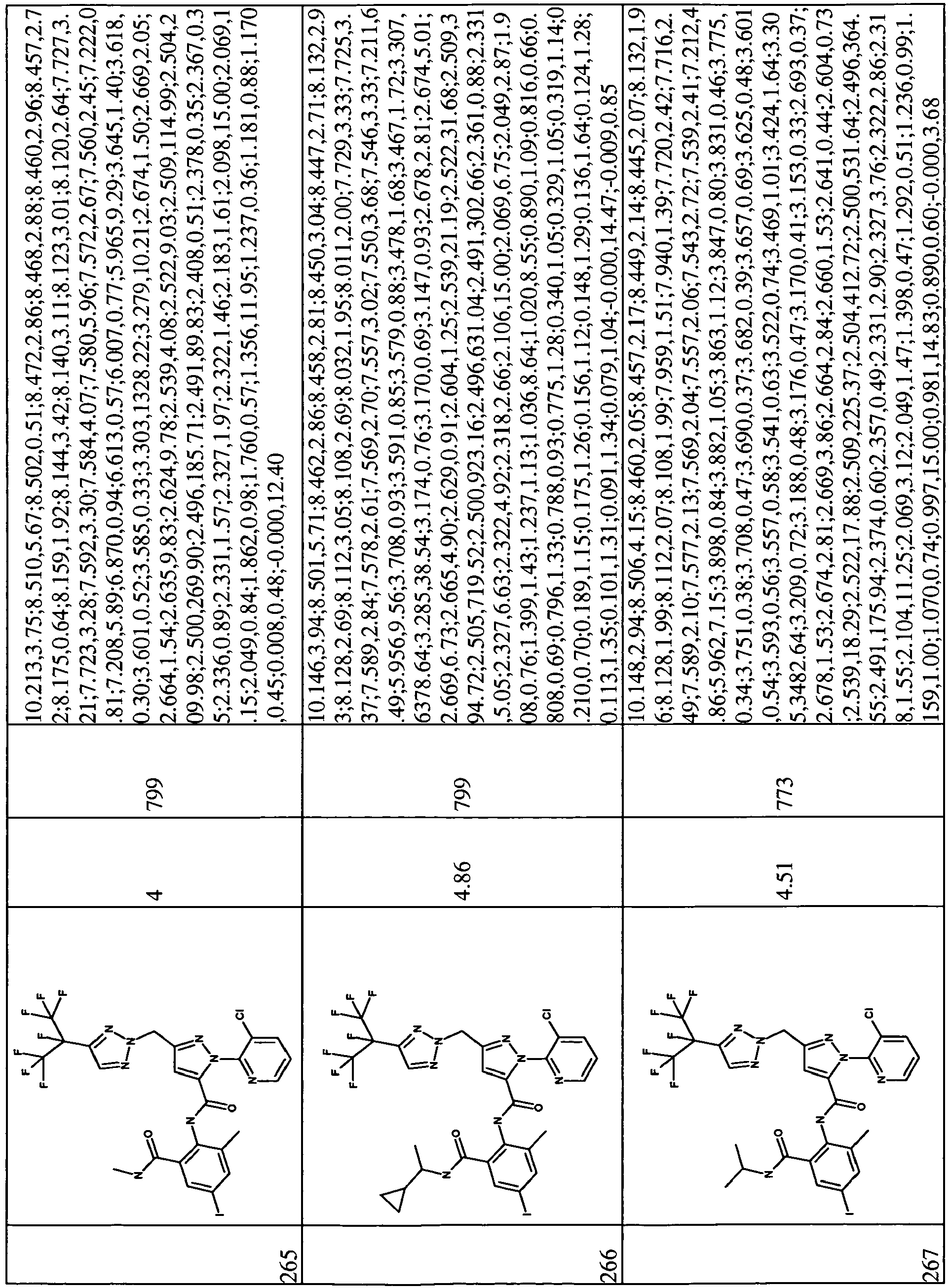

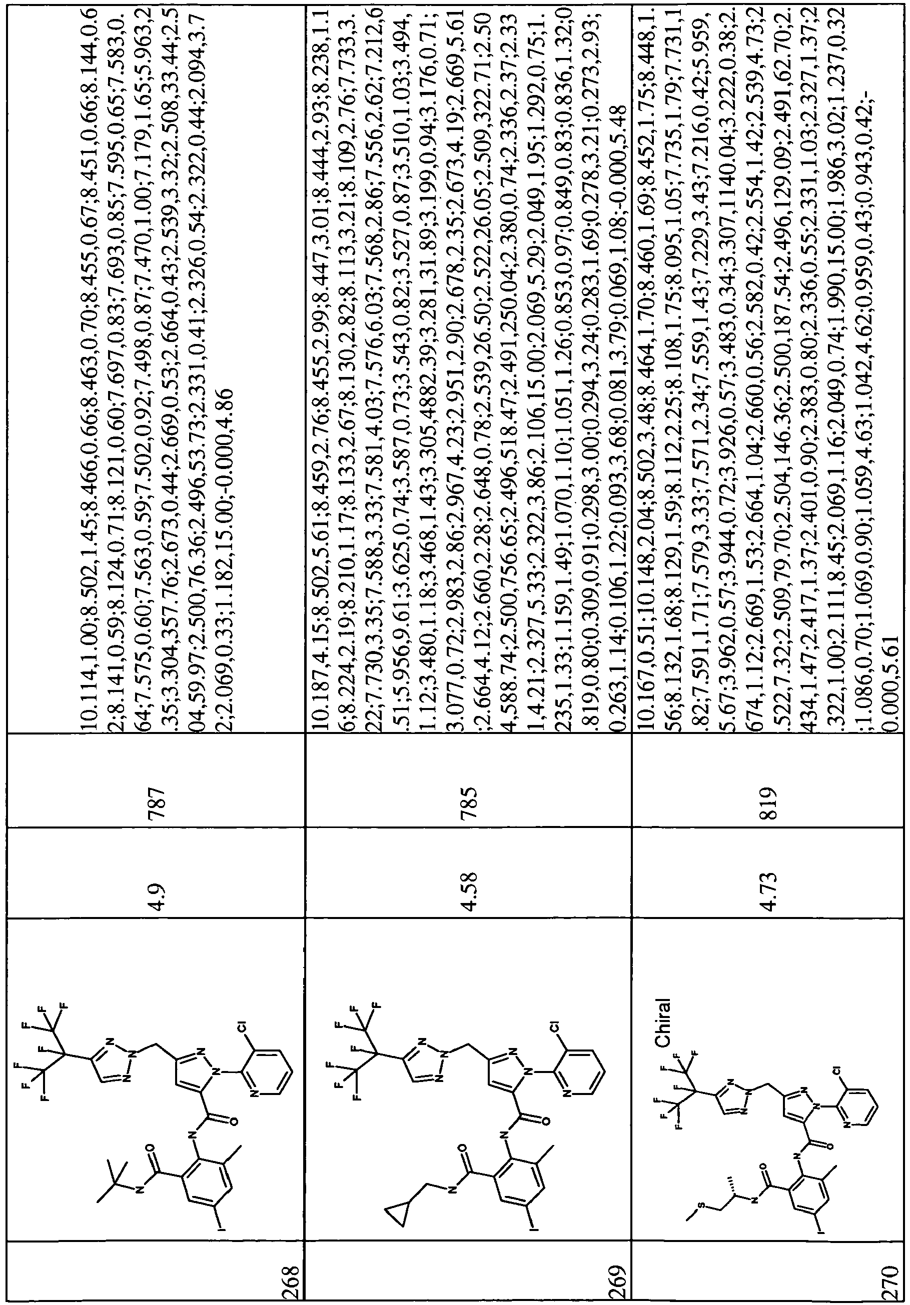

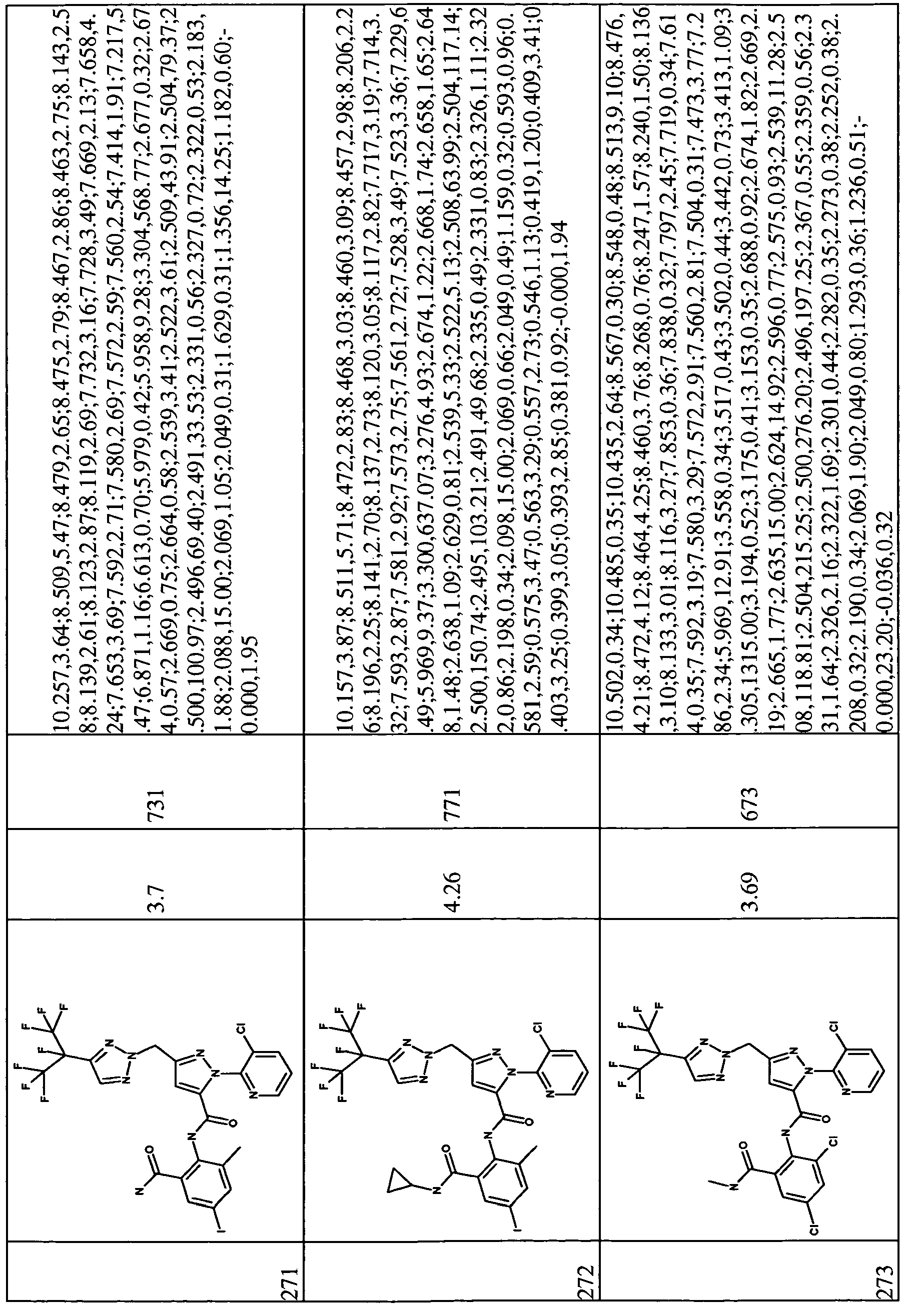

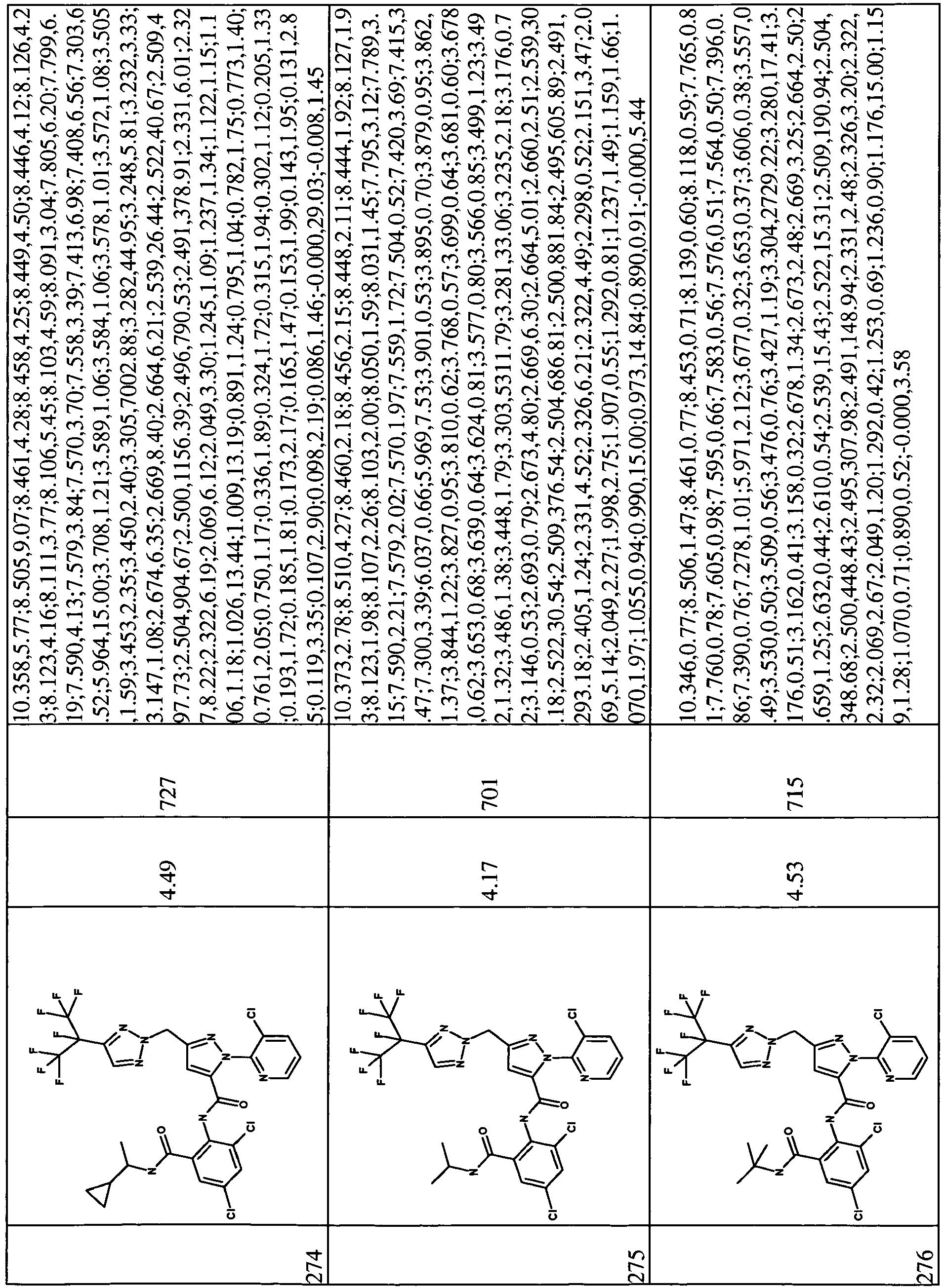

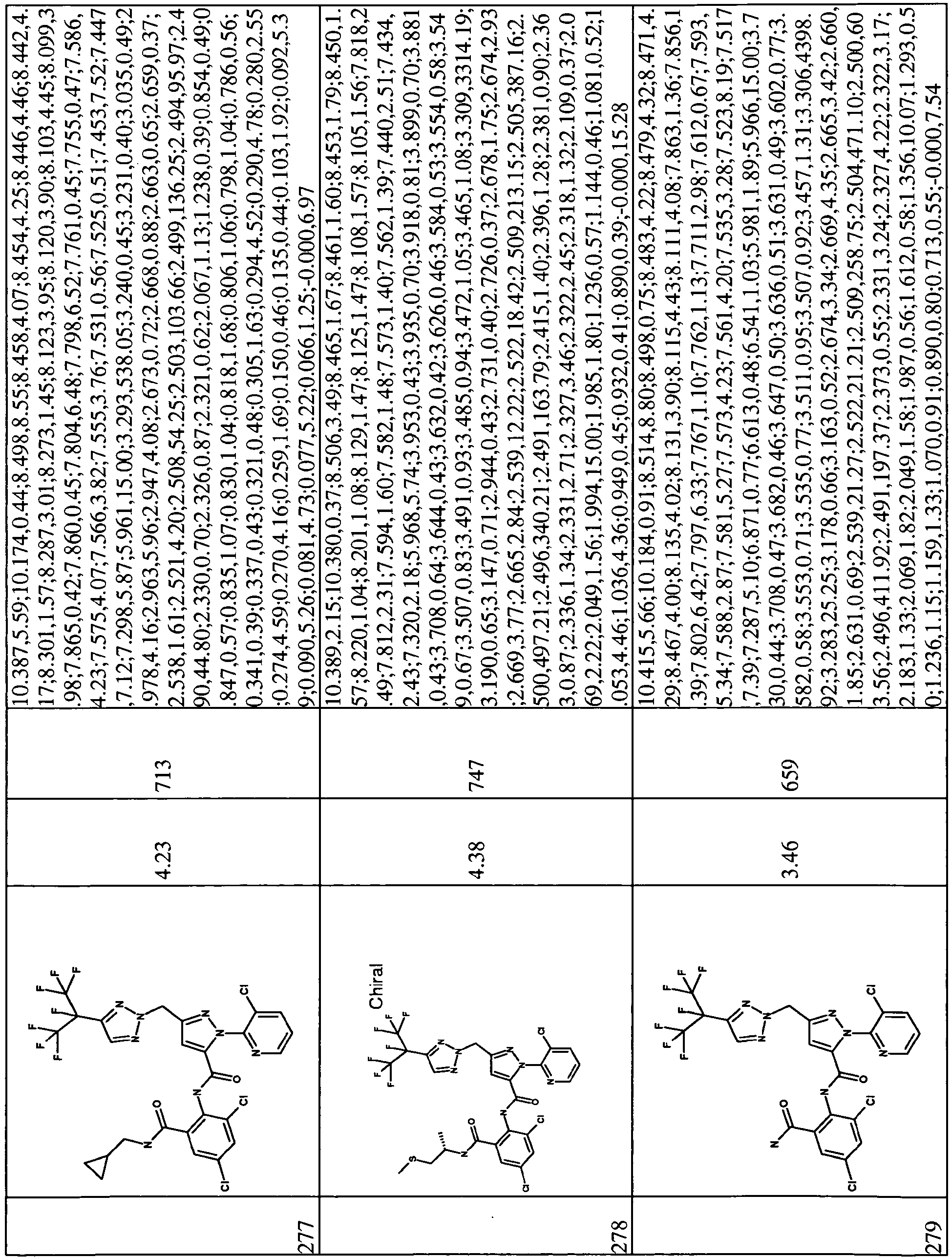

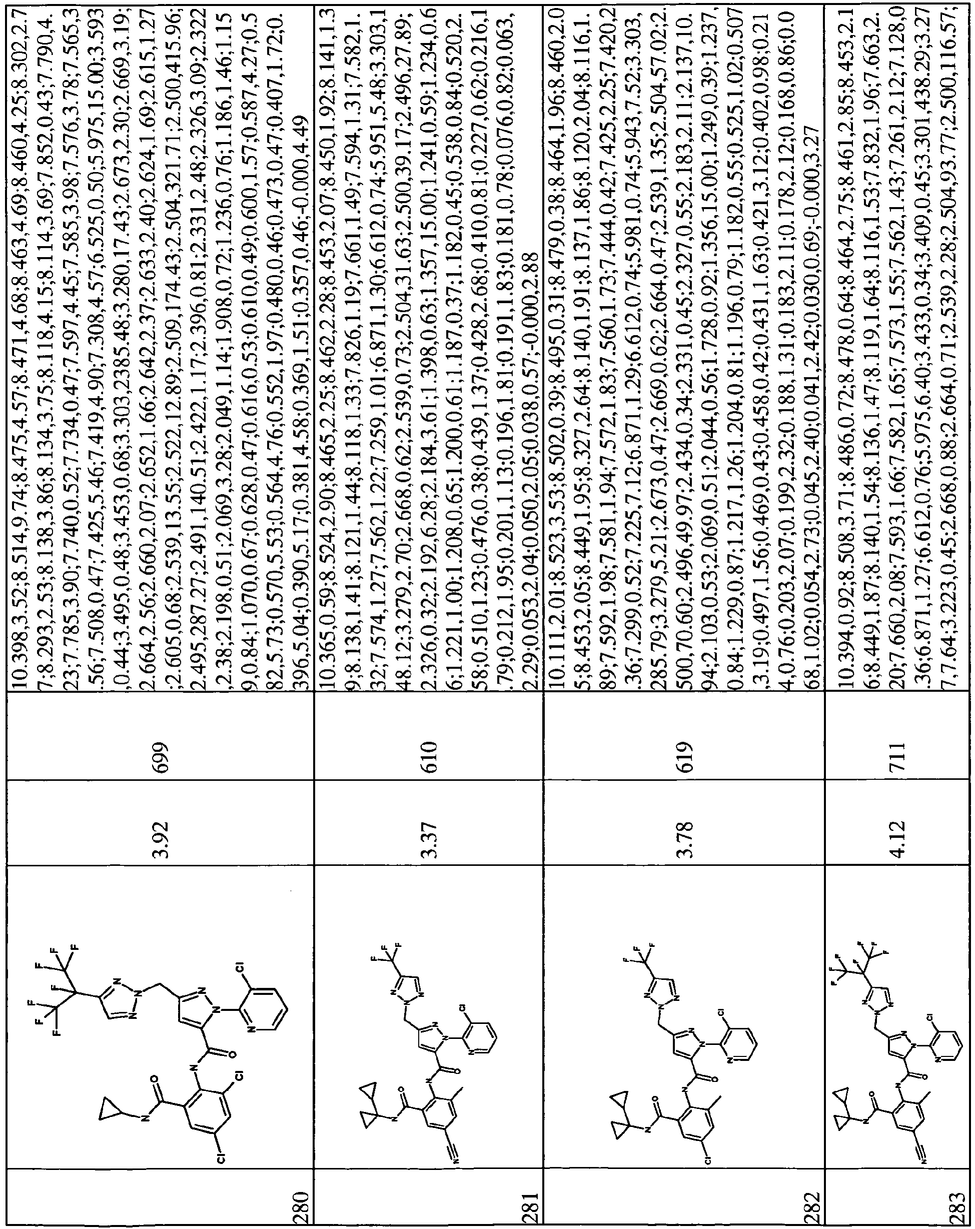

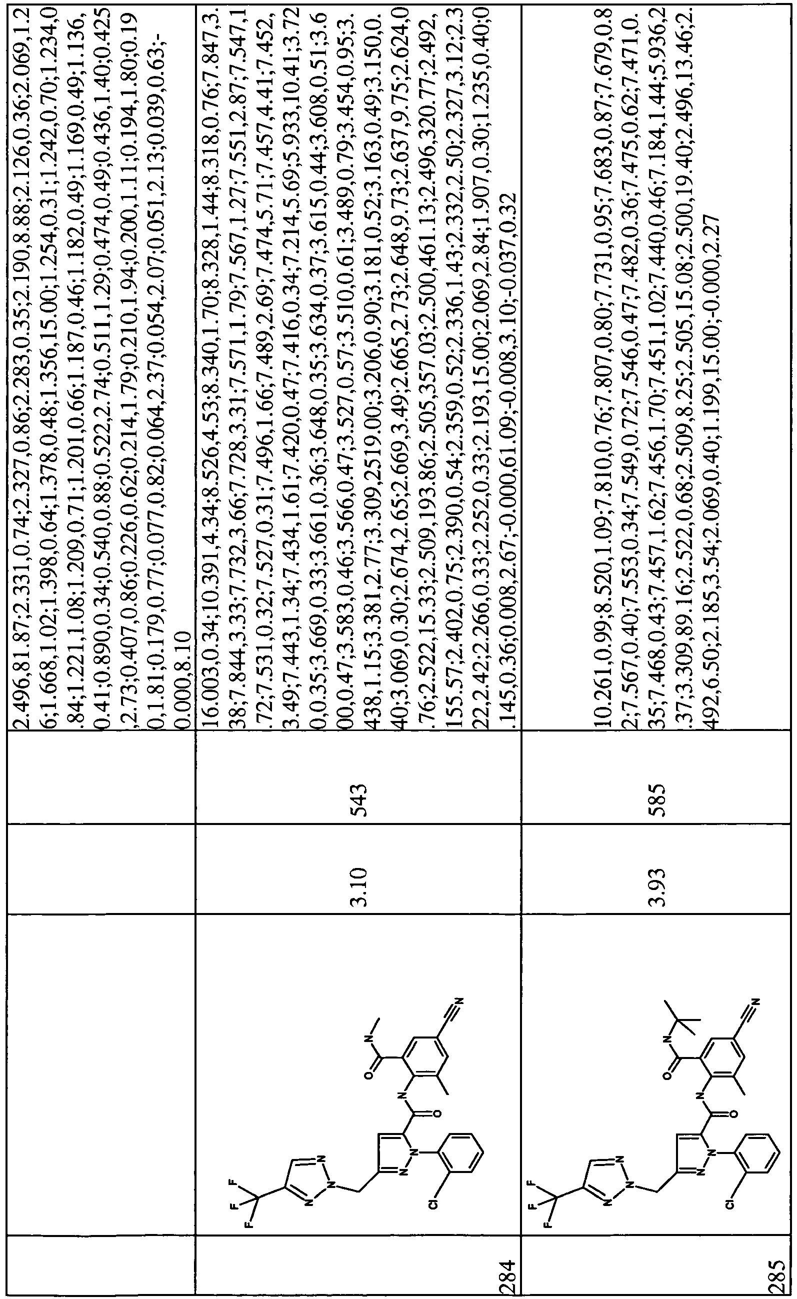

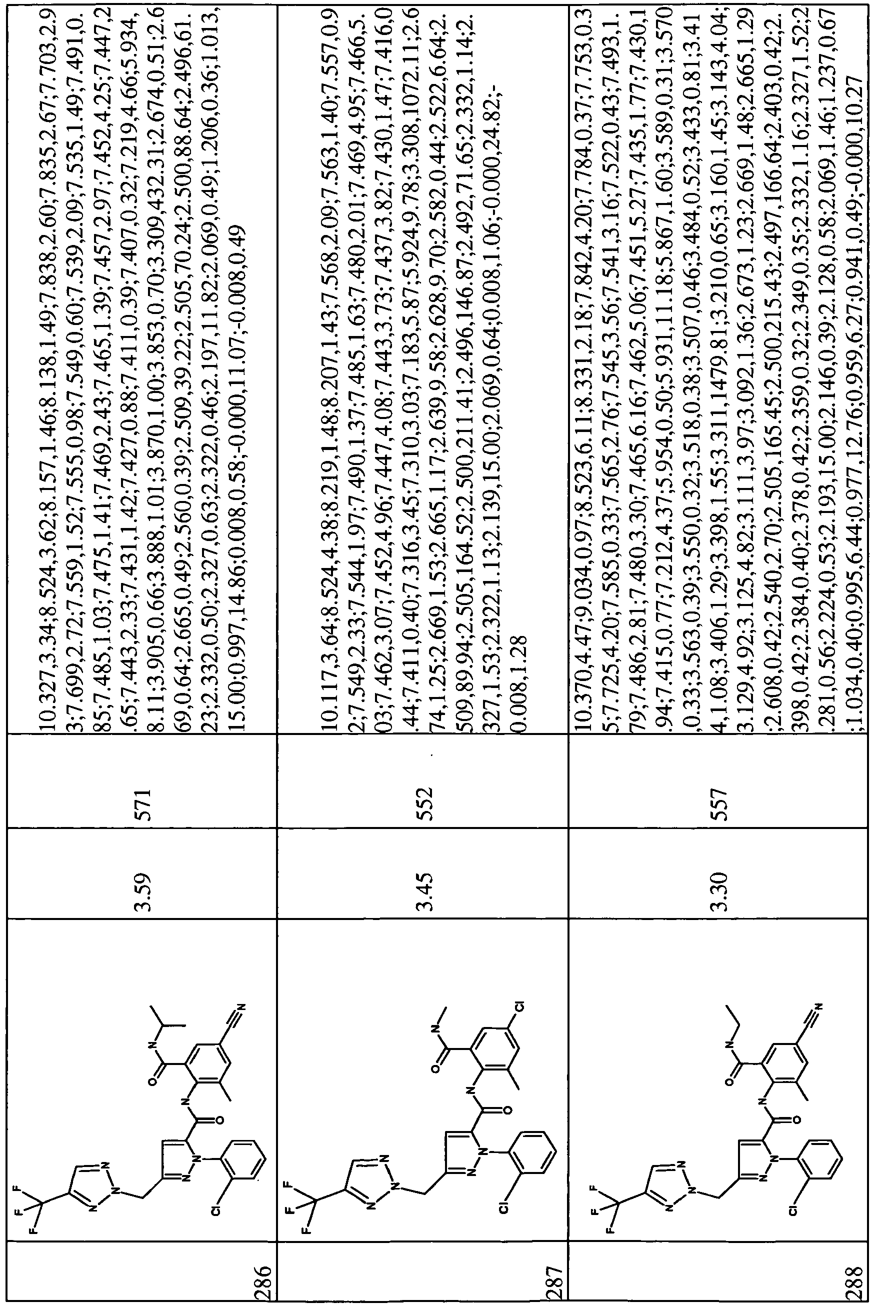

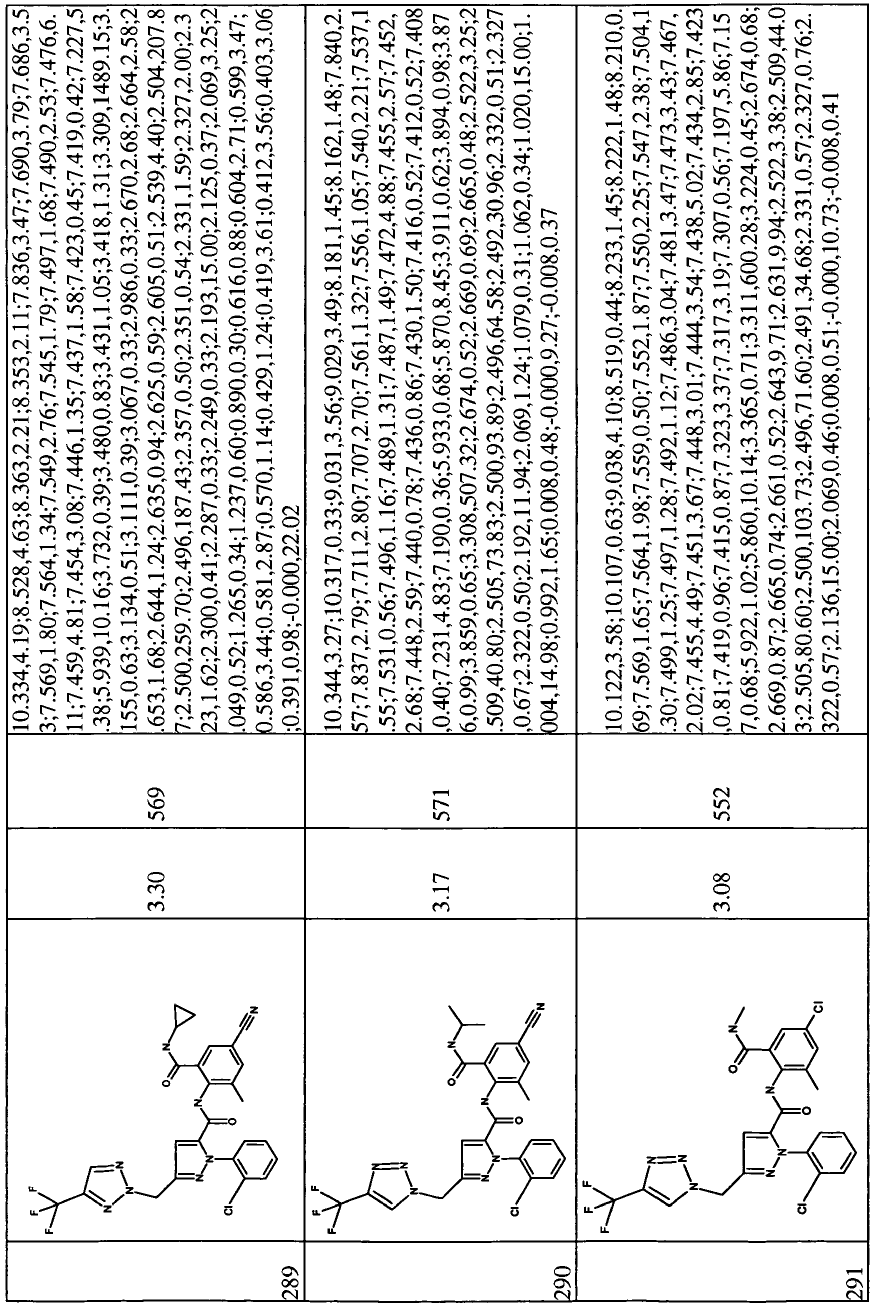

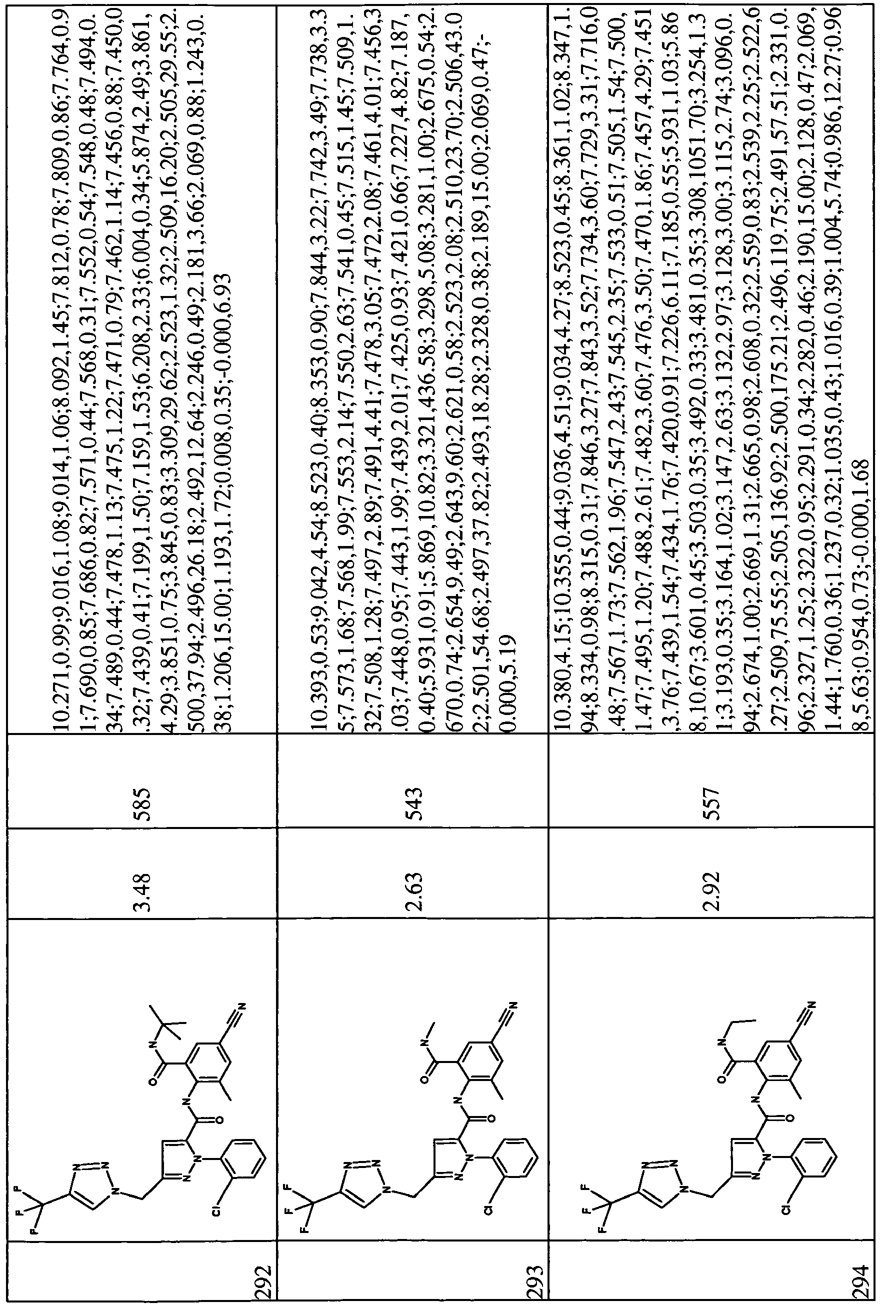

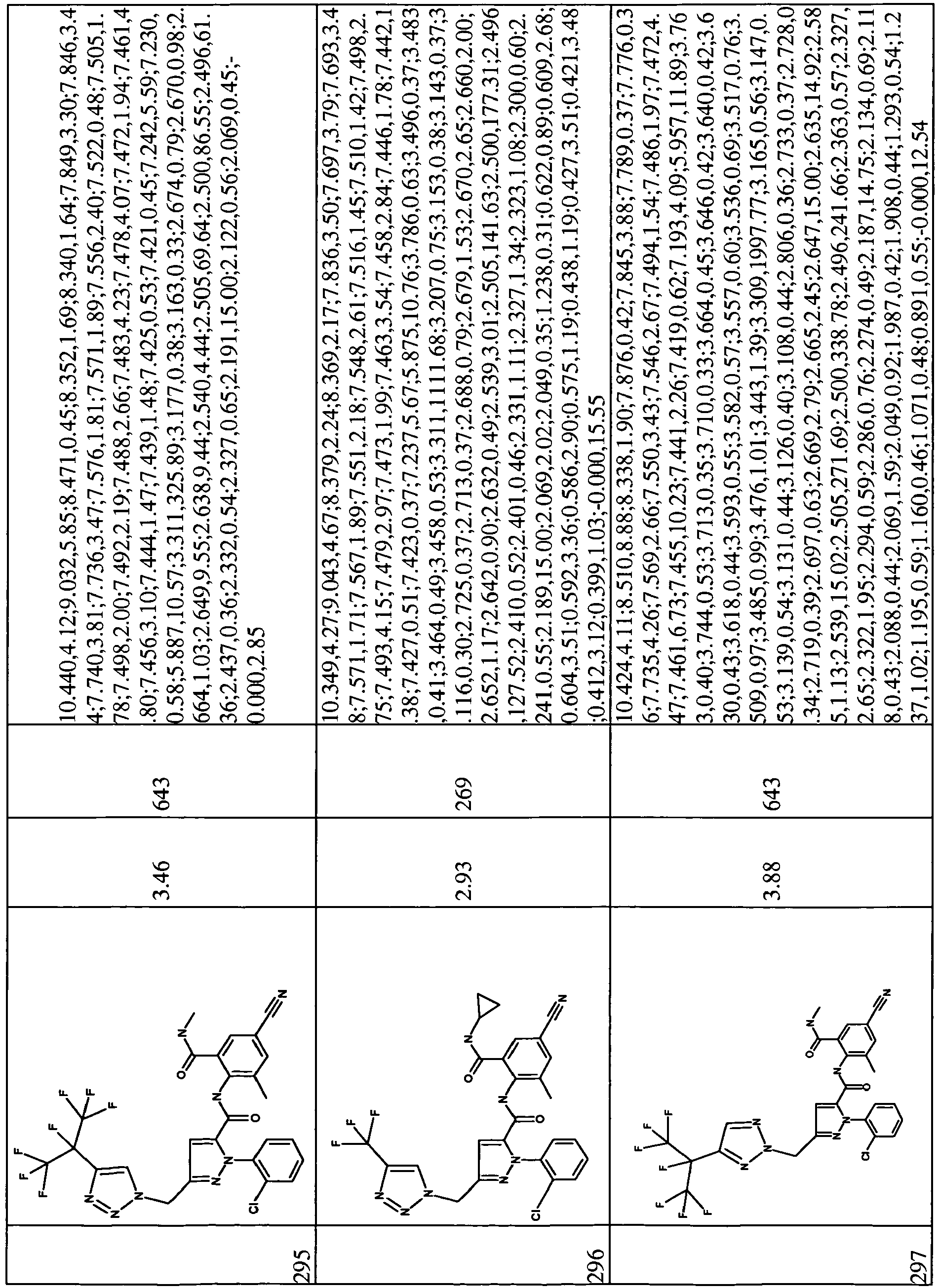

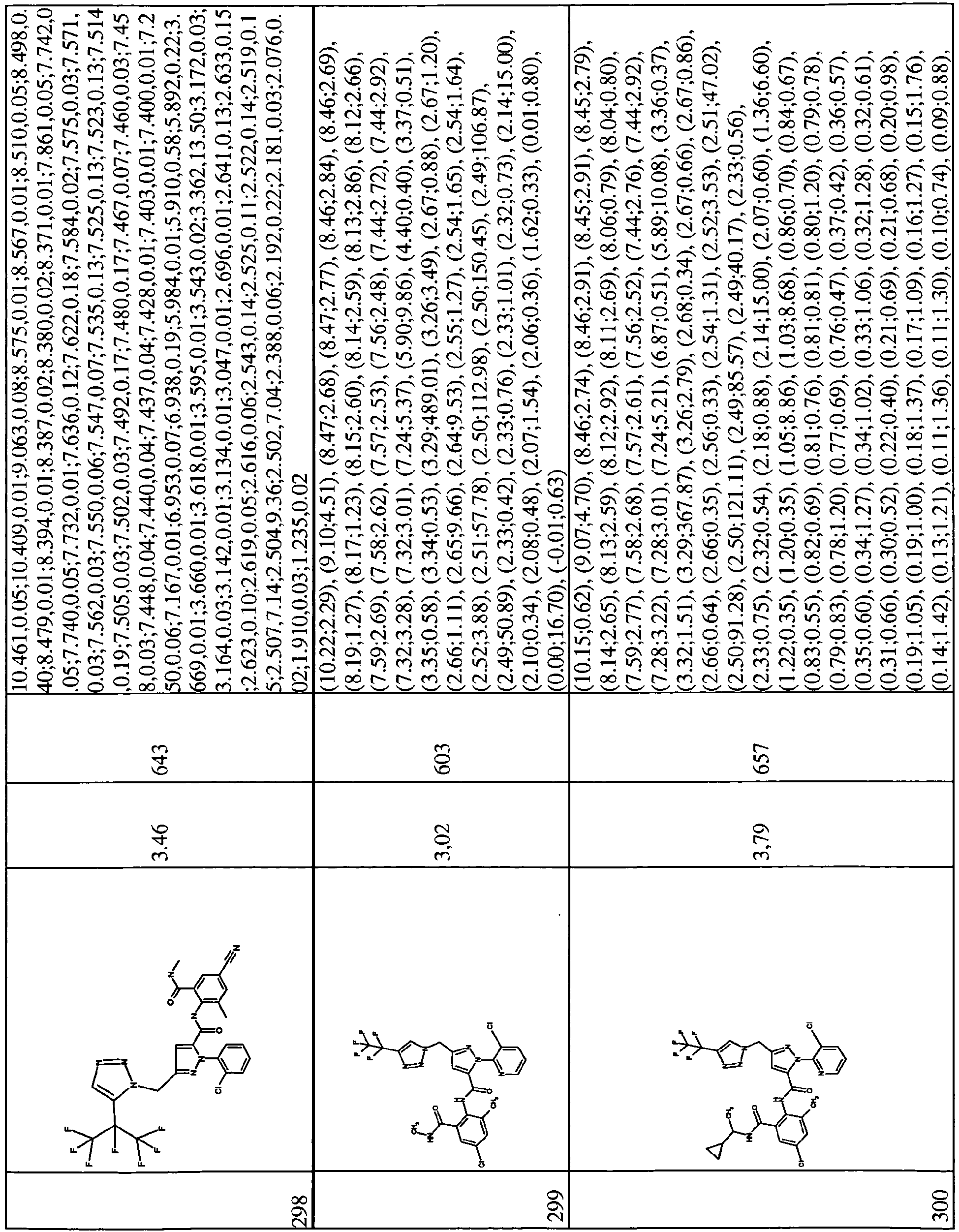

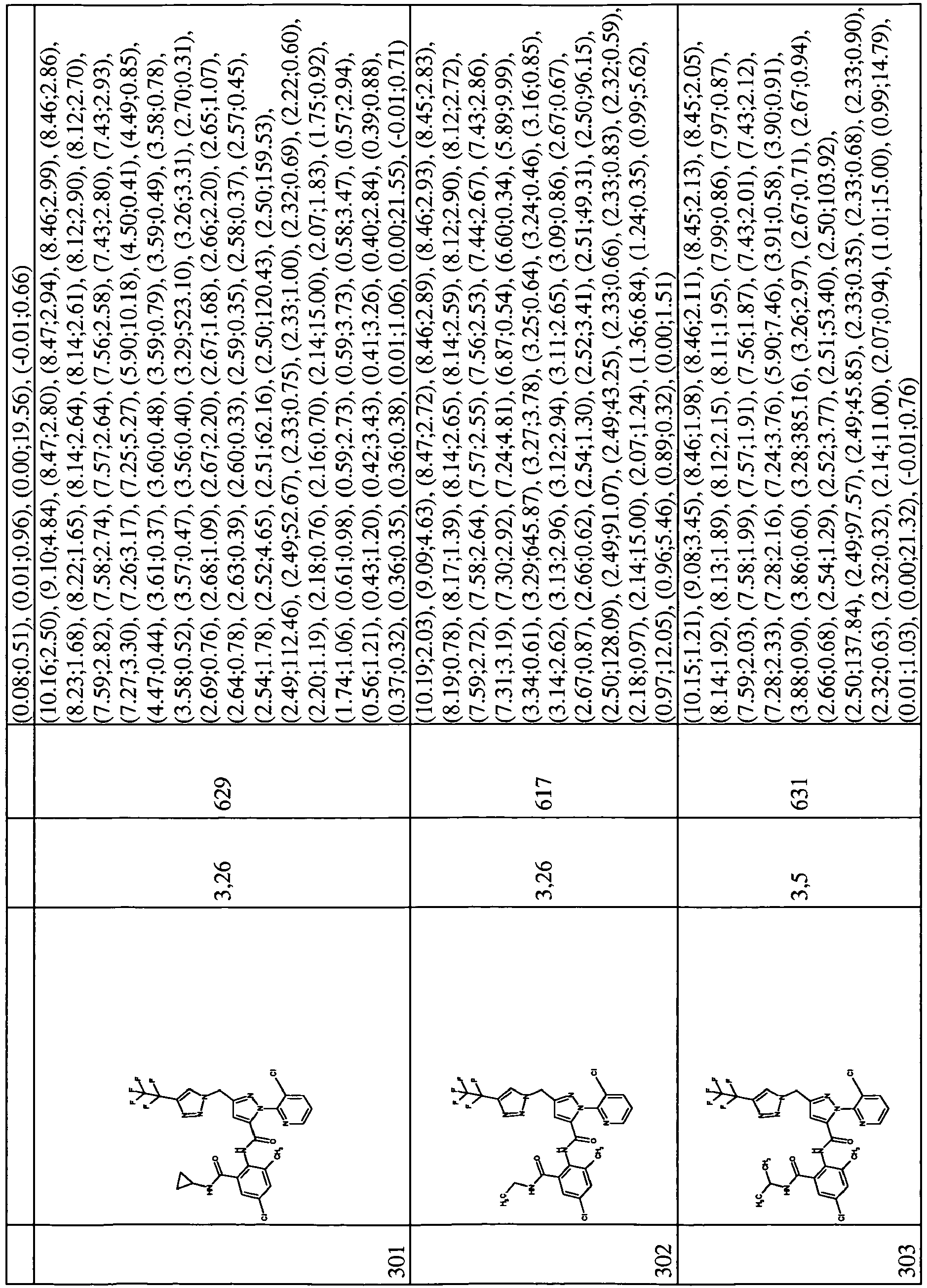

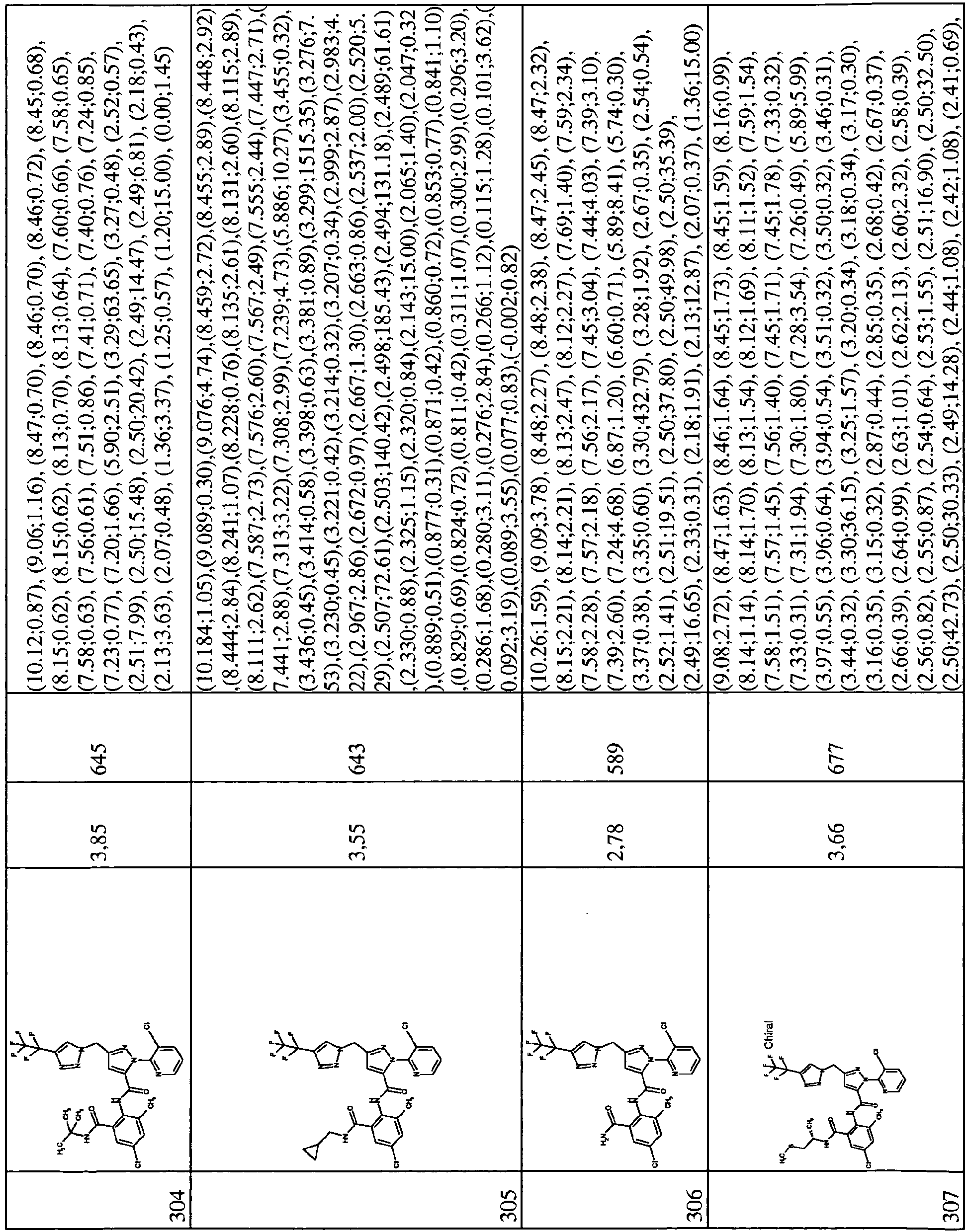

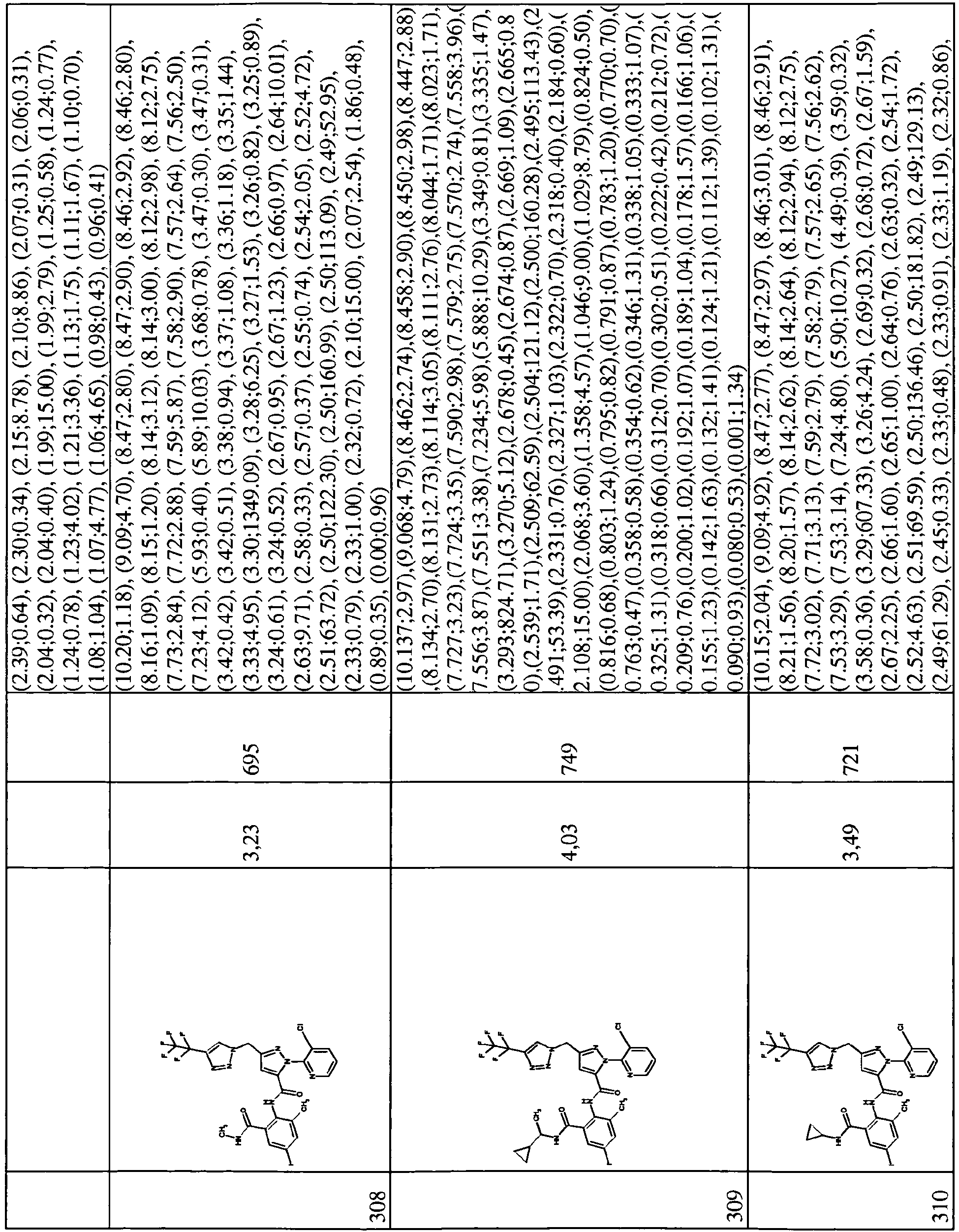

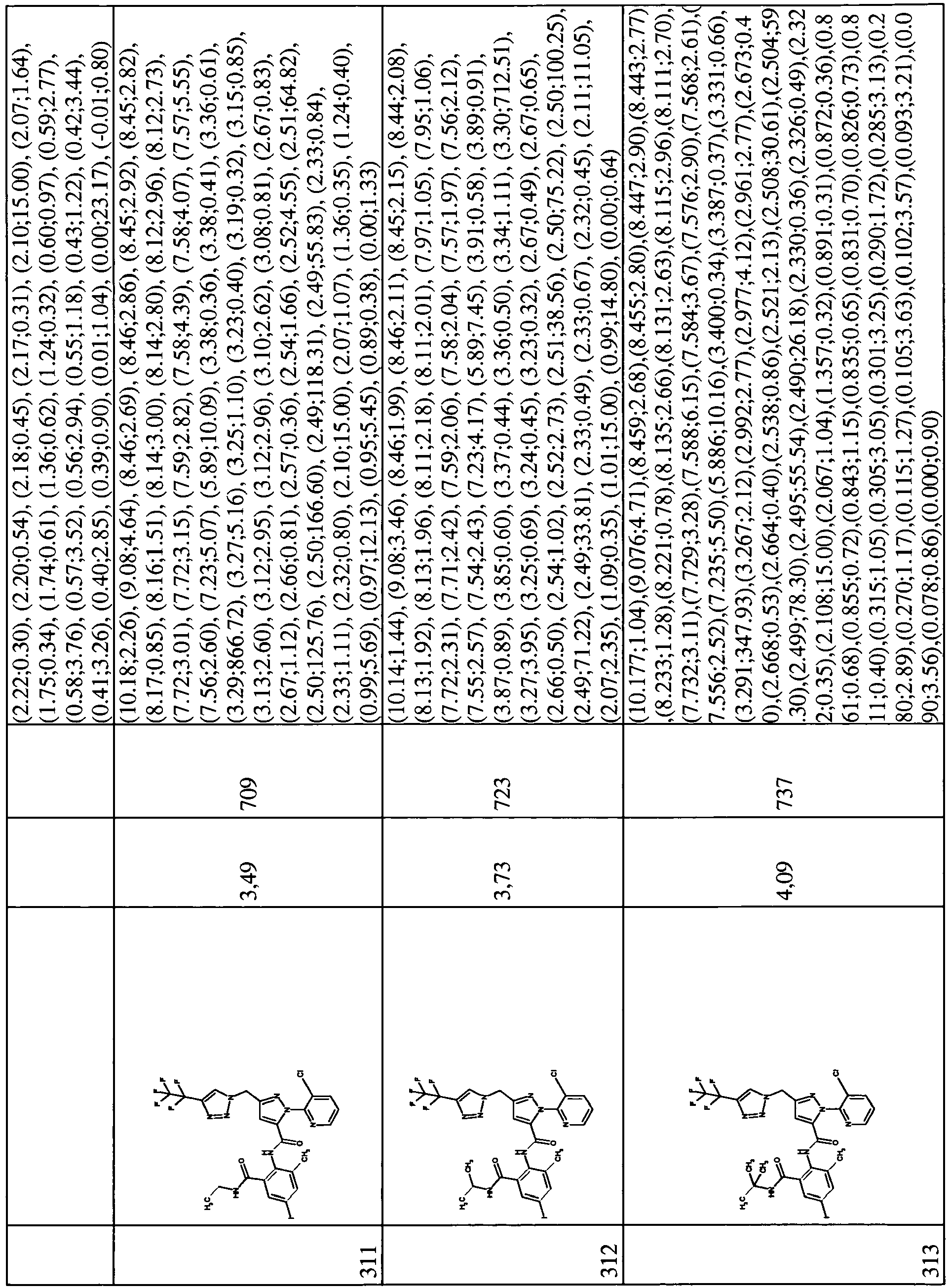

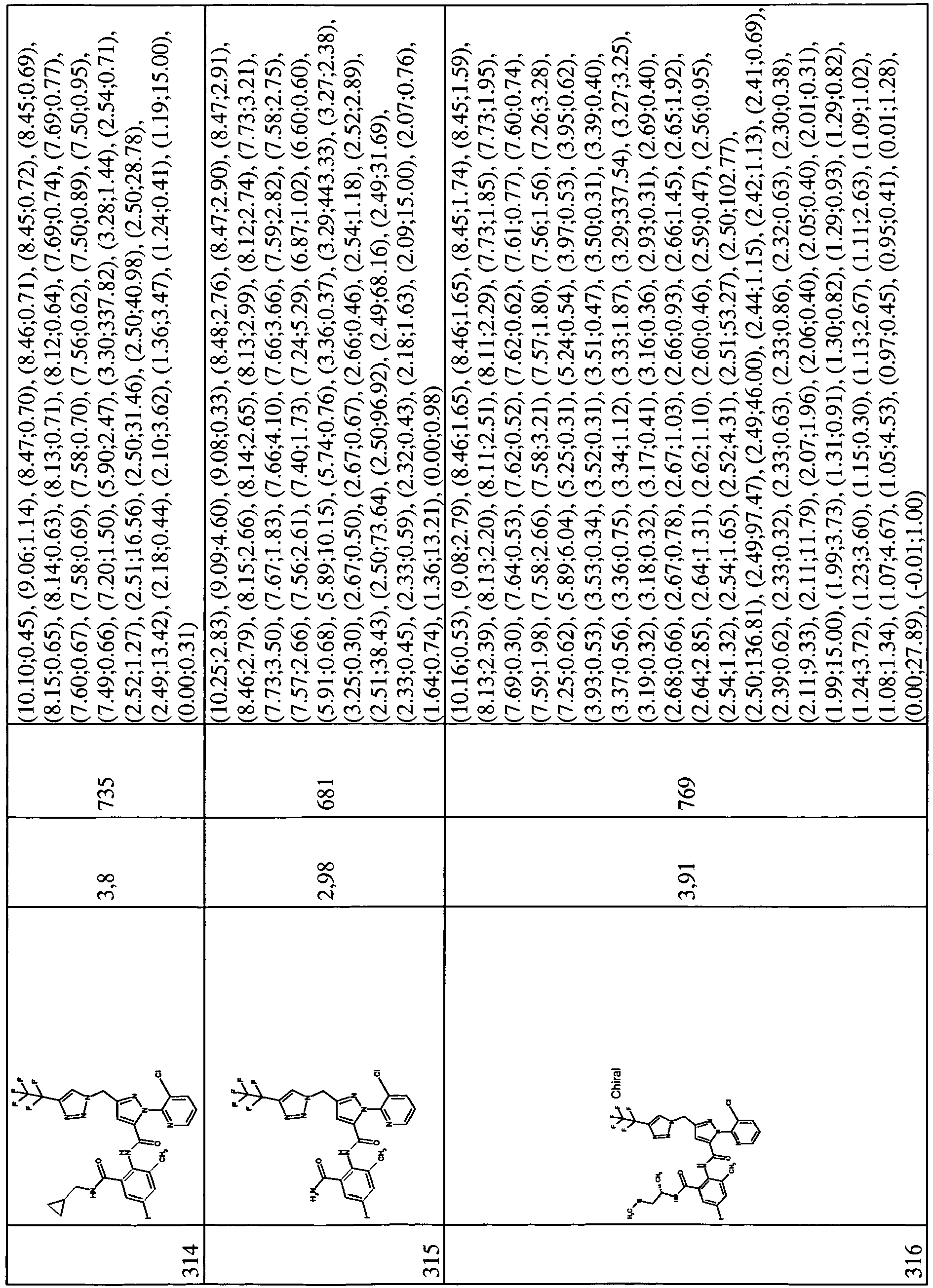

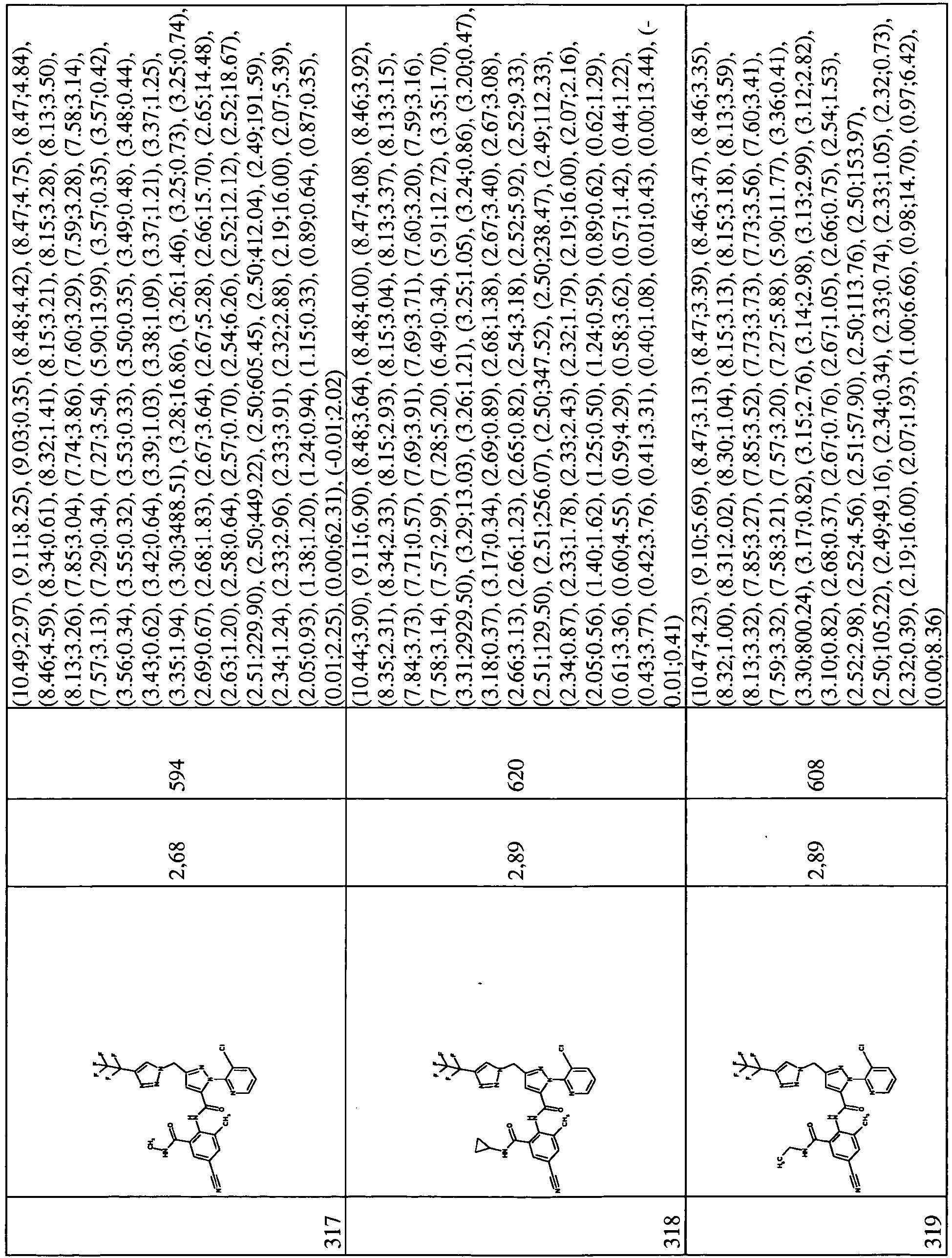

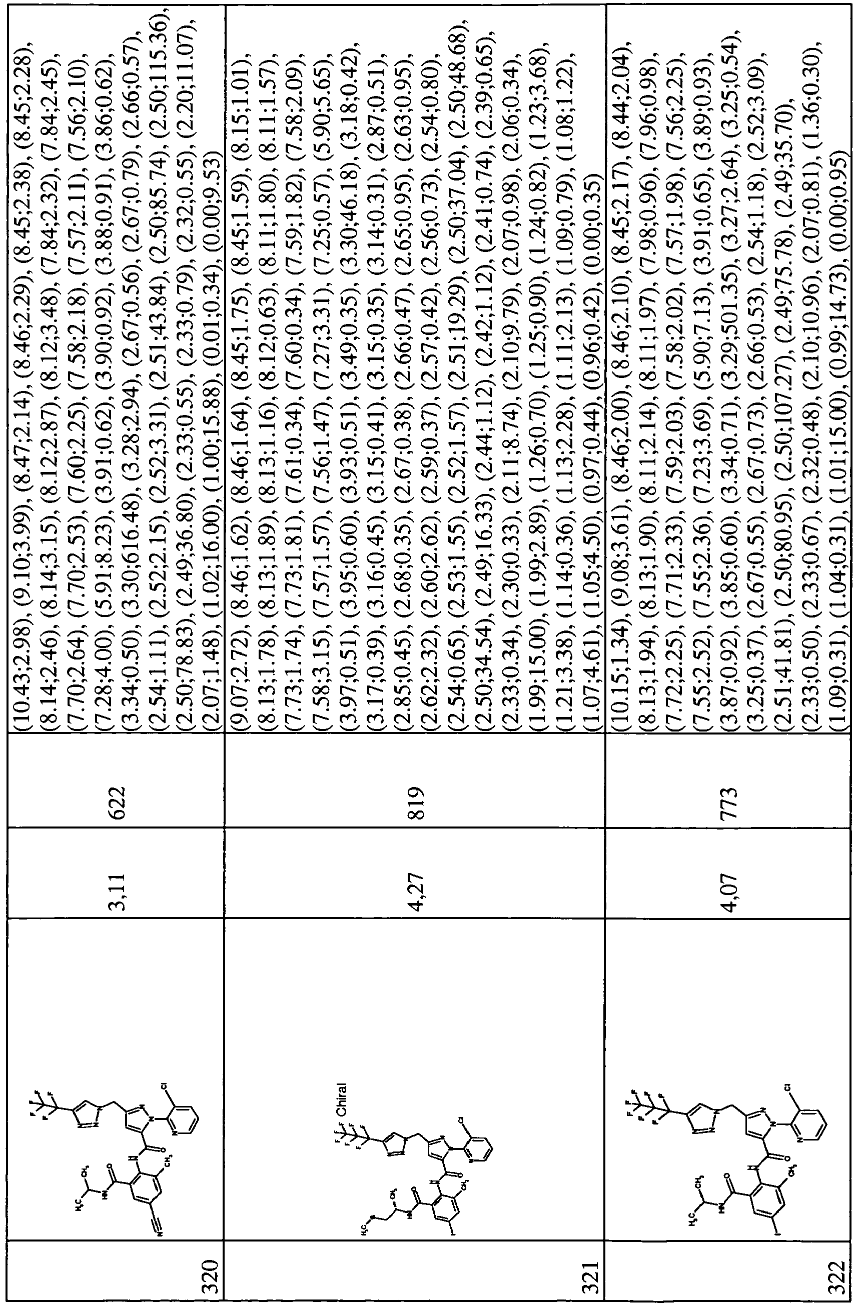

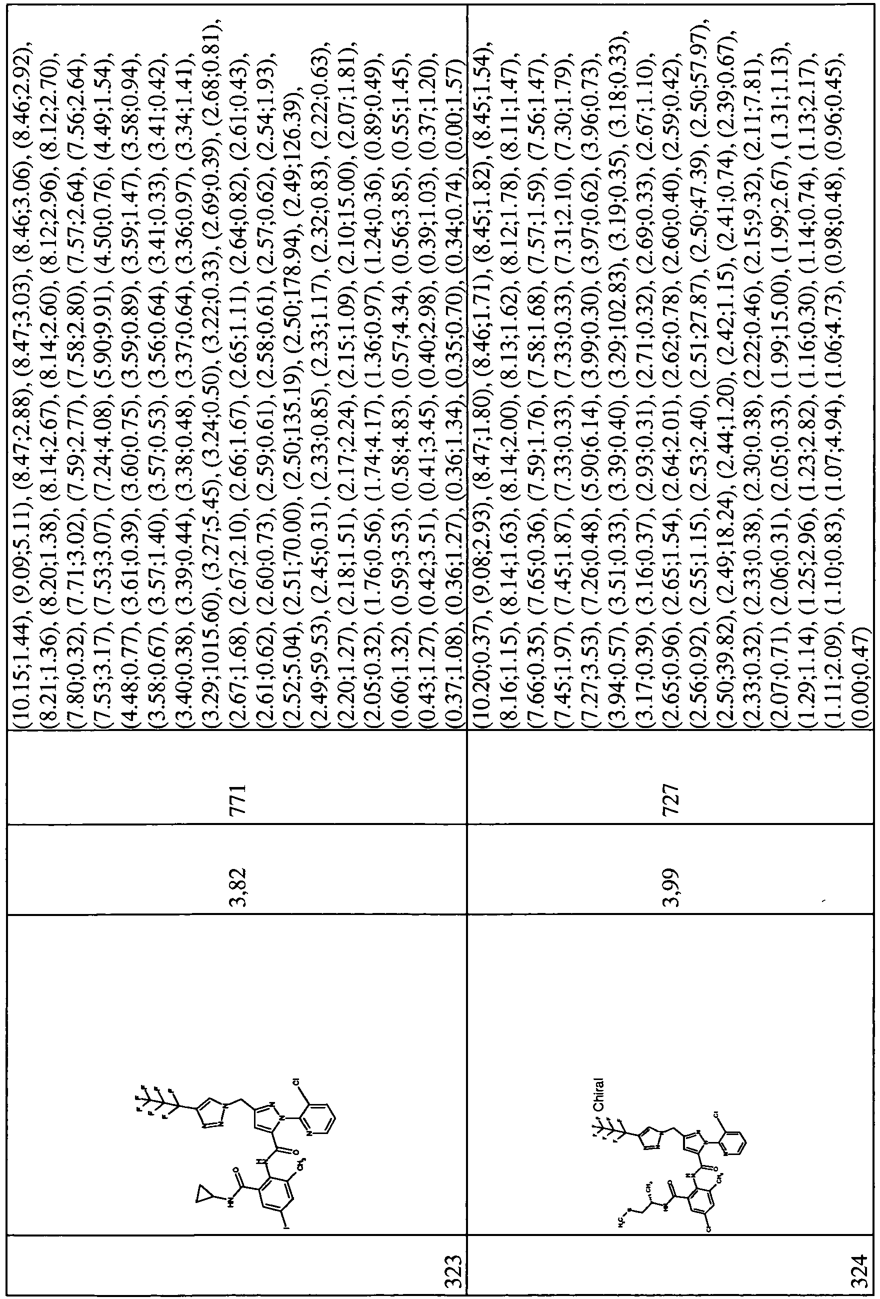

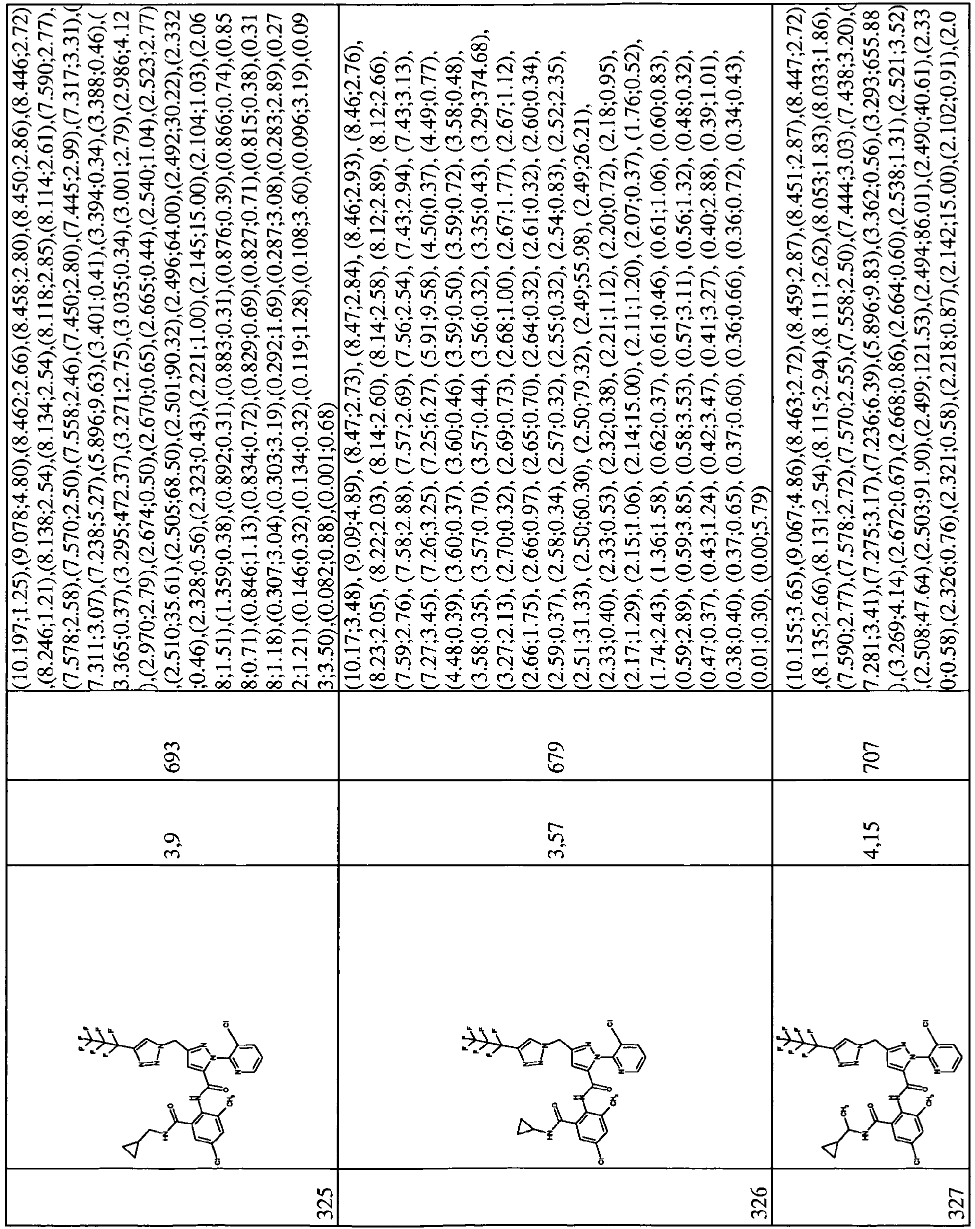

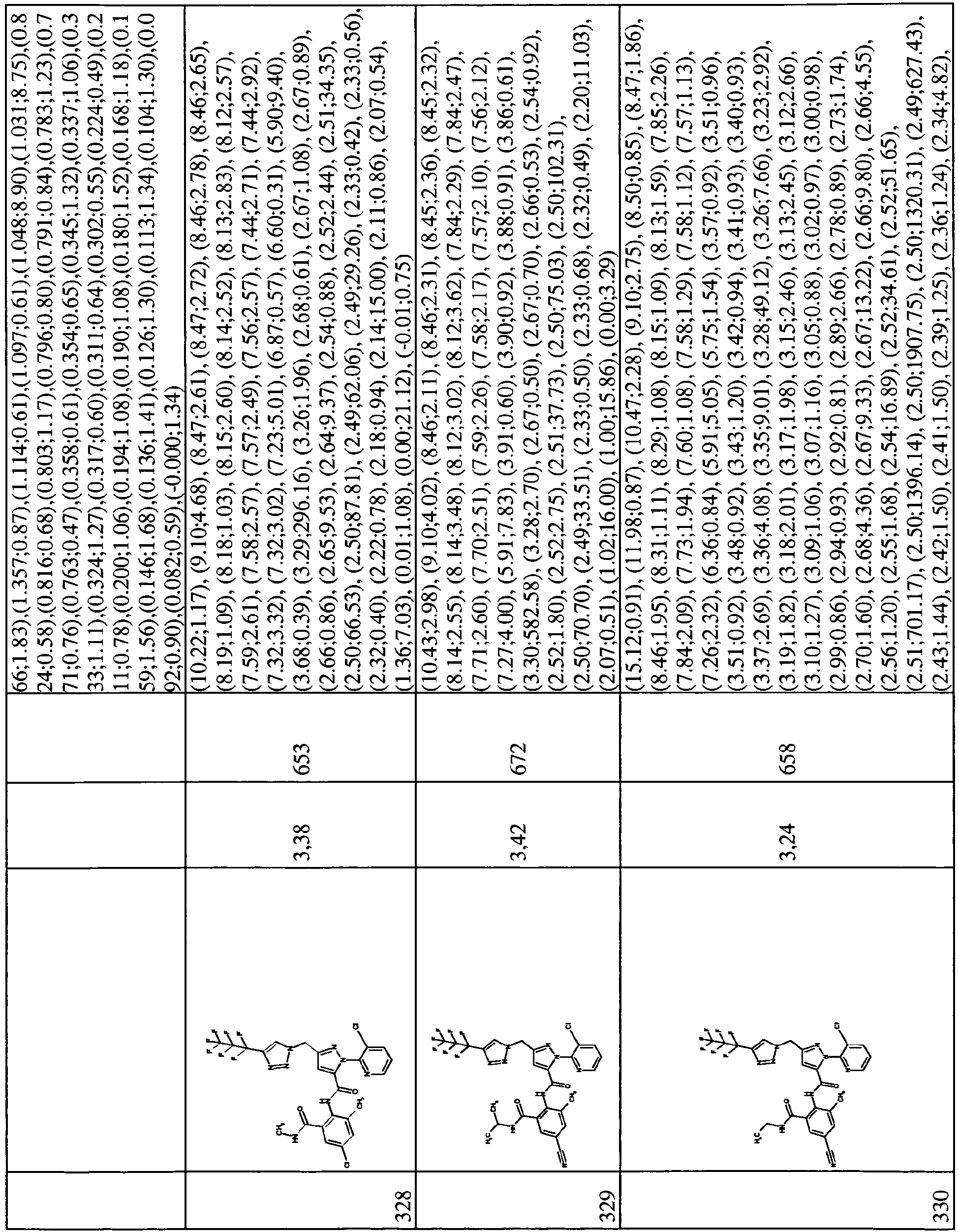

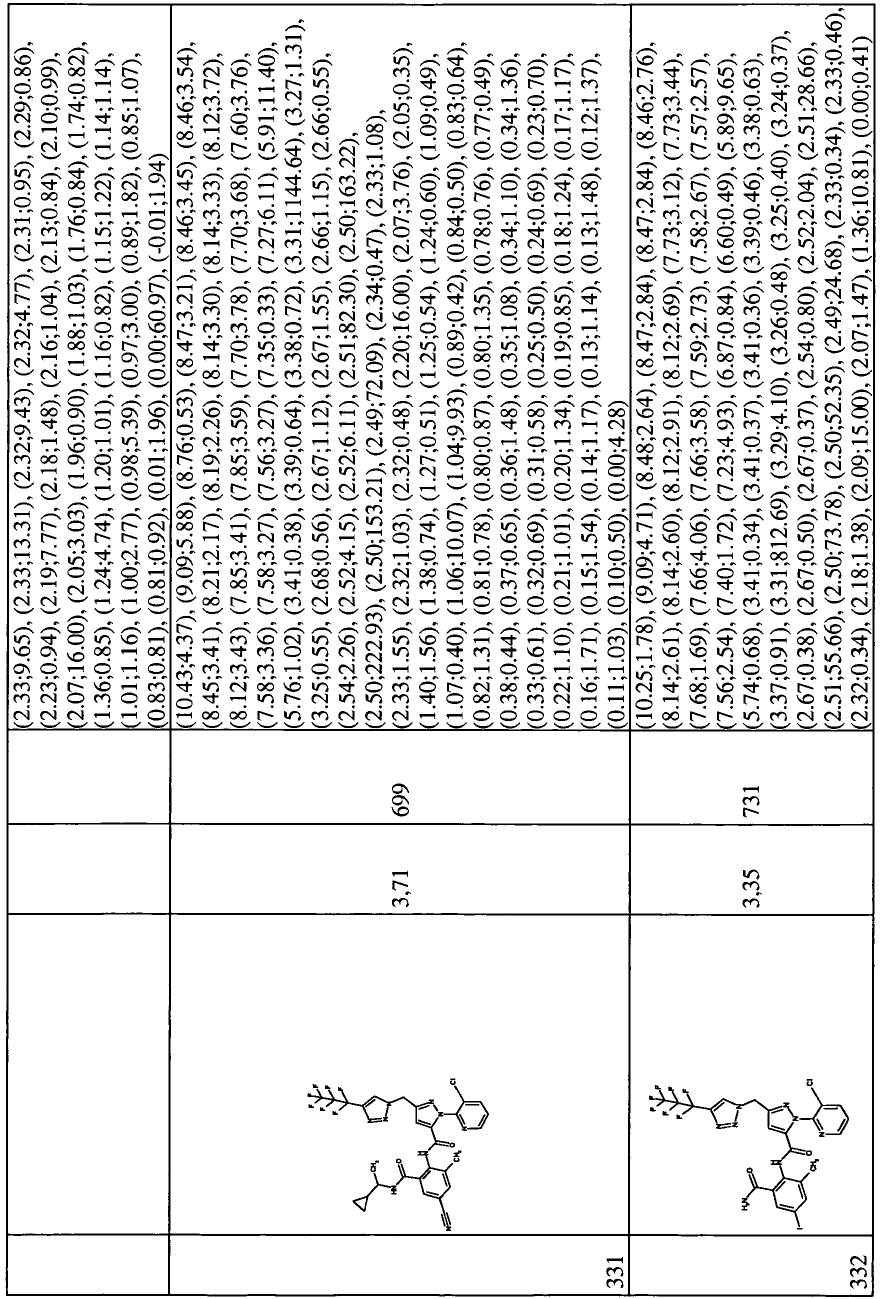

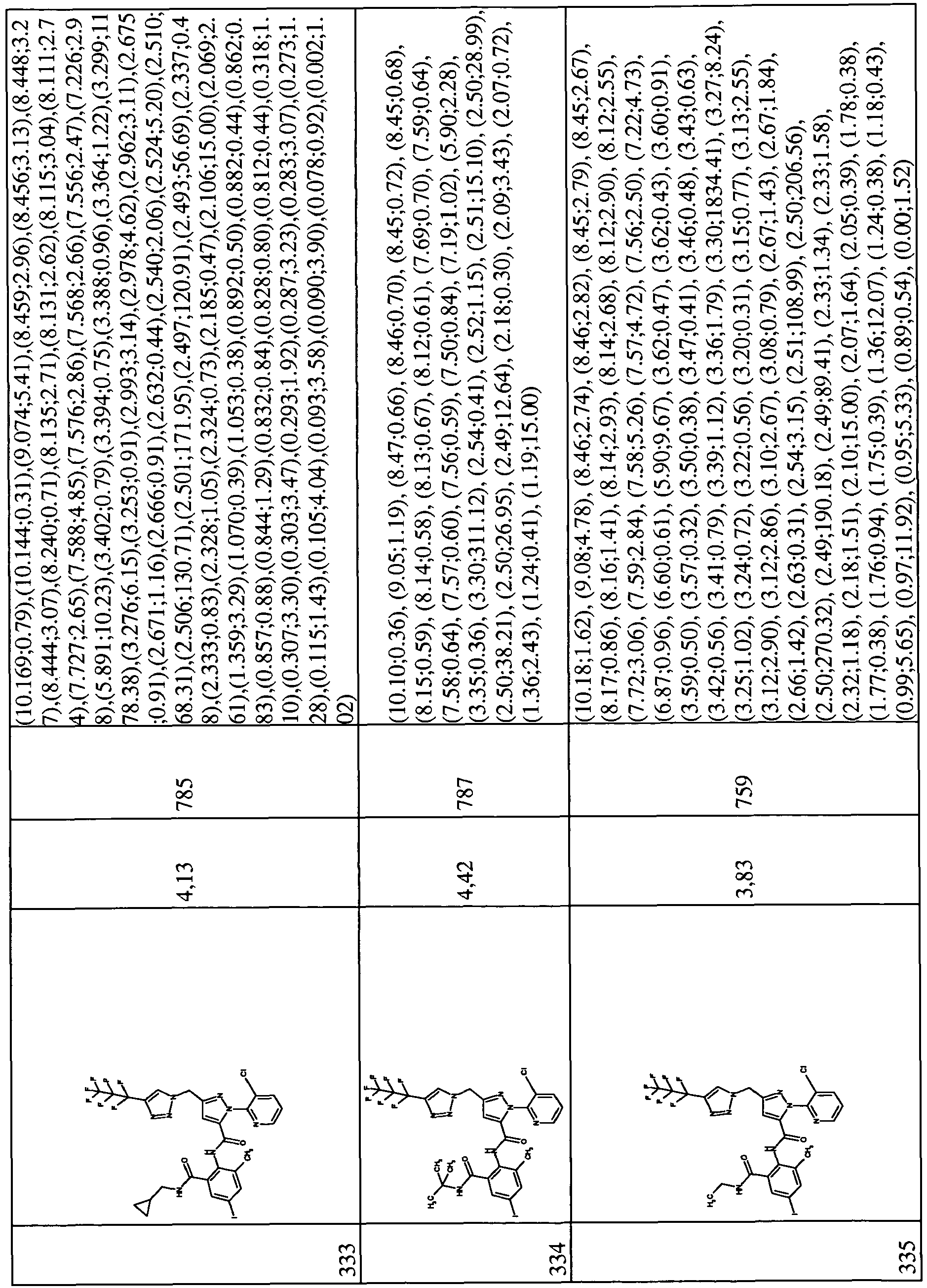

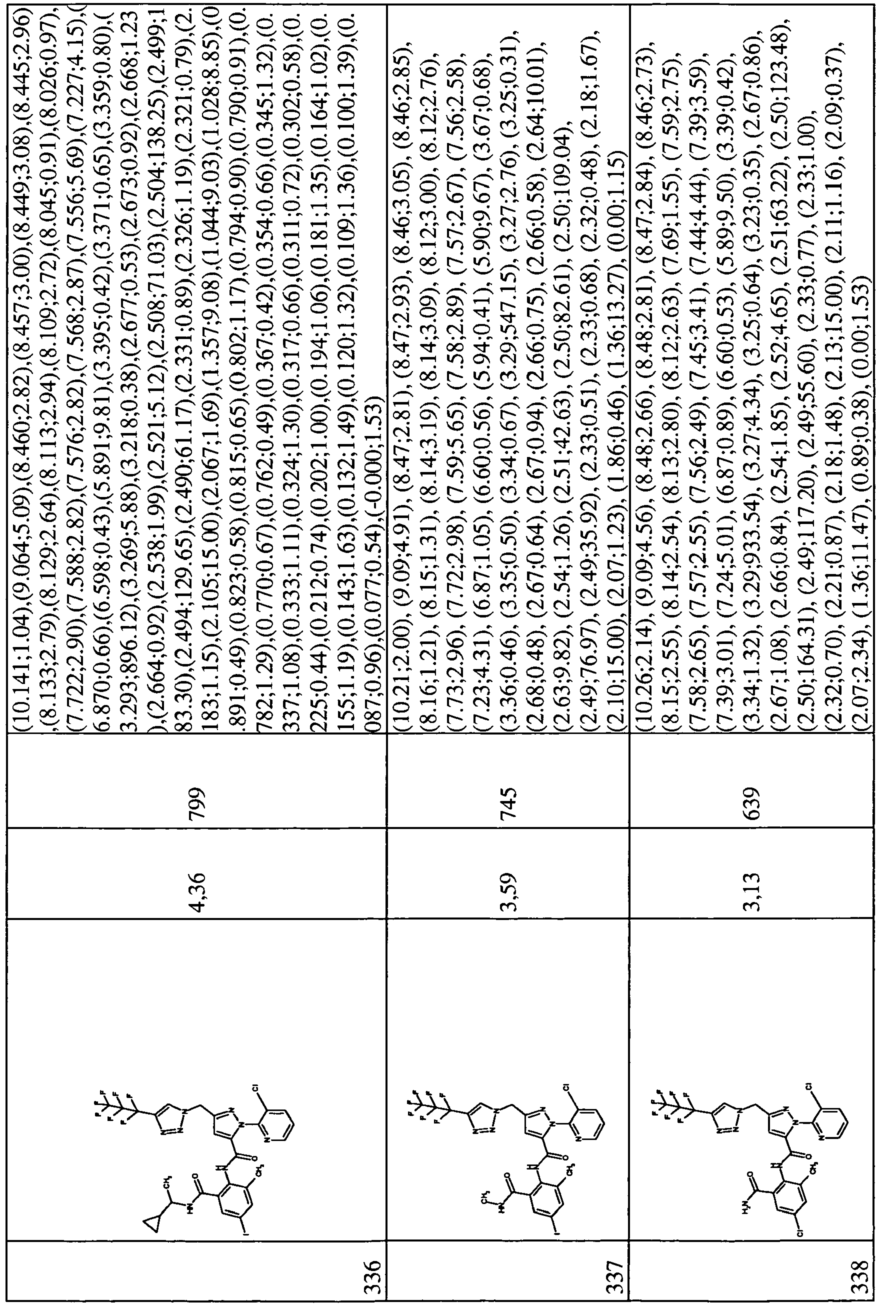

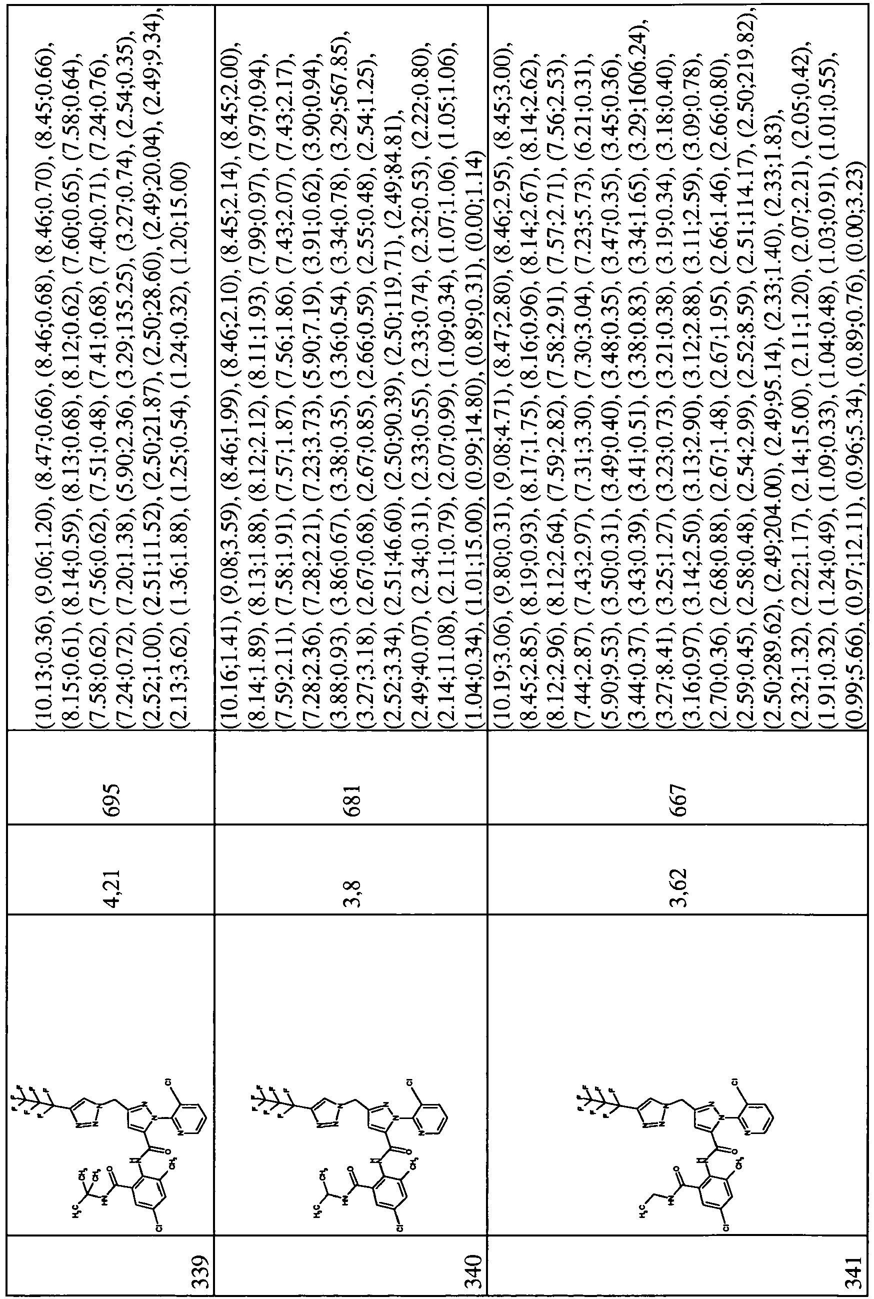

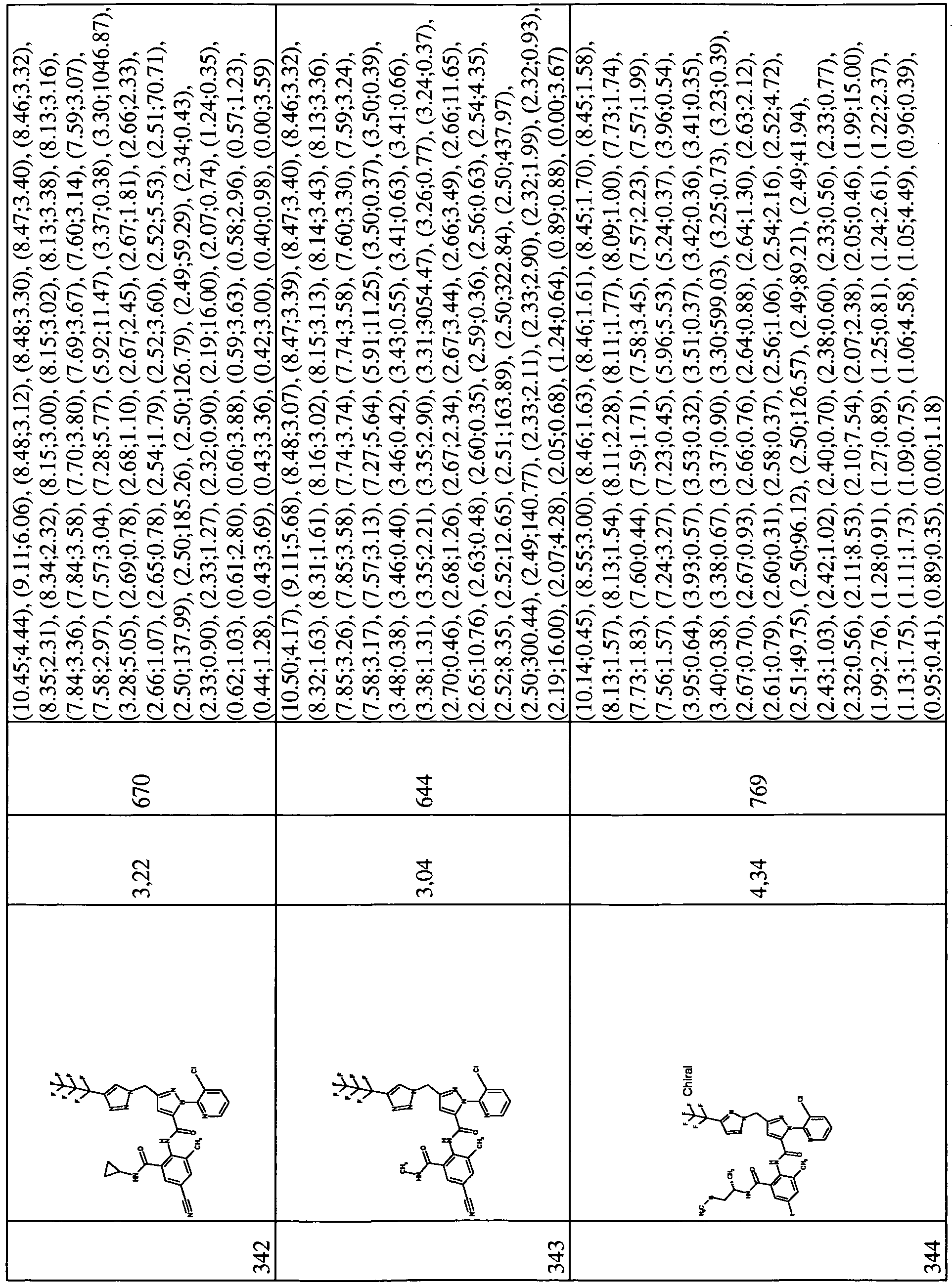

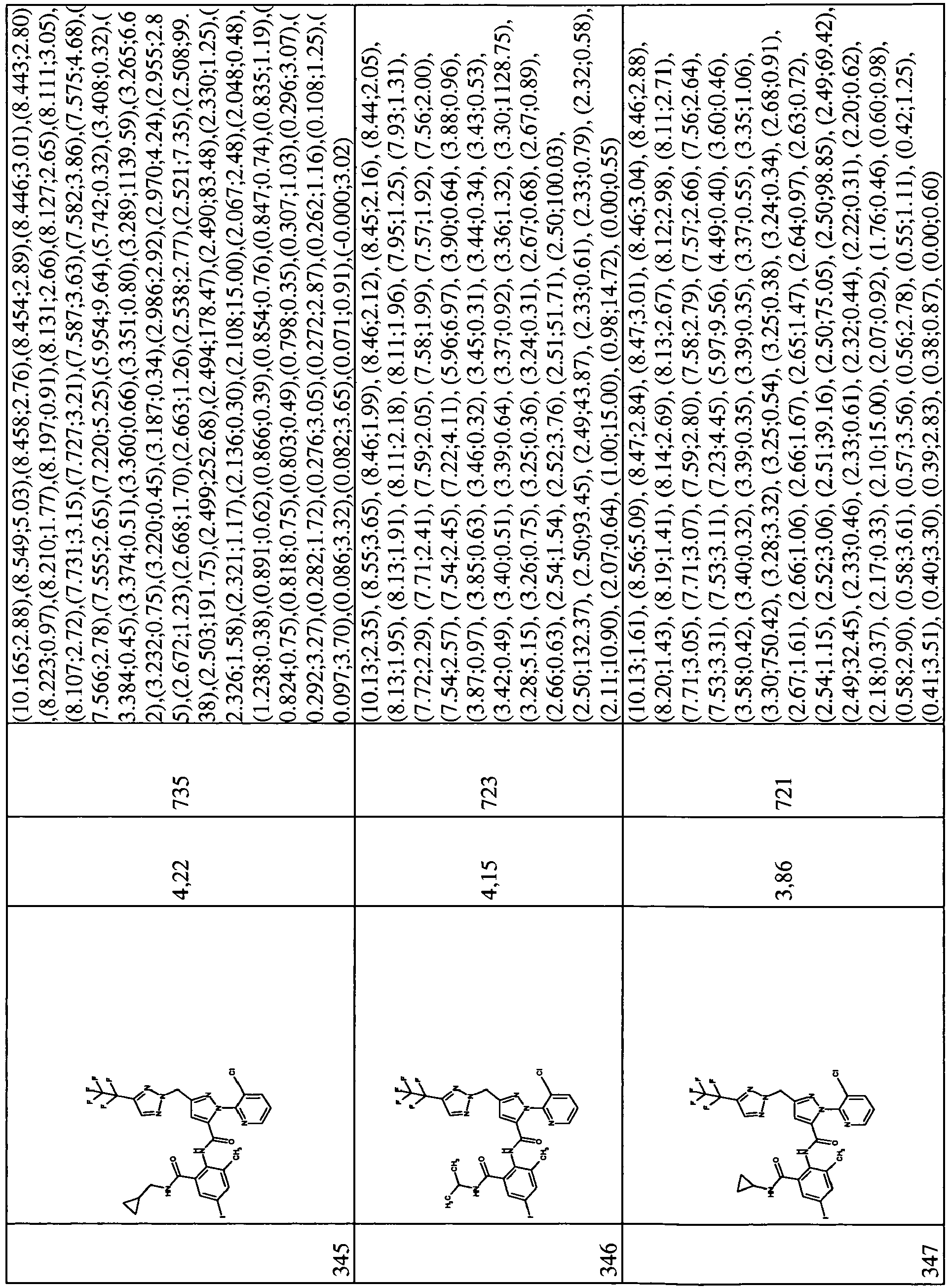

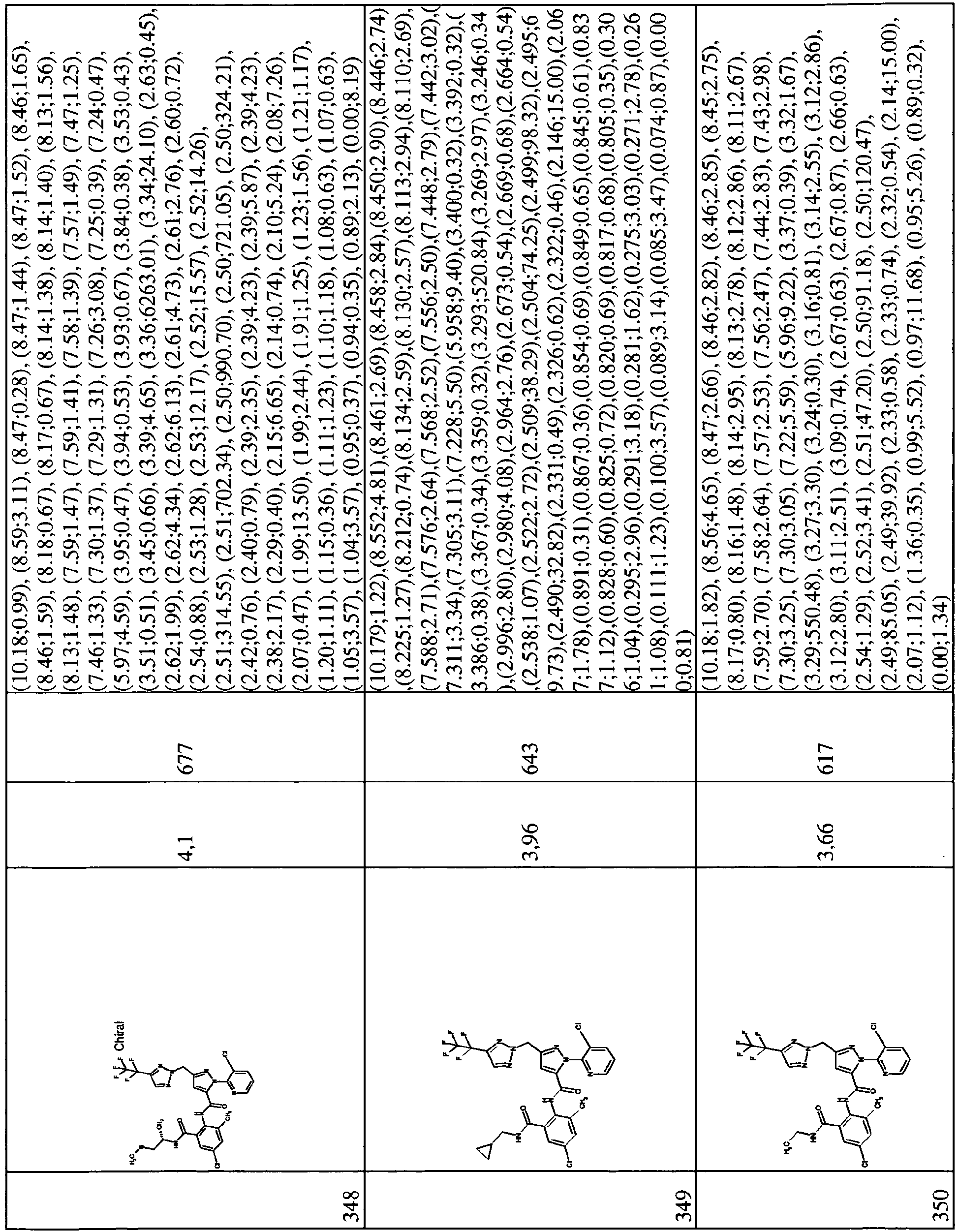

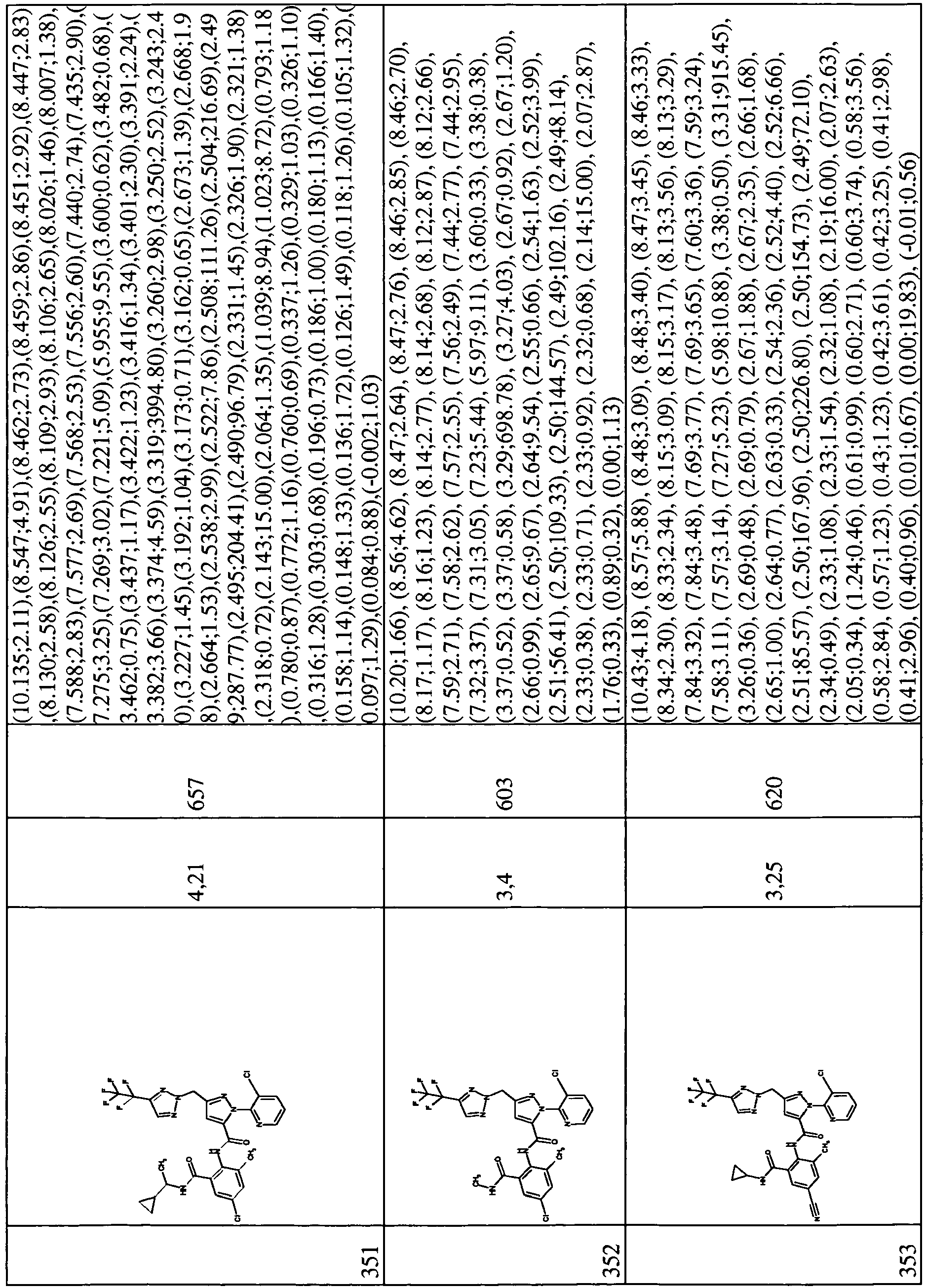

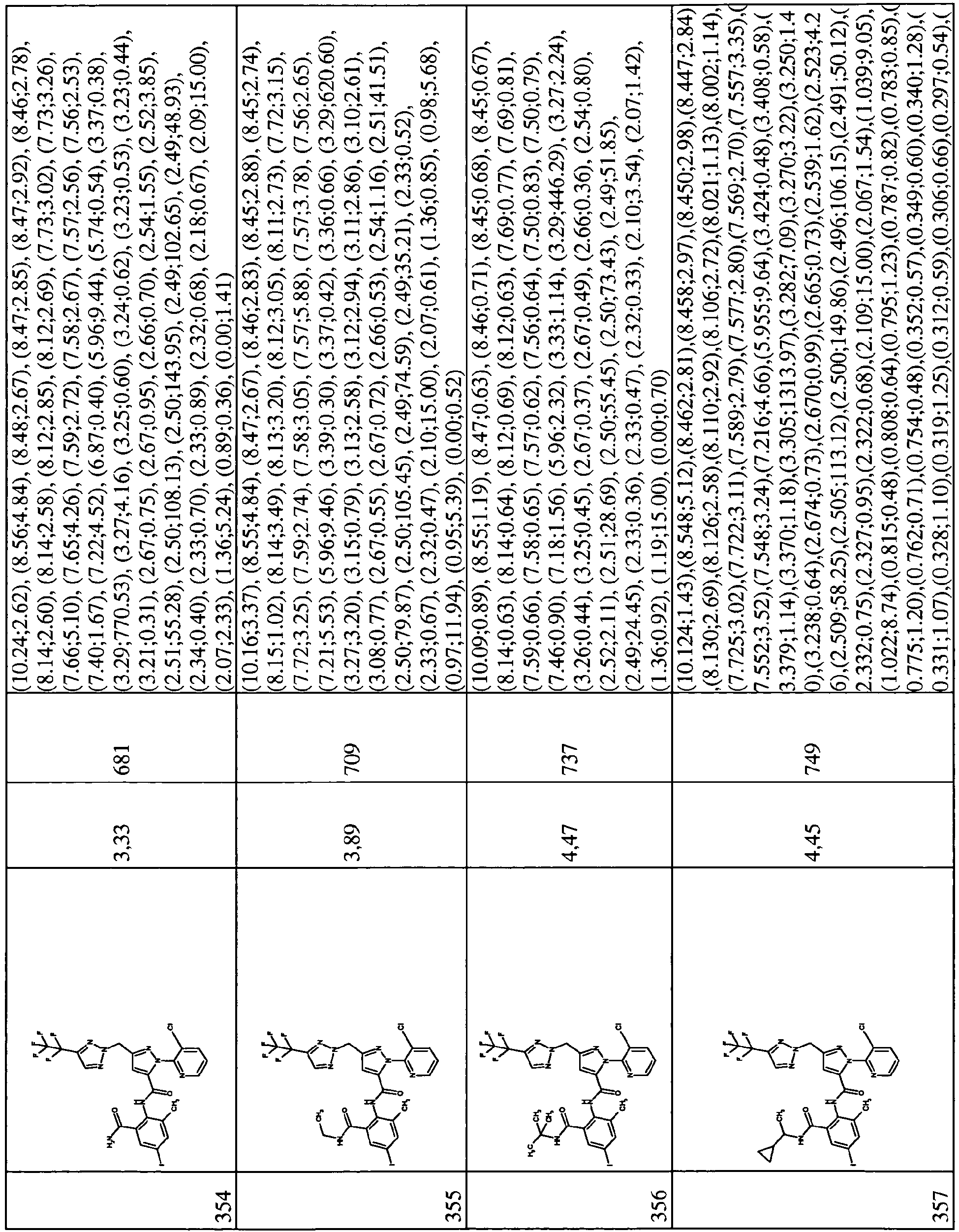

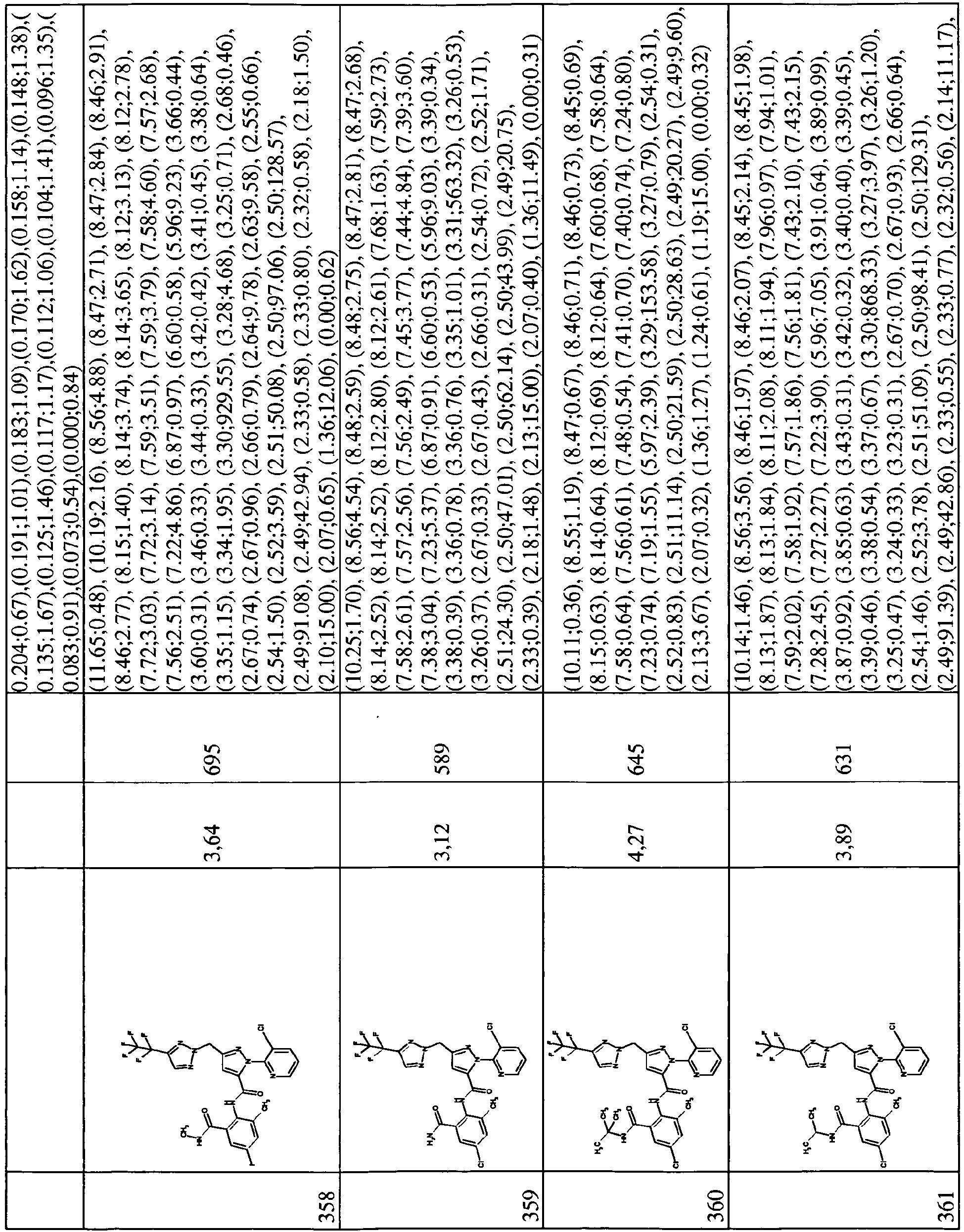

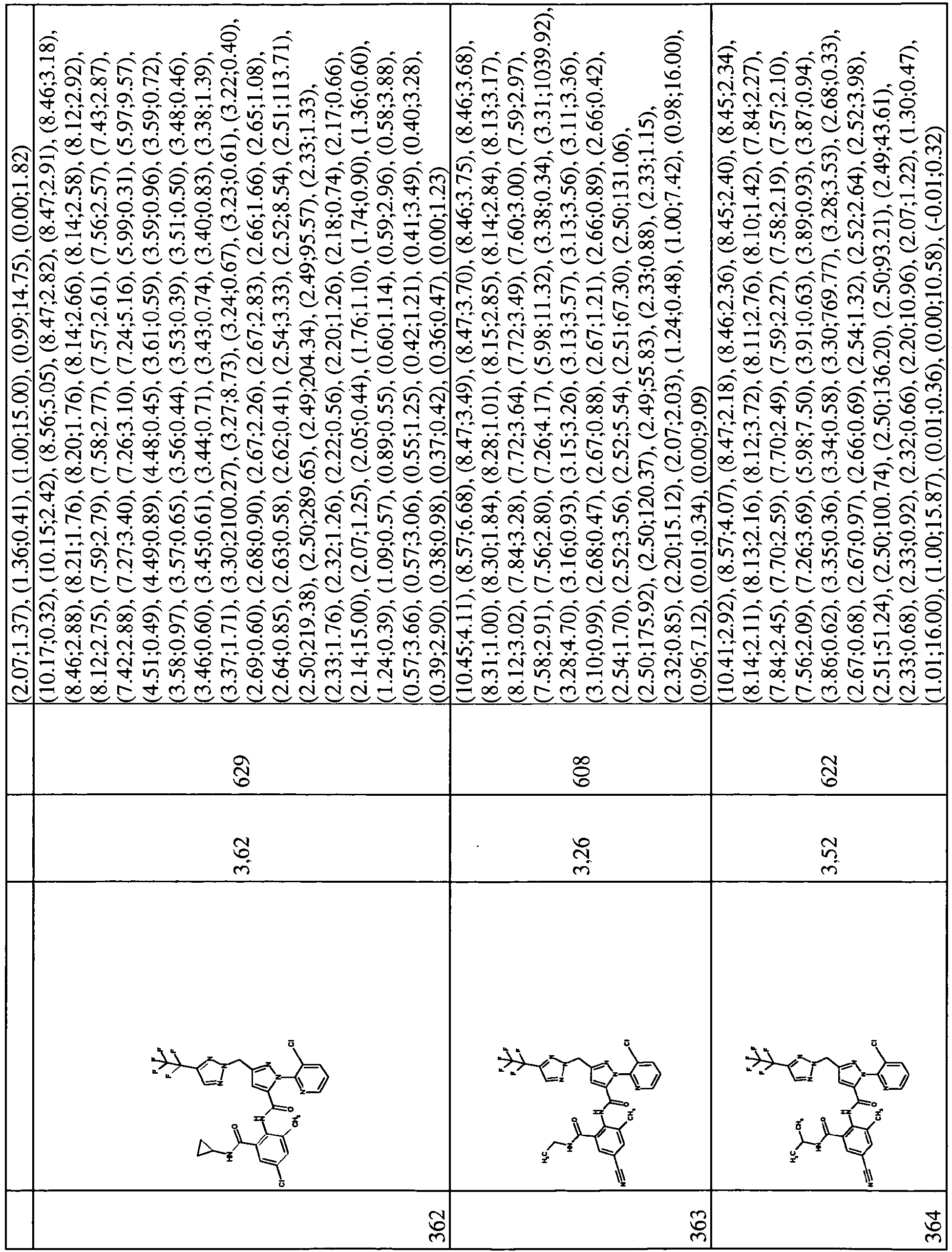

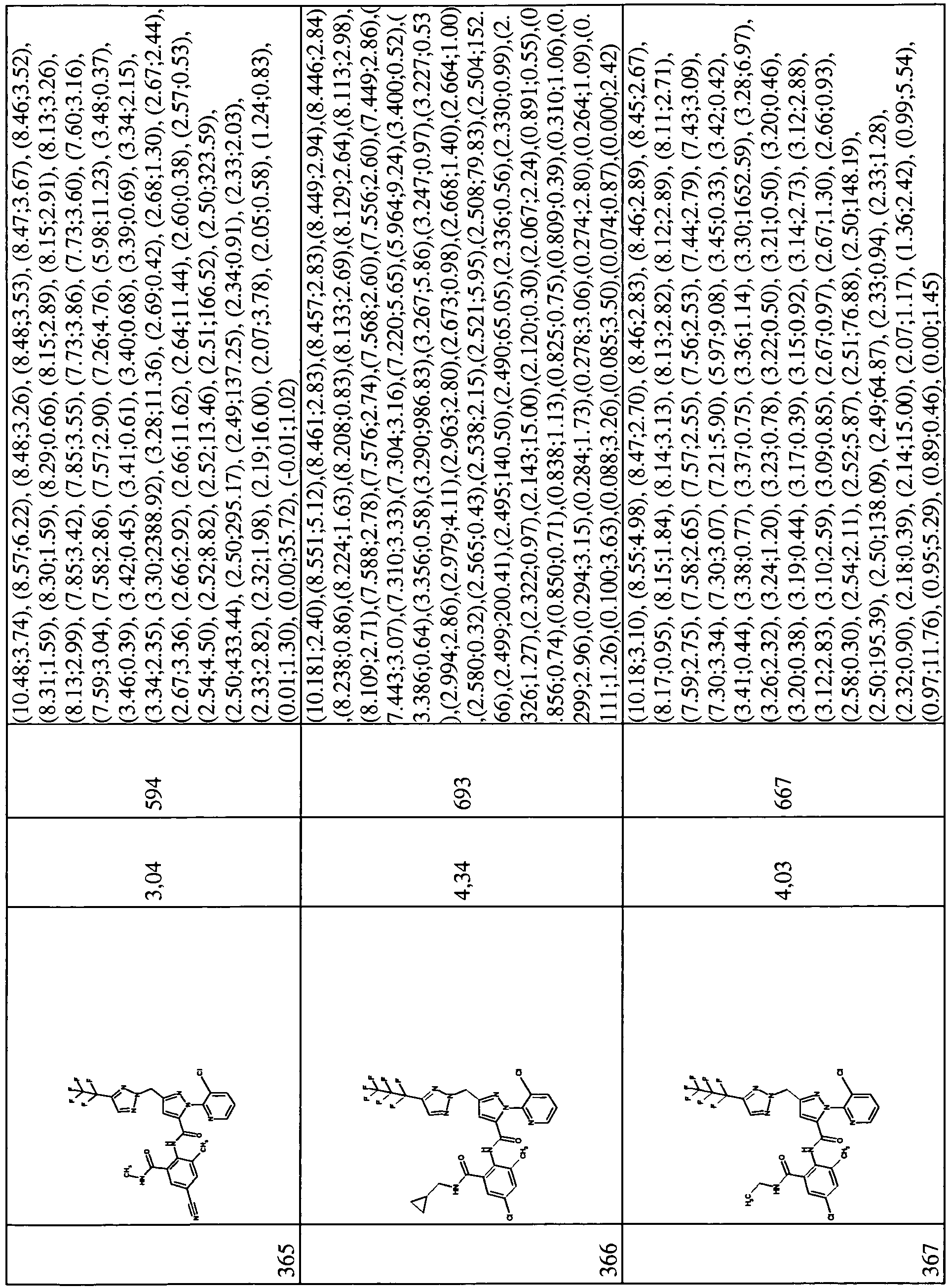

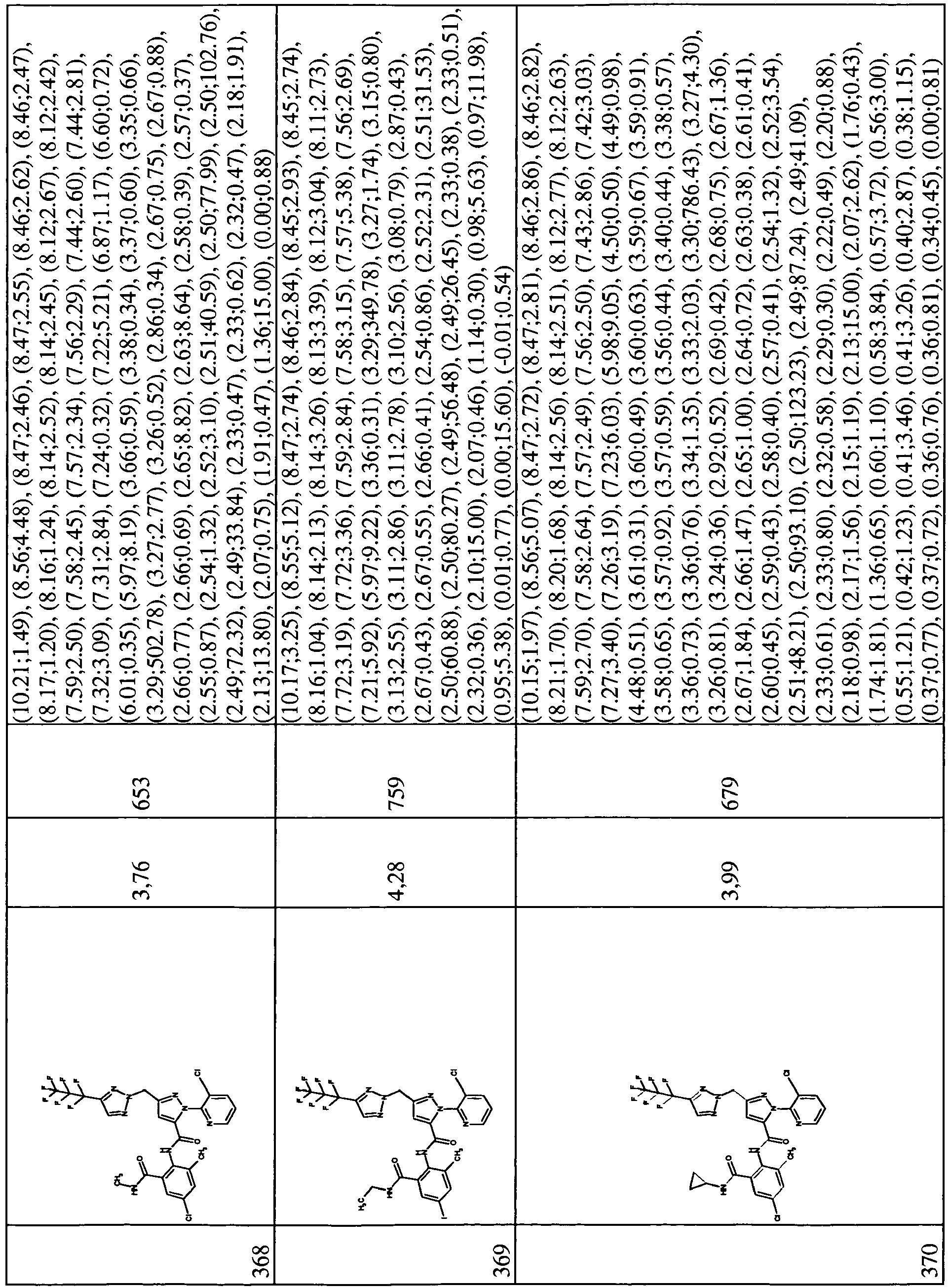

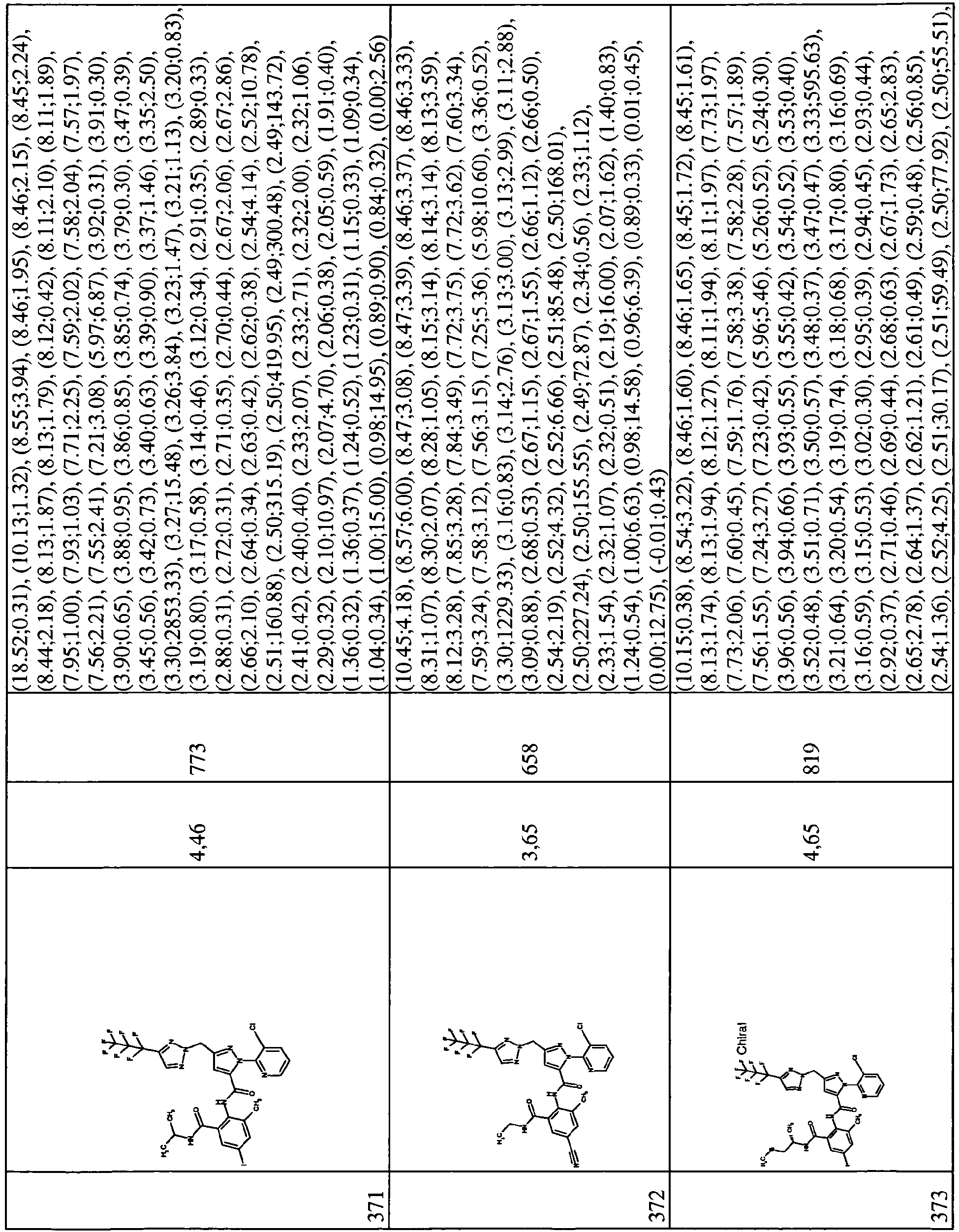

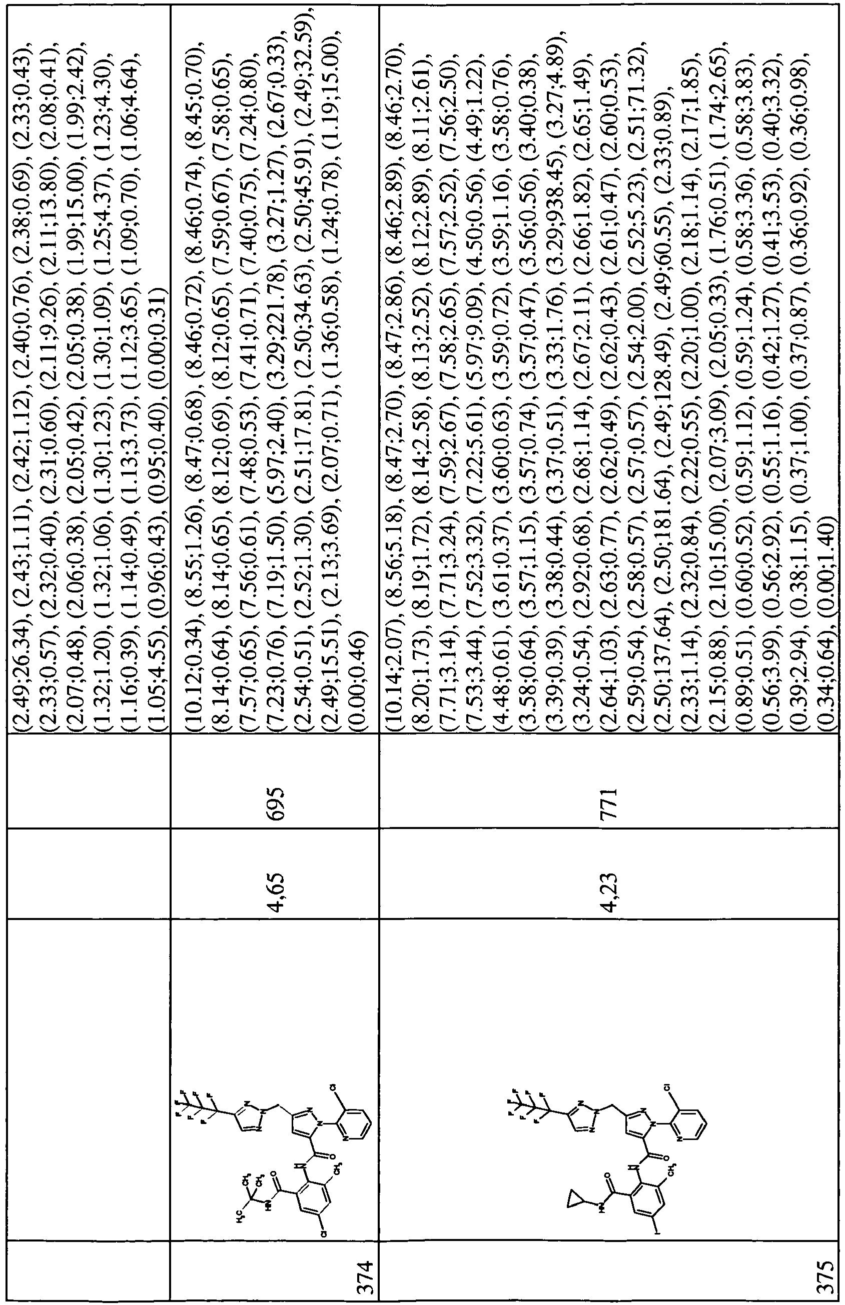

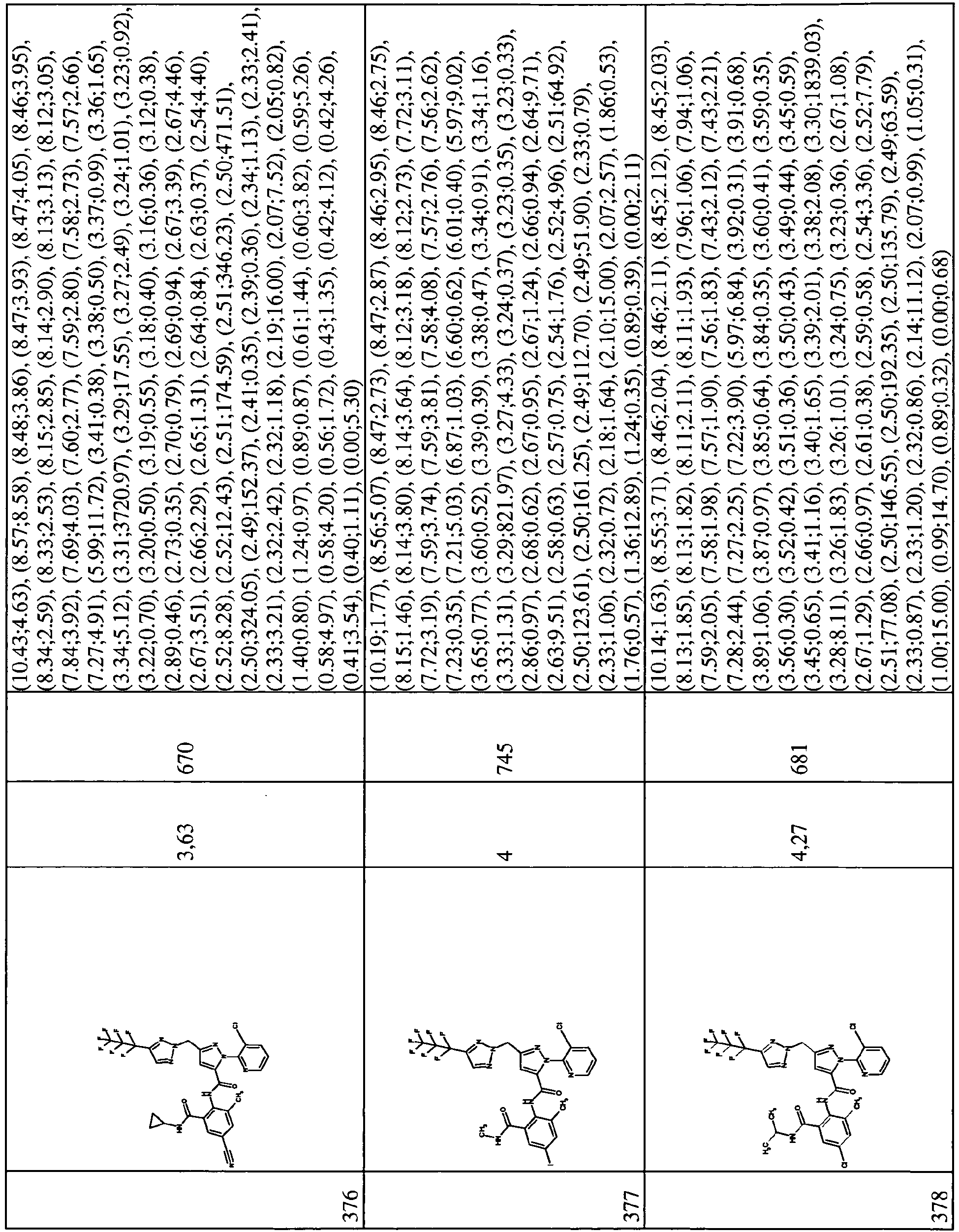

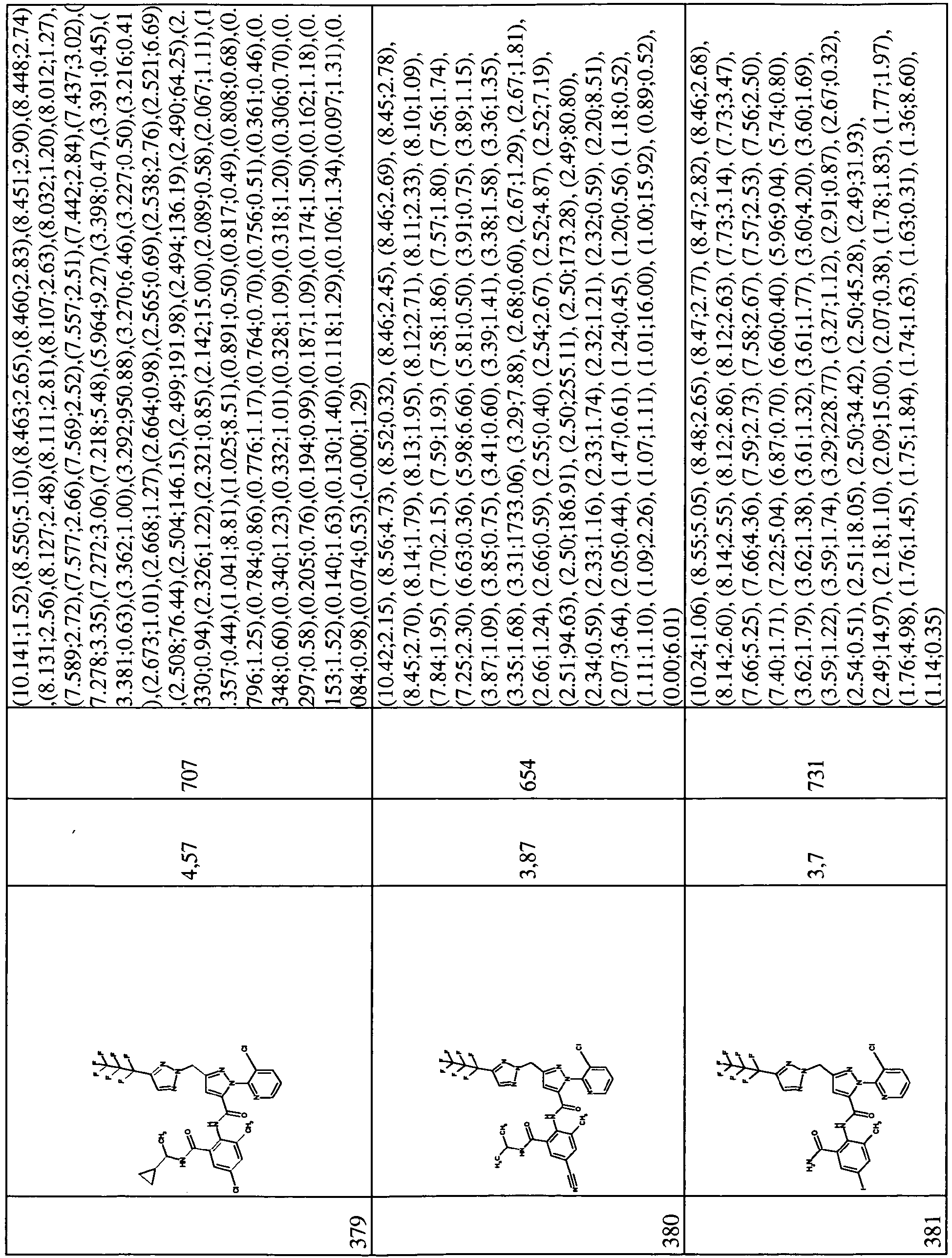

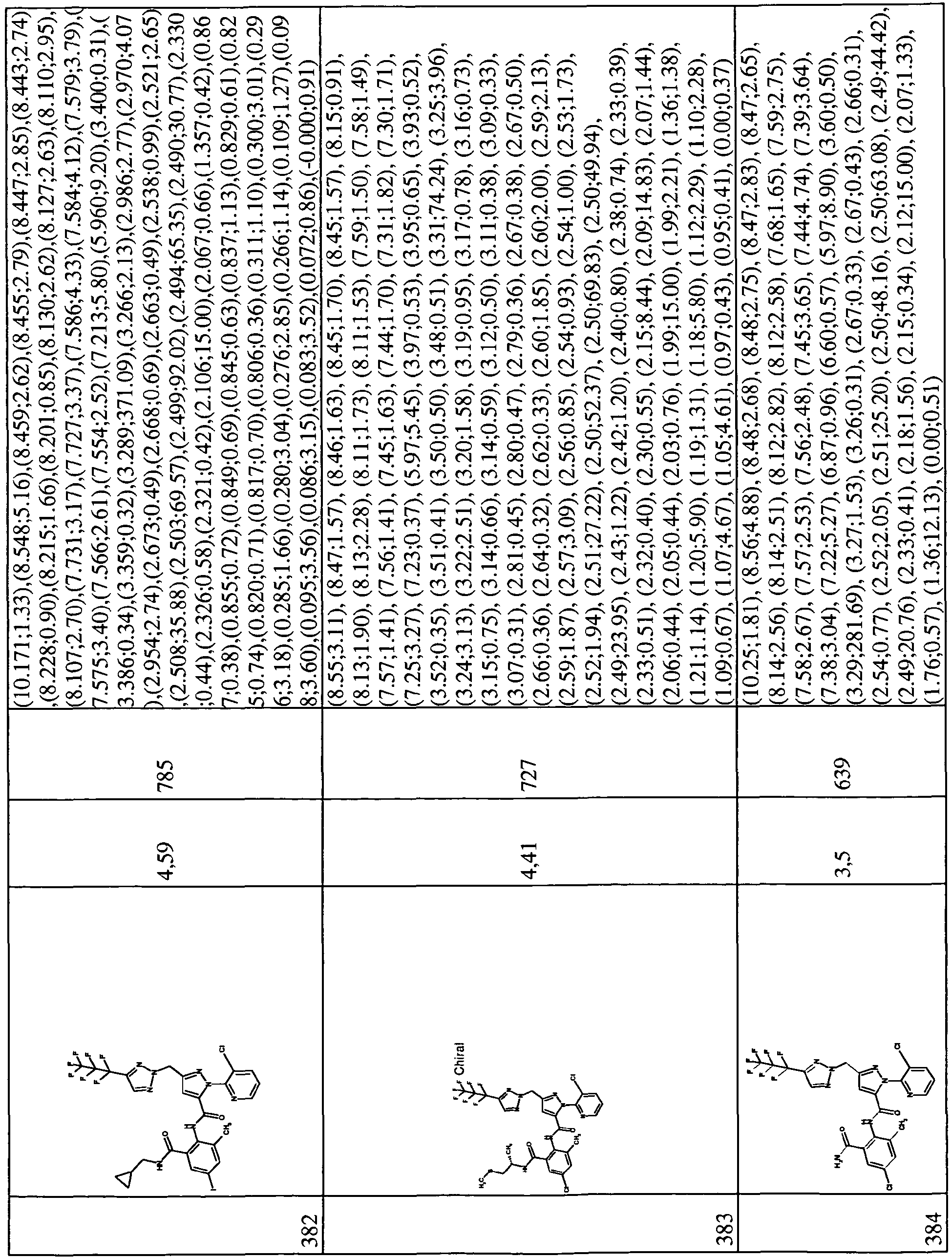

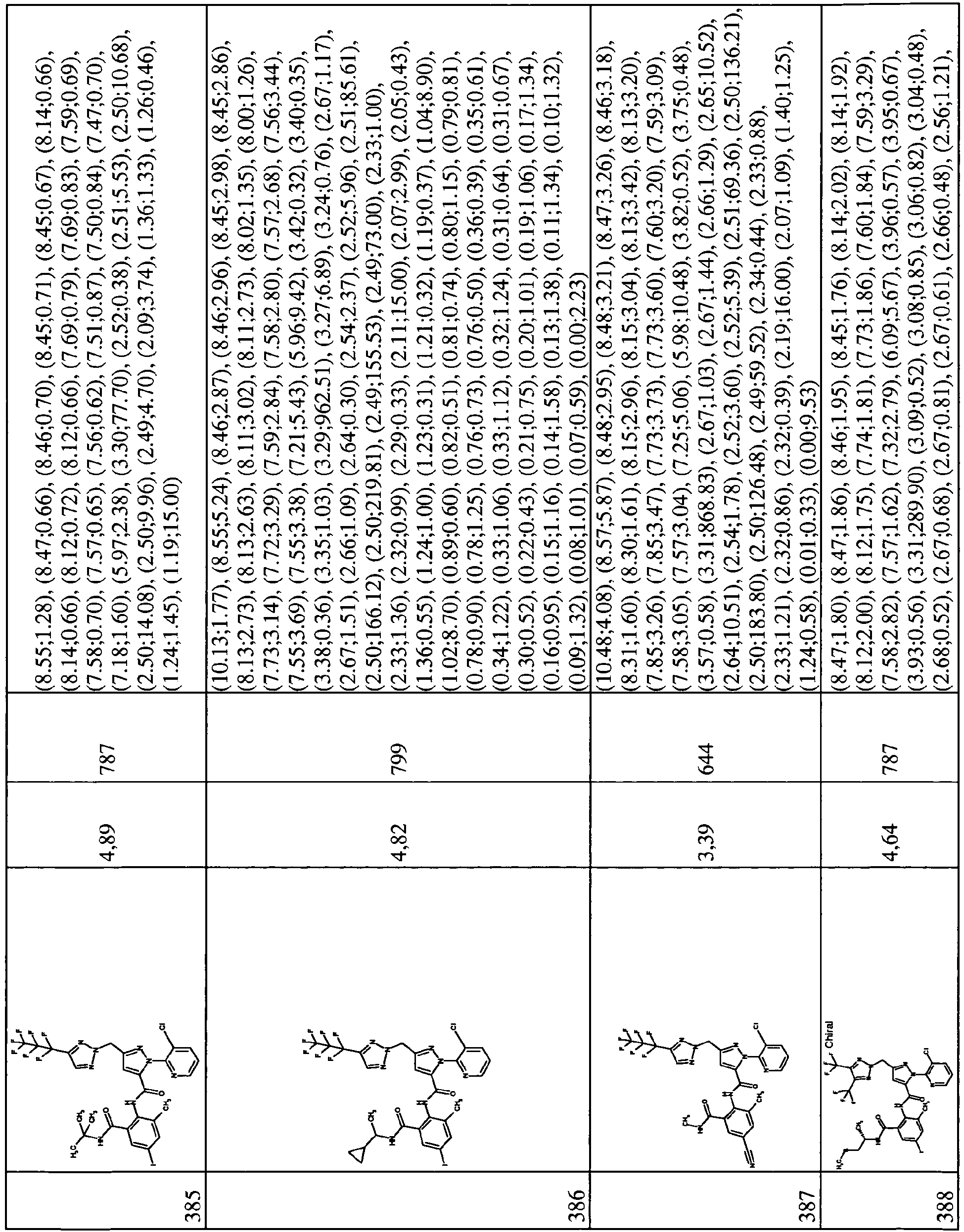

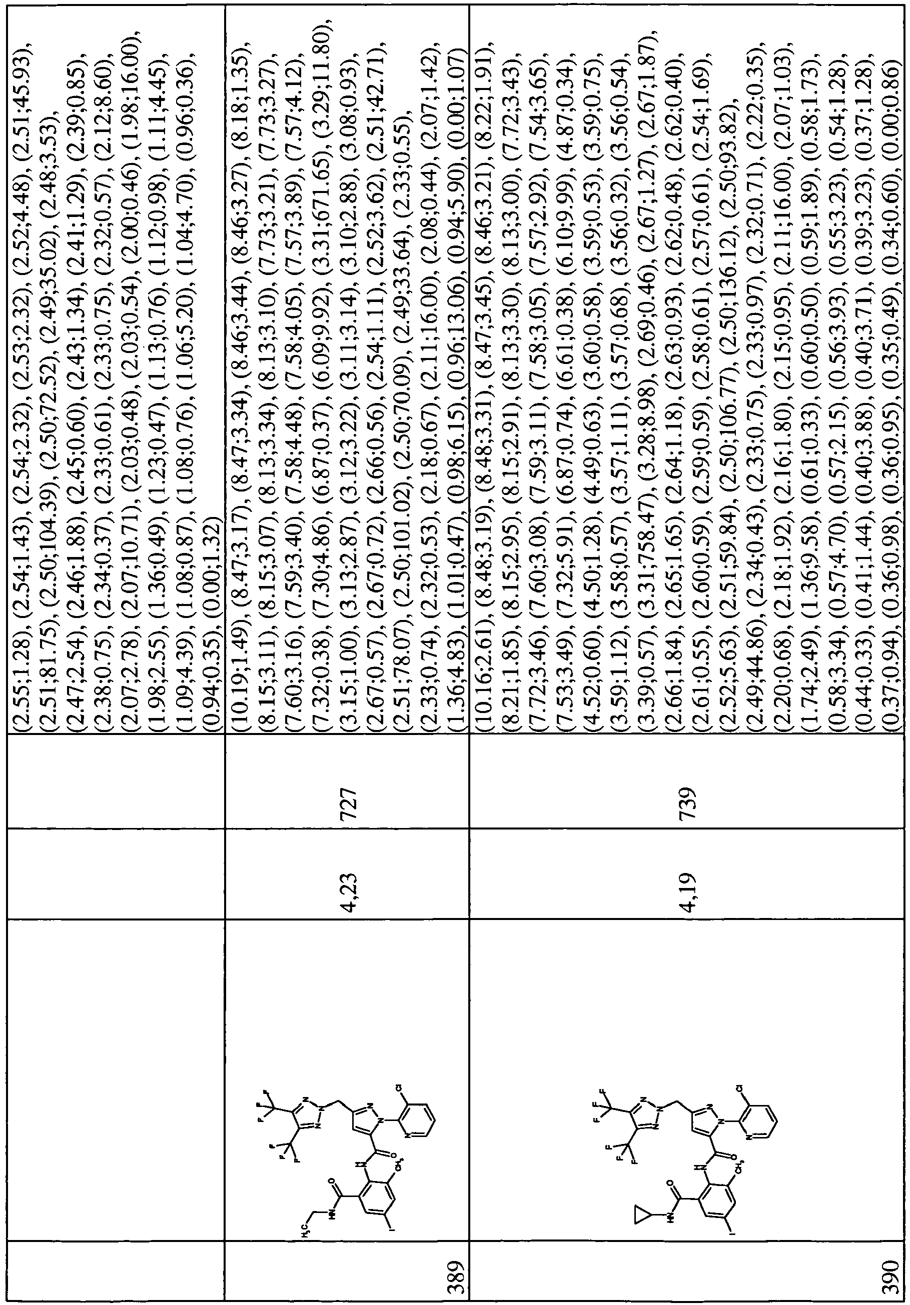

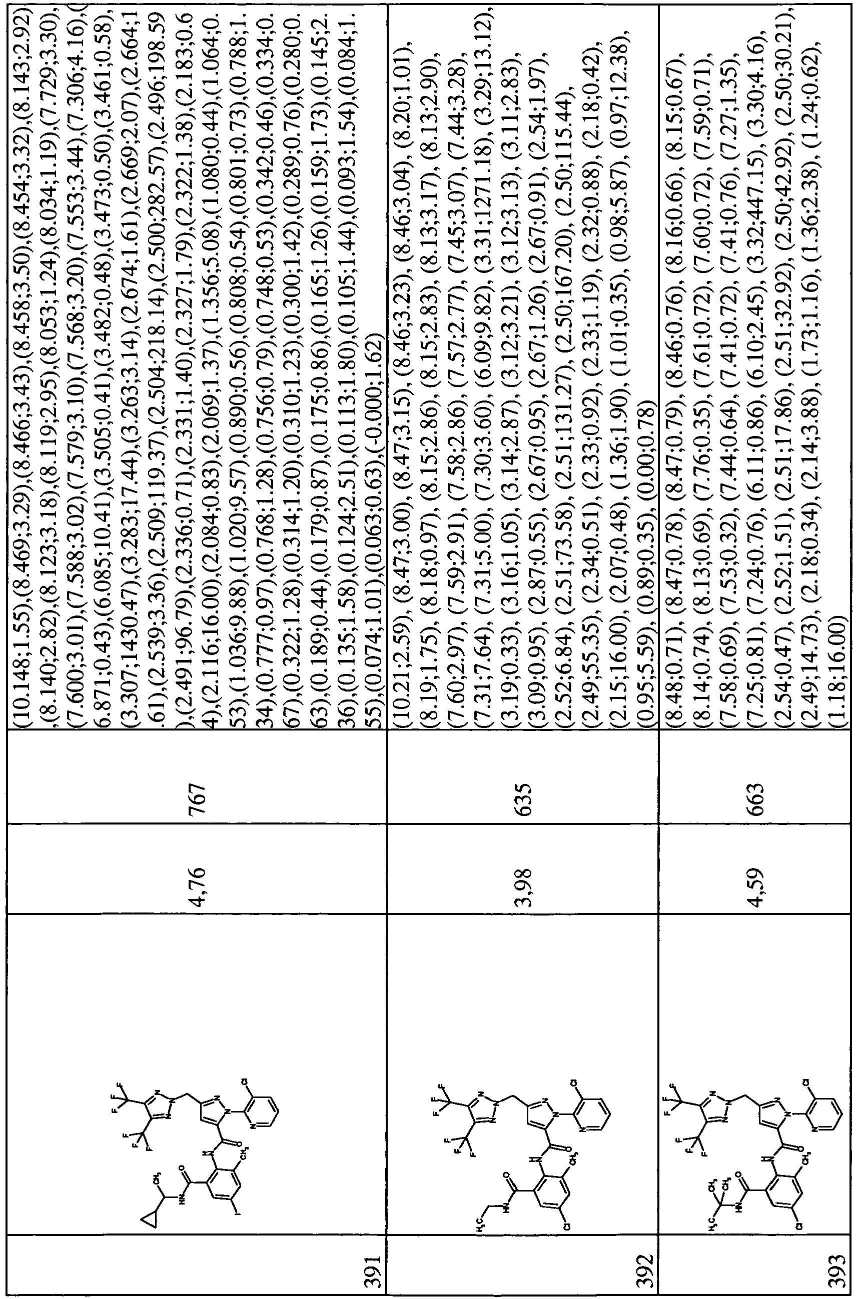

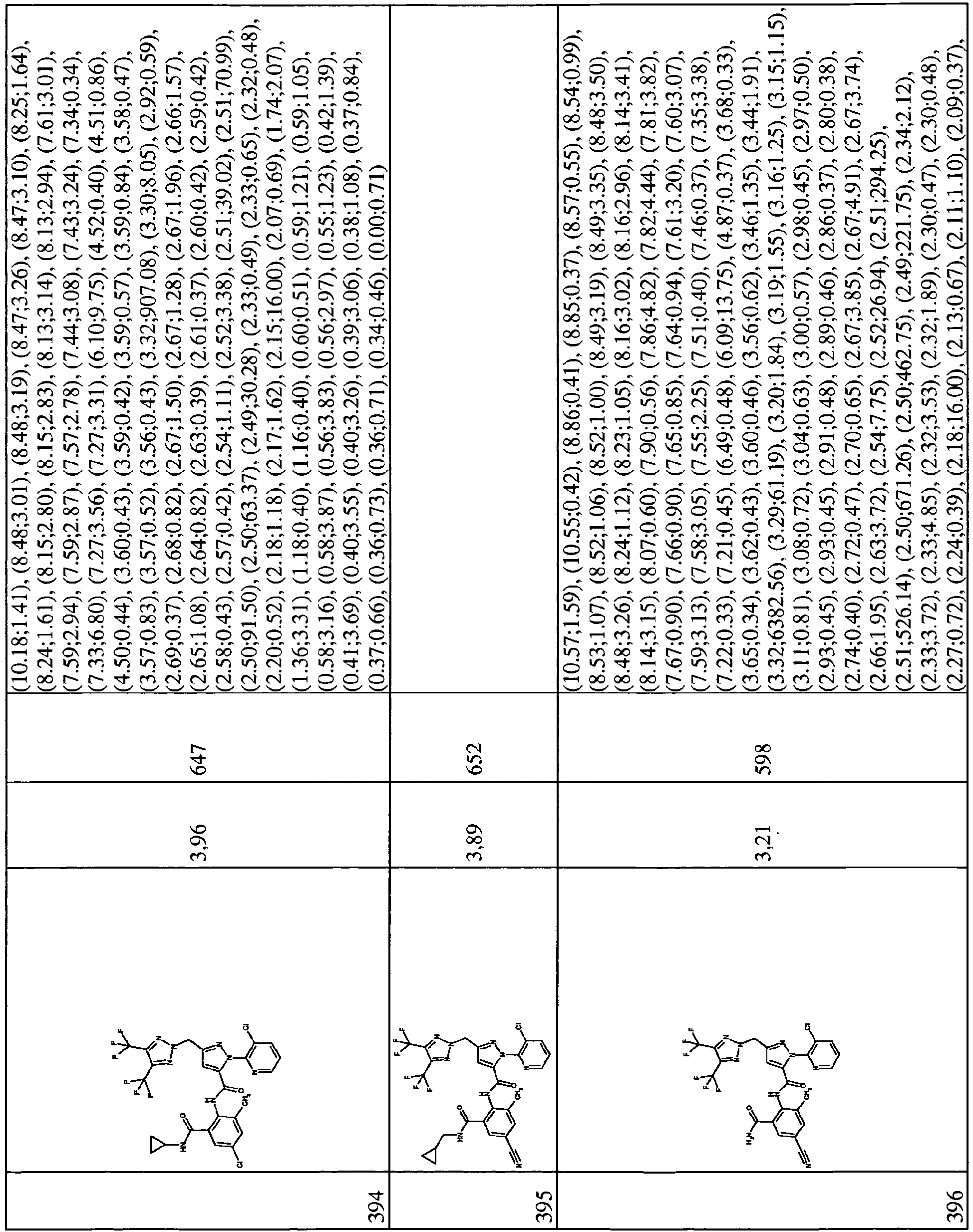

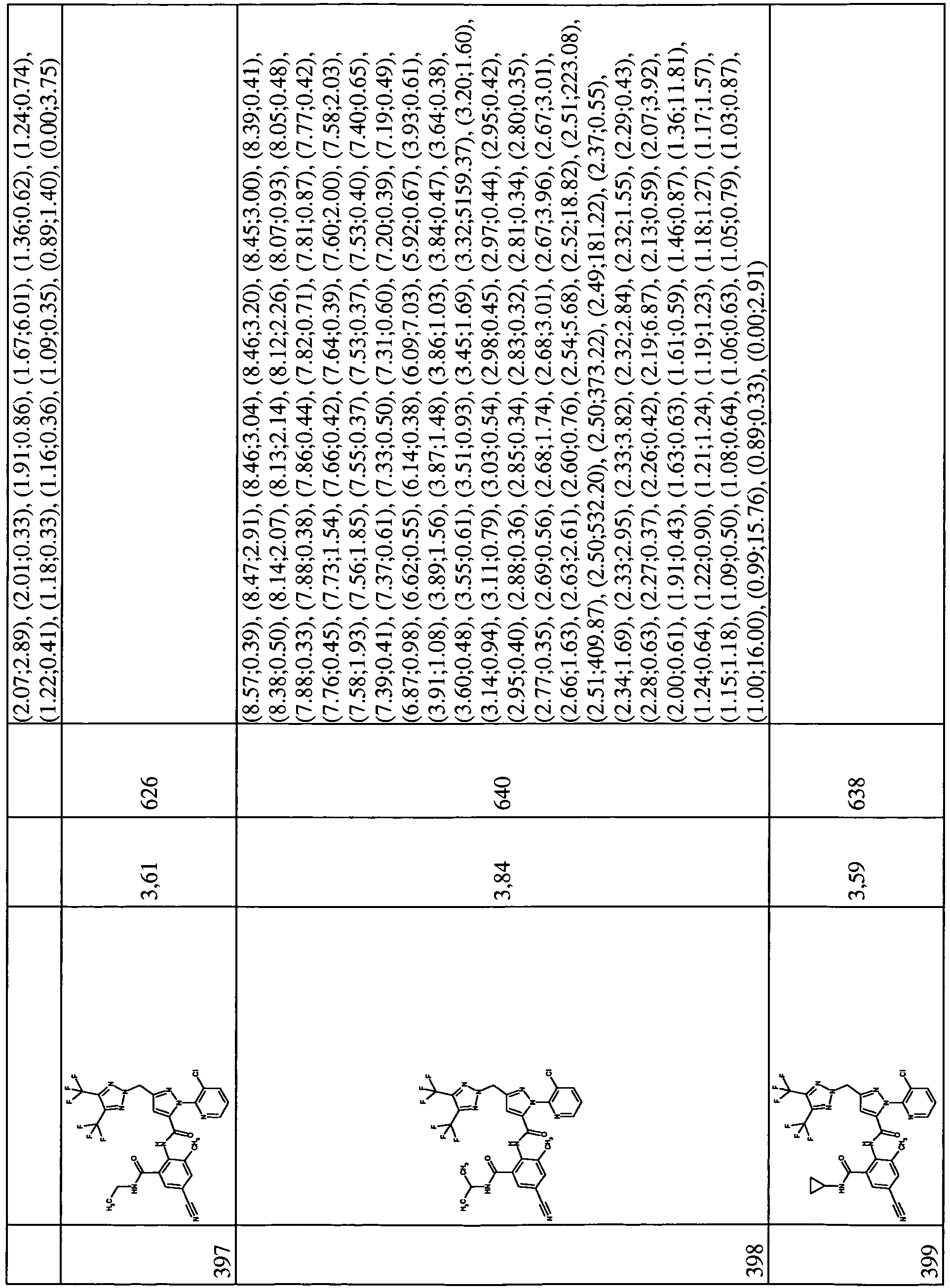

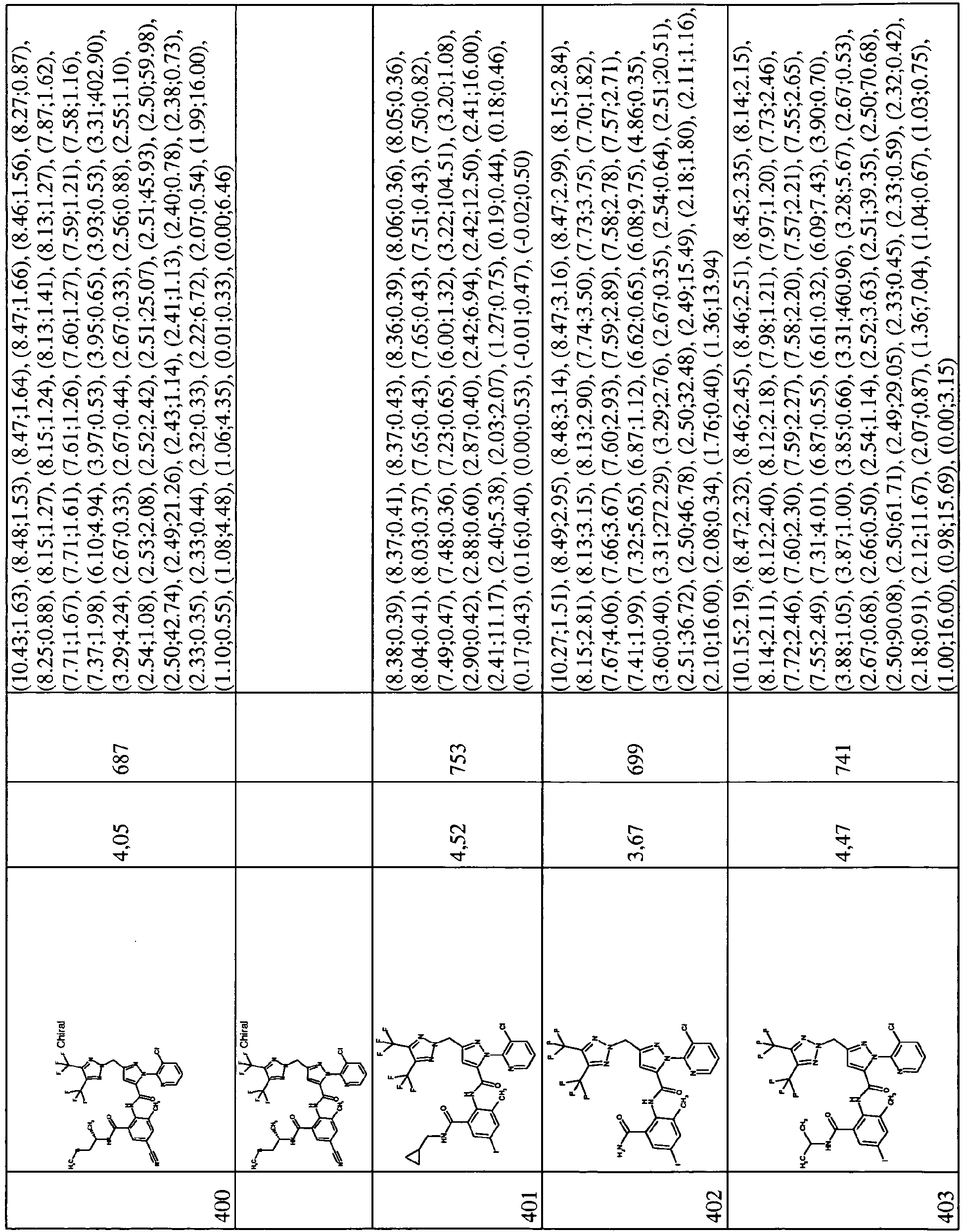

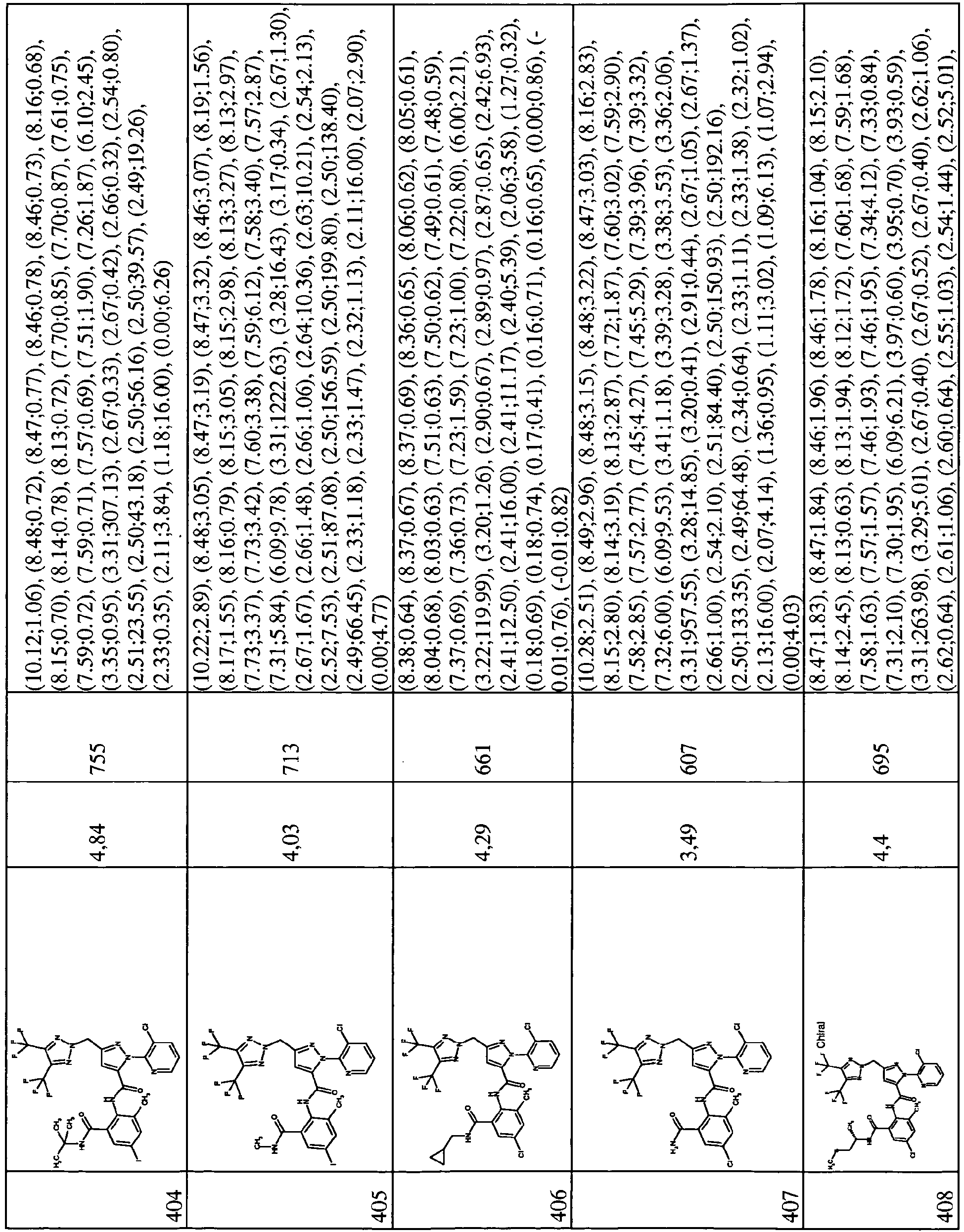

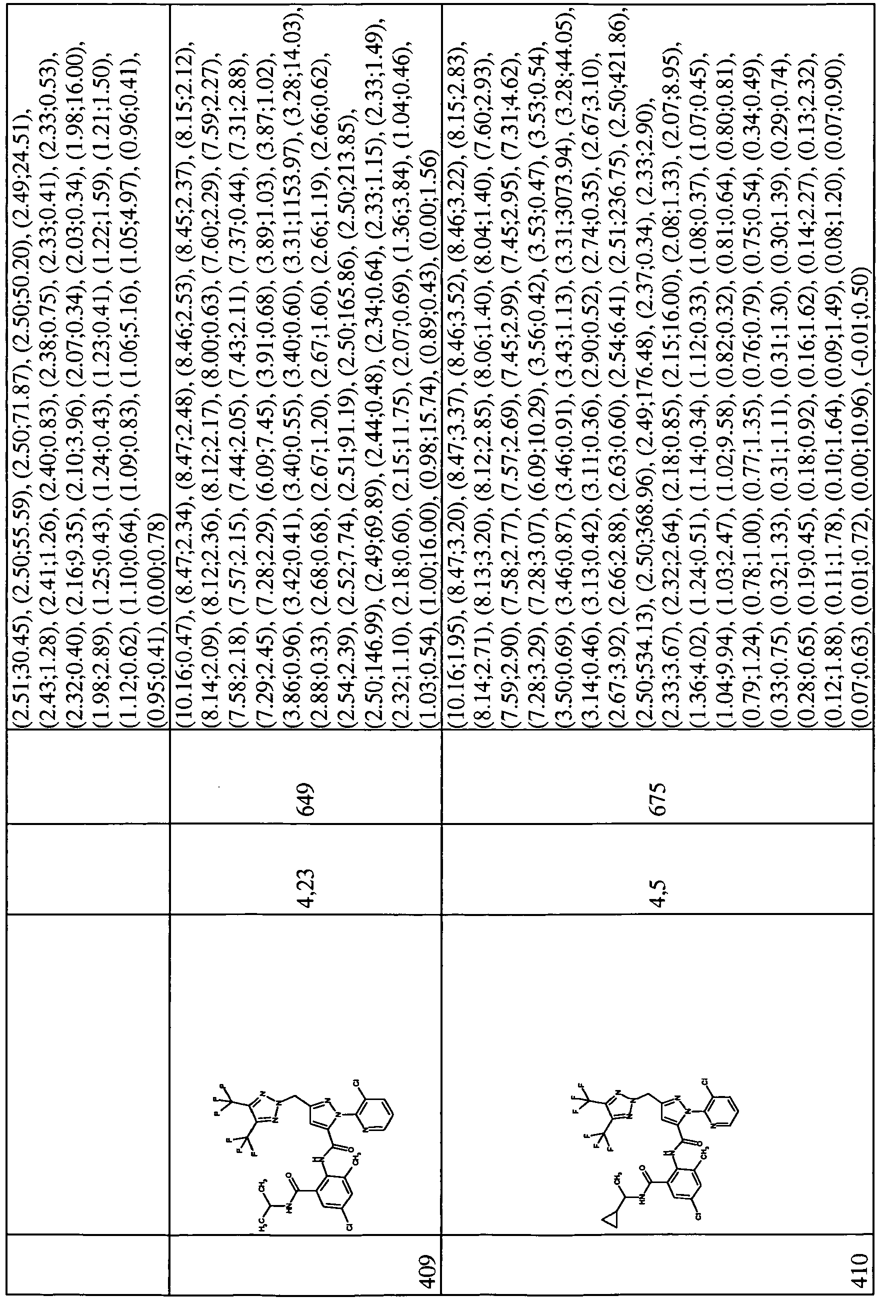

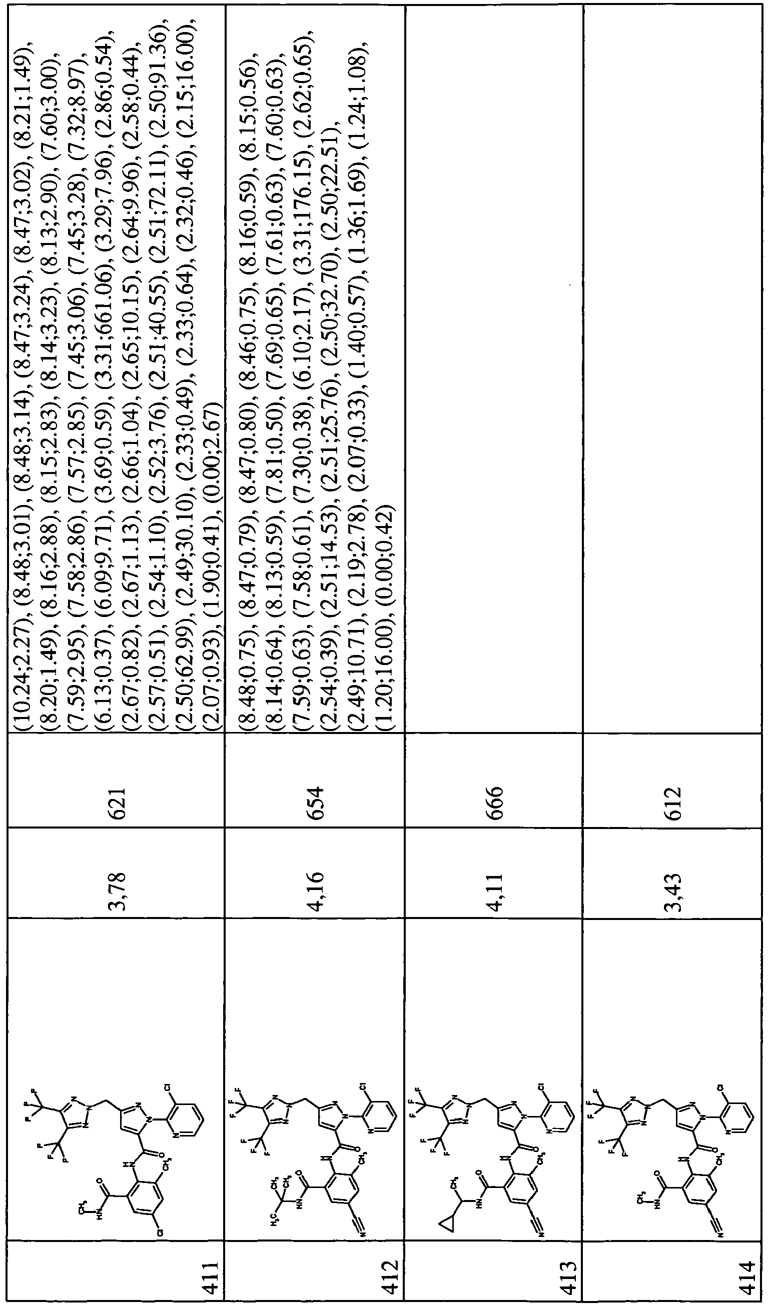

### Analytische Methoden

Die Bestimmung der in der voranstehenden Tabelle und den Herstellungsbeispielen angegebenen logP Werten erfolgte gemäß EEC Directive 79/831 Annex V.A8 durch HPLC (High Performance Liquid Chromatography) an reversed-phase Säulen (C 18), mit nachfolgenden Methoden:
Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril.
Die Kalibrierung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).
Die lambda-maX Werte wurden an Hand der UV-Spektren von 200 nm bis 400 nm in den Maxima der chromatographischen Signale ermittelt.
Die MH⁺-Signale wurden mit einem Agilent MSD-System mit ESI und positiver oder negativer Ionisation bestimmt.
Die NMR-Spektren wurden
   a) mit einem Bruker Avance 400, ausgestattet mit einem Durchflussprobenkopf (60 µl Volumen), bestimmt. Als Lösungsmittel wurden CD₃CN oder d₆-DMSO verwendet, wobei als Referenz Tetramethylsilan (0.00 ppm) eingesetzt wurde.
   b) mit einem Bruker Avance II 600 bestimmt. Als Lösungsmittel wurden CD₃CN oder d₆-DMSO verwendet, wobei als Referenz Tetramethylsilan (0.00 ppm) eingesetzt wurde.

Für alle der in obiger Tabelle aufgelisteten Beispiele wurde d₆-DMSO als Lösungsmittel verwendet, mit Ausnahme der Beispiele Nr. 121, 140, 162 und 177, für welche CD₃CN als Lösungsmittel zur Erstellung der NMR- Spektren verwendet wurde.

### Anwendungsbeispiele

### Beispiel 1

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 10 mg Wirkstoff mit 0,5mol Lösungsmittel und verdünnt das Konzentrat mit Lösungsmittel auf die gewünschte Konzentration. Die Wirkstofflösung wird in das Abdomen (*Boophilus microplus*) injiziert, die Tiere werden in Schalen überführt und in einem klimatisierten Raum aufbewahrt. Die Wirkungskontrolle erfolgt auf Ablage fertiler Eier.

Nach 7 Tagen wird die Wirkung in % bestimmt. Dabei bedeutet 100%, dass keine Zecke fertile Eier gelegt hat.

Bei diesem Test zeigen z.B. die folgenden Verbindungen der Herstellungsbeispiele eine Wirkung von 100% bei einer Aufwandmenge von 20µg/Tier: 7,70, 124, 128, 129,134, 135, 136, 147, 151, 155, 162, 172, 174, 175, 218, 231, 232, 239, 241, 247, 263, 281, 282

### Beispiel 2

### Lucilia cuprina-Test (LUCICU)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit ca 20 *Lucilia cuprina* Larven besetzt.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: 7, 70, 124, 128, 129, 134, 135, 136, 147, 151, 155, 162, 172, 174, 175, 218, 231, 232, 239, 241, 247, 263, 281, 282

### Beispiel 3

### Musca domestica-Test (MUSCDO)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration. Gefäße, die einen Schwamm enthalten, der mit der Wirkstoffzubereitung der gewünschten Konzentration behandelt wurde, werden mit adulten *Musca domestica* besetzt.

Nach 2 Tagen wird die Abtötung in % bestimmt. Dabei bedeutet 100 %, dass alle Fliegen abgetötet wurden; 0 % bedeutet, dass keine Fliegen abgetötet wurden.

Bei diesem Test zeigen z.B. die folgenden Verbindungen der Herstellungsbeispiele eine Wirkung von 100% bei einer Aufwandmenge von 100ppm: 134, 232, 239, 247, 263, 281

Bei diesem Test zeigen z.B. die folgenden Verbindungen der Herstellungsbeispiele eine Wirkung von 85% bei einer Aufwandmenge von 100ppm: 218

Bei diesem Test zeigen z.B. die folgenden Verbindungen der Herstellungsbeispiele eine Wirkung von 80% bei einer Aufwandmenge von 100ppm: 124, 128, 129, 172, 231, 241, 282

### Beispiel 4

### Myzus-Test (MYZUPE Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 500g/ha: 400

Bei diesem Test zeigen z. B. die folgenden Verbindungen der Herstellungsbeispiele Wirkung von 100% bei einer Aufwandmenge von 100g/ha: 5, 7, 9, 11, 12, 21, 70, 101, 121, 124, 125, 126, 128, 129, 130, 132, 134, 137, 143, 144, 146, 151, 155, 156, 157, 165, 166, 170, 172, 174, 177, 178, 182, 187, 192, 193, 194, 196, 201, 203, 212, 213, 217, 220, 222, 223, 225, 231, 238, 239, 241, 273, 281, 288, 289, 294, 303, 304, 305, 306, 307, 308, 313, 316, 317, 318, 319, 320, 321, 324, 325, 326, 327, 328, 329, 331, 332, 337, 338, 340, 341, 342, 343, 344, 353, 359 ,363, 364, 365, 368, 372, 373, 381, 384,387,414

Bei diesem Test zeigen z. B. die folgenden Verbindungen der Herstellungsbeispiele Wirkung von 90% bei einer Aufwandmenge von 100g/ha: 6, 50, 110, 120, 127, 131, 133, 135, 139, 150, 181, 185, 197, 215, 300, 301, 315, 339, 393, 396, 408

Bei diesem Test zeigen z. B. die folgenden Verbindungen der Herstellungsbeispiele Wirkung von 80% bei einer Aufwandmenge von 100g/ha: 8, 53, 54, 189, 219, 221, 224, 228, 230, 232, 246, 249, 254, 255, 262, 263, 271, 282, 284, 286, 291, 293, 298, 299, 302, 309, 312, 314, 348, 349, 350, 352, 356, 358, 360, 361, 362, 383, 402, 412

Bei diesem Test zeigen z. B. die folgenden Verbindungen der Herstellungsbeispiele Wirkung von 100% bei einer Aufwandmenge von 20g/ha: 123, 180, 184

### Beispiel 5

### Phaedon -Test (PHAECO Spritzbehandlung)

| | |
|---|---|
| Lösungsmittel: | 78,0 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 500g/ha: 180

Bei diesem Test zeigen z. B. die folgenden Verbindungen der Herstellungsbeispiele Wirkung von 100% bei einer Aufwandmenge von 100g/ha: 2, 3, 4, 5, 6, 7, 8, 9, 11, 12, 16, 17, 18, 19, 20, 21, 24, 25, 26, 27, 28, 29, 30, 31, 33, 34, 36, 37, 38, 40, 41, 42, 43, 44, 45, 46, 50, 53, 54, 55, 57, 61, 62, 63, 64, 65, 70, 71, 72, 74, 75, 77, 79, 81, 86, 87, 88, 89, 90, 91, 92, 94, 95, 96, 97, 98, 99, 100, 101, 102, 107, 108, 109, 110, 111, 112, 113, 114, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 172, 174, 175, 176, 177, 178, 182, 183, 184, 185, 186, 187, 189, 190, 192, 193, 194, 195, 196, 197, 198, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414

Bei diesem Test zeigen z. B. die folgenden Verbindungen der Herstellungsbeispiele Wirkung von 83% bei einer Aufwandmenge von 100g/ha: 14, 39

### Beispiel 6

### Spodoptera frugiperda -Test (SPODFR Spritzbehandlung)

| | |
|---|---|
| Lösungsmittel: | 78,0 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frasgiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 100g/ha: 1, 2, 3, 4, 5, 6, 7, 8, 9, 10, 11, 12, 17, 18, 19, 20, 21, 22, 24, 25, 26, 27, 28, 32, 33, 34, 35, 36, 40, 41, 42, 43, 44, 45, 53, 54, 55, 58, 61, 62, 63, 64, 65, 66, 67, 68, 69, 70, 71, 72, 74, 75, 77, 78, 79, 80, 81, 82, 83, 84, 86, 87, 88, 89, 90, 91, 92, 94, 95, 96, 97, 98, 99, 100, 101, 102, 103, 106, 107, 108, 109, 110, 111, 112, 113, 114, 115, 117, 118, 119, 120, 121, 122, 123, 124, 125, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 165, 166, 167, 168, 169, 170, 171, 172, 174, 175, 176, 177, 178, 180, 181, 182, 183, 184, 185, 187, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 221, 222, 224, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 273, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414 Bei diesem Test zeigen z. B. die folgenden Verbindungen der Herstellungsbeispiele Wirkung von 83% bei einer Aufwandmenge von 100g/ha: 16, 23, 50, 56, 57, 126

Bei diesem Test zeigen z. B. die folgenden Verbindungen der Herstellungsbeispiele Wirkung von 100% bei einer Aufwandmenge von 20g/ha: 220, 223, 225, 226, 270, 271, 272, 274

### Beispiel 7

### Tetranychus - test, OP-resistent (TETRUR Spritzbehandlung)

| | |
|---|---|
| Lösungsmittel: | 78,0 GewichtsteileAceton |
| | 1,5 Gewichtsteile Dimethylformamid |
| Emulgator : | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bohnenblattscheiben (*Phaseolus vulgaris*), die von allen Stadien der Gemeinen Spinnmilbe (*Tetranychus urticae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 90% bei einer Aufwandmenge von 100g/ha: 39, 321, 375

Bei diesem Test zeigen z. B. die folgenden Verbindungen der Herstellungsbeispiele eine Wirkung von 80% bei einer Aufwandmenge von 100g/ha: 50

### Beispiel 8

### Myzus-Test (MYZUPE Spritzbehandlung)

| | |
|---|---|
| Lösungsmittel: | 78,0 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)*, die von allen Stadien der Grünen Pfirsichblattlaus *(Myzus persicae)* befallen sind, werden mit einer Wirkstoffzubereitung der gewünschten Konzentration gespritzt.
Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.
Bei diesem Test zeigen z. B. die folgenden Verbindungen der Herstellungsbeispiele überlegene Wirksamkeit gegenüber dem Stand der Technik: siehe Tabelle

| Substanz | Struktur | Objekt | Konzentration | % Wirkung |
|---|---|---|---|---|
| erfindungsgemäß | 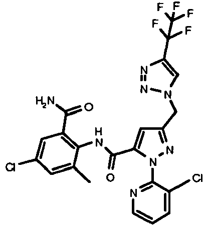 | MYZUPE | 100 g / ha | 100 6d |
| bekannt (WO 2007/144100) | 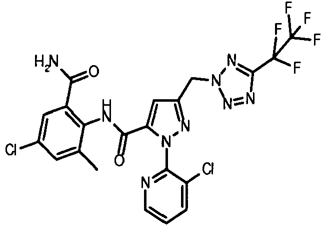 | MYZUPE | 100g/ha | 80 6d |

## Patentansprüche

1. Triazolsubstituierte Anthranilsäureamide der allgemeinen Formel (I), 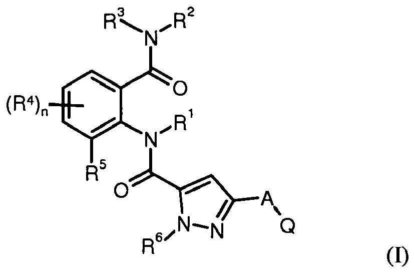 in welcher
R¹ für Wasserstoff, Amino, Hydroxy oder für jeweils gegebenenfalls einfach oder mehrfach, gleich oder verschieden substituiertes C₁-C₆-Alkyl, C₂-C₆-Alkenyl, C₂-C₆-Alkinyl oder C₃-C₆-Cycloalkyl steht, wobei die Substituenten unabhängig voneinander ausgewählt sein können aus Halogen, Cyano, Nitro, Hydroxy, C₁-C₄-Alkoxy, C₁-C₄-Alkylthio, C₁-C₄-Alkylsulfinyl, C₁-C₄-Al-kylsulfonyl, (C₁-C₄-Alkoxy)carbonyl, C₁-C₄-Alkylamino, Di-(C₁-C₄-alkyl)amino, C₃-C₆-Cycloalkyl-amino oder (C₁-C₄-Alkyl)C₃-C₆-cycloalkylamino,
R² für Wasserstoff, C₁-C₆-Alkyl, C₂-C₆-Alkenyl, C₂-C₆-Alkinyl, C₃-C₆-Cycloalkyl, C₁-C₄-Alkoxy, C₁-C₄-Alkylamino, Di-(C₁-C₄-alkyl)amino, C₃-C₆-Cycloalkylamino, C₂-C₆-Alkoxycarbonyl oder C₂-C₆-Alkylcarbonyl steht,
R³ für Wasserstoff oder für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl steht, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
R³ weiterhin für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl steht, wobei die Substituenten unabhängig voneinander ausgewählt sein können aus Amino, C₃-C₆-Cycloalkylamino oder einem 5- oder 6-gliedrigen heteroaromatischen Ring,
R³ ebenfalls weiterhin für C₃-C₁₂-Cycloalkyl, C₃-C₁₂-Cycloalkyl-C₁-C₆-Alkyl und C₄-C₁₂-Bicycloalkyl steht, wobei die Substituenten unabhängig voneinander ausgewählt sein können aus Halogen, Cyano, Nitro, Hydroxy, Amino, C₁-C₆-Alkyl, C₃-C₆-Cycloalkyl, C₃-C₆-Cycloalkylamino, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl, C₃-C₆-Trialkyl-silyl oder einem 5- oder 6-gliedrigen heteroaromatischen Ring,
R² und R³ miteinander über zwei bis sechs Kohlenstoffatome verbunden sein können und einen Ring ausbilden, der gegebenenfalls zusätzlich ein weiteres Stickstoff-, Schwefel- oder Sauerstoffatom enthält und gegebenenfalls einfach bis vierfach mit C₁-C₂-Alkyl, Halogen, Cyano, Amino oder C₁-C₂-Alkoxy substituiert sein kann,
R², R³ weiterhin gemeinsam für =S(C₁-C₄-Alkyl)₂, =S(O)(C₁-C₄-Alkyl)₂, stehen,
R⁴ für Wasserstoff, Halogen, Cyano, Nitro C₁-C₄-Alkyl, C₁-C₄-Haloalkyl, C₂-C₆-Alkenyl, C₂-C₆-Haloalkenyl, C₂-C₆-Alkinyl, C₁-C₄-Alkoxy, C₁-C₄-Haloalkoxy, SF₅, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₄-Alkoxy)imino, (C₁-C₄-Alkyl)(C₁-C₄-Alkoxy)imino, (C₁-C₄-Haloakyl)(C₁-C₄-Alkoxy)imino oder C₃-C₆-Trialkylsilyl steht, oder
zwei R⁴ über benachbarte Kohlenstoffatome einen Ring ausbilden, der für -(CH₂)₃-,-(CH₂)₄-, -(CH₂)₅-, -(CH=CH-)₂-, -OCH₂O- -O(CH₂)₂O-, -OCF₂O-, -(CF₂)₂O-, -O(CF₂)₂O-, -(CH=CH-CH=N)- oder -(CH=CH-N=CH)- steht,
zwei R⁴ weiterhin über benachbarte Kohlenstoffatome die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, C₁-C₆-Alkyl, C₃-C₆-Cycloalkyl, C₁-C₆-Haloalkyl, C₃-C₆-Halocycloalkyl, Halogen, C₁-C₆-Alkoxy, C₁-C₄-Alkylthio(C₁-C₆-alkyl), C₁-C₄-Alkylsulfinyl(C₁-C₆-alkyl), C₁-C₄-Alkylsulfonyl(C₁-C₆-alkyl), C₁-C₄-Alkylamino, Di-(C₁-C₄-alkyl)amino oder C₃-C₆-Cycloalkylamino, 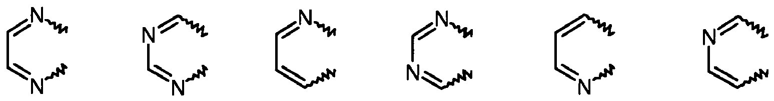 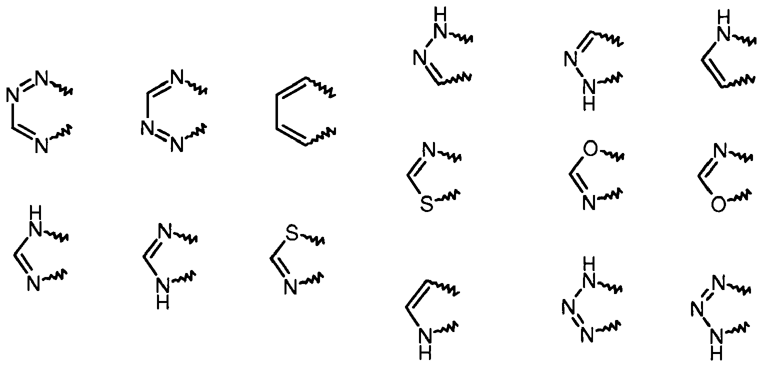
n für 0 bis 3 steht,
R⁵ für C₁-C₆-Alkyl, C₃-C₆-Cycloalkyl, C₁-C₆-Haloalkyl, C₁-C₆-Halocycloalkyl, C₂-C₆-Alkenyl, C₂-C₆-Haloalkenyl, C₂-C₆-Alkinyl, C₂-C₆-Haloalkinyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, Halogen, Cyano, Nitro oder C₃-C₆-Trialkylsilyl steht,
R⁶ für Wasserstoff, C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl oder 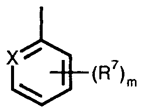 steht,
R⁶ weiterhin für C₃-C₆-Cycloalkoxy steht,
R⁷ steht unabhängig voneinander für Wasserstoff, C₁-C₆-Alkyl, C₃-C₆-Cycloalkyl, C₁-C₆-Haloalkyl, Halogen, Cyano, Nitro, C₁-C₄-Alkoxy, C₁-C₄-Haloalkoxy, C₁-C₄-Alkylthio oder C₁-C₄-Haloalkylthio,
m für 0 bis 4 steht,
X für N, CH, CF, CCl, CBr oder CI steht,
A für -CH₂-, -CH₂O-, -CH₂OCH₂-, -CH₂S-, -CH₂SCH₂-, -CH₂N(C₁-C₆-Alkyl)-,-CH₂N(C₁-C₆-Alkyl)CH₂-, -CH[CO₂(C₁-C₆-Alkyl)]-, -CH(CN)-, -CH(C₁-C₆-Alkyl)-, -C(Di-C₁-C₆-Alkyl)-, -CH₂CH₂-, -C=NO(C₁-C₆-Alkyl)- steht,
Q für einen 5-gliedrigen heteroaromatischen Ring oder ein aromatisches 9-gliedriges annelliertes heterobicyclisches Ringsystem steht, wobei der Ring oder das Ringsystem drei Stickstoffe enthalten und wobei die Stickstoffe im Ring oder Ringsystem benachbarte Positionen einnehmen und die Bindung des Rings oder des Ringsystems an den Rest A in Formel (I) über Stickstoff erfolgt. Der Ring oder das Ringsystem sind gegebenenfalls einfach oder mehrfach, gleich oder verschieden substituiert, wobei die Substituenten unabhängig voneinander ausgewählt sein können aus Wasserstoff, C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, CO₂H, CO₂NH₂, NO₂, OH, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino,
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, OH, C₁-C₄-Alkoxy, C₁-C₄-Haloalkoxy substituiert sein können,
die Verbindungen der allgemeinen Formel (I) außerdem N-Oxide und Salze umfassen.

2. Anthranilsäureamide der allgemeinen Formel (I) gemäß Anspruch 1, in welcher
R¹ steht für Wasserstoff, C₁-C₆-Alkyl, C₂-C₆-Alkenyl, C₂-C₆-Alkinyl, C₃-C₆-Cyclo-alkyl, Cyano(C₁-C₆-alkyl), C₁-C₆-Haloalkyl, C₂-C₆-Haloalkenyl, C₂-C₆-Haloalkinyl, C₁-C₄-Alkoxy-C₁-C₄-alkyl, C₁-C₄-Alkylthio-C₁-C₄-alkyl, C₁-C₄-Alkylsulfinyl-C₁-C₄-alkyl oder C₁-C₄-Alkylsulfonyl-C₁-C₄-alkyl,
R² steht für Wasserstoff oder C₁-C₆-Alkyl,
R³ steht für Wasserstoff oder für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfinüno-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
R³ steht weiterhin für C₃-C₁₂-Cycloalkyl und C₄-C₁₀-Bicycloalkyl, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
R⁴ steht für Wasserstoff, C₁-C₄-Alkyl, C₁-C₄-Haloalkyl, Halogen, Cyano, C₁-C₄-Alkoxy, C₁-C₄-Haloalkoxy, C₁-C₄-Alkylthio oder C₁-C₄-Haloalkylthio,
ausserdem zwei benachbarte Reste R⁴ stehen für -(CH₂)₃-, -(CH₂)₄-, -(CH₂)₅-, -(CH=CH-)₂-, -OCH₂O-, -O(CH₂)₂O-, -OCF₂O-, -(CF₂)₂O-, -O(CF₂)₂O-, -(CH=CH-CH=N)- oder - (CH=CH-N=CH)-,
n steht für 1,2 oder 3,
R⁵ steht für C₁-C₄-Alkyl, C₃-C₆-Cycloalkyl, C₁-C₄-Haloalkyl, C₁-C₆-Halocycloalkyl, C₂-C₆-Alkenyl, C₂-C₄-Haloalkenyl, C₂-C₄-Alkinyl, C₂-C₄-Haloalkinyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, Halogen, Cyano, Nitro oder C₃-C₆-Trialkylsilyl.
R⁶ steht für C₁-C₆-Alkyl oder 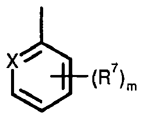
R⁶ steht weiterhin für C₃-C₆-Cycloalkoxy,
R⁷ steht unabhängig voneinander für Wasserstoff, Halogen, Cyano, C₁-C₄-Alkyl, C₁-C₄-Alkoxy, C₁-C₄-Haloalkyloder C₁-C₄-Haloalkoxy
m steht für 1, 2 oder 3,
X steht für N, CH, CF, CCl, CBr oder CI,
A steht für -CH₂-, -CH₂O-, -CH₂OCH₂-, -CH₂S-, -CH₂SCH₂-, -CH₂N(C₁-C₆-Alkyl)-, -CH₂N(C₁-C₆-Alkyl)CH₂-, -CH(CN)-, -CH(C₁-C₆-Alkyl)-, -C(Di-C₁-C₆-Alkyl)-, -CH₂CH₂-, -C=NO(C₁-C₆-Alkyl)-,
Q für einen 5-gliedrigen heteroaromatischen Ring oder ein aromatisches 9-gliedriges annelliertes heterobicyclisches Ringsystem steht, wobei der Ring oder das Ringsystem drei Stickstoffe enthalten und wobei die Stickstoffe im Ring oder Ringsystem benachbarte Positionen einnehmen und die Bindung des Rings oder des Ringsystems an den Rest A in Formel (I) über Stickstoff erfolgt. Der Ring oder das Ringsystem sind gegebenenfalls einfach oder mehrfach, gleich oder verschieden substituiert, wobei die Substituenten unabhängig voneinander ausgewählt sein können aus Wasserstoff, C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, CO₂H, CO₂NH₂, NO₂, OH, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino,
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, OH, C₁-C₄-Alkoxy, C₁-C₄-Haloalkoxy substituiert sein können,

3. Anthranilsäureamide der allgemeinen Formel (I) gemäß Anspruch 1 oder 2, in welcher
R¹ steht für Wasserstoff, Methyl, Cyclopropyl, Cyanomethyl, Methoxymethyl, Methylthiomethyl, Methylsulfinylmethyl oder Methylsulfonylmethyl,
R² steht für Wasserstoff oder Methyl,
R³ steht für Wasserstoff oder für jeweils gegebenenfalls einfach oder mehrfach, gleich oder verschieden substituiertes C₁-C₆-Alkyl, C₁-C₆-Alkoxy, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
R³ steht weiterhin für gegebenenfalls einfach oder mehrfach, gleich oder verschieden substituiertes C₃-C₆-Cycloalkyl wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkyl-sulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
R⁴ steht für Wasserstoff, C₁-C₄-Alkyl, C₁-C₂-Haloalkyl, Halogen, Cyano oder C₁-C₂-Haloalkoxy.
Ausserdem zwei benachbarte Reste R⁴ stehen für -(-CH₂)₄-, -(CH=CH-)₂-, -O(CH₂)₂O-, -O(CF₂)₂O-, -(CH=CH-CH=N)- oder -(CH=CH-N=CH)-.
n steht für 1 oder 2,
R⁵ steht für C₁-C₄-Alkyl, C₃-C₆-Cycloalkyl, C₁-C₄-Haloalkyl, C₁-C₆-Halocycloalkyl, C₂-C₆-Alkenyl, C₂-C₄-Haloalkenyl, C₂-C₄-Alkinyl, C₂-C₄-Haloalkinyl, C₁-C₄-Alkoxy, C₁-C₄-Haloalkoxy, Fluor, Chlor, Brom, Iod, Cyano, Nitro oder C₃-C₆-Trialkylsilyl.
R⁶ steht für Methyl oder 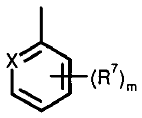
R⁷ steht unabhängig voneinander für Wasserstoff, Halogen oder C₁-C₄-Haloalkyl,
m steht für 1 oder 2,
X steht für N, CH, CF, CCl oder CBr,
A steht für -CH₂-, -CH(CH₃), C(CH₃)₂ oder CH₂CH₂,
A steht weiterhin für -CH(CN)-,
Q steht für einen 5-gliedrigen heteroaromatischen Ring oder ein aromatisches 9-gliedriges annelliertes heterobicyclisches Ringsystem, wobei der Ring oder das Ringsystem drei Stickstoffe enthalten und wobei die Stickstoffe im Ring oder Ringsystem benachbarte Positionen einnehmen und die Bindung des Rings oder des Ringsystems an den Rest A in Formel (I) über Stickstoff erfolgt. Der Ring oder das Ringsystem sind gegebenenfalls einfach oder mehrfach, gleich oder verschieden substituiert, wobei die Substituenten unabhängig voneinander ausgewählt sein können aus Wasserstoff, C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, CO₂H, CO₂NH₂, NO₂, OH, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino,
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, OH, C₁-C₄-Alkoxy, C₁-C₄-Haloalkoxy substituiert sein können,.

4. Mischungen von Verbindungen der allgemeinen Formel 1 gemäß einem der Ansprüche 1 bis 3, wobei der Ring oder das Ringsystem Q an den Rest (A) jeweils über unterschiedliche Kohlenstoff- oder Stickstoffatome gebunden ist.

5. Verfahren zur Herstellung von Verbindungen der allgemeinen Formel (I) gemäß einem der Ansprüche 1 bis 3, **dadurch gekennzeichnet, dass** man
(A) Aniline der Formel (II) 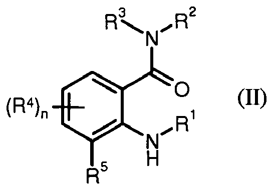 in welcher A, R¹, R², R³, R⁴, R⁵ und n die oben angegebenen Bedeutungen haben,
mit Carbonsäurechloriden der Formel (III) 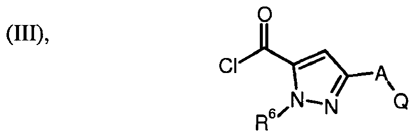 in welcher R⁶, A und Q die oben angegebenen Bedeutungen haben, in Gegenwart eines Säurebindemittels umsetzt,
(B) Aniline der Formel (II) 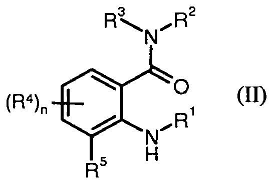 in welcher A, R¹, R², R³, R⁴, R⁵ und n die oben angegebenen Bedeutungen haben,
mit einer Carbonsäure der Formel (IV) 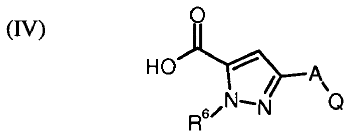 in welcher R⁶, A und Q die oben angegebenen Bedeutungen hat,
in Gegenwart eines Kondensationsmittels umsetzt oder indem man
(C) zur Synthese von Anthranilamiden der Formel (I), in welcher R¹ für Wasserstoff steht, Benzoxazinone der Formel (V) 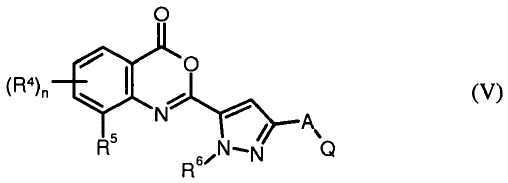 in welcher R⁴, R⁵, R⁶, A, Q und n die oben angegebenen Bedeutungen haben,
mit einem Amin der Formel (VI) 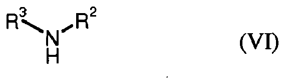 in welcher R² und R³ die oben angegebenen Bedeutungen haben,
in Gegenwart eines Verdünnungsmittels umsetzt.

6. Agrochemische Zusammensetzungen enthaltend mindestens eine Verbindung der Formel (I) oder eine Mischung von Verbindungen der Formel (I) gemäß einem der Ansprüche 1 bis 3 oder 4, sowie Streckmittel und/oder oberflächenaktive Stoffe.

7. Zusammensetzungen enthaltend mindestens eine Verbindung der Formel (I) oder eine Mischung von Verbindungen der Formel (I) gemäß einem der Ansprüche 1 bis 3 oder 4 sowie mindestens ein Salz der Formel (XI) 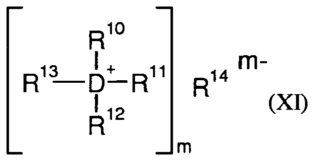 in welcher
D für Stickstoff oder Phosphor steht,
R¹⁰, R¹¹, R¹² und R¹³ unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
m für 1, 2, 3 oder 4 steht,
R¹⁴ für ein anorganisches oder organisches Anion steht.

8. Zusammensetzungen enthaltend mindestens eine Verbindung der Formel (I) oder eine Mischung von Verbindungen der Formel (I) gemäß einem der Ansprüche 1 bis 3 oder 4 sowie mindestens einen Penetrationsförderer der Formel (XII)
R-O-(-AO)ᵥ-R' (XII),
in welcher
R für geradkettiges oder verzweigtes Alkyl mit 4 bis 20 Kohlenstoffatomen steht,
R' für Wasserstoff, Methyl, Ethyl, n-Propyl, i-Propyl, n-Butyl, i-Butyl, t-Butyl, n-Pentyl oder n-Hexyl steht,
AO für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
V für Zahlen von 2 bis 30 steht.

9. Verfahren zur Herstellung agrochemischer Zusammensetzungen, **dadurch gekennzeichnet, dass** mindestens eine Verbindung der allgemeinen Formel (I) oder eine Mischung von Verbindungen der allgemeinen Formel (I) gemäß einem der Ansprüche 1 bis 3 oder 4 mit Streckmitteln und/oder oberflächenaktiven Stoffen gemischt wird.

10. Verwendung einer Verbindung der allgemeinen Formel (I) gemäß einem der Ansprüche 1 bis 3, einer Mischung von Verbindungen gemäß Anspruch 4 oder einer Zusammensetzung gemäß einem der Ansprüche 6 bis 8 zur Bekämpfung tierischer Schädlinge ausgenommen zur therapeutischen Behandlung des tierischen und menschlichen Körpers.

11. Verfahren zur Bekämpfung tierischer Schädlinge, **dadurch gekennzeichnet, dass** man eine Verbindung der allgemeinen Formel (I) gemäß einem der Ansprüche 1 bis 3, eine Mischung von Verbindungen der allgemeinen Formel (I) gemäß Anspruch 4 oder eine Zusammensetzung gemäß einem der Ansprüche 6 bis 8 auf tierische Schädlinge und/oder phytopathogene Pilze und/oder deren Lebensraum und/oder Saatgut einwirken lässt ausgenommen zur therapeutischen Behandlung des tierischen und menschlichen Körpers.

## Claims

1. Triazole-substituted anthranilamides of the general formula (I), 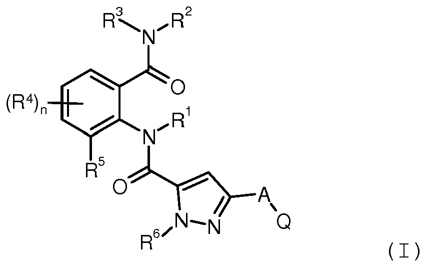 in which
R¹ is hydrogen, amino or hydroxyl or is C₁-C₆-alkyl, C₂-C₆-alkenyl, C₂-C₆-alkynyl or C₃-C₆-cycloalkyl each optionally substituted one or more times by identical or different substituents, the substituents being selectable independently of one another from halogen, cyano, nitro, hydroxyl, C₁-C₄-alkoxy, C₁-C₄-alkylthio, C₁-C₄-alkylsulphinyl, C₁-C₄-alkylsulphonyl, (C₁-C₄-alkoxy)carbonyl, C₁-C₄-alkylamino, di(C₁-C₄-alkyl)amino, C₃-C₆-cycloalkyl-amino or (C₁-C₄-alkyl)C₃-C₆-cycloalkylamino,
R² is hydrogen, C₁-C₆-alkyl, C₂-C₆-alkenyl, C₂-C₆-alkynyl, C₃-C₆-cycloalkyl, C₁-C₄-alkoxy, C₁-C₄-alkylamino, di (C₁-C₄-alkyl)amino, C₃-C₆-cycloalkylamino, C₂-C₆-alkoxycarbonyl or C₂-C₆-alkylcarbonyl,
R³ is hydrogen or is C₁-C₆-alkyl, C₁-C₆-alkoxy, C₂-C₆-alkenyl or C₂-C₆-alkynyl each optionally substituted one or more times by identical or different substituents, the substituents being selectable independently of one another from halogen, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₂-C₆-alkoxycarbonyl, C₂-C₆-alkylcarbonyl or C₃-C₆-trialkylsilyl,
R³ additionally is C₁-C₆-alkyl, C₁-C₆-alkoxy, C₂-C₆-alkenyl, C₂-C₆-alkynyl each optionally substituted one or more times by identical or different substituents, the substituents being selectable independently of one another from amino, C₃-C₆-cycloalkylamino or a 5- or 6-membered heteroaromatic ring,
R³ likewise additionally is C₃-C₁₂-cycloalkyl, C₃-C₁₂-cycloalkyl-C₁-C₆-alkyl and C₄-C₁₂-bicycloalkyl, the substituents being selectable independently of one another from halogen, cyano, nitro, hydroxyl, amino, C₁-C₆-alkyl, C₃-C₆-cycloalkyl, C₃-C₆-cycloalkylamino, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₂-C₆-alkoxycarbonyl, C₂-C₆-alkylcarbonyl, C₃-C₆-trialkylsilyl or a 5- or 6-membered heteroaromatic ring, R² and R³ may be joined to one another via two to six carbon atoms and form a ring which optionally further comprises another nitrogen, sulphur or oxygen atom and may optionally be substituted one to four times by C₁-C₂-alkyl, halogen, cyano, amino or C₁-C₂-alkoxy,
R², R³ additionally together are =S(C₁-C₄-alkyl)₂, =S(O)(C₁-C₄-alkyl)₂,
R⁴ is hydrogen, halogen, cyano, nitro, C₁-C₄-alkyl, C₁-C₄-haloalkyl, C₂-C₆-alkenyl, C₂-C₆-haloalkenyl, C₂-C₆-alkynyl, C₁-C₄-alkoxy, C₁-C₄-haloalkoxy, SF₅, C₁-C₄-alkylthio, C₁-C₄-alkylsulphinyl, C₁-C₄-alkylsulphonyl, C₁-C₄-haloalkylthio, C₁-C₄-halo-alkylsulphinyl, C₁-C₄-haloalkylsulphonyl, C₁-C₄-alkylamino, di (C₁-C₄-alkyl)amino, C₃-C₆-cycloalkylamino, (C₁-C₄-alkoxy)imino, (C₁-C₄-alkyl) (C₁-C₄-alkoxy) imino, (C₁-C₄-haloalkyl) (C₁-C₄-alkoxy)imino or C₃-C₆-trialkylsilyl, or
two radicals R⁴ form, via adjacent carbon atoms, a ring which is -(CH₂)₃-, -(CH₂)₄-, -(CH₂)₅-, -(CH=CH-)₂-, -OCH₂O-, -O(CH₂)₂O-, -OCF₂O-, -(CF₂)₂O-,-O(CF₂)₂O-, -(CH=CH-CH=N)- or -(CH=CH-N=CH)-, two radicals R⁴ additionally, via adjacent carbon atoms, form the fused-on rings below, which are optionally substituted one or more times by identical or different substituents, the substituents being selectable independently of one another from hydrogen, C₁-C₆-alkyl, C₃-C₆-cycloalkyl, C₁-C₆-haloalkyl, C₃-C₆-halocycloalkyl, halogen, C₁-C₆-alkoxy, C₁-C₄-alkylthio (C₁-C₆-alkyl), C₁-C₄-alkylsulphinyl (C₁-C₆-alkyl), C₁-C₄-alkylsulphonyl(C₁-C₆-alkyl), C₁-C₄-alkylamino, di(C₁-C₄-alkyl)amino or C₃-C₆-cycloalkylamino, 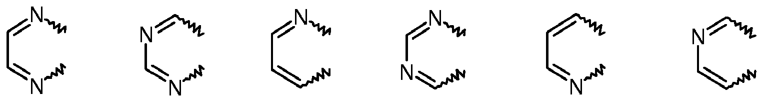 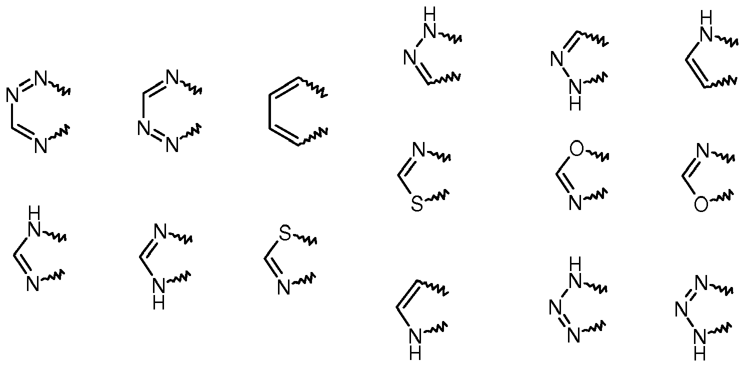
n is 0 to 3,
R⁵ is C₁-C₆-alkyl, C₃-C₆-cycloalkyl, C₁-C₆-haloalkyl, C₁-C₆-halocycloalkyl, C₂-C₆-alkenyl, C₂-C₆-haloalkenyl, C₂-C₆-alkynyl, C₂-C₆-haloalkynyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-haloalkylthio, C₁-C₄-haloalkylsulphinyl, C₁-C₄-haloalkylsulphonyl, halogen, cyano, nitro or C₃-C₆-trialkylsilyl,
R⁶ is hydrogen, C₁-C₆-alkyl, C₂-C₆-alkenyl, C₂-C₆-alkynyl, C₃-C₆-cycloalkyl, C₁-C₆-haloalkyl, C₂-C₆-haloalkenyl or 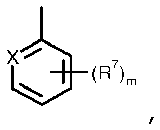
R⁶ additionally is C₃-C₆-cycloalkoxy,
R⁷ independently at each occurrence is hydrogen, C₁-C₆-alkyl, C₃-C₆-cycloalkyl, C₁-C₆-haloalkyl, halogen, cyano, nitro, C₁-C₄-alkoxy, C₁-C₄-haloalkoxy, C₁-C₄-alkylthio or C₁-C₄-haloalkylthio,
m is 0 to 4,
X is N, CH, CF, CC1, CBr or CI,
A is -CH₂-, -CH₂O-, -CH₂OCH₂-, -CH₂S-, -CH₂SCH₂-, - CH₂N(C₁-C₆-alkyl)-, -CH₂N(C₁-C₆-alkyl)CH₂-,-CH[CO₂(C₁-C₆-alkyl)]-, -CH(CN)-, -CH(C₁-C₆-alkyl)-, -C(di-C₁-C₆-alkyl)-, -CH₂CH₂-, -C=NO(C₁-C₆-alkyl)-,
Q is a 5-membered heteroaromatic ring or an aromatic 9-membered fused-on heterobicyclic ring system, the ring or the ring system comprising three nitrogens and the nitrogens in the ring or ring system occupying adjacent positions, and the bond of the ring or ring system to the radical A in formula (I) being via nitrogen; the ring or the ring system are optionally substituted one or more times by identical or different substituents, the substituents being selectable independently of one another from hydrogen, C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, CO₂H, CO₂NH₂, NO₂, OH, C₁-C₄-alkoxy, C₁-C₄-haloalkoxy, C₁-C₄-alkylthio, C₁-C₄-alkylsulphinyl, C₁-C₄-alkylsulphonyl, C₁-C₄-haloalkylthio, C₁-C₄-haloalkylsulphinyl, C₁-C₄-haloalkylsulphonyl, C₁-C₄-alkylamino, di(C₁-C₄-alkyl)amino, C₃-C₆-cycloalkylamino, (C₁-C₆-alkyl)carbonyl, (C₁-C₆-alkoxy)carbonyl, (C₁-C₆-alkyl)aminocarbonyl, di(C₁-C₄-alkyl)aminocarbonyl, tri (C₁-C₂) alkylsilyl, (C₁-C₄-alkyl) (C₁-C₄-alkoxy)imino,
or where the substituents are selectable independently of one another from phenyl or a 5- or 6-membered heteroaromatic ring, where phenyl or the ring may optionally be substituted one or more times by identical or different C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, NO₂, OH, C₁-C₄-alkoxy and/or C₁-C₄-haloalkoxy substituents;
the compounds of the general formula (I) further comprise N-oxides and salts.

2. Anthranilamides of the general formula (I) according to Claim 1, in which
R¹ is hydrogen, C₁-C₆-alkyl, C₂-C₆-alkenyl, C₂-C₆-alkynyl, C₃-C₆-cycloalkyl, cyano(C₁-C₆-alkyl), C₁-C₆-haloalkyl, C₂-C₆-haloalkenyl, C₂-C₆-haloalkynyl, C₁-C₄-alkoxy-C₁-C₄-alkyl, C₁-C₄-alkylthio-C₁-C₄-alkyl, C₁-C₄-alkylsulphinyl-C₁-C₄-alkyl or C₁-C₄-alkylsulphonyl-C₁-C₄-alkyl,
R² is hydrogen or C₁-C₆-alkyl,
R³ is hydrogen or is C₁-C₆-alkyl, C₁-C₆-alkoxy, C₂-C₆-alkenyl or C₂-C₆-alkynyl each optionally substituted one or more times by identical or different substituents, the substituents being selectable independently of one another from halogen, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₂-C₆-alkoxycarbonyl, C₂-C₆-alkylcarbonyl or C₃-C₆-tri-alkylsilyl,
R³ additionally is C₃-C₁₂-cycloalkyl and C₄-C₁₀-bicycloalkyl, the substituents being selectable independently of one another from halogen, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₂-C₆-alkoxycarbonyl, C₂-C₆-alkylcarbonyl or C₃-C₆-trialkylsilyl,
R⁴ is hydrogen, C₁-C₄-alkyl, C₁-C₄-haloalkyl, halogen, cyano, C₁-C₄-alkoxy, C₁-C₄-haloalkoxy, C₁-C₄-alkylthio or C₁-C₄-haloalkylthio,
furthermore, two adjacent radicals R⁴ are -(CH₂)₃-, -(CH₂)₄-, -(CH₂)₅-, -(CH=CH-)₂-, -OCH₂O-, -O(CH₂)₂O-, -OCF₂O-, -(CF₂)₂O-, -O(CF₂)₂O-, -(CH=CH-CH=N)- or - (CH=CH-N=CH)-,
n is 1, 2 or 3,
R⁵ is C₁-C₄-alkyl, C₃-C₆-cycloalkyl, C₁-C₄-haloalkyl, C₁-C₆-halocycloalkyl, C₂-C₆-alkenyl, C₂-C₄-haloalkenyl, C₂-C₄-alkynyl, C₂-C₄-haloalkynyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-haloalkylthio, C₁-C₄-haloalkylsulphinyl, C₁-C₄-haloalkylsulphonyl, halogen, cyano, nitro or C₃-C₆-trialkylsilyl,
R⁶ is C₁-C₆-alkyl or 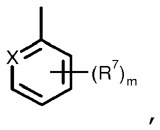
R⁶ additionally is C₃-C₆-cycloalkoxy,
R⁷ independently at each occurrence is hydrogen, halogen, cyano, C₁-C₄-alkyl, C₁-C₄-alkoxy, C₁-C₄-haloalkyl or C₁-C₄-haloalkoxy,
m is 1, 2 or 3,
X is N, CH, CF, CC1, CBr or CI,
A is -CH₂-, -CH₂O-, -CH₂OCH₂-, -CH₂S-, -CH₂SCH₂-,-CH₂N(C₁-C₆-alkyl) -CH₂N (C₁-C₆-alkyl) CH₂-, -CH(CN)-, -CH (C₁-C₆-alkyl)-, -C(di-C₁-C₆-alkyl)-, -CH₂CH₂-,-C=NO(C₁-C₆-alkyl)-,
Q is a 5-membered heteroaromatic ring or an aromatic 9-membered fused-on heterobicyclic ring system, the ring or the ring system comprising three nitrogens and the nitrogens in the ring or ring system occupying adjacent positions, and the bond of the ring or ring system to the radical A in formula (I) being via nitrogen; the ring or the ring system are optionally substituted one or more times by identical or different substituents, the substituents being selectable independently of one another from hydrogen, C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, CO₂H, CO₂NH₂, NO₂, OH, C₁-C₄-alkoxy, C₁-C₄-haloalkoxy, C₁-C₄-alkylthio, C₁-C₄-alkylsulphinyl, C₁-C₄-alkylsulphonyl, C₁-C₄-haloalkylthio, C₁-C₄-haloalkylsulphinyl, C₁-C₄-haloalkylsulphonyl, C₁-C₄-alkylamino, di(C₁-C₄-alkyl)amino, C₃-C₆-cycloalkylamino, (C₁-C₆-alkyl)carbonyl, (C₁-C₆-alkoxy)carbonyl, (C₁-C₆-alkyl)aminocarbonyl, di(C₁-C₄-alkyl)aminocarbonyl, tri (C₁-C₂) alkylsilyl, (C₁-C₄-alkyl) (C₁-C₄-alkoxy)imino,
or where the substituents may be selected independently of one another from phenyl or a 5-or 6-membered heteroaromatic ring, where phenyl or the ring may optionally be substituted one or more times by identical or different C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, NO₂, OH, C₁-C₄-alkoxy and/or C₁-C₄-haloalkoxy substituents.

3. Anthranilamides of the general formula (I) according to Claim 1 or 2, in which
R¹ is hydrogen, methyl, cyclopropyl, cyanomethyl, methoxymethyl, methylthiomethyl, methylsulphinylmethyl or methylsulphonylmethyl,
R² is hydrogen or methyl,
R³ is hydrogen or is C₁-C₆-alkyl or C₁-C₆-alkoxy each optionally substituted one or more times by identical or different substituents, the substituents being selectable independently of one another from halogen, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₂-C₆-alkoxycarbonyl, C₂-C₆-alkylcarbonyl or C₃-C₆-tri-alkylsilyl,
R³ additionally is C₃-C₆-cycloalkyl optionally substituted one or more times by identical or different substituents, the substituents being selectable independently of one another from halogen, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₂-C₆-alkoxy-carbonyl, C₂-C₆-alkylcarbonyl or C₃-C₆-trialkylsilyl,
R⁴ is hydrogen, C₁-C₄-alkyl, C₁-C₂-haloalkyl, halogen, cyano or C₁-C₂-haloalkoxy,
moreover, two adjacent radicals R⁴ are -(CH₂)₄-, - (CH=CH-)₂-, -O(CH₂)₂O-, -O(CF₂)₂O-, -(CH=CH-CH=N)-or -(CH=CH-N=CH)-,
n is 1 or 2,
R⁵ is C₁-C₄-alkyl, C₃-C₆-cycloalkyl, C₁-C₄-haloalkyl, C₁-C₆-halocycloalkyl, C₂-C₆-alkenyl, C₂-C₄-haloalkenyl, C₂-C₄-alkynyl, C₂-C₄-haloalkynyl, C₁-C₄-alkoxy, C₁-C₄-haloalkoxy, fluorine, chlorine, bromine, iodine, cyano, nitro or C₃-C₆-trialkylsilyl,
R⁶ is methyl or 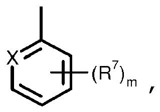
R⁷ independently at each occurrence is hydrogen, halogen or C₁-C₄-haloalkyl,
m is 1 or 2,
X is N, CH, CF, CC1 or CBr,
A is -CH₂-, -CH(CH₃)₂, C(CH₃)₂ or CH₂CH₂,
A additionally is -CH(CN)-,
Q is a 5-membered heteroaromatic ring or an aromatic 9-membered fused-on heterobicyclic ring system, the ring or the ring system comprising three nitrogens and the nitrogens in the ring or ring system occupying adjacent positions, and the bond of the ring or ring system to the radical A in formula (I) being via nitrogen; the ring or the ring system are optionally substituted one or more times by identical or different substituents, the substituents being selectable independently of one another from hydrogen, C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, CO₂H, CO₂NH₂, NO₂, OH, C₁-C₄-alkoxy, C₁-C₄-haloalkoxy, C₁-C₄-alkylthio, C₁-C₄-alkylsulphinyl, C₁-C₄-alkylsulphonyl, C₁-C₄-haloalkylthio, C₁-C₄-haloalkylsulphinyl, C₁-C₄-haloalkylsulphonyl, C₁-C₄-alkylamino, di(C₁-C₄-alkyl)amino, C₃-C₆-cycloalkylamino, (C₁-C₆-alkyl)carbonyl, (C₁-C₆-alkoxy)carbonyl, (C₁-C₆-alkyl)aminocarbonyl, di(C₁-C₄-alkyl)aminocarbonyl, tri (C₁-C₂) alkylsilyl, (C₁-C₄-alkyl) (C₁-C₄-alkoxy)imino,
or where the substituents may be selected independently of one another from phenyl or a 5-or 6-membered heteroaromatic ring, where phenyl or the ring may be optionally substituted one or more times by identical or different C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, NO₂, OH, C₁-C₄-alkoxy and/or C₁-C₄-haloalkoxy substituents.

4. Mixtures of compounds of the general formula (I) according to any of Claims 1 to 3, the ring or the ring system Q being bonded to the radical (A) in each case via different carbon atoms or nitrogen atoms.

5. Process for preparing compounds of the general formula (I) according to any of Claims 1 to 3, **characterized in that**
(A) anilines of the formula (II) 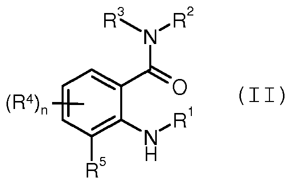 in which A, R¹, R², R³, R⁴, R⁵ and n have the definitions indicated above
are reacted with carbonyl chlorides of the formula (III) 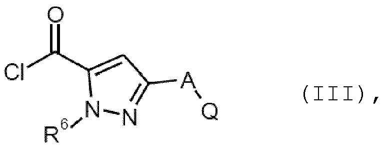 in which R⁶, A and Q have the definitions indicated above, in the presence of an acid-binding agent,
(B) anilines of the formula (II) 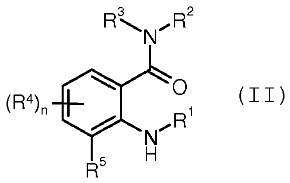 in which A, R¹, R², R³, R⁴, R⁵ and n have the definitions indicated above
are reacted with a carboxylic acid of the formula (IV) 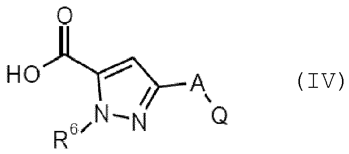 in which R⁶, A and Q have the definitions indicated above
in the presence of a condensing agent, or
(C) for the synthesis of anthranilamides of the formula (I) in which R¹ is hydrogen, benzoxazinones of the formula (V) 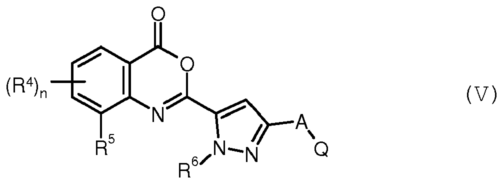 in which R4, R5, R6, A, Q and n have the definitions indicated above
are reacted with an amine of the formula (VI) 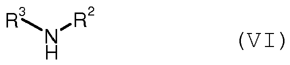 in which R² and R³ have the definitions indicated above,
in the presence of a diluent.

6. Agrochemical compositions comprising at least one compound of the formula (I) or a mixture of compounds of the formula (I) according to any of Claims 1 to 3 and 4, and also extenders and/or surfactants.

7. Compositions comprising at least one compound of the formula (I) or a mixture of compounds of the formula (I) according to any of Claims 1 to 3 and 4, and also at least one salt of the formula (XI) 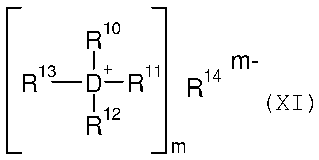 in which
D is nitrogen or phosphorus,
R¹⁰, R¹¹, R¹² and R¹³ independently of one another are hydrogen or each optionally substituted C₁-C₈-alkyl or singly or multiply unsaturated, optionally substituted C₁-C₈-alkylene, the substituents being selectable from halogen, nitro and cyano,
m is 1, 2, 3 or 4,
R¹⁴ is an inorganic or organic anion.

8. Compositions comprising at least one compound of the formula (I) or a mixture of compounds of the formula (I) according to any of Claims 1 to 3 and 4, and also at least one penetrant of the formula (XII)
R-O-(-AO)ᵥ-R' (XII),
in which
R is straight-chain or branched alkyl having 4 to 20 carbon atoms,
R' is hydrogen, methyl, ethyl, n-propyl, isopropyl, n-butyl, isobutyl, tert-butyl, n-pentyl or n-hexyl, AOis an ethylene oxide radical, a propylene oxide radical, or a butylene oxide radical or is mixtures of ethylene oxide and propylene oxide radicals or butylene oxide radicals, and
V stands for numbers from 2 to 30.

9. Process for preparing agrochemical compositions, **characterized in that** at least one compound of the general formula (I) or a mixture of compounds of the general formula (I) according to any of Claims 1 to 3 and 4 is mixed with extenders and/or surfactants.

10. Use of a compound of the general formula (I) according to any of Claims 1 to 3, of a mixture of compounds according to Claim 4 or of a composition according to any of Claims 6 to 8 for controlling animal pests, with the exception of the therapeutic treatment of the animal and human body.

11. Method for controlling animal pests, **characterized in that** a compound of the general formula (I) according to any of Claims 1 to 3, a mixture of compounds of the general formula (I) according to Claim 4 or a composition according to any of Claims 6 to 8 is caused to act on animal pests and/or phytopathogenic fungi and/or their habitat and/or seed, with the exception of the therapeutic treatment of the animal and human body.

## Revendications

1. Amides de l'acide anthranilique à substitution triazole de formule générale (I) 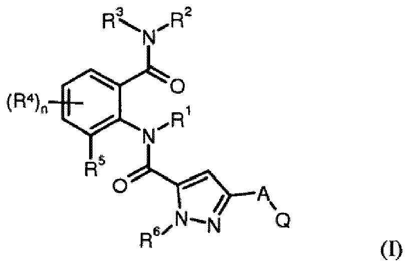 dans lesquels
R¹ représente hydrogène, amino, hydroxy ou alkyle en C₁-C₆, alcényle en C₂-C₆, alcynyle en C₂-C₆ ou cycloalkyle en C₃-C₆, chacun éventuellement substitués une ou plusieurs fois, de manière identique ou différente, les substituants pouvant être choisis indépendamment les uns des autres parmi halogène, cyano, nitro, hydroxy, alcoxy en C₁-C₄, alkylthio en C₁-C₄, alkylsulfinyle en C₁-C₄, alkylsulfonyle en C₁-C₄, (alcoxy en C₁-C₄)carbonyle, alkylamino en C₁-C₄, di-(alkyle en C₁-C₄)amino, cycloalkylamino en C₃-C₆ ou (alkyle en C₁-C₄)-cycloalkylamino en C₃-C₆,
R² représente hydrogène, alkyle en C₁-C₆, alcényle en C₂-C₆, alcynyle en C₂-C₆, cycloalkyle en C₃-C₆, alcoxy en C₁-C₄, alkylamino en C₁-C₄, di- (alkyle en C₁-C₄)amino, cycloalkylamino en C₃-C₆, alcoxycarbonyle en C₂-C₆ ou alkylcarbonyle en C₂-C₆,
R³ représente hydrogène ou alkyle en C₁-C₆, alcoxy en C₁-C₆, alcényle en C₂-C₆, alcynyle en C₂-C₆, chacun éventuellement substitués une ou plusieurs fois, de manière identique ou différente, les substituants pouvant être choisis indépendamment les uns des autres parmi halogène, cyano, nitro, hydroxy, alkyle en C₁-C₆, cycloalkyle en C₃-C₆, alcoxy en C₁-C₄, haloalcoxy en C₁-C₄, alkylthio en C₁-C₄, alkylsulfinyle en C₁-C₄, alkylsulfonyle en C₁-C₄, alkylsulfimino en C₁-C₄, alkylsulfimino en C₁-C₄-alkyle en C₁-C₄, alkylsulfimino en C₁-C₄-alkylcarbonyle en C₂-C₅, alkylsulfoximino en C₁-C₄, alkylsulfoximino en C₁-C₄-alkyle en C₁-C₄, alkylsulfoximino en C₁-C₄-alkylcarbonyle en C₂-C₅, alcoxycarbonyle en C₂-C₆, alkylcarbonyle en C₂-C₆ ou trialkylsilyle en C₃-C₆,
R³ représente également alkyle en C₁-C₆, alcoxy en C₁-C₆, alcényle en C₂-C₆, alcynyle en C₂-C₆, chacun éventuellement substitués une ou plusieurs fois, de manière identique ou différente, les substituants pouvant être choisis indépendamment les uns des autres parmi amino, cycloalkylamino en C₃-C₆ ou un cycle hétéroaromatique à 5 ou 6 éléments,
R³ représente également cycloalkyle en C₃-C₁₂, cycloalkyle en C₃-C₁₂-alkyle en C₁-C₆ et bicycloalkyle en C₄-C₁₂, les substituants pouvant être choisis indépendamment les uns des autres parmi halogène, cyano, nitro, hydroxy, amino, alkyle en C₁-C₆, cycloalkyle en C₃-C₆, cycloalkylamino en C₃-C₆, alcoxy en C₁-C₄, haloalcoxy en C₁-C₄, alkylthio en C₁-C₄, alkylsulfinyle en C₁-C₄, alkylsulfonyle en C₁-C₄, alkylsulfimino en C₁-C₄, alkylsulfimino en C₁-C₄-alkyle en C₁-C₄, alkylsulfimino en C₁-C₄-alkylcarbonyle en C₂-C₅, alkylsulfoximino en C₁-C₄, alkylsulfoximino en C₁-C₄-alkyle en C₁-C₄, alkylsulfoximino en C₁-C₄-alkylcarbonyle en C₂-C₅, alcoxycarbonyle en C₂-C₆, alkylcarbonyle en C₂-C₆, trialkylsilyle en C₃-C₆ ou un cycle hétéroaromatique à 5 ou 6 éléments,
R² et R³ peuvent être reliés l'un à l'autre par le biais de deux à six atomes de carbone et former un cycle, qui contient éventuellement en outre un atome d'azote, de soufre ou d'oxygène supplémentaire et qui peut éventuellement être substitué une à quatre fois avec alkyle en C₁-C₂, halogène, cyano, amino ou alcoxy en C₁-C₂,
R², R³ représentent également ensemble =S(alkyle en C₁-C₄)₂, =S(O) (alkyle en C₁-C₄)₂,
R⁴ représente hydrogène, halogène, cyano, nitro, alkyle en C₁-C₄, haloalkyle en C₁-C₄, alcényle en C₂-C₆, haloalcényle en C₂-C₆, alcynyle en C₂-C₆, alcoxy en C₁-C₄, haloalcoxy en C₁-C₄, SF₅, alkylthio en C₁-C₄, alkylsulfinyle en C₁-C₄, alkylsulfonyle en C₁-C₄, haloalkylthio en C₁-C₄, haloalkylsulfinyle en C₁-C₄, haloalkylsulfonyle en C₁-C₄, alkylamino en C₁-C₄, di-(alkyle en C₁-C₄)amino, cycloalkylamino en C₃-C₆, (alcoxy en C₁-C₄)imino, (alkyle en C₁-C₄) (alcoxy en C₁-C₄)imino, (haloalkyle en C₁-C₄) (alcoxy en C₁-C₄)imino ou trialkylsilyle en C₃-C₆, ou
deux R⁴ forment un cycle par le biais d'atomes de carbone voisins, qui représente -(CH₂)₃-, -(CH₂)₄-, - (CH₂)₅-, -(CH=CH-)₂-, -OCH₂O-, -O(CH₂)₂O-, -OCF₂O-, (CF₂)₂O-, -O(CF₂)₂O-, -(CH=CH-CH=N)- ou -(CH=CH-N=CH)-, deux R⁴ forment également par le biais d'atomes de carbone voisins les cycles annelés suivants, qui sont éventuellement substitués une ou plusieurs fois, de manière identique ou différente, les substituants pouvant être choisis indépendamment les uns des autres parmi hydrogène, alkyle en C₁-C₆, cycloalkyle en C₃-C₆, haloalkyle en C₁-C₆, halocycloalkyle en C₃-C₆, halogène, alcoxy en C₁-C₆, alkylthio en C₁-C₄-(alkyle en C₁-C₆), alkylsulfinyle en C₁-C₄-(alkyle en C₁-C₆), alkylsulfonyle en C₁-C₄-(alkyle en C₁-C₆), alkylamino en C₁-C₄, di-(alkyle en C₁-C₄)amino ou cycloalkylamino en C₃-C₆, 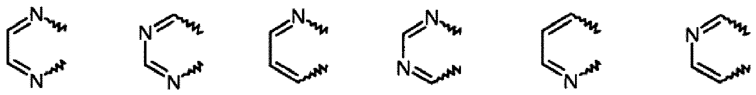 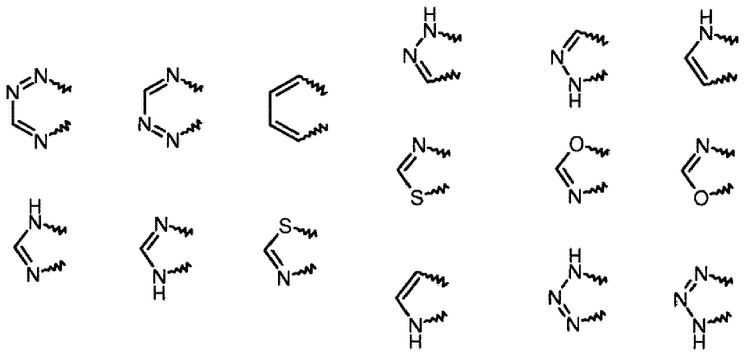
n représente 0 à 3,
R⁵ représente alkyle en C₁-C₆, cycloalkyle en C₃-C₆, haloalkyle en C₁-C₆, halocycloalkyle en C₁-C₆, alcényle en C₂-C₆, haloalcényle en C₂-C₆, alcynyle en C₂-C₆, haloalcynyle en C₂-C₆, alcoxy en C₁-C₄, haloalcoxy en C₁-C₄, alkylthio en C₁-C₄, alkylsulfinyle en C₁-C₄, alkylsulfonyle en C₁-C₄, haloalkylthio en C₁-C₄, haloalkylsulfinyle en C₁-C₄, haloalkylsulfonyle en C₁-C₄, halogène, cyano, nitro ou trialkylsilyle en C₃-C₆,
R⁶ représente hydrogène, alkyle en C₁-C₆, alcényle en C₂-C₆, alcynyle en C₂-C₆, cycloalkyle en C₃-C₆, haloalkyle en C₁-C₆, haloalcényle en C₂-C₆, ou 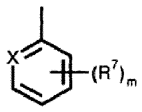
R⁶ représente également cycloalcoxy en C₃-C₆,
les R⁷ représentent indépendamment les uns des autres hydrogène, alkyle en C₁-C₆, cycloalkyle en C₃-C₆, haloalkyle en C₁-C₆, halogène, cyano, nitro, alcoxy en C₁-C₄, haloalcoxy en C₁-C₄, alkylthio en C₁-C₄ ou haloalkylthio en C₁-C₄,
m représente 0 à 4,
X représente N, CH, CF, CCl, CBr ou CI,
A représente -CH₂-, -CH₂O-, -CH₂OCH₂-, -CH₂S-, - CH₂SCH₂-, -CH₂N (alkyle en C₁-C₆)-, -CH₂N(alkyle en C₁-C₆)CH₂-, -CH[CO₂(alkyle en C₁-C₆)]-, -CH(CN)-, -CH (alkyle en C₁-C₆)-, -C(di-alkyle en C₁-C₆)-, -CH₂CH₂-, - C=NO (alkyle en C₁-C₆)-,
Q représente un cycle hétéroaromatique à 5 éléments ou un système cyclique hétérobicyclique annelé aromatique à 9 éléments, le cycle ou le système cyclique contenant trois azotes et les azotes occupant des positions voisines dans le cycle ou le système cyclique et la liaison du cycle ou du système cyclique au radical A dans la formule (I) ayant lieu par un azote ; le cycle ou le système cyclique étant éventuellement substitué une ou plusieurs fois, de manière identique ou différente, les substituants pouvant être choisis indépendamment les uns des autres parmi hydrogène, alkyle en C₁-C₆, alcényle en C₂-C₆, alcynyle en C₂-C₆, cycloalkyle en C₃-C₆, haloalkyle en C₁-C₆, haloalcényle en C₂-C₆, haloalcynyle en C₂-C₆, halocycloalkyle en C₃-C₆, halogène, CN, CO₂H, CO₂NH₂, NO₂, OH, alcoxy en C₁-C₄, haloalcoxy en C₁-C₄, alkylthio en C₁-C₄, alkylsulfinyle en C₁-C₄, alkylsulfonyle en C₁-C₄, haloalkylthio en C₁-C₄, haloalkylsulfinyle en C₁-C₄, haloalkylsulfonyle en C₁-C₄, alkylamino en C₁-C₄, di-(alkyle en C₁-C₄)amino, cycloalkylamino en C₃-C₆, (alkyle en C₁-C₆)carbonyle, (alcoxy en C₁-C₆)carbonyle, (alkyle en C₁-C₆)aminocarbonyle, di-(alkyle en C₁-C₄)aminocarbonyle, tri-alkylsilyle en (C₁-C₂), (alkyle en C₁-C₄) (alcoxy en C₁-C₄)imino,
ou les substituants pouvant être choisis indépendamment les uns des autres parmi phényle ou un cycle hétéroaromatique à 5 ou 6 éléments, le phényle ou le cycle pouvant éventuellement être substitué une ou plusieurs fois, de manière identique ou différente, avec alkyle en C₁-C₆, alcényle en C₂-C₆, alcynyle en C₂-C₆, cycloalkyle en C₃-C₆, haloalkyle en C₁-C₆, haloalcényle en C₂-C₆, haloalcynyle en C₂-C₆, halocycloalkyle en C₃-C₆, halogène, CN, NO₂, OH, alcoxy en C₁-C₄, haloalcoxy en C₁-C₄,
les composés de formule générale (I) comprenant en outre les N-oxydes et les sels.

2. Amides de l'acide anthranilique de formule générale (I) selon la revendication 1, dans lesquels
R¹ représente hydrogène, alkyle en C₁-C₆, alcényle en C₂-C₆, alcynyle en C₂-C₆, cycloalkyle en C₃-C₆, cyano(alkyle en C₁-C₆), haloalkyle en C₁-C₆, haloalcényle en C₂-C₆, haloalcynyle en C₂-C₆, alcoxy en C₁-C₄-alkyle en C₁-C₄, alkylthio en C₁-C₄-alkyle en C₁-C₄, alkylsulfinyle en C₁-C₄-alkyle en C₁-C₄ ou alkylsulfonyle en C₁-C₄-alkyle en C₁-C₄,
R² représente hydrogène ou alkyle en C₁-C₆,
R³ représente hydrogène ou alkyle en C₁-C₆, alcoxy en C₁-C₆, alcényle en C₂-C₆, alcynyle en C₂-C₆, chacun éventuellement substitués une ou plusieurs fois, de manière identique ou différente, les substituants pouvant être choisis indépendamment les uns des autres parmi halogène, cyano, nitro, hydroxy, alkyle en C₁-C₆, cycloalkyle en C₃-C₆, alcoxy en C₁-C₄, haloalcoxy en C₁-C₄, alkylthio en C₁-C₄, alkylsulfinyle en C₁-C₄, alkylsulfonyle en C₁-C₄, alkylsulfimino en C₁-C₄, alkylsulfimino en C₁-C₄-alkyle en C₁-C₄, alkylsulfimino en C₁-C₄-alkylcarbonyle en C₂-C₅, alkylsulfoximino en C₁-C₄, alkylsulfoximino en C₁-C₄-alkyle en C₁-C₄, alkylsulfoximino en C₁-C₄-alkylcarbonyle en C₂-C₅, alcoxycarbonyle en C₂-C₆, alkylcarbonyle en C₂-C₆ ou trialkylsilyle en C₃-C₆,
R³ représente également cycloalkyle en C₃-C₁₂ et bicycloalkyle en C₄-C₁₀, les substituants pouvant être choisis indépendamment les uns des autres parmi halogène, cyano, nitro, hydroxy, alkyle en C₁-C₆, cycloalkyle en C₃-C₆, alcoxy en C₁-C₄, haloalcoxy en C₁-C₄, alkylthio en C₁-C₄, alkylsulfinyle en C₁-C₄, alkylsulfonyle en C₁-C₄, alkylsulfimino en C₁-C₄, alkylsulfimino en C₁-C₄-alkyle en C₁-C₄, alkylsulfimino en C₁-C₄-alkylcarbonyle en C₂-C₅, alkylsulfoximino en C₁-C₄, alkylsulfoximino en C₁-C₄-alkyle en C₁-C₄, alkylsulfoximino en C₁-C₄-alkylcarbonyle en C₂-C₅, alcoxycarbonyle en C₂-C₆, alkylcarbonyle en C₂-C₆ ou trialkylsilyle en C₃-C₆,
R⁴ représente hydrogène, alkyle en C₁-C₄, haloalkyle en C₁-C₄, halogène, cyano, alcoxy en C₁-C₄, haloalcoxy en C₁-C₄, alkylthio en C₁-C₄ ou haloalkylthio en C₁-C₄,
deux radicaux R⁴ voisins représentent également -(CH₂)₃-, -(CH₂)₄-, -(CH₂)₅-, -(CH=CH-)₂-, -OCH₂O-, -O(CH₂)₂O-, - OCF₂O-, -(CF₂)₂O-, -O(CF₂)₂O-, -(CH=CH-CH=N)- ou -(CH=CH-N=CH)-,
n représente 1, 2 ou 3,
R⁵ représente alkyle en C₁-C₄, cycloalkyle en C₃-C₆, haloalkyle en C₁-C₄, halocycloalkyle en C₁-C₆, alcényle en C₂-C₆, haloalcényle en C₂-C₄, alcynyle en C₂-C₄, haloalcynyle en C₂-C₄, alcoxy en C₁-C₄, haloalcoxy en C₁-C₄, alkylthio en C₁-C₄, alkylsulfinyle en C₁-C₄, alkylsulfonyle en C₁-C₄, haloalkylthio en C₁-C₄, haloalkylsulfinyle en C₁-C₄, haloalkylsulfonyle en C₁-C₄, halogène, cyano, nitro ou trialkylsilyle en C₃-C₆,
R⁶ représente alkyle en C₁-C₆ ou 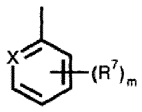 R⁶ représente également cycloalcoxy en C₃-C₆,
les R⁷ représentent indépendamment les uns des autres hydrogène, halogène, cyano, alkyle en C₁-C₄, alcoxy en C₁-C₄, haloalkyle en C₁-C₄ ou haloalcoxy en C₁-C₄,
m représente 1, 2 ou 3,
X représente N, CH, CF, CCl, CBr ou CI,
A représente -CH₂-, -CH₂O-, -CH₂OCH₂-, -CH₂S-, - CH₂SCH₂-, -CH₂N (alkyle en C₁-C₆)-, -CH₂N (alkyle en C₁-C₆)CH₂-, -CH(CN)-, -CH (alkyle en C₁-C₆)-, -C(di-alkyle en C₁-C₆)-, -CH₂CH₂-, -C=NO(alkyle en C₁-C₆)-,
Q représente un cycle hétéroaromatique à 5 éléments ou un système cyclique hétérobicyclique annelé aromatique à 9 éléments, le cycle ou le système cyclique contenant trois azotes et les azotes occupant des positions voisines dans le cycle ou le système cyclique et la liaison du cycle ou du système cyclique au radical A dans la formule (I) ayant lieu par un azote ; le cycle ou le système cyclique étant éventuellement substitué une ou plusieurs fois, de manière identique ou différente, les substituants pouvant être choisis indépendamment les uns des autres parmi hydrogène, alkyle en C₁-C₆, alcényle en C₂-C₆, alcynyle en C₂-C₆, cycloalkyle en C₃-C₆, haloalkyle en C₁-C₆, haloalcényle en C₂-C₆, haloalcynyle en C₂-C₆, halocycloalkyle en C₃-C₆, halogène, CN, CO₂H, CO₂NH₂, NO₂, OH, alcoxy en C₁-C₄, haloalcoxy en C₁-C₄, alkylthio en C₁-C₄, alkylsulfinyle en C₁-C₄, alkylsulfonyle en C₁-C₄, haloalkylthio en C₁-C₄, haloalkylsulfinyle en C₁-C₄, haloalkylsulfonyle en C₁-C₄, alkylamino en C₁-C₄, di-(alkyle en C₁-C₄)amino, cycloalkylamino en C₃-C₆, (alkyle en C₁-C₆)carbonyle, (alcoxy en C₁-C₆)carbonyle, (alkyle en C₁-C₆)aminocarbonyle, di-(alkyle en C₁-C₄)aminocarbonyle, tri-alkylsilyle en (C₁-C₂), (alkyle en C₁-C₄) (alcoxy en C₁-C₄)imino,
ou les substituants pouvant être choisis indépendamment les uns des autres parmi phényle ou un cycle hétéroaromatique à 5 ou 6 éléments, le phényle ou le cycle pouvant éventuellement être substitué une ou plusieurs fois, de manière identique ou différente, avec alkyle en C₁-C₆, alcényle en C₂-C₆, alcynyle en C₂-C₆, cycloalkyle en C₃-C₆, haloalkyle en C₁-C₆, haloalcényle en C₂-C₆, haloalcynyle en C₂-C₆, halocycloalkyle en C₃-C₆, halogène, CN, NO₂, OH, alcoxy en C₁-C₄, haloalcoxy en C₁-C₄.

3. Amides de l'acide anthranilique de formule générale (I) selon la revendication 1 ou 2, dans lesquels
R¹ représente hydrogène, méthyle, cyclopropyle, cyanométhyle, méthoxyméthyle, méthylthiométhyle, méthylsulfinylméthyle ou méthylsulfonylméthyle,
R² représente hydrogène ou méthyle,
R³ représente hydrogène ou alkyle en C₁-C₆, alcoxy en C₁-C₆, chacun éventuellement substitués une ou plusieurs fois, de manière identique ou différente, les substituants pouvant être choisis indépendamment les uns des autres parmi halogène, cyano, nitro, hydroxy, alkyle en C₁-C₆, cycloalkyle en C₃-C₆, alcoxy en C₁-C₄, haloalcoxy en C₁-C₄, alkylthio en C₁-C₄, alkylsulfinyle en C₁-C₄, alkylsulfonyle en C₁-C₄, alkylsulfimino en C₁-C₄, alkylsulfimino en C₁-C₄-alkyle en C₁-C₄, alkylsulfimino en C₁-C₄-alkylcarbonyle en C₂-C₅, alkylsulfoximino en C₁-C₄, alkylsulfoximino en C₁-C₄-alkyle en C₁-C₄, alkylsulfoximino en C₁-C₄-alkylcarbonyle en C₂-C₅, alcoxycarbonyle en C₂-C₆, alkylcarbonyle en C₂-C₆ ou trialkylsilyle en C₃-C₆,
R³ représente également cycloalkyle en C₃-C₆ éventuellement substitué une ou plusieurs fois, de manière identique ou différente, les substituants pouvant être choisis indépendamment les uns des autres parmi halogène, cyano, nitro, hydroxy, alkyle en C₁-C₆, cycloalkyle en C₃-C₆, alcoxy en C₁-C₄, haloalcoxy en C₁-C₄, alkylthio en C₁-C₄, alkylsulfinyle en C₁-C₄, alkylsulfonyle en C₁-C₄, alkylsulfimino en C₁-C₄, alkylsulfimino en C₁-C₄-alkyle en C₁-C₄, alkylsulfimino en C₁-C₄-alkylcarbonyle en C₂-C₅, alkylsulfoximino en C₁-C₄, alkylsulfoximino en C₁-C₄-alkyle en C₁-C₄, alkylsulfoximino en C₁-C₄-alkylcarbonyle en C₂-C₅, alcoxycarbonyle en C₂-C₆, alkylcarbonyle en C₂-C₆ ou trialkylsilyle en C₃-C₆,
R⁴ représente hydrogène, alkyle en C₁-C₄, haloalkyle en C₁-C₂, halogène, cyano ou haloalcoxy en C₁-C₂,
deux radicaux R⁴ voisins représentent également -(CH₂)₄-, -(CH=CH-)₂-_{,} -O(CH₂)₂O-, -O(CF₂)₂O-, -(CH=CH-CH=N)- ou -(CH=CH-N=CH)-,
n représente 1 ou 2,
R⁵ représente alkyle en C₁-C₄, cycloalkyle en C₃-C₆, haloalkyle en C₁-C₄, halocycloalkyle en C₁-C₆, alcényle en C₂-C₆, haloalcényle en C₂-C₄, alcynyle en C₂-C₄, haloalcynyle en C₂-C₄, alcoxy en C₁-C₄, haloalcoxy en C₁-C₄, fluor, chlore, brome, iode, cyano, nitro ou trialkylsilyle en C₃-C₆,
R⁶ représente méthyle ou 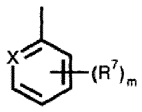 les R⁷ représentent indépendamment les uns des autres hydrogène, halogène ou haloalkyle en C₁-C₄,
m représente 1 ou 2,
X représente N, CH, CF, CCl ou CBr,
A représente -CH₂-, -CH(CH₃)-, C(CH₃)₂ ou CH₂CH₂,
A représente également -CH(CN)-,
Q représente un cycle hétéroaromatique à 5 éléments ou un système cyclique hétérobicyclique annelé aromatique à 9 éléments, le cycle ou le système cyclique contenant trois azotes et les azotes occupant des positions voisines dans le cycle ou le système cyclique et la liaison du cycle ou du système cyclique au radical A dans la formule (I) ayant lieu par un azote ; le cycle ou le système cyclique étant éventuellement substitué une ou plusieurs fois, de manière identique ou différente, les substituants pouvant être choisis indépendamment les uns des autres parmi hydrogène, alkyle en C₁-C₆, alcényle en C₂-C₆, alcynyle en C₂-C₆, cycloalkyle en C₃-C₆, haloalkyle en C₁-C₆, haloalcényle en C₂-C₆, haloalcynyle en C₂-C₆, halocycloalkyle en C₃-C₆, halogène, CN, CO₂H, CO₂NH₂, NO₂, OH, alcoxy en C₁-C₄, haloalcoxy en C₁-C₄, alkylthio en C₁-C₄, alkylsulfinyle en C₁-C₄, alkylsulfonyle en C₁-C₄, haloalkylthio en C₁-C₄, haloalkylsulfinyle en C₁-C₄, haloalkylsulfonyle en C₁-C₄, alkylamino en C₁-C₄, di-(alkyle en C₁-C₄)amino, cycloalkylamino en C₃-C₆, (alkyle en C₁-C₆)carbonyle, (alcoxy en C₁-C₆)carbonyle, (alkyle en C₁-C₆)aminocarbonyle, di-(alkyle en C₁-C₄)aminocarbonyle, tri-alkylsilyle en (C₁-C₂), (alkyle en C₁-C₄) (alcoxy en C₁-C₄)imino,
ou les substituants pouvant être choisis indépendamment les uns des autres parmi phényle ou un cycle hétéroaromatique à 5 ou 6 éléments, le phényle ou le cycle pouvant éventuellement être substitué une ou plusieurs fois, de manière identique ou différente, avec alkyle en C₁-C₆, alcényle en C₂-C₆, alcynyle en C₂-C₆, cycloalkyle en C₃-C₆, haloalkyle en C₁-C₆, haloalcényle en C₂-C₆, haloalcynyle en C₂-C₆, halocycloalkyle en C₃-C₆, halogène, CN, NO₂, OH, alcoxy en C₁-C₄, haloalcoxy en C₁-C₄.

4. Mélanges de composés de formule générale 1 selon l'une quelconque des revendications 1 à 3, dans lesquels le cycle ou le système cyclique Q est à chaque fois relié au radical (A) par des atomes de carbone ou d'azote différents.

5. Procédé de fabrication de composés de formule générale (I) selon l'une quelconque des revendications 1 à 3, **caractérisé en ce que**
(A) des anilines de formule (II) 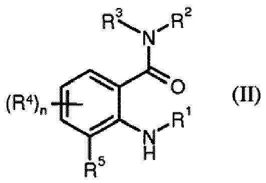 dans laquelle A, R¹, R², R³, R⁴, R⁵ et n ont les significations indiquées précédemment,
sont mises en réaction avec des chlorures d'acides carboxyliques de formule (III) 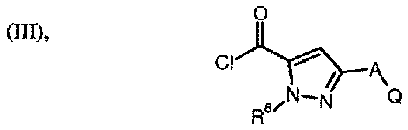 dans laquelle R⁶, A et Q ont les significations indiquées précédemment, en présence d'un liant d'acide,
(B) des anilines de formule (II) 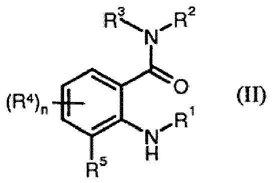 dans laquelle A, R¹, R², R³, R⁴, R⁵ et n ont les significations indiquées précédemment,
sont mises en réaction avec un acide carboxylique de formule (IV) 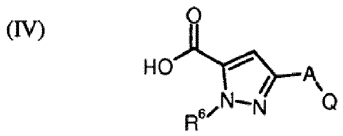 dans laquelle R⁶, A et Q ont les significations indiquées précédemment,
en présence d'un agent de condensation, ou
(C) pour la synthèse d'amides anthraniliques de formule (I) dans lesquels R¹ représente hydrogène, des benzoxazinones de formule (V) 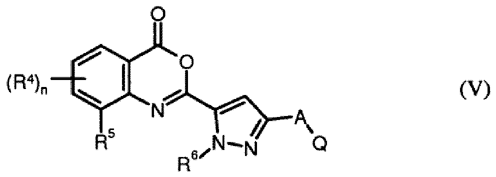 dans laquelle R⁴, R⁵, R⁶, A, Q et n ont les significations indiquées précédemment,
sont mises en réaction avec une amine de formule (VI) 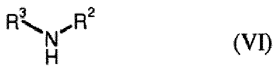 dans laquelle R² et R³ ont les significations indiquées précédemment,
en présence d'un diluant.

6. Compositions agrochimiques contenant au moins un composé de formule (I) ou un mélange de composés de formule (I) selon l'une quelconque des revendications 1 à 3 ou 4, ainsi que des extendeurs et/ou des substances tensioactives.

7. Compositions contenant au moins un composé de formule (I) ou un mélange de composés de formule (I) selon l'une quelconque des revendications 1 à 3 ou 4, ainsi qu'au moins un sel de formule (XI) 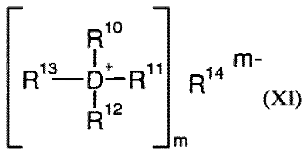 dans laquelle
D représente azote ou phosphore,
R¹⁰, R¹¹, R¹² et R¹³ représentent indépendamment les uns des autres hydrogène ou alkyle en C₁-C₈ à chaque fois éventuellement substitué ou alkylène en C₁-C₈ mono-ou polyinsaturé, éventuellement substitué, les substituants pouvant être choisis parmi halogène, nitro et cyano,
m représente 1, 2, 3 ou 4,
R¹⁴ représente un anion inorganique ou organique.

8. Compositions contenant au moins un composé de formule (I) ou un mélange de composés de formule (I) selon l'une quelconque des revendications 1 à 3 ou 4, ainsi qu'au moins un promoteur de pénétration de formule (XII)
R-O-(-AO)ᵥ-R' (XII)
dans laquelle
R représente alkyle linéaire ou ramifié contenant 4 à 20 atomes de carbone,
R' représente hydrogène, méthyle, éthyle, n-propyle, i-propyle, n-butyle, i-butyle, t-butyle, n-pentyle ou n-hexyle,
AO représente un radical oxyde d'éthylène, un radical oxyde de propylène, un radical oxyde de butylène ou des mélanges de radicaux oxyde d'éthylène et oxyde de propylène ou de radicaux oxyde de butylène, et
V représente des nombres de 2 à 30.

9. Procédé de fabrication de compositions agrochimiques, **caractérisé en ce qu'**au moins un composé de formule générale (I) ou un mélange de composés de formule générale (I) selon l'une quelconque des revendications 1 à 3 ou 4 est mélangé avec des extendeurs et/ou des substances tensioactives.

10. Utilisation d'un composé de formule générale (I) selon l'une quelconque des revendications 1 à 3, d'un mélange de composés selon la revendication 4 ou d'une composition selon l'une quelconque des revendications 6 à 8 pour lutter contre les animaux nuisibles, à l'exception du traitement thérapeutique du corps animal et humain.

11. Procédé de lutte contre des animaux nuisibles, **caractérisé en ce qu'**un composé de formule générale (I) selon l'une quelconque des revendications 1 à 3, un mélange de composés de formule générale (I) selon la revendication 4 ou une composition selon l'une quelconque des revendications 6 à 8 est laissé agir sur des animaux nuisibles et/ou des champignons phytopathogènes et/ou leur habitat et/ou des graines, à l'exception du traitement thérapeutique du corps animal et humain.
